# EUROPÄISCHE PATENTSCHRIFT

(11) **EP 0 335 297 B1**
(45) Veröffentlichungstag und Bekanntmachung des Hinweises auf die Patenterteilung: **13.03.1996**
(21) Anmeldenummer: 89105359.7
(22) Anmeldetag: 29.03.1989
(51) Int. Cl.: C07D 501/26, C07D 501/38, A61K 31/545

(54) **Cephalosporinderivate**
Cephalosporin derivatives
Dérivés de céphalosporine

(30) Priorität: 31.03.1988 US 175471; 24.05.1988 US 197943; 07.02.1989 EP 89102071
(43) Veröffentlichungstag der Anmeldung: 04.10.1989
(73) Patentinhaber: F. HOFFMANN-LA ROCHE AG, CH-4002 Basel (CH)
(72) Erfinder: Albrecht, Harry Allen, Towaco, N.J. 07082 (US); Keith, Dennis Dalton, Montclair, N.J. 07042 (US); Konzelmann, Frederick Martin, West Paterson, N.J. 07424 (US); Rossman, Pamela Loreen, Nutley, N.J. 07110 (US); Wei, Chung-Chen, Cedar Knolls, N.J. 07927 (US); Weigele, Manfred, North Caldwell, N.J. 07006 (US); Yang, Roxana, West Caldwell, N.J. 07006 (US)
(74) Vertreter: Lederer, Franz, Dr.

(56) Entgegenhaltungen:
- EP-A- 0 121 244
- EP-A- 0 137 440
- EP-A- 0 192 176
- EP-A- 0 251 330
- DE-A- 1 954 516

## Beschreibung

Die vorliegende Erfindung betrifft Acylderivate der allgemeinen Formel 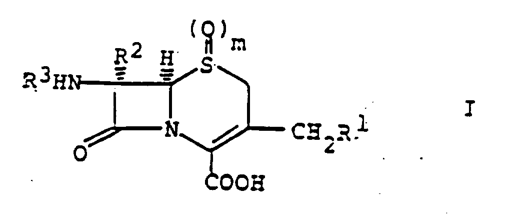 in der R¹ einen substituierten N-Heterocyclus der Formeln 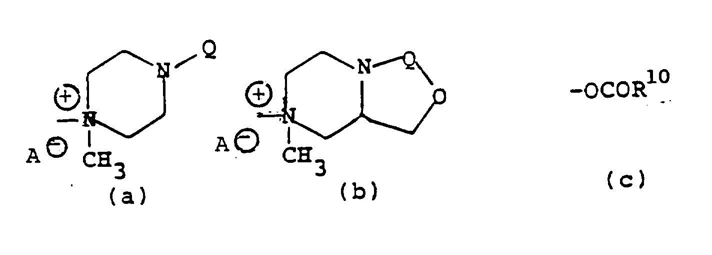 bedeutet, worin R¹⁰ eine der Gruppen 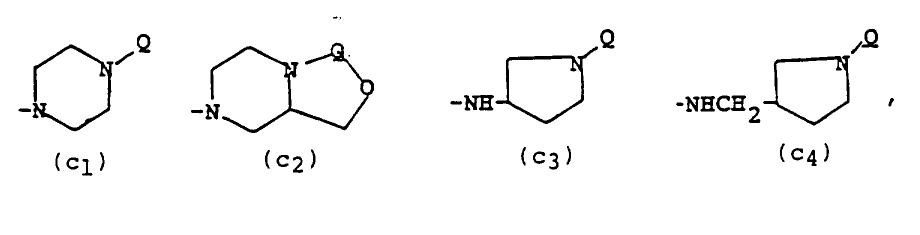 und Q eine substituierte Chinolinyl- oder Naphthyridinylgruppe darstellt, wobei der N-Heterocyclus gegebenenfalls durch eine oder mehrere C₁-C₈ Alkylgruppen substituiert sein kann; und worin ferner R Wasserstoff, C₁-C₈-Alkoxy, C₁-C₈-Alkylthio oder C₁-C₇-Alkanoylamino, R³ eine Acylgruppe, m 0, 1 oder 2 und A ein pharmazeutisch verträgliches Anion darstellt,
sowie leicht hydrolysierbare Ester und pharmazeutisch verträgliche Salze dieser Verbindungen und Hydrate der Verbindungen der Formel I bzw. von deren Estern und Salzen.

Der Ausdruck "C₁-C₈-Alkyl" bezieht sich auf gerad- und verzweigtkettige Kohlenwasserstoffgruppen mit 1-8, bevorzugt 1-4 Kohlenstoffatomen, z.B. Methyl, Aethyl, n-Propyl, Isopropyl, t-Butyl oder dergleichen.

Der Ausdruck "C₁-C₈-Alkoxy" bezieht sich auf gerad- oder verzweigtkettige Kohlenwasserstoffoxygruppen, worin der Alkylrest die oben gegebene Bedeutung hat. Beispiele sind Methoxy, Aethoxy, n-Propoxy und dergleichen.

Der Ausdruck "Halogen" bezieht sich auf Chlor, Brom, Jod oder Fluor, sofern nicht anderweitig definiert.

Der Ausdruck "Aryl" bedeutet einen gegebenenfalls substituierten aromatischen Rest, wie z.B. Phenyl, Tolyl, Xylyl, Mesityl, Cumyl (Isopropylphenyl), Naphthyl und dergleichen, wobei die Arylgruppe z.B. 1-3 geeignete Substituenten tragen kann, wie Halogen (z.B. Fluor, Chlor, Brom), Hydroxy oder dergleichen.

Der Ausdruck "C₁-C₇-Alkanoyl" bezeichnet einen Rest der allgemeinen Formel

R⁵-CO-

in der R⁵ Wasserstoff oder C₁-C₆ Alkyl darstellt. Beispiele dieser Gruppen sind Formyl, Acetyl, Propionyl, n-Butyryl und dergleichen.

Der Ausdruck "substituiertes Phenyl" bedeutet eine mono- oder disubstituierte Phenylgruppe, wobei als Substituenten Halogen (z.B. Chlor, Brom, Fluor), C₁-C₈-Alkyl Amino, Nitro oder Trifluormethyl in Frage kommen.

Der Ausdruck "substituiertes Alkyl" bedeutet eine C₁-C₈-Alkylgruppe, die z.B. durch Halogen (z.B. Chlor, Fluor, Brom), Trifluormethyl, Amino, Cyan etc. substituiert sein kann.

Der Ausdruck "C₂-C₆-Alkenyl" bedeutet gerad- oder verzweigtkettige Kohlenwasserstoffgruppen mit einer olefinischen Doppelbindung und 2-6 Kohlenstoffatomen, d.h. den Rest von Verbindungen der Formel CₙH₂ₙ, worin n 2-6 ist, z.B. Allyl, Vinyl etc.

Der Ausdruck "Aryl-C₁-C₈-alkyl" bedeutet eine Kohlenwasserstoffgruppe mit aromatischer und aliphatischer Struktur, d.h. eine Kohlenwasserstoffgruppe, worin ein Wasserstoffatom der C₁-C₈-Alkylgruppe durch eine monocyclische Arylgruppe substituiert ist, z.B. durch Phenyl, Tolyl etc.

Der Ausdruck "5-, 6 oder 7-gliedriger heterocyclischer Ring mit 1-4 Sauerstoff-, Stickstoff- und/oder Schwefelatomen" bedeutet z.B. die folgenden Gruppen: 6-gliedrige, Stickstoff-enthaltende heterocyclische Ringe, wie Pyridyl, Piperidyl, Piperidino, N-oxido-pyridyl, Pyrimidyl, Piperazinyl, Pyridazinyl, N-oxido-pyridazinyl, etc.; 5-gliedrige Stickstoff-enthaltende heterocyclische Ringe, z.B. Pyrazolyl, Pyrrolidinyl, Imidazolyl, Thiazolyl, 1,2,3-Thiadiazolyl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl, 1,2,3-Triazolyl, 1,2,4-Triazolyl, 1H-Tetrazolyl, 2H-Tetrazolyl etc. Diese heterocyclischen Ringe können weiter substituiert sein, wobei z.B. folgende Substituenten in Frage kommen: C₁-C₈-Alkyl wie Methyl, Aethyl, n-Propyl, etc., C₁-C₈-Alkoxy, z.B. Methoxy, Aethoxy, etc., Halogen wie Chlor, Brom, etc., Halogen- C₁-C₈-Alkyl wie Trifluormethyl, Trichloräthyl, etc., Amino, Mercapto, Hydroxy, Carbamoyl oder Carboxy, etc..

Der Ausdruck "C₃-C₇-Cycloalkyl" bedeutet einen 3-7-gliedrigen gesättigten carbocyclischen Rest, z.B. Cyclopropyl, Cyclobutyl, Cyclohexyl, etc..

Der Ausdruck "Acyl" (Substituent R³) steht für alle organischen Reste, welche durch Entfernung der Hydroxygruppe aus einer Carbonsäure gebildet werden. Gewisse Acylreste sind bevorzugt: Beispiele solcher bevorzugten Acylgruppen sind diejenigen, welche bisher zur Acylierung von β-Lacta-mantibiotika, einschliesslich der 6-Aminopenicillansäure und deren Derivaten, sowie der 7-Aminocephalosporansäure und deren Derivaten, verwendet worden sind; siehe z.B. Cephalosporins and Penicillins, Verlag Flynn, Academic Press (1972), Belgische Patentschrift 866,038, veröffentlicht am 17, Oktober 1978, Belgische Patentschrift 867,994, veröffentlicht am 11. Dezember 1978, U.S. Patentschrift 4,152,432, veröffentlicht am 1. Mai 1979, U.S. Patentschrift 3,971,778, veröffentlicht am 27. Juli 1976 und U.S. Patentschrift 4,173,199, veröffentlicht am 23. Oktober 1979. In diesen Publikationen werden verschiedene Acylreste, die im vorliegenden Falle Verwendung finden, erwähnt. Die nachfolgend aufgeführten Acylreste veranschaulichen weiter den Begriff Acyl, beschränkt ihn jedoch in keiner Weise. Beispiele für Acylreste sind:

### a) aliphatische Acylgruppen der Formel

R⁵-CO-

in der R⁵ Wasserstoff, C₁-C₈-Alkyl, C₃-C₇-Cycloalkyl, C₁-C₈-Alkoxy, C₂-C₆-Alkenyl, C₃-C₇-Cycloalkenyl oder Cyclohexadienyl; oder durch einen oder mehrere der Reste Halogen, Cyan, Nitro, Amino, Mercapto, C₁-C₈- Alkylthio oder Cyanmethylthio substituiertes C₁-C₈-Alkyl oder C₂-C₆-Alkenyl darstellt;

### (b) aromatische Acylgruppen der allgemeinen Formeln

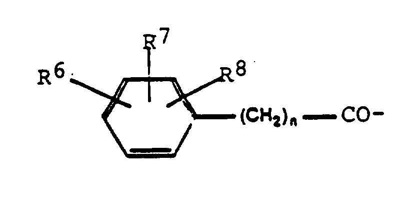

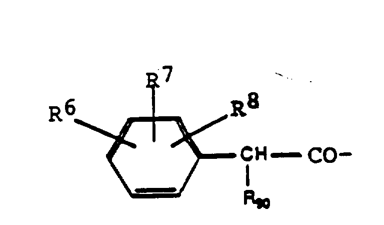

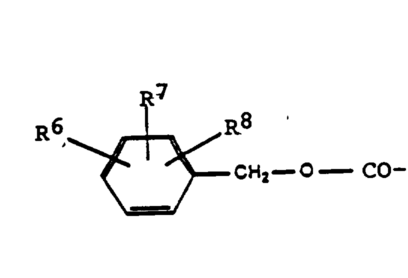

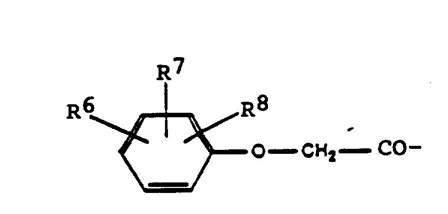

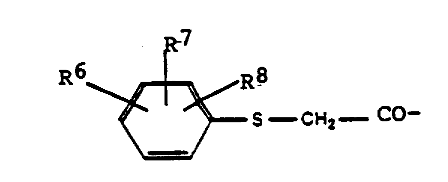

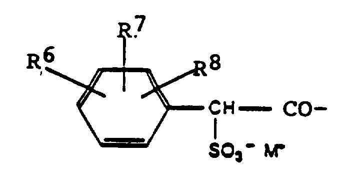

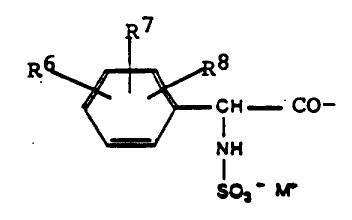

in denen n 0, 1, 2 oder 3; R⁶, R⁷ und R⁸ je Wasserstoff, Halogen, Hydroxy, Nitro, Amino, Cyan, Trifluormethyl, C₁-C₄-Alkyl, C₁-C₄-Alkoxy oder Aminomethyl, R⁹⁰ Amino, Acylamino, Hydroxy, ein Carboxysalz, geschütztes Carboxy, wie z.B. Benzyloxycarbonyl, Formyloxy, Azido oder ein Sulfosalz und M ein Kation darstellen.

Bevorzugte carboxyclische aromatische Acylgruppen sind diejenigen der Formeln 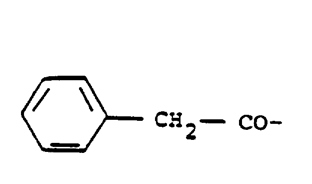 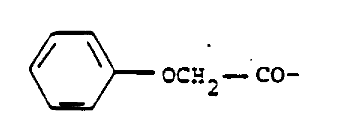 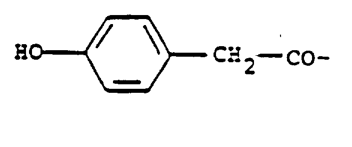 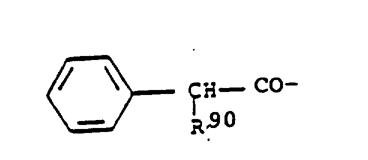 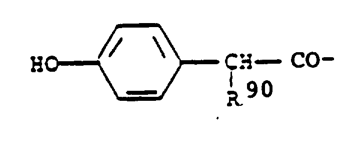 Besonders bevorzugt ist die zweite der obigen Gruppe (Phenoxyacetyl).

R⁹⁰ ist bevorzugt eine Aminogruppe, eine Hydroxygruppe oder ein Carboxy- oder Sulfosalz.

Beispiele anderer erfindungsgemäss einsetzbarer Acylgruppen sind:
Sulfophenylacetyl,
Hydroxysulfonyloxyphenylacetyl,
Sulfamoylphenylacetyl,
(Phenoxycarbonyl)phenylacetyl,
(p-Tolyloxycarbonyl)phenylacetyl,
Formyloxyphenylacetyl,
Carboxyphenylacetyl,
Formylaminophenylacetyl,
Benzyloxycarbonylphenylacetyl,
2-(N,N-Dimethylsulfamoyl)-2-phenylacetyl, etc.

### c) Heteroaromatische Acylgruppen der allgemeinen Formeln

R¹⁰¹-(CH₂)ₙ-CO-

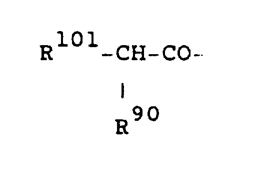

R¹⁰¹-O-CH₂-CO-

R¹⁰¹-S-CH₂-CO-

oder

R¹⁰¹-CO-CO-

in denen R⁹⁰ die oben gegebene Bedeutung hat, n 0, 1, 2 oder 3 und R¹⁰¹ einen gegebenenfalls substituierten 5-, 6- oder 7-gliedrigen heterocyclischen Ring mit 1, 2, 3 oder 4 (vorzugsweise 1 oder 2) Stickstoff-, Sauerstoff- und/oder Schwefelatomen darstellt.

Beispiele heterocyclischer Ringe sind Thienyl, Furyl, Pyrrolyl, Pyridinyl, Pyrazinyl, Thiazolyl, Pyrimidinyl und Tetrazolyl. Beispiele für Substituenten am heterocyclischen Ring sind Halogen, Hydroxy, Nitro, Amino, Cyan, Trifluormethyl, C₁-C₄-Alkyl sowie C₁-C₄-Alkoxy.

Bevorzugte heteroaromatische Acylgruppen sind diejenigen, worin R¹⁰¹ 2-Amino-4-thiazolyl, 2-Amino-5-halogen-4-thiazolyl, 4-Aminopyridin-2-yl, 2-Amino-1,3,4-thiadiazol-5-yl, 2-Thienyl, 2-Furanyl, 4-Pyridinyl oder 2,6-Dichlor-4-pyridinyl bedeutet.

### d) [[4-Substituierte-2,3-dioxo-1-piperazinyl)carbonyl]amino]arylacetylgruppen der Formel

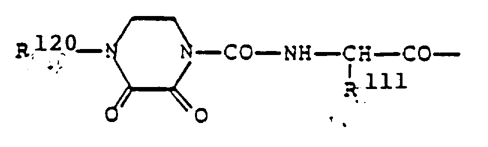

in der R¹¹¹ C₁-C₈-Alkyl, Hydroxy-C₁-C₈-Alkyl oder eine aromatische Gruppe der allgemeinen Formel 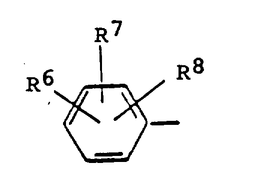 (in der R⁶, R⁷ und R⁸ die obige Bedeutung haben) oder einen wie für R¹⁰¹ definierten heterocyclischen Ring und R¹⁰ gegebenenfalls durch einen oder mehrere der Reste Halogen, Cyan, Nitro, Amino und/oder Mercapto substituiertes C₁-C₈-Alkyl darstellen, z.B. 4-niederes Alkyl (vorzugsweise Aethyl oder Methyl)-2,3-dioxo-1-piperazincarbonyl-D-phenylglycyl.

### e) (Substituierte Oxyimino)arylacetylgruppen der allgemeinen Formel

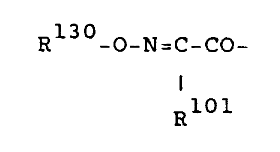

in der R¹⁰¹ die obige Bedeutung hat und R¹³⁰ Wasserstoff, C₁-C₈-Alkyl, C₃-C₇-Cycloalkyl, Carboxy-C₃-C₇-cycloalkyl oder substituiertes
C₁-C₈-Alkyl darstellt, wobei die C₁-C₈-Alkylgruppe substituiert ist durch einen oder mehrere der Reste Halogen, Cyan, Nitro, Amino, Mercapto, C₁-C₈-Alkylthio, eine durch R¹¹¹ oben definierte aromatische Gruppe, Carboxy (einschliesslich dessen Salze), C₁-C₇-Alkanoylamino, Carbamoyl, C₂-C₉-Alkoxycarbonyl, Phenylmethoxycarbonyl, Diphenylmethoxycarbonyl, Hydroxy( C₁-C₈-alkoxy)phosphinyl, Dihydroxyphosphinyl, Hydroxy(phenylmethoxy)phosphinyl oder di(niederes Alkoxy)phosphinyl.
Beispiele für Reste 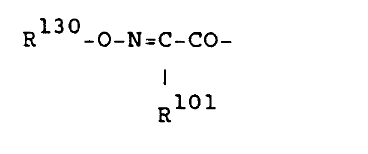 sind: [2-[(Chloracetyl)amino]-4-thiazolyl](methoxyamino)acetyl, (2-Amino-4-thiazolyl)(1-methyläthoxyimino)acetyl, (2-Amino-4-thiazolyl)(methoxyimino)acetyl, (2-Furyl)(methoxyimino)acetyl, (4-Hydroxyphenyl)(methoxyimino)acetyl, (Methoxyimino)(phenyl)acetyl, (Hydroxyimino)(phenyl)acetyl, (Hydroxyimino)(2-thienyl)acetyl, [[(Dichloracetyl)oxy]imino](2-thienyl)acetyl, [5-Chlor-2-[(chloracetyl)amino]-4-thiazolyl](methoxyimino)acetyl, (2-Amino-5-chlor-4-thiazolyl)(methoxyimino)acetyl, [[[1-(1,1-Dimethyläthoxy)carbonyl]-1-methyläthoxy]imino]-2-sulfoamino-4-thiazolyl)acetyl, [[[1-(1,1-Dimethyläthoxycarbonyl]-1-methylethoxy]imino]-[[2-(triphenylmethyl)amino]-4-thiazolyl]acetyl, (Methoxyimino)(2-sulfoamino-4-thiazolyl)acetyl, [(1-Methyläthoxy)imino]-[2-[(methylsulfonyl)amino]-4-thiazolyl]acetyl, [(3-Methylsulfonyl)-2[3H]-thiazolimin-4-yl]-[1-(methylethoxy)imino]acetyl, [[2-(Chloracetyl)amino]-4-thiazolyl][[[[(4-nitrophenyl)methoxy]carbonyl]methoxy]imino]acetyl, (2-Amino-4-thiazolyl)[(carboxymethoxy)imino]acetyl, (2-Amino-4-thiazolyl)-[1-carboxy-(1-methyläthoxy)imino]acetyl, (2-Amino-4-thiazolyl)[[(aminocarbonyl)methoxy]imino]acetyl.

### (f) (Acylamino)acetylgruppen der allgemeinen Formel

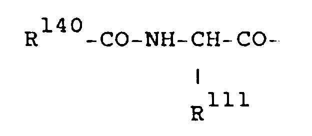

in der R¹¹¹ die obige Bedeutung hat und R¹⁴⁰ eine Gruppe der allgemeinen Formel 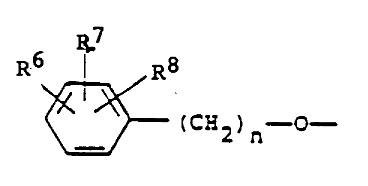 (worin R⁶, R⁷, R⁸ und n die obige Bedeutung haben), Wasserstoff, C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkyl)amino, Hydrazino, C₁-C₈-Alkyl-Hydrazino, Aryl-Hydrazino oder Acyl-Hydrazino darstellt.

Bevorzugte (Acylamino)acetylgruppen der obigen Formel sind diejenigen, worin R¹⁴⁰ Amino oder Acylamino darstellt. Ebenfalls bevorzugt sind diejenigen Gruppen, worin R¹¹¹ Phenyl oder 2-Thienyl darstellt.

### (g) (Substituierte Oxyimino)acetylgruppen der allgemeinen Formel

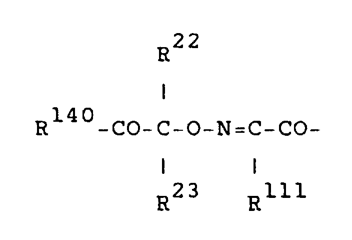

in der R¹¹¹ und R¹⁴⁰ die oben gegebenen Bedeutung haben und R und R³ je einzeln Wasserstoff oder C₁-C₈-Alkyl bedeuten oder R und R³ zusammen mit dem benachbarten Kohlenstoffatom einen C₃-C₇ carbocyclischen Ring, z.B. Cyclopropyl, Cyclobutyl oder Cyclopentyl, bilden.

Bevorzugte substituierte Oxyiminoacetylgruppen der obigen Formel sind diejenigen, worin R¹⁴⁰ Amino darstellt. Ebenfalls bevorzugt sind diejenigen Gruppen, worin R¹¹¹ 2-Amino-4-thiazolyl darstellt.

### (h) [[[3-Substituierte-2-oxo-1-imidazolidinyl]carbonyl]amino]acetylgruppen der allgemeinen Formel

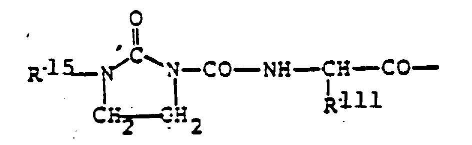

in der R¹¹¹ die oben gegebene Bedeutung hat, und R¹⁵ Wasserstoff, C₁-C₈-Alkylsulfonyl, -N=CH-R¹¹¹ (worin R¹¹¹ die oben gegebene Bedeutung hat), R¹⁶-CO- (worin R¹⁶ Wasserstoff oder gegebenenfalls durch Halogen substituiertes C₁-C₈-alkyl darstellt), eine wie unter R¹¹¹ oben definierte aromatische Gruppe oder gegebenenfalls durch einen oder mehrere der Reste Halogen, Cyan, Nitro, Amino und/oder Mercapto substituiertes C₁-C₈-Alkyl darstellt.

Bevorzugte [[[3-substituierte-2-oxo -1-imidazolidinyl]carbonyl]amino]acetylgruppen der obigen Formel sind diejenigen, worin R¹¹¹ Phenyl oder 2-Thienyl darstellt. Ebenfalls bevorzugt sind diejenigen Gruppen, worin R¹⁵ Wasserstoff, Methylsulfonyl, Phenylmethylenamino oder 2-Furylmethylenamino darstellt.

R¹ in Formel I stellt eine der oben erwähnten substituierten N-heterocyclischen Gruppen (a), (b) und (c) dar, insbesondere die Gruppe (a) oder die Gruppe (c), worin R¹⁰ die Gruppe (c₁) darstellt. R¹ kann eine der folgenden Gruppen darstellen:

### (i) Gruppen (a) und (b) (quaternäre Piperazinium-Salze)

Das Symbol A bezeichnet ein pharmazeutisch verträgliches, von einer pharmazeutisch verträglichen organischen oder anorganischen Säure abgeleitetes Anion. Beispiele solcher Säuren sind Halogenwasserstoffsäuren (z.B. Chlorwasserstoffsäure, Bromwasserstoffsäure und Iodwasserstoffsäure) sowie auch andere Mineralsäuren wie z.B. Schwefelsäure, Salpetersäure, Phosphorsäure oder dergleichen; C₁-C₈-Alkansulfonsäuren und Arylsulfonsäuren wie z.B. Aethan-, Toluol- und Benzolsulfonsäuren sowie auch andere organische Säuren, wie Essigsäure, Trifluoressigsäure, Weinsäure, Maleinsäure, Apfelsäure, Zitronensäure, Benzoesäure, Salicylsäure, Ascorbinsäure und dergleichen. A schliesst ebenfalls das Anion ein, welches aus der 2-Carboxygruppe des Cephalosporinkerns nach Dissociation eines Protons gebildet wird und dabei ein Zwitterion mit der positiven Ladung des quaternären Stickstoffatoms bildet.

Bevorzugte Ausführungsformen des Anions A sind das Iodid-Anion, das Trifluoracetat-Anion und das 2-Carboxylat-Anion am Cephalosporinkern, welches ein inneres Salz mit dem Piperaziniumrest R¹ bildet.

Q in den Gruppen (a) und (b) ist eine substituierte Chinolinyl- oder Naphthyridinylgruppe, bevorzugt mit der Formel 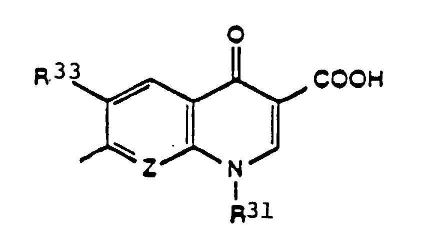 worin Z R³⁰-C oder Stickstoff; R³⁰ Wasserstoff, Halogen oder eine Oxymethylenbrücke (-CH₂O-) zum Piperazinrest unter Bildung eines ankondensierten 6-gliedrigen Ringes; R₃₁ Wasserstoff, C₁-C₈-Alkyl, C₂-C₆-Alkenyl, C₃-C₇-Cycloalkyl, Halogen-C₁-C₈-Alkyl oder Mono-, Di- oder Trihalogenphenyl; oder R³⁰ und R³¹ zusammen eine C₃-C₅-Alkylengruppe, eine C₂-C₄-Alkylenmonooxygruppe, eine C₁-C₂-Alkylendioxygruppe oder eine Gruppe der Formel -OCH₂N(CH₃)-; und R³³ Wasserstoff oder Halogen darstellen.

In einer bevorzugten Ausführungsform stellt Z die Gruppe R³⁰-C dar, worin R³⁰ Wasserstoff, Brom, Chlor oder Fluor, insbesondere Wasserstoff oder Fluor, und R³¹ C₁-C₈-Alkyl, insbesondere Aethyl, oder Halogen-substituiertes C₁-C₈-Alkyl, insbesondere Fluoräthyl, oder wahlweise C₃-C₇-Cycloalkyl, insbesondere Cyclopropyl, und R³³ Wasserstoff, Chlor oder Fluor, insbesondere Wasserstoff oder Fluor, bedeutet.

Beispiele der substituierten Piperaziniumreste R¹ als Gruppen (a) und (b) sind beispielsweise Gruppen der nachstehenden Formeln:

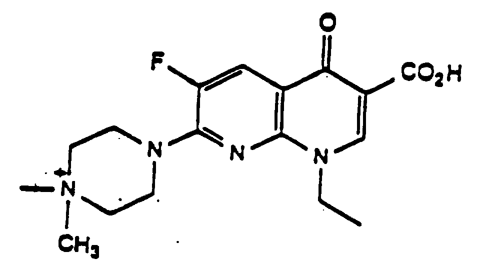

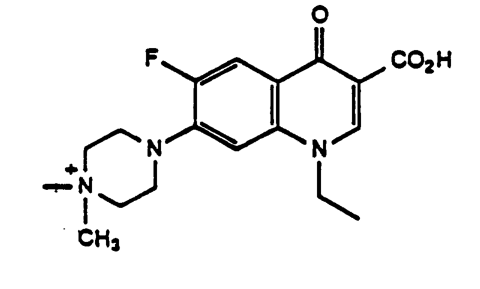

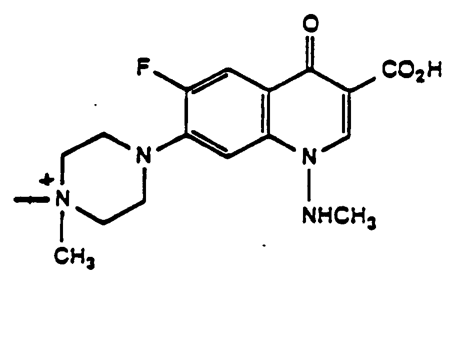

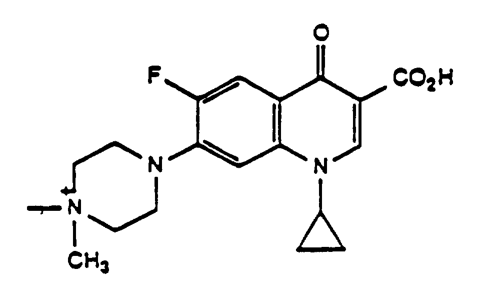

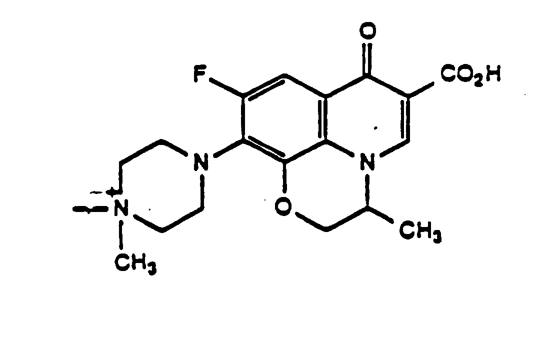

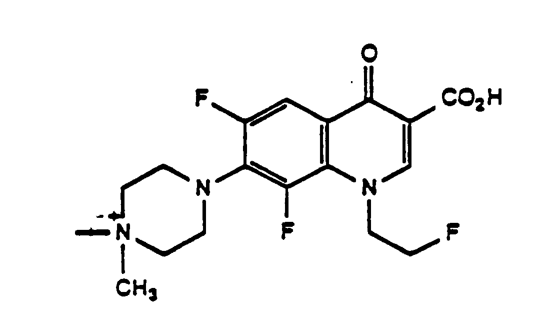

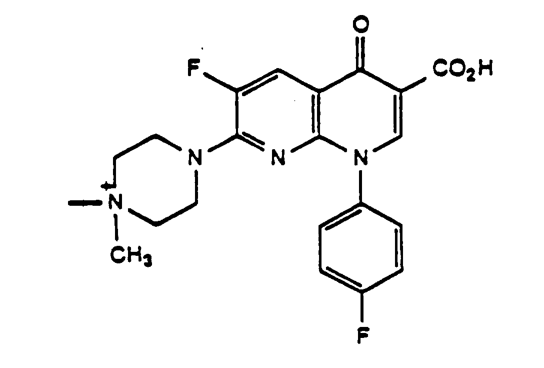

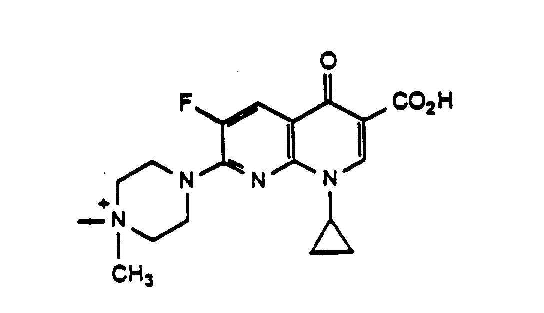

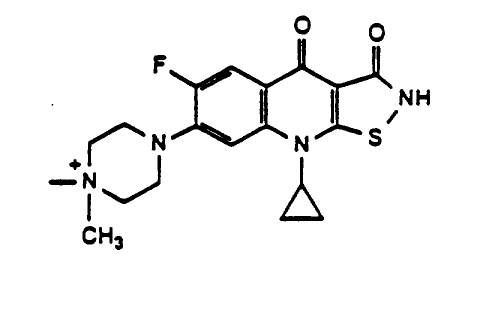

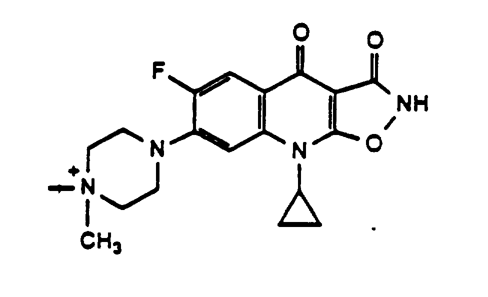

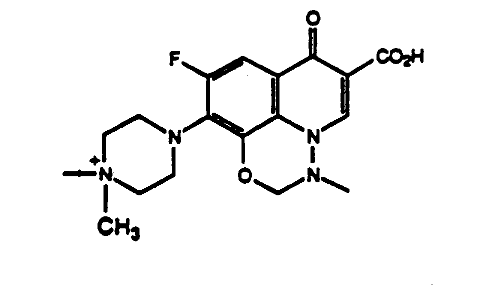

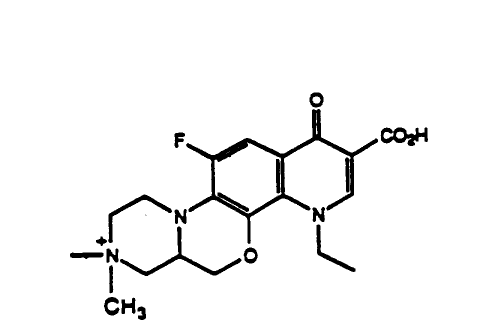

### (ii) Gruppe (c) (Urethane)

Q in der Gruppe (c) ist vorzugsweise der Formel 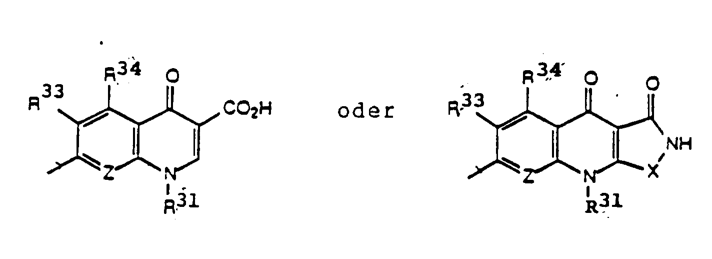 worin Z R³⁰-C oder Stickstoff; X Sauerstoff oder Schwefel; R³⁰ Wasserstoff, Halogen oder eine Oxomethylenbrücke (-CH₂O-) zum Piperazinrest unter 31 Bildung eines ankondensierten 6-gliedrigen Ringes; R³¹ Wasserstoff, C₁-C₈-Alkyl, C₂-C₆-Alkenyl, C₃-C₇-Cycloalkyl, Halogen-C₁-C₈-Alkyl oder Mono- Di- oder Tri-halogenphenyl; R³⁰ und R³¹ zusammen eine C₃-C₅-Alkylenegruppe, eine C₂-C₄-Alkylenmonooxygruppe, eine C₁-C₂-Alkylendioxygruppe oder eine Gruppe der Formel -OCH₂N(CH₃)-; R³³ Wasserstoff oder Halogen;und R³⁴ Wasserstoff oder Amino darstellen.

In einer bevorzugten Ausführungsform stellt Z die Gruppe R³⁰-C dar, worin R³⁰ Wasserstoff, Chlor, Brom oder Fluor insbesondere Wasserstoff oder Fluor, R³¹ C₁-C₈-Alkyl, insbesondere Aethyl, oder Halogen-C₁-C₈-Alkyl, insbesondere Fluoräthyl, oder C₃-C₇-Cycloalkyl, insbesondere Cyclopropyl, und R³³ Wasserstoff, Chlor oder Fluor, insbesondere Wasserstoff oder Fluor, bedeutet.

Die Urethangruppen (c) in der Bedeutung R¹ sind unter anderem Gruppen der Formeln

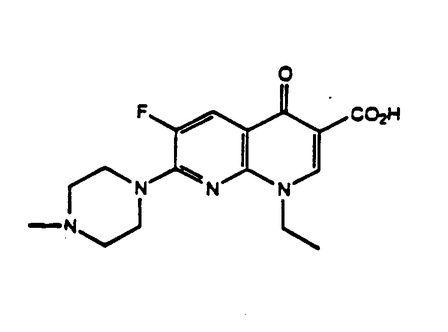

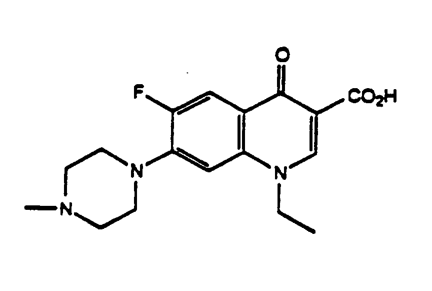

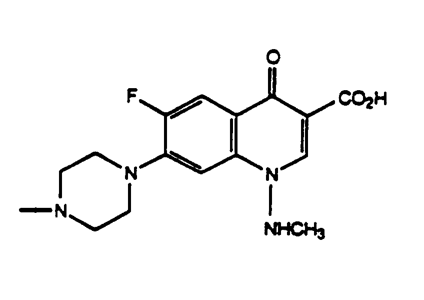

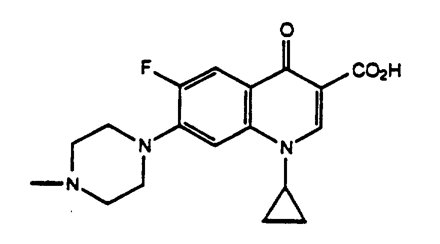

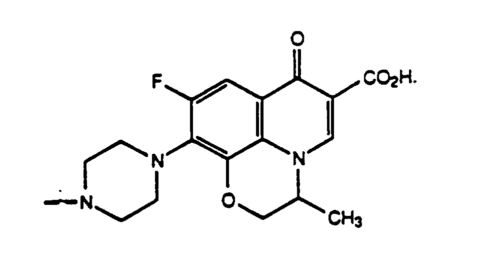

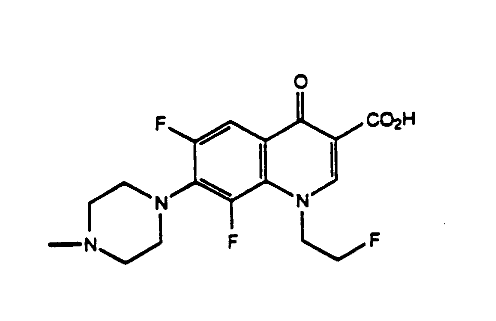

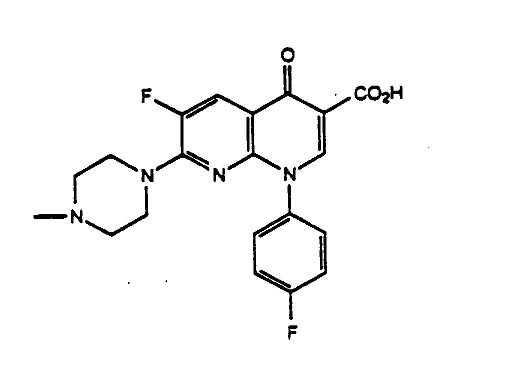

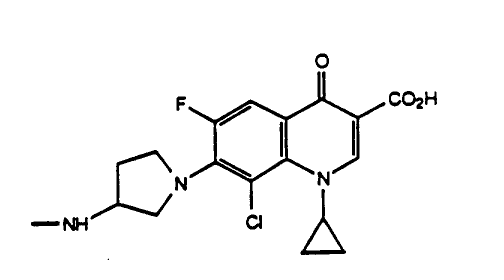

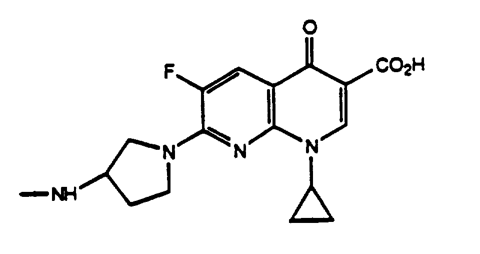

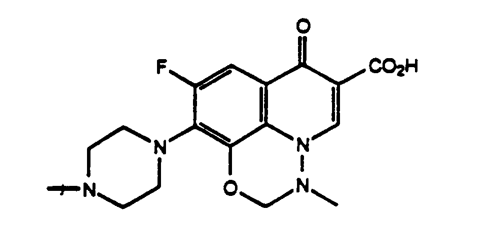

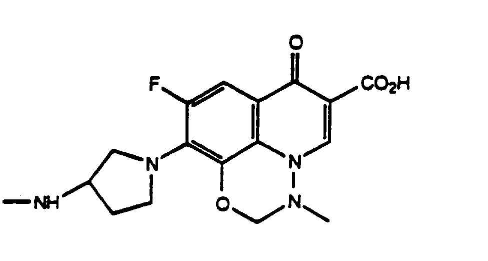

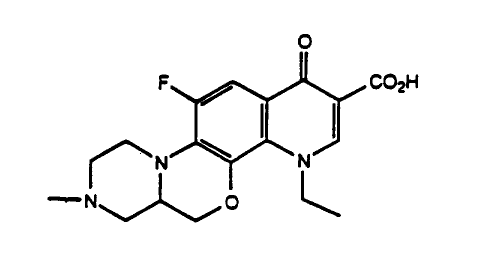

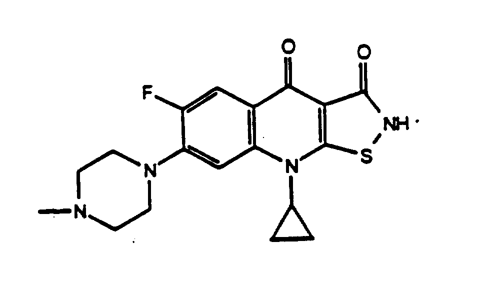

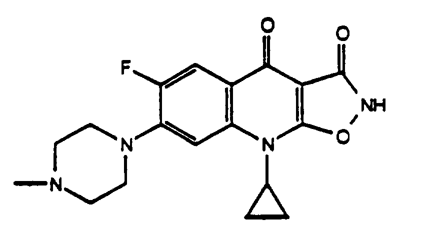

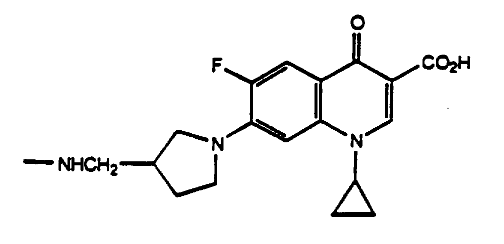

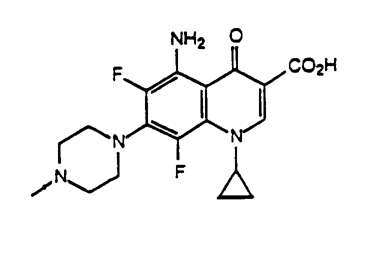

Bevorzugte Verbindungen der vorliegenden Erfindung sind diejenigen der Formel I, worin m 0 und R Wasserstoff darstellt. Insbesondere bevorzugt sind Verbindungen der Formel 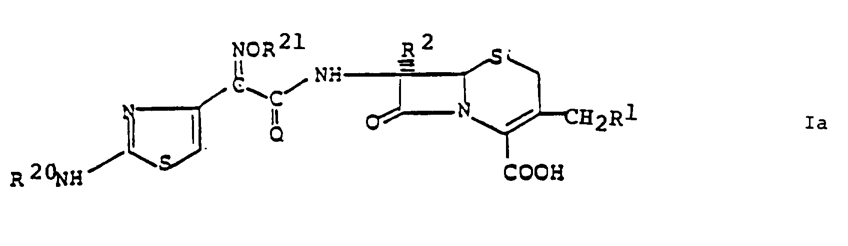 worin R¹ und R³ die obigen Bedeutungen haben, R⁰ Wasserstoff oder eine Aminoschutzgruppe, z.B. Trityl oder Chloracetyl, und R¹ Wasserstoff, C₁-C₈-Alkyl oder eine Gruppe der allgemeinen Formel 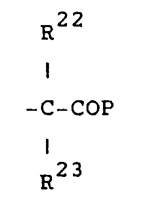 worin R und R³ die oben gegebene Bedeutung haben und P Hydroxy oder -NHR¹⁹, worin R¹⁹ Wasserstoff, C₁-C₈-Alkyl, Amino, C₁-C₈-Alkylamino, Arylamino oder Acylamino darstellt, bedeutet.

Besonders bevorzugt sind Verbindungen der Formel Ia, worin R⁰ Wasserstoff und R¹ Methyl oder eine Gruppe der Formel 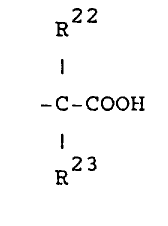 worin R und R³ Wasserstoff oder Methyl darstellen, bedeutet.

Bevorzugt liegen die Gruppen 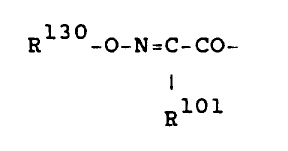 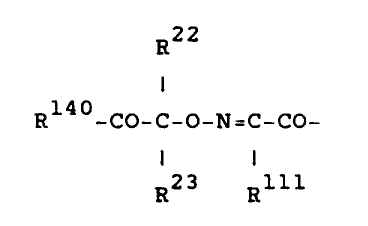 und 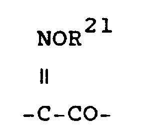 in der syn-Form, d.h. in der Z-Form, oder als Mischungen vor, in welchen die syn-Form überwiegt.

Eine weitere bevorzugte Verbindungsgruppe stellt diejenige der Formel 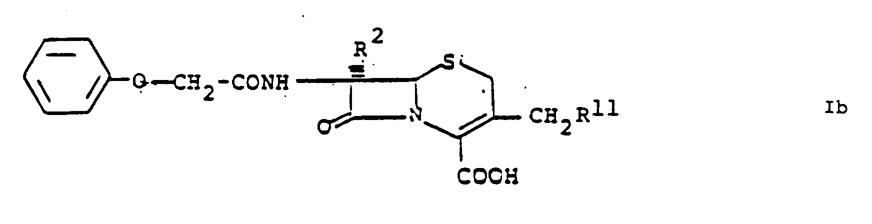 in der R die oben gegebene Bedeutung hat und R¹¹ eine der obigen Gruppen (a) und (b) bedeutet,
dar.

Als leicht hydrolysierbare Ester der Verbindungen der Formel I sind Verbindungen der Formel I zu verstehen, deren Carboxygruppe bzw. Carboxygruppen (z.B. die 2-Carboxygruppe) in Form einer leicht hydrolysierbaren Estergruppe vorliegt/vorliegen. Beispiele solcher Ester, die herkömmlicher Art sein können, sind die C₁-C₇-Alkanoyl-C₁-C₈-Alkylester, z.B. der Acetoxymethyl-, Pivaloyloxymethyl-1-Acetoxyäthyl- und der 1-Pivaloxyloxyäthylester; die C₂-C₉-Alkoxycarbonyloxy-C₁-C₈-Alkylester, z.B. der Methoxycarbonyloxymethyl, 1-Aethoxycarbonyloxyäthyl- und der 1-Isopropoxycarbonyloxyäthylester; die Lactonylester, z.B. der Phthylidyl und der Thiophthalidylester; die C₂-C₉-Alkoxymethylester, z.B. der Methoxymethylester und die C₁-C₇-Alkanoylaminomethylester, z.B. der Acetamidomethylester. Auch andere Ester, z.B. die Benzyl- und Cyanmethylester können brauchbar sein.

Beispiele von Salzen der Verbindungen der Formel I sind Alkalimetallsalze, wie das Natrium- und das Kaliumsalz, Ammoniumsalze; Erdalkalimetallsalze, wie das Calciumsalz; Salze mit organischen Basen, wie die Salze mit Aminen, z.B. Salze mit N-Aethylpiperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin.

Die Verbindungen der Formel I, sofern sie eine basische funktionelle Gruppe, wie z.B. eine Aminogruppe besitzen, bilden ebenfalls Additionssalze mit organischen oder anorganischen Säuren. Beispiele für solche Salze sind Hydrohalogenide, beispielsweise Hydrochloride, Hydrobromide, Hydrojodide, sowie andere Mineralsäuresalze, wie Sulfate, Nitrate, Phosphate und dergleichen, C₁-C₈-Alkyl- und Monoarylsulfonate, wie Aethansulfonate, Toluolsulfonate, Benzolsulfonate und dergleichen und auch andere organische Säuresalze wie Acetate, Tartrate, Maleate, Citrate, Benzoate, Salicylate, Ascorbate und dergleichen.

Die Verbindungen der Formel I einschliesslich deren Salze und leicht hydrolysierbare Ester können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die obigen Acylderivate werden erfindungsgemäss dadurch hergestellt, dass man
a) zur Herstellung einer Verbindung der Formel I, in der m 0 darstellt, eine Verbindung der allgemeinen Formel 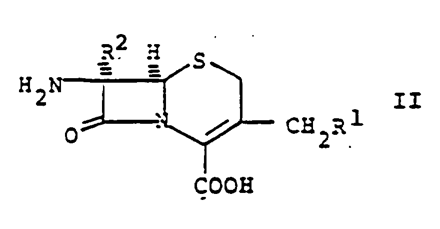 in der R¹ und R die oben gegebene Bedeutung haben, oder einen leicht hydrolysierbaren Ester oder Salz dieser Verbindung mit einer Carbonsäure der allgemeinen Formel R³OH oder mit einem reaktionsfähigen Derivat davon umsetzt, oder dass man
b) zur Herstellung einer Verbindung der Formel I, in der m 0 und R¹ eine der Gruppen (a) und (b) darstellt, eine Verbindung der Formel 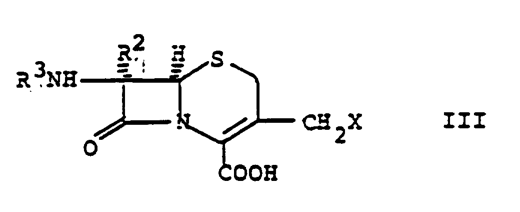 in der R und R³ die oben gegebene Bedeutung haben,
   X eine Abgangsgruppe darstellt, und Amino-, Hydroxy- und Carboxygruppen geschützt sein können,
   oder einen leicht hydrolysierbaren Ester oder Salz davon mit einer Verbindung der Formel 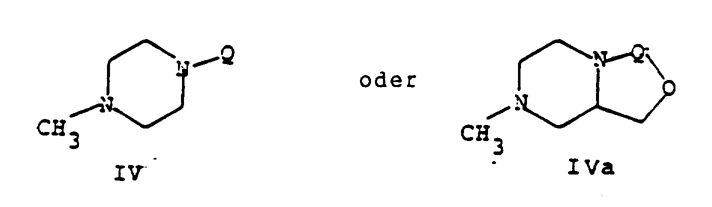 in der Q die oben gegebene Bedeutung hat und die Stickstoffatome geschützt sein können,
   oder dass man
c) zur Herstellung eines leicht hydrolysierbaren Esters einer Carbonsäure der Formel I, in der m 0 und R¹ die Gruppe (c) darstellt, eine Verbindung der Formel 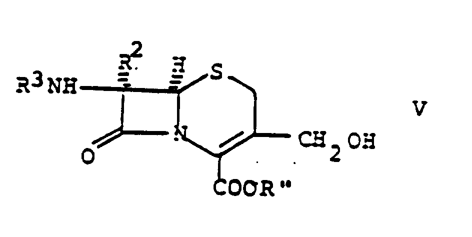 in der R und R³ die oben gegebene Bedeutung haben und R˝ den Rest eines leicht hydrolysierbaren Esters darstellt,
   in Gegenwart einer Base mit Phosgen umsetzt, das erhaltene Reaktionsprodukt mit einer Verbindung der Formel R¹⁰H, worin R¹⁰ die oben gegebene Bedeutung hat, umsetzt, wobei jedoch allfällige Amino-, Hydroxy- und/oder Carboxyfunktionen davon geschützt sein können, wonach man allfällige Schutzgruppen abspaltet, oder dass man
d) zur Herstellung einer Carbonsäure der Formel I einen Ester der Formel 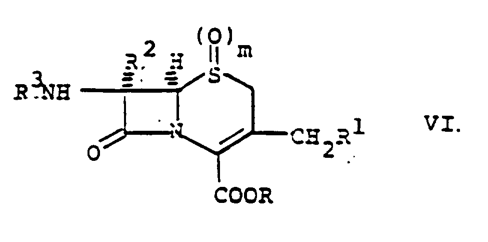 in der R¹, R, R³ und m die oben gegebene Bedeutung haben und R eine Carbonsäureschutzgruppe darstellt,
   in die Carbonsäure der Formel I umwandelt, oder dass man
e) zur Herstellung einer Verbindung der Formel I, worin m 0 darstellt, oder eines leicht hydrolysierbaren Esters oder Salzes davon eine Verbindung der Formel 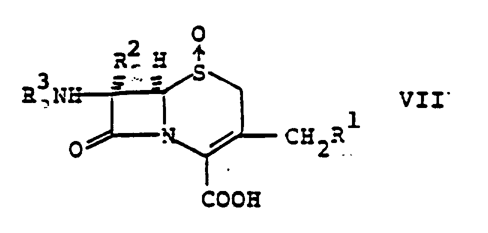 in der R¹, R und R³ die oben gegebene Bedeutung haben,
   oder einen leicht hydrolysierbaren Ester oder Salz davon reduziert, oder dass man
f) zur Herstellung einer Verbindung der Formel I, worin m 1 oder 2 darstellt oder eines leicht hydrolysierbaren Esters oder Salzes davon eine Verbindung der Formel 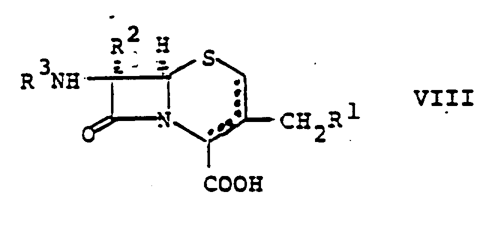 in der R¹, R und R³ die oben gegebene Bedeutung haben und die gestrichelten Linien die Anwesenheit einer Δ2- oder Δ3-Doppelbindung anzeigen,
   oder eines ihrer Ester oder Salze oxidiert, oder dass man
g) zur Herstellung einer Verbindung der Formel I, worin R³ einer Aminosubstituenten enthält oder eines ihrer Ester oder Salze die Aminoschutzgruppe in Substituenten R³⁰ einer Verbindung der allgemeinen Formel 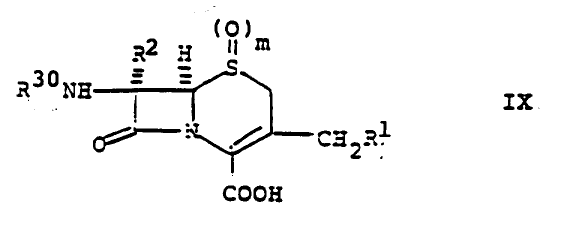 in der R¹, R und m die oben gegebene Bedeutung haben und R³⁰ einen eine geschützte Aminogruppe enthaltenden Acylrest darstellt,
   oder eines ihrer Ester oder Salze abspaltet, oder dass man
h) zur Herstellung eines leicht hydrolysierbaren Esters einer Verbindung der Formel I eine Carbonsäure der Formel I einer entsprechenden Veresterung unterwirft, oder dass man
i) zur Herstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

Die Umsetzung der Verbindung II mit dem Säuren R³OH oder ihren reaktionsfähigen Derivaten gemäss Variante (a) kann in sich bekannter Weise durchgeführt werden. Die Carboxygruppe der Verbindungen II kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden. In Gruppen R³ vorhandene Aminogruppen können geschützt sein. Mögliche Schutzgruppen sind beispielsweise sauer-hydrolytisch abspaltbare Schutzgruppen, wie z.B. t-Butoxycarbonyl oder Trityl, oder auch basisch-hydrolytisch abspaltbare Schutzgruppen wie z.B. Trifluoracetyl. Bevorzugte Schutzgruppen sind Chlor-, Brom- und Jodacetyl, insbesondere Chloracetyl. Letztere Schutzgruppen können durch Behandeln mit Thioharnstoff abgespalten werden. Die 7-Aminogruppe in Verbindungen II kann geschützt sein, z.B. durch eine Silylschutzgruppe, wie z.B. Trimethylsilyl.

Als reaktionsfähige Derivate von Säuren der Formel R³OH kommen z.B. Halogenide (d.h. Chloride, Bromide und Fluoride) Azide, Anhydride, insbesondere gemischte Anhydride mit stärkeren Säuren, reaktionsfähige Ester, wie z.B. N-Hydroxysuccinimidester, und Amide, z.B. Imidazolide, in Betracht.

Die Umsetzung der 7-Aminoverbindung der Formel II mit einer Säure der Formel R³OH oder einem reaktionsfähigen Derivat davon erfolgt beispielsweise derart, dass man eine freie Säure der Formel R³OH mit einem der erwähnten Ester entsprechend Formel II mittels eines Carbodiimids, wie Dicyclohexylcarbodiimid, in einem inerten Lösungsmittel, wie Aethylacetat, Acetonitril, Dioxan, Chloroform, Methylenchlorid, Benzol oder Dimethylformamid, kondensiert und anschliessend die Estergruppe abspaltet. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel II, z.B. ein Tri-(C₁-C₈-alkyl)-ammoniumsalz wie das Triäthylammoniumsalz, mit einem reaktionsfähigen Derivat einer Säure der Formel R³OH wie oben erwähnt in einem inerten Lösungsmittel, z.B. einem der oben genannten Lösungsmittel, um.

Nach einer weiteren Ausführungsform wird ein Säurehalogenid, vorzugsweise das Chlorid, einer Säure der Formel R³OH mit dem Amin der Formel II umgesetzt. Die Umsetzung erfolgt vorzugsweise in Gegenwart eines säurebindenden Mittels, z.B. in Gegenwart von wässrigem Alkali, vorzugsweise Natriumhydroxid, oder auch in Gegenwart eines Alkalimetallcarbonats, wie Kaliumcarbonat, oder in Gegenwart eines C₁-C₈-alkylierten Amins, wie Triäthylamin. Als Lösungsmittel wird vorzugsweise Wasser verwendet, gegebenenfalls im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel II wird in wasserfreiem Medium gearbeitet.

Bevorzugte Varianten der Acylierung, wobei eine vorhandene Aminogruppe in der Gruppe R³ nicht geschützt zu sein braucht, betrifft die Verwendung eines 2-Benzothiazolylthioesters oder eines 1-Hydroxybenzotriazolesters der Säure R³OH. Vorzugsweise kann der 2-Benzothiazolylthioester mit einer Verbindung II in einem inerten organischen Lösungsmittel, wie z.B. in einem chlorierten Kohlenwasserstoff, z.B. in Methylenchlorid, in Aceton, Aethylacetat oder in einer Mischung solcher Lösungsmittel mit Wasser, umgesetzt werden. Der 1-Hydroxybenzotriazolester kann in der Weise verwendet werden, dass man die Säure R³OH mit 1-Hydroxybenzotriazol und einem Carbodiimid, insbesondere N,N'-Dicyclohexylcarbodiimid oder N,N'-Diisopropylcarbodiimid, in einem inerten organischen Lösungsmittel, vorzugsweise Methylenchlorid, Dimethylformamid, Tetrahydrofuran, Acetonitril oder Aethylacetat, umsetzt.

Die erfindungsgemässe Umsetzung der 7-Aminoverbindung der Formel II mit einer Säure der Formel R³OH oder einem reaktionsfähigen Derivat hiervon kann zweckmässig bei einer Temperatur zwischen etwa -40°C und +60°C, z.B. bei Zimmertemperatur, durchgeführt werden.

Die Umsetzung der Verbindungen III und ihrer leicht hydrolysierbarer Ester und Salze mit den Piperazinderivaten IV oder IVa gemäss Alternative (b) des vorliegenen Verfahrens wird vorzugsweise derart vollzogen, dass man anfänglich die Ausgangsverbindungen III bzw. ihre leicht hydrolysierbaren Ester und Salze schützt, vorzugsweise mit einem Trimethylsilylierungsmittel, wie z.B. N-Methyl-N-(trimethylsilyl)trifluoracetamid (MSTFA), wobei allfällige Amino-, Hydroxy- und Carboxygruppen geschützt werden. Die Abgangsgruppe X in Verbindung III ist vorzugsweise ein Halogenatom, z.B. Chlor, Brom oder, insbesondere, Jod, eine niedere Alkyl- oder Arylsulfonyloxygruppe, z.B. Mesyloxy oder Tosyloxy, oder die Azidogruppe. Die Verbindungen III können ebenfalls wie für die Ausgangsverbindungen der Formel II beschrieben geschützt werden. Die Piperazinderiate IV und IVa sind vorzugsweise N-geschützt, z.B. mit MSTFA. Die Reaktion wird vorzugsweise bei etwa 0°C bis Zimmertemperatur und in einem inerten organischen Lösungsmittel, wie Chloroform, Methylenchlorid oder Acetonitril, durchgeführt. Zwitterionische Formen der Verbindung I erhält man durch Behandeln eines erhaltenen Halogenidsalzes mit einer Base, z.B. Natriumbicarbonat oder Natriumhydroxid oder mit Natriumphosphatpuffer.

Die Umsetzung der Verbindungen V mit einer Base und Phosgen und anschliessend mit Verbindungen R¹⁰H gemäss Alternative (c) des vorliegenden Verfahrens wird vorzugsweise in einem inerten organischen Lösungsmittel durchgeführt, wie z.B. in Methylenchlorid oder Chloroform, sowie bei einer Temperatur zwischen etwa 0°C und etwa 30°C. Allfällige Aminogruppen können geschützt sein, z.B. als Trimethylsilylderivate in der oben beschriebenen Weise; Carboxygruppen können geschützt sein, z.B. als leicht hydrolysierbare Ester, wie oben beschrieben, oder als Ester, wie sie in der Esterverseifungsstufe (d) nachstehend beschrieben werden, z.B. als p-Nitrobenzylester.

Esterschutzgruppen R der in Variante d) des erfindungsgemässen Verfahrens verwendeten Ester der Formel VI sind vorzugsweise solche, welche unter milden Bedingungen leicht in die freie Carboxygruppe umgewandelt werden können, z.B. t-Butyl, p-Nitrobenzyl, Benzhydryl, Allyl, etc. Auch die oben erläuterten Reste eines leicht hydrolysierbaren Esters können verwendet werden. Die Esterschutzgruppen werden z.B. wie folgt abgespalten: p-Nitrobenzyl durch Hydrolyse in Gegenwart von Natriumsulfid bei (oder unterhalb) 0°C bis Zimmertemperatur in einem Lösungsmittel, wie z.B. Dimethylformamid (vorzugsweise wässrig); t-Butyl und Benzhydryl durch Umsetzung mit Trifluoressigsäure, gegebenenfalls in Gegenwart von Anisol, bei etwa 0°C bis Zimmertemperatur, mit oder ohne zusätzliches Lösungsmittel, wie z.B. Methylenchlorid; Allyl durch Palladium-(O)katalysierte Transallylierung in Gegenwart eines Natrium- oder Kaliumsalzes der 2-Aethylcapronsäure, siehe z.B. J. Org. Chem. 1982, 47, 587.

Die Reduktion der Sulfoxide der allgemeinen Formel VII nach Variante e) des erfindungsgemässen Verfahrens kann nach einer Vielzahl von Reaktionen erfolgen, z.B. durch Behandlung mit Phosphortrichlorid in Dimethylformamid oder mit Trifluoressigsäureanhydrid in Gegenwart von Natriumjodid in Aceton/Methylenchlorid. Die Temperatur dieser Reaktionen liegt bevorzugt bei etwa 0 bis -20°C, insbesondere bei 0°C.

Durch die Oxidation der Verbindungen der Formel VIII gemäss Variante f) des erfindungsgemässen Verfahrens wird ein allfälliges Δ2-Isomeres der Formel VIII in das entsprechende Δ3-Isomere der Formel I, worin m 1 oder 2 darstellt, übergeführt. Diese Oxidation wird durch Behandeln mit einem organischen oder anorganischen Oxidationsmittel durchgeführt. Als Oxidationsmittel dienen verschiedene, Sauerstoff leicht abgebende Verbindungen, wie z.B. organische Peroxide, z.B. monosubstituierte organische Peroxide wie C₁-C₄-Alkyl- oder C₁-C₄-Alkanoylhydroperoxide, wie t-Butylhydroperoxid, Perameisensäure oder Peressigsäure; sowie phenylsubstituierte Derivate dieser Hydroperoxide, wie Cumolhydroperoxid oder Perbenzoesäure. Der Phenylsubstituent kann gegebenenfalls eine weitere niedere Gruppe, z.B. C₁-C₄-Alkyl oder C₁-C₄-Alkoxy oder auch Halogen oder eine Carboxygruppe tragen, z.B. 4-Methylperbenzoesäure, 4-Methoxyperbenzoesäure, 3-Chlorperbenzoesäure oder Monoperphthalsäure. Als Oxidationsmittel können auch verschiedene anorganische Oxidationsmittel verwendet werden, z.B. Wasserstoffperoxid; Oxon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen, inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol oder Aethanol, oder einer niederen, gegebenenfalls halogenierten Alkancarbonsäure mit bis zu 7 Kohlenstoffatomen, z.B. Ameisensäure, Essigsäure oder Trifluoressigsäure. Die Reaktionstemperatur bewegt sich vornehmlich im Bereiche von -20°C bis +50°C.

Bei Verwendung von äquimolaren Mengen Oxidationsmittel bzw. leichtem Ueberschuss im Verhältnis zum Ausgangsmaterial erhält man vornehmlich das entsprechende Sulfoxid, d.h. eine Verbindung der Formel I, worin m die Zahl 1 darstellt. Erhöht man die Menge des Oxidationsmittels auf das Doppelte des stöchiometrischen Verhältnisses oder mehr, bildet sich das entsprechende Sulfon, d.h. eine Verbindung der Formel I, worin m die Zahl 2 darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Durch Abspaltung der Aminoschutzgruppe im Substituenten R³⁰ einer Verbindung der Formel IX gemäss Variante g) des erfindungsgemässen Verfahrens erhält man entsprechende Verbindungen der Formel I mit einer freien Aminogruppe. Mögliche Aminoschutzgruppen sind diejenigen, welche in der Peptidchemie verwendet werden, z.B. C₂-C₉-Alkoxycarbonylgruppen, z.B. t-Butoxycarbonyl etc., substituierte C₂-C₉-Alkoxycarbonylgruppen, z.B. Trichloräthoxycarbonyl etc., substituierte Aralkoxycarbonylgruppen, z.B. p-Nitrobenzyloxycarbonyl, ArylC₁-C₈-alkylqruppen, z.B. Trityl oder Benzhydryl, oder Halogen-C₂-C₇-Alkanoylgruppen, wie z.B. Chloracetyl, Bromacetyl, Jodacetyl oder Trifluoracetyl.

Bevorzugte Aminoschutzgruppen sind t-Butoxycarbonyl (t-BOC) und Trityl.

Die Aminoschutzgruppen sind beispielsweise durch saure Hydrolyse abspaltbar (z.B. t-Butoxycarbonyl oder Trityl) oder auch durch basische Hydrolyse abspaltbar (z.B. Trifluoracetyl). Die Chlor-, Brom- und Jodacetylgruppen können durch Behandeln mit Thioharnstoff abgespalten werden.

Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperaturen angewendet werden können, z.B. im Bereiche von etwa 0°C bis +40°C. Alkalisch abspaltbare Schutzgruppen werden im allgemeinen mit verdünnt wässriger Lauge bei 0°C bis +30°C hydrolysiert. Die Chlor-, Brom- und Jodacetylschutzgruppen können mittels Thioharnstoff in saurem, neutralem oder alkalischem Milieu bei etwa 0°C bis +30°C abgespalten werden.

Zur Herstellung der leicht hydrolysierbaren Ester der Carbonsäuren der Formel I gemäss Variante h) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Die Veresterungsreaktion wird vorzugsweise in einem inerten organischen Lösungsmittel, wie Dimethylacetamid, Hexamethylphosphorsäuretriamid, Dimethylsulfoxid oder, vorzugsweise, Dimethylformamid durchgeführt. Die Temperatur liegt vorzugsweise im Bereiche von etwa 0-40°C.

Die Herstellung der Salze und Hydrate der Verbindungen der Formel I bzw. der Hydrate dieser Salze gemäss Variante g) des erfindungsgemässen Verfahrens kann in an sich bekannter Weise erfolgen, z.B. durch Umsetzen der Carbonsäure der Formel I mit einer äquimolaren Menge der gewünschten Base, zweckmässig in einem Lösungsmittel, wie Wasser, oder in einem organischen Lösungsmittel wie Aethanol, Methanol, Aceton und dergleichen. Entsprechend wird Salzbildung durch Addition einer organischen oder anorganischen Säure herbeigeführt. Die Temperatur der Salzbildung ist nicht kritisch, sie liegt im allgemeinen bei Raumtemperatur, kann aber auch leicht darüber oder darunter, etwa im Bereiche von 0°C bis +50°C, liegen.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I oder eines ihrer Salze) einer feuchten Atmosphäre, z.B. bei etwa +10°C bis +40°C, ausgesetzt werden.

Die folgenden Reaktionsschemata veranschaulichen das Verfahren zur Herstellung der erfindungsgemässen Produkte.

Die dabei verwendeten Verbindungen der Formel IV sind substituierte Piperazine der Formel 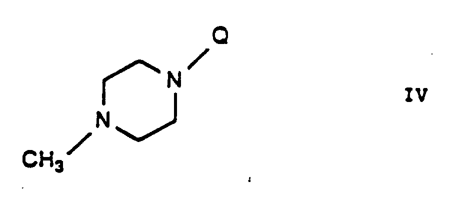 worin der Piperazinkern durch eine oder mehrere C₁-C₈, vorzugsweise C₁-C₄, Alkylgruppen substituiert sein kann.

Anstelle dieser substituierten Piperazine der Formel IV können substituierte Piperazine der Formel 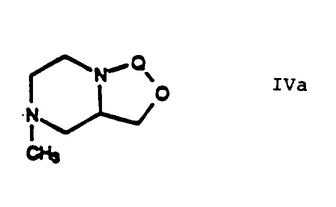 verwendet werden.

In den nachstehenden Schemas stellt R' eine leicht abspaltbare Aminoschutzgruppe dar, wie z.B. t-Butoxycarbonyl; R, R¹ und R haben die obige Bedeutung. Reaktionsfähige Gruppen sind vorzugsweise geschützt: beispielsweise können Aminogruppen durch in der Peptidchemie leicht abspaltbare Gruppen geschützt sein, z.B. durch C₂-C₉-Alkoxycarbonylgruppen, wie t-Butoxycarbonyl und dergleichen; substituierte C₂-C₉-Alkoxycarbonylgruppen, z.B. Trichloräthoxycarbonyl etc.; substituierte C₂-C₉-Alkylcacbonylgruppen, z.B. Monochlormethylcarbonyl substituierte Aryl-C₂-C₉-Alkoxycarbonylgruppen, z.B. p-Nitrobenzyloxycarbonyl, oder Aryl-C₁-C₈-alkylgruppen, z.B. Triphenylmethyl.

Bevorzugte Schutzgruppen sind t-Butoxycarbonyl (t-BOC) oder Triphenylmethyl.

Carbonsäureschutzgruppen sind beispielsweise Esterformen, welche unter milden Bedingungen leicht in freie Carboxygruppen übergeführt werden können. Beispiele solcher Gruppen sind z.B. t-Butyl, p-Nitrobenzyl, Benzhydryl, Allyl etc. Ebenfalls verwendbar sind Trimethylsilylestergruppen.

Die Verbindungen der Formeln X und IIIa, d.h. die Ausgangsmaterialien in den Schemas IV und V sind bekannt und können durch herkömmliche Verfahren hergestellt werden; einige sind Handelsprodukte, z.B. 7-Aminocephalosporansäure. Siehe Gordon, E.M., und Sykes, R.B. in "Chemistry and Biology of β-Lactam Antibiotics", Band 1, Morin, R.B. und Gorman, M., Verlag; Academic Press: New York, 1982; Kapitel 3 sowie darin enthaltene Literaturangaben; und Ponsford, R.J. et al. in "Recent Advances in the Chemistry of β-Lactam Antibiotics, Proceedings of the Third International Symposium," Brown A.G. und Roberts S.M., Verlag: Royal Society of Chemistry, Burlington House: London, 1985; Kapitel 3 und darin enthaltene Literaturangaben.

Verbindungen der Formel I worin m 0 darstellt, können erwünschtenfalls in die entsprechenden Verbindungen mit m = 1 oder 2 übergeführt werden, und zwar durch Oxidation gemäss allgemein bekannten Methoden, z.B. durch Umsetzung mit m-Chlorperbenzoesäure.

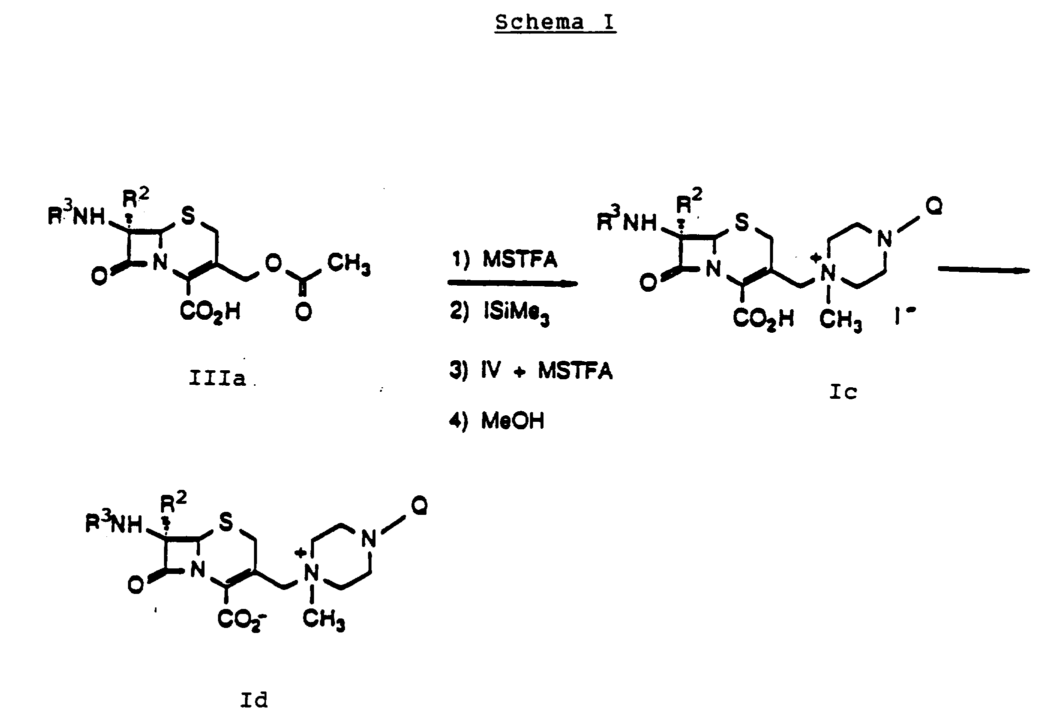

### Schema I

Die Ausgangsverbindung IIIa wird vorgängig geschützt durch Umsetzung mit einem Trimethylsilylierungsmittel, wie N-Methyl-N-(trimethylsilyl)trifluoracetamid (MSTFA), in einem inerten Lösungsmittel, wie z.B. Chloroform oder Methylenchlorid. Der erhaltene Trimethylsilylester, in welchem alle potentiell reaktiven Stellen, wie Amino, Hydroxy und Carboxy, durch Trimethylsilylgruppen geschützt sind, wird anschliessend mit Jodtrimethylsilan bei etwa 0°C bis Zimmertemperatur während etwa 20 Minuten bis etwa 2 Stunden umgesetzt. Das Reaktionsgemisch wird anschliessend unter vermindertem Druck eingedampft und der Rückstand in einem geeigneten nicht-hydroxylierten Lösungsmittel, wie z.B. Acetonitril, gelöst. Durch Zugabe einer geringfügigen Menge Tetrahydrofuran (THF) werden Restmengen an Jodtrimethylsilan zersetzt. Das erhaltene geschützte, iodierte Zwischenprodukt wird anschliessend in situ mit einem geschützten Piperazinderivat der Formel IV umgesetzt, welches seinerseits hergestellt wurde aus dem Piperazin IV durch Behandlung mit einem Trimethylsilylierungsmittel, wie MSTFA, in einem verträglichen Lösungsmittel, wie Acetonitril. Die Quaternisierungsreaktion wird bei etwa Zimmertemperatur während etwa 30 Minuten bis 24 Stunden, bevorzugt während etwa 2 Stunden, durchgeführt. Anschliessend wird ein hydroxyliertes Lösungsmittel, wie Methanol, unter Eiskühlung hinzugefügt, wodurch die Trimethylsilylschutzgruppen solvolytisch entfernt werden und das quaternäre Jodid der Formel Ic ausgefällt wird. Nach weiterer Behandlung unter wässrigen Bedingungen mit einer Base, wie Natriumbicarbonat oder Natriumhydroxid, oder mit einem Natriumsulfatpuffer, geht die Verbindung Ic in die zwitterionische Form Id über, worin je nach der Menge hinzugefügter Base oder des pH-Werts des Puffers andere saure Funktionen in R³ und Q in Salze übergeführt werden können.

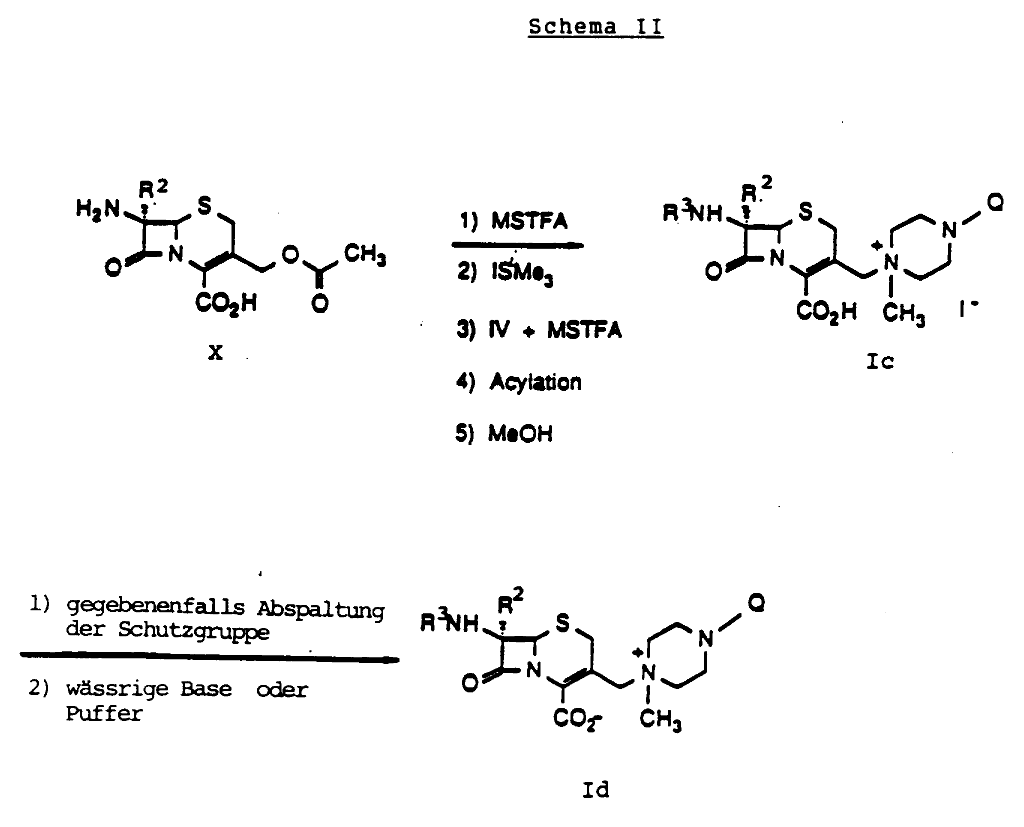

### Schema II

Die Ausgangsverbindungen der Formel X werden zunächst geschützt, und zwar durch Umsetzung mit einem Trimethylsilylierungsmittel, wie MSTFA, in einem inerten Lösungsmittel, wie Acetonitril, unter wasserfreien Bedingungen bei Zimmertemperatur während etwa 10 Minuten bis 2 Stunden, vorzugsweise etwa 30 Minuten. Nach Zugabe von Jodtrimethylsilan lässt man letzteres während etwa 15 Minuten bis 3 Stunden, vorzugsweise etwa 30 Minuten, bei Zimmertemperatur einwirken. Eine geringfügige Menge THF wird anschliessend hinzugefügt, wodurch Restmengen Jodtrimethylsilan zerstört werden. Es folgt die Quaternisierungsstufe, welche durch Zugabe eines geschützten Piperazinderivats der Formel IV (erhalten aus einer Verbindung IV durch Behandeln mit einem Trimethylsilylierungsmittel, wie z.B. MSTFA in einem geeigneten Lösungsmittel, wie Acetonitril) ausgeführt wird. Die Quaternisierungsreaktion läuft während etwa 30 Minuten bis 24 Stunden, vorzugsweise etwa 2-4 Stunden, insbesondere bei Zimmertemperatur. Anschliessend wird ein Acylierungsmittel, d.h. eine aktivierte Form einer Carbonsäure, z.B. ein Thioester wie z.B. 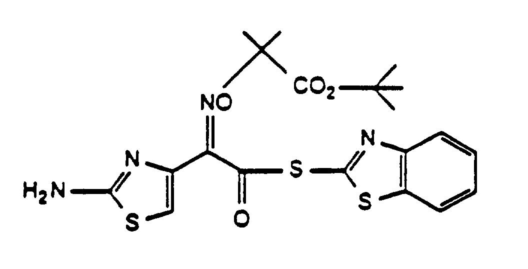 hinzugegeben. Das Reaktionsgemisch wird während etwa 2-24 Stunden gerührt. Das Gemisch wird anschliessend unter vermindertem Druck zur Trockene eingedampft und der Rückstand in Acetonitril aufgelöst. Die Zugabe von Methanol spaltet die Trimethylsilylschutzgruppen solvolytisch ab und fällt das quaternäre Jodid der Formel Ic aus. Allfällige weitere Schutzgruppen, z.B. im Substituenten R³, werden nach an sich bekannten Methoden entfernt. Beispielsweise wird ein t-Butylester durch Behandeln mit Trifluoressigsäure/Anisol abgespalten. Die Verbindungen der Formel Id werden wie in Schema I erhalten, d.h. durch wässriges Umsetzen mit einer Base, z.B. Natriumbicarbonat oder Natriumphosphatpuffer.

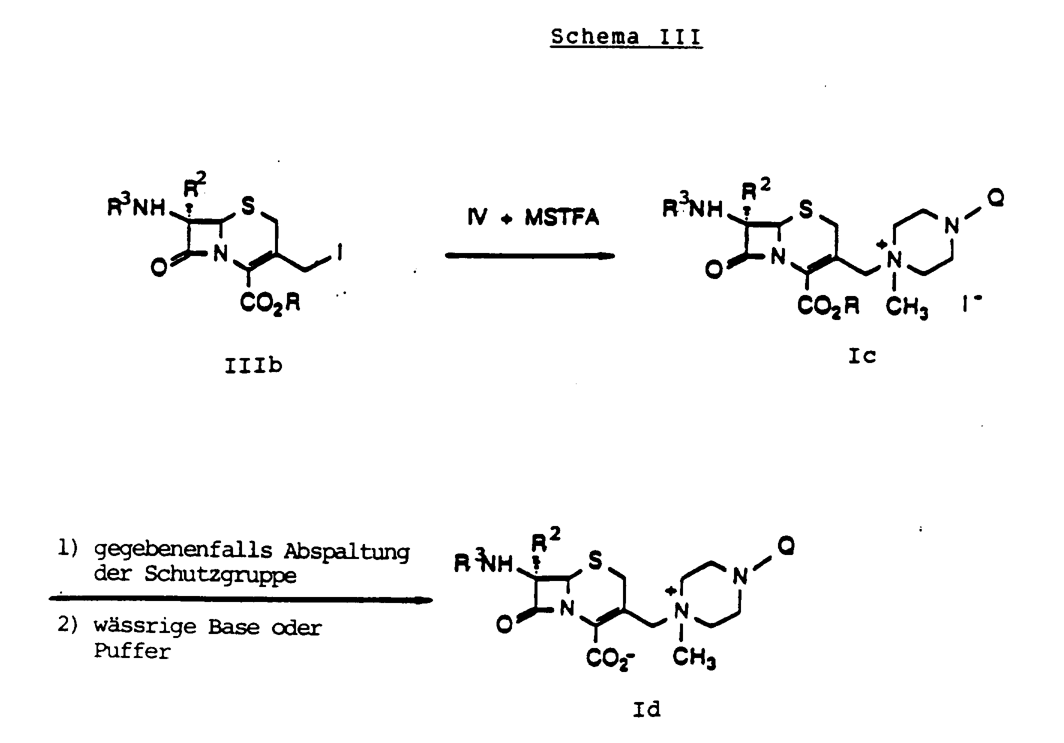

### Schema III

Verbindungen der Formel IIIb sind bekannt und können beispielsweise gemäss U.S.-Patent 4,266,049 (R. Bonjouklian, 5. Mai 1981) hergestellt werden. Das Piperazinderivat der Formel IV wird vorerst geschützt, und zwar durch Umsetzung mit einem Trimethylsilylierungsmittel, wie MSTFA, in einem geeigneten Lösungsmittel, wie Acetonitril, unter wasserfreien Bedingungen. Wahlweise können andere herkömmliche, unter milden Bedingungen abspaltbare Schutzgruppen verwendet werden. Beispielsweise kann eine Carboxygruppe in die t-Butyl- oder p-Nitrobenzylesterform umgewandelt werden. Nach Umsetzung von Verbindungen IIIb mit der geschützten Form der Piperazinderivate IV in einem inerten Lösungsmittel, wie Acetonitril, erhält man das quaternäre Jodid Ic. Nötigenfalls werden Schutzgruppen abgespalten; beispielsweise, falls R die t-Butylgruppe darstellt, kann diese mit Hilfe von Trifluoressigsäure/Anisol abgespalten werden. Nach anschliessender Behandlung mit wässriger Natriumbicarbonatlösung oder Natriumphosphatpufferlösung erhält man eine Verbindung der Formel I, worin andere Carboxyfunktionen in R³ und Q, je nach dem pH, in Salze umgewandelt werden können.

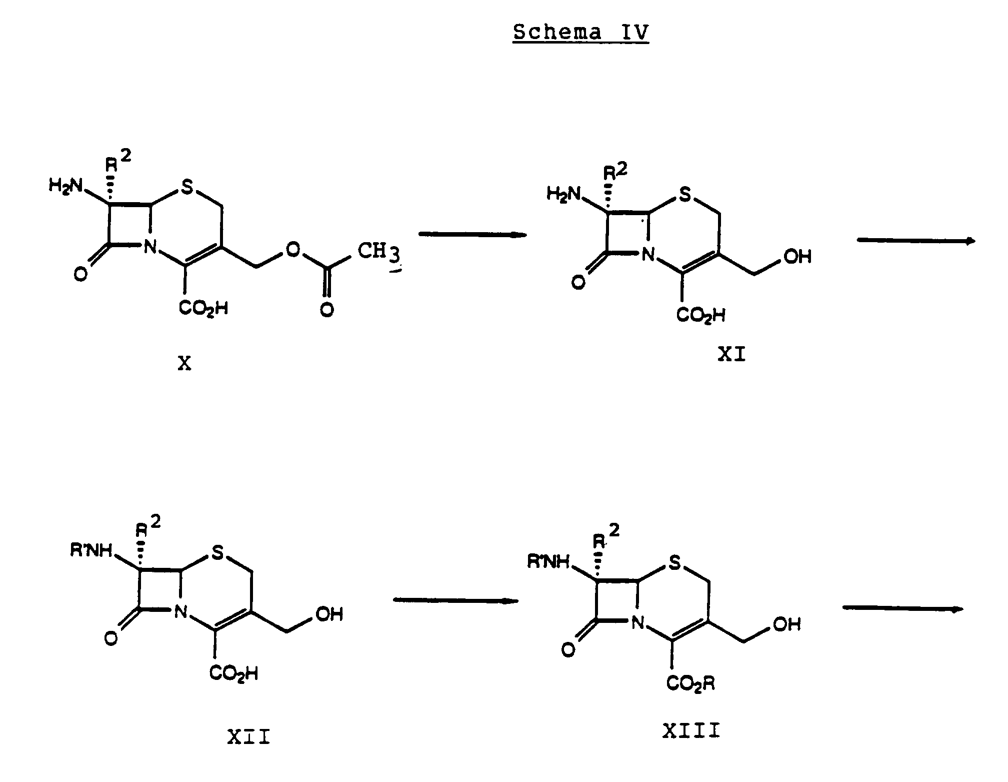

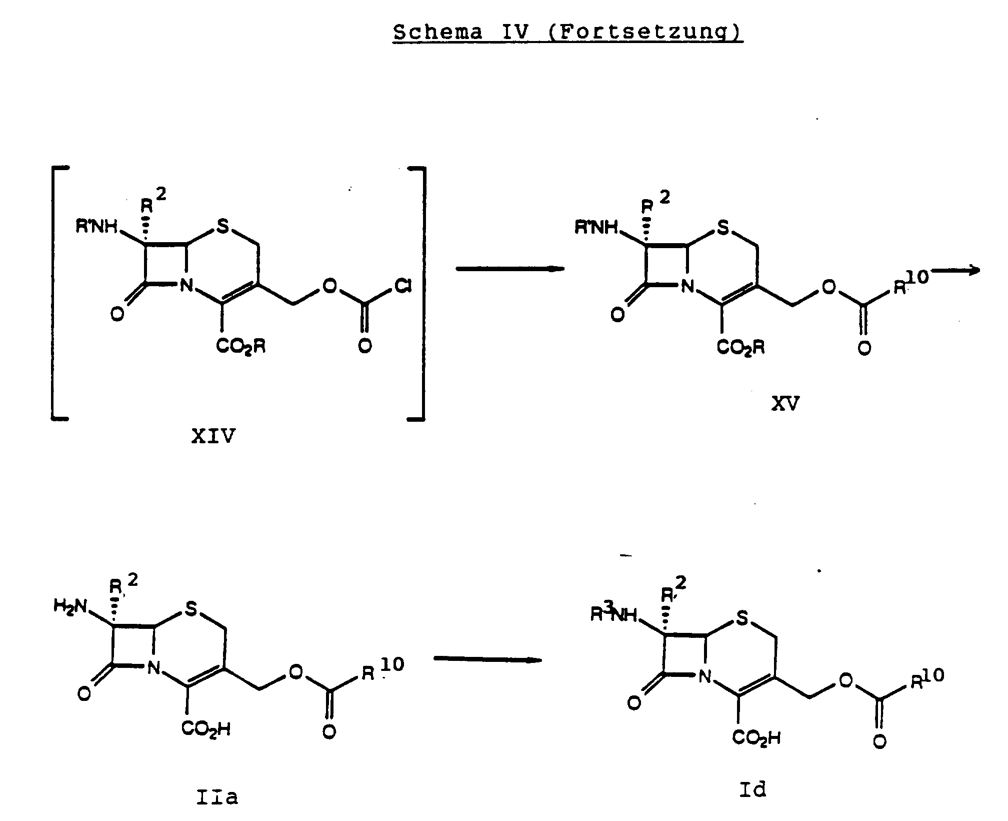

### Schema IV

### X → XIII

Die Verbindung der Formel X wird unter kontrollierten Bedingungen bei -5° bis +5°C mit wässriger Natriumhydroxidlösung zu Verbindung XI hydrolysiert. Die Verbindung X wird nicht isoliert sondern weiter umgesetzt, wobei eine leicht abspaltbare Aminoschutzgruppe R′ eingeführt wird. Beispielsweise liefert Umsetzung mit Di-t-butyldicarbonat während etwa 1-4 Tage die Verbindung der Formel XII, worin R′ t-Butoxycarbonyl darstellt. Nach Veresterung in an sich bekannter Weise erhält man die Verbindung XIII, worin R eine unter milden Bedingungen leicht abspaltbare Schutzgruppe darstellt. Beispielsweise erhält man durch Umsetzung der Verbindung XII mit Diphenyldiazomethan eine Verbindung XIII, worin R Diphenylmethyl darstellt.

### XIII → XV

Durch Umsetzung der Verbindung XIII mit Phosgen in Gegenwart einer Base, wie N,N-Diisopropyläthylamin oder Triäthylamin, in einem inerten Lösungsmittel, wie Methylenchlorid oder Chloroform, bei einer Temperatur von etwa 0°C bis etwa 30°C erhält man als Zwischenprodukt ein Chloroformat-Ester der Formel XIV, welches nicht isoliert sondern in situ verwendet wird. Diese Verbindung XIV wird mit einem substituierten Piperazin der Formel 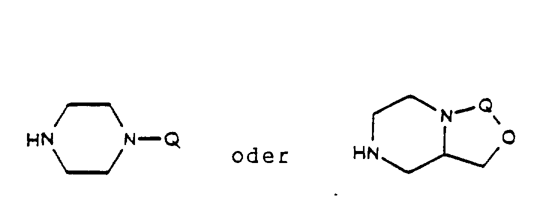 oder einem substituierten Pyrrolidinamin der Formel 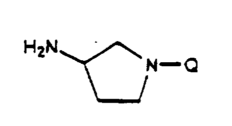 oder einem substituierten Pyrrolidinylmethylamin der Formel 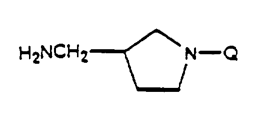 umgesetzt, wobei die Piperazin- oder Pyrrolidinkerne gegebenenfalls durch eine oder mehrere C₁-C₈-Alkylgruppen substituiert sein können, und wobei Q eine substituierte Chinolinyl- oder Naphthyridinylgruppe darstellt, worin Carboxygruppen vorzugsweise als Ester geschützt sind, z.B. als p-Nitrobenzylester. Man erhält das Carbamat der Formel XV. Wahlweise können Carboxygruppen in Q als Dimethylsilylester geschützt werden; ferner können die Aminofunktionen am Piperazin, Pyrrolidinamin bzw. Pyrrolidinylmethylamin vor der Umsetzung mit Verbindungen XIV trimethylsilyliert werden.

### XV → IIa

R′, R und andere Schutzgruppen in R¹⁰ werden anschliessend nach herkömmlichen Methoden entfernt. Je nach der Natur und Verschiedenartigkeit der Gruppen können mehr als eine Reaktion für die Schutzgruppenabspaltung nötig sein. Beispielsweise, falls R Diphenylmethyl und R′ t-Butoxycarbonyl darstellt, werden diese Gruppen durch Umsetzung mit Trifluoressigsäure/Anisol bei etwa 0°C bis etwa Zimmertemperatur in einem Lösungsmittel, wie Methylenchlorid oder Chloroform, entfernt. Falls R¹⁰ z.B. eine p-Nitrobenzylestergruppe enthält, kann diese durch Hydrogenolyse in einer separaten Stufe entfernt werden, vorzugsweise vorgängig der Entfernung von R und R′.

### IIa → Id

In der letzten Stufe des Schemas IV wird die Aminogruppe der Verbindung der Formel IIa durch Umsetzung mit einer aktivierten Carbonsäure nach an sich bekannten Methoden acyliert (Einführung von R³, d.h. man erhält die Verbindung Id). Beispielsweise wird ein Acylierungsmittel, wie z.B. ein Säurechlorid oder ein Thioester, wie z.B. 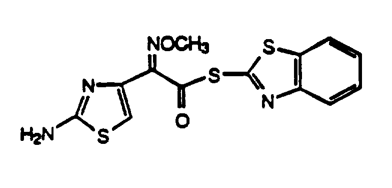 unter Verwendung eines geeigneten Lösungsmittels, wie wässriges Aceton oder wässriges Tetrahydrofuran, mit der Verbindung IIa in Gegenwart einer Base, wie Natriumbicarbonat oder Triäthylamin, umgesetzt. Die Reaktion wird zwischen etwa 0°C bis etwa 30°C während etwa 2-24 Stunden durchgeführt. Falls R³ unter Verwendung von Schutzgruppen eingeführt wird, werden diese Schutzgruppen anschliessend in an sich bekannter Weise entfernt.

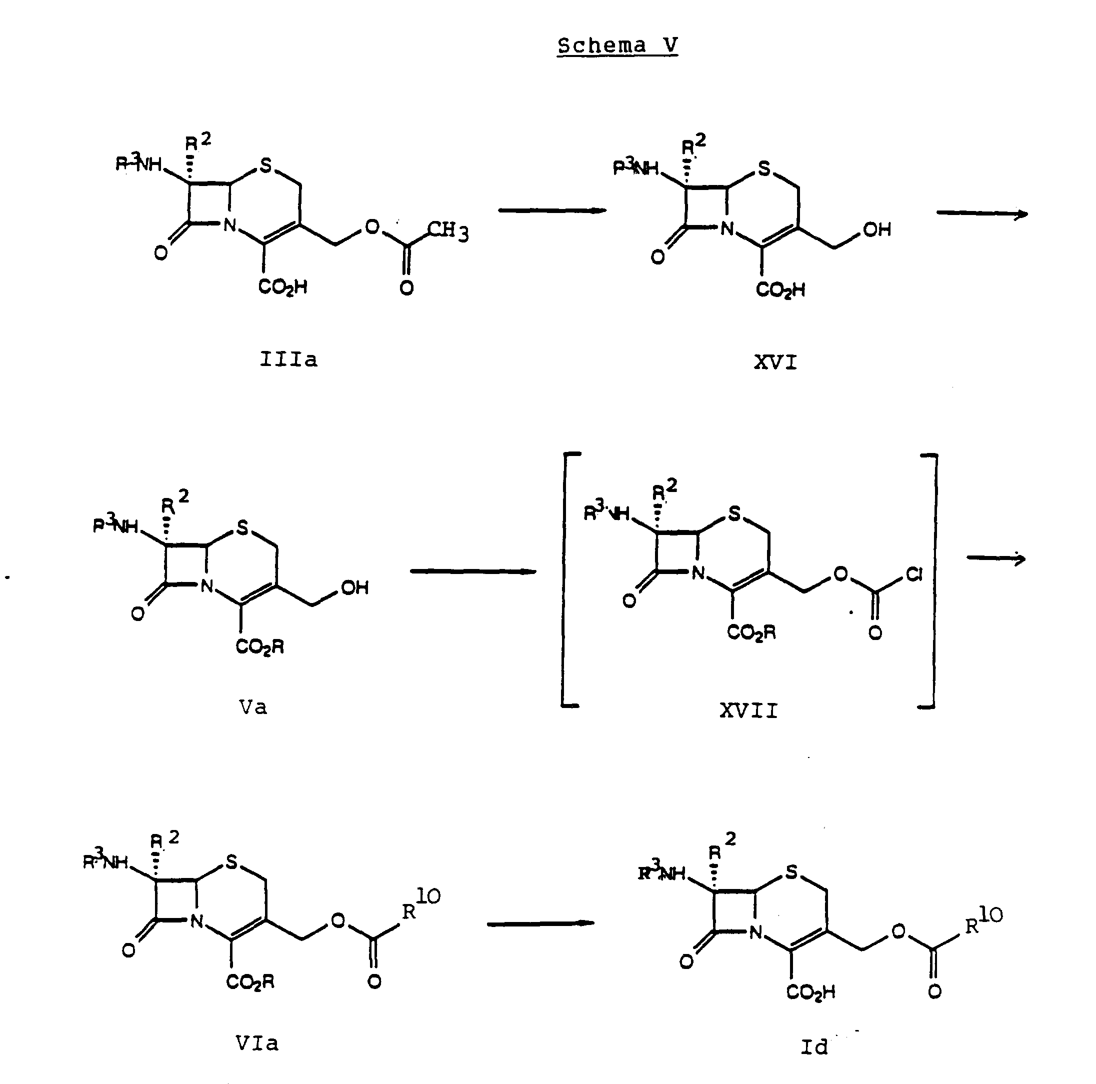

### Schema V

### IIIa → XVI

Eine Verbindung der Formel IIIa wird zu einer Verbindung der Formel XVI hydrolysiert, entweder mit Hilfe von wässriger Natriumhydroxidlösung oder enzymatisch durch Umsetzung mit einer Esterase, wie Zitrus-acetylesterase oder einer Esterase aus Bacterium subtilis, oder Weizenkleie, nach bekanntem Verfahren, siehe z.B.: H. Peter und H. Bickel, Helvetica Chimica Acta, 57, 2044 (1974); and U.S. Patent 4,406,899. Beispielsweise wird eine Verbindung der Formel IIIa mit R Wasserstoff und R³ Phenylacetyl in wässriger Mischung mit Acetylesterase aus Orangenschalen (von Sigma Chemical Company) während 18 bis 72 Stunden bei Zimmertemperatur hydrolysiert.

### XVI → Va

Die Verbindung der Formel XVI wird in an sich bekannter Weise zu einer Verbindung Va verestert, z.B. durch Umsetzung mit Diphenyldiazomethan, wobei der Diphenylmethylester erhalten wird. Vorgängig der weiteren Umwandlung der Verbindung Va sollten allfällige reaktive Gruppen, wie Aminogruppen, wie oben beschrieben geschützt werden.

### Va → VIa

Durch Umsetzung der Verbindung der Formel Va mit Phosgen in Gegenwart einer Base, wie N,N-Diisopropyläthylamin oder Triäthylamin, in einem inerten Lösungsmittel, wie Chloroform, Methylenchlorid, Dioxan oder Acetonitril, bei einer Temperatur zwischen etwa 0 und etwa 30°C, erhält man als Zwischenprodukt das Clorformatester der Formel XVII, welches nicht isoliert sondern in situ direkt verwendet wird. Durch Umsetzung der Verbindung XVII mit einem substituierten Piperazin der Formel 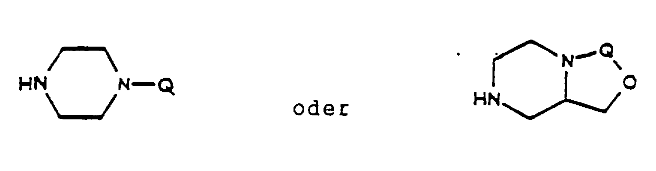 oder einem substituierten Pyrrolidinamin der Formel 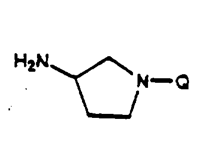 oder einem substituierten Pyrrodinylmethylamin der Formel 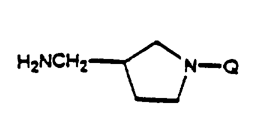 wobei die Piperazin- oder Pyrrolidinkerne wahlweise durch eine oder mehrere C₁-C₈-Alkylgruppen substituiert sein können, und wobei Q eine substituierte Chinolinyl- oder Naphthyridinylgruppe darstellt, worin Carboxygruppen vorzugsweise als Ester geschützt sind, z.B. als p-Nitrobenzylester (welche in die freie Säure leicht übergeführt werden können) erhält man das Carbamat der Formel VIa. Wahlweise können Carboxygruppen in Q als Trimethylsilylester geschützt werden; auch können Aminofunktionen des Piperazins, Pyrrolidinamins bzw. Pyrrolidinylmethylamins vor der Umsetzung mit der Verbindung XVII trimethylsilyliert werden.

### VIa → Id

Als letzte Reaktionsstufe in Schema V wird die Schutzgruppe R sowie allfällige andere Schutzgruppen in R¹⁰ und R³ nach an sich bekannten Methoden entfernt. Beispielsweise wird eine Nitrobenzylestergruppe durch Hydrolyse, eine t-Butyl- oder Diphenylmethylestergruppe durch Umsetzen mit Trifluoressigsäure/Anisol und eine N-Triphenylmethylgruppe durch Umsetzen mit wässriger Ameisensäure entfernt. Man erhält die Verbindung der Formel Id.

Verbindungen der Formel I mit den Gruppen 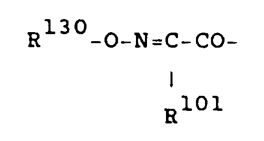 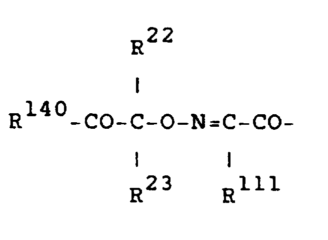 und 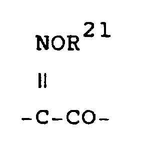 liegen vorzugsweise als syn-Formen vor. Solche syn-Formen können erhalten werden durch Verwendung von Ausgangsmaterialien, worin diese syn-Form bereits vorliegt. Wahlweise kann ein erhaltenes syn/anti-Gemisch einer Verbinung der Formel I in üblicher Weise in die entsprechenden syn- und anti-Formen aufgetrennt werden, beispielsweise durch Umkristallisation oder durch chromatographische Methoden unter Verwendung eines geeigneten Lösungsmittels bzw. Lösungsmittelsgemisches.

Die Verbindungen der Formel I sowie die entsprechenden Salze bzw. die Hydrate dieser Produkte und Ester sind antibiotisch, insbesondere bakterizid wirksam. Sie können als Agenzien zur Bekämpfung von bakteriellen Infektionen (einschliesslich Harn- und Atemweginfektionen) bei Säugern, z.B. Hunden, Katzen, Pferden usw., sowie beim Menschen verwendet werden. Diese Cephalosporine sind gegen einen breiten Bereich sowohl gram-negativer als auch gram-positiver Bakterien wirksam.

Die in vitro Wirksamkeit der erfindungsgemässen Verbindungen gegen eine Vielzahl gram-positiver und gram-negativer Mikroorganismen, ausgedrückt als Mindesthemmkonzentration in Mikrogramm/ml und ermittelt unter Verwendung der Reihenverdünnungsmethode (in Flüssigmedium) ist wie folgt:
Verbindung A: [6R-[6α,7β(Z)]]-1-[7-[[[(2-Amino-4-thiazolyl)(methoxyimino)acetyl]amino]-2-carboxy-8-oxo-5-thia-1-azabicyclo[4.2.0]oct-2-en-3-yl]-methyl]-4-[3-carboxy-6,8-difluor-1-(2-fluoräthyl)-1,4-dihydro-4-oxo-7-chinolinyl]-1-methylpiperaziniumjodid
Verbindung B: (6R-trans)-4-[3-Carboxy-1-(2-fluoräthyl)-6,8-difluor-1,4-dihydro-4-oxochinolin-7-yl]-1-[[2-carboxy-8-oxo-7-[(phenoxyacetyl)amino]-5-thia-1-azabicyclo[4.2.0]oct-2-en-3-yl]methyl]-1-methyl-piperaziniumjodid
Verbindung C: [6R-[6α,7β(Z)]]-1-[[7-[[[(2-Amino-4-thiazolyl)[1-(1-carboxy-1-methyl)äthoxy]imino]acetyl]amino]-8-oxo-5-thia-1-azabicyclo[4.2.0]oct-2-en-3-yl]methyl]-4-[3-carboxy-1-(2-fluoräthyl)-6,8-difluor-1,4-dihydro-4-oxochinolin-7-yl]-1-methylpiperaziniumhydroxid-inneres Salz-Mononatriumsalz
Verbindung D: [6R-[6α,7β(Z)]]-1-[[7-[[(2-Amino-4-thiazolyl)(methoxyimino)acetyl]amino]-2-carboxy-8-oxo-5-thia-1-azabicyclo[4.2.0]-oct-2-en-3-yl]methyl]-4-(3-carboxy-1-äthyl-6-fluor-1,4-dihydro-4-oxo-7-chinolinyl)-1-methylpiperaziniumjodid
Verbindung E: (6R-trans)-4-[3-Carboxy-6,8-difluor-1-(2-fluoräthyl)-1,4-dihydro-7-chinolinyl]-1-[[2-carboxy-7-(formylamino)-8-oxo-5-thia-1-azabicyclo[4.2.0]oct-2-en-3-yl]methyl]-1-methylpiperaziniumtrifluoracetat

**Tabelle 1**

| In Vitro MHK (µg/ml), Reihenverdünnungsmethode in Flüssigmedium | | | | | |
|---|---|---|---|---|---|
| | Verbindungen | | | | |
| Kultur | A | B | C | D | E |
| E. coli ATCC 25922 | 0.25 | 0.5 | 0.25 | 0.5 | 0.5 |
| E. coli TEM 1 | 0.25 | 0.5 | 0.5 | 0.5 | 0.5 |
| E. cloacae 5699 | 0.5 | 0.5 | 0.5 | 1 | 0.5 |
| E. cloacae P99 | 0.5 | 0.5 | 2 | 1 | 0.5 |
| S. marcescens 1071 | 0.5 | 0.5 | 0.5 | 0.5 | 1 |
| P. aeruginosa 8710 | 8 | 8 | 8 | 8 | 8 |
| P. aeruginosa 18 S/H | 8 | 4 | 8 | 32 | 8 |
| E. faecalis ATCC 29212 | 64 | 32 | 32 | 32 | 32 |
| S. pneumoniae 6301 | 0.063 | 0.25 | 1 | 0.063 | 1 |
| S. aureus 1059B | 2 | 0.5 | 8 | 2 | 0.5 |
| S. aureus 95 | 4 | 1 | 4 | 4 | 1 |
| S. aureus ATCC 29213 | 4 | 0.5 | 4 | 4 | 0.5 |

Die in vitro Wirksamkeit der erfinungsgemässen Verbindungen gegen eine Vielzahl grampositiver und gramnegativer Mikroorganismen ausgedrückt als Mindesthemmkonzentration in Mikrogramm/ml und ermittelt unter Verwendung der Reihenverdünnungsmethode (in Agarmedium) ist wie folgt:
Verbindung F: [6R-[6α,7β(Z)]]-7-[[(2-Amino-4-thiazolyl)-(methoxyimino)acetyl]amino]-3-[[[[4-(3-carboxy-1-äthyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure-Dinatriumsalz.
Verbindung G: [6R-[6α,7β(Z)]]-7-[[(2-Amino-4-thiazolyl)-(methoxyimino)acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure-Dinatriumsalz.

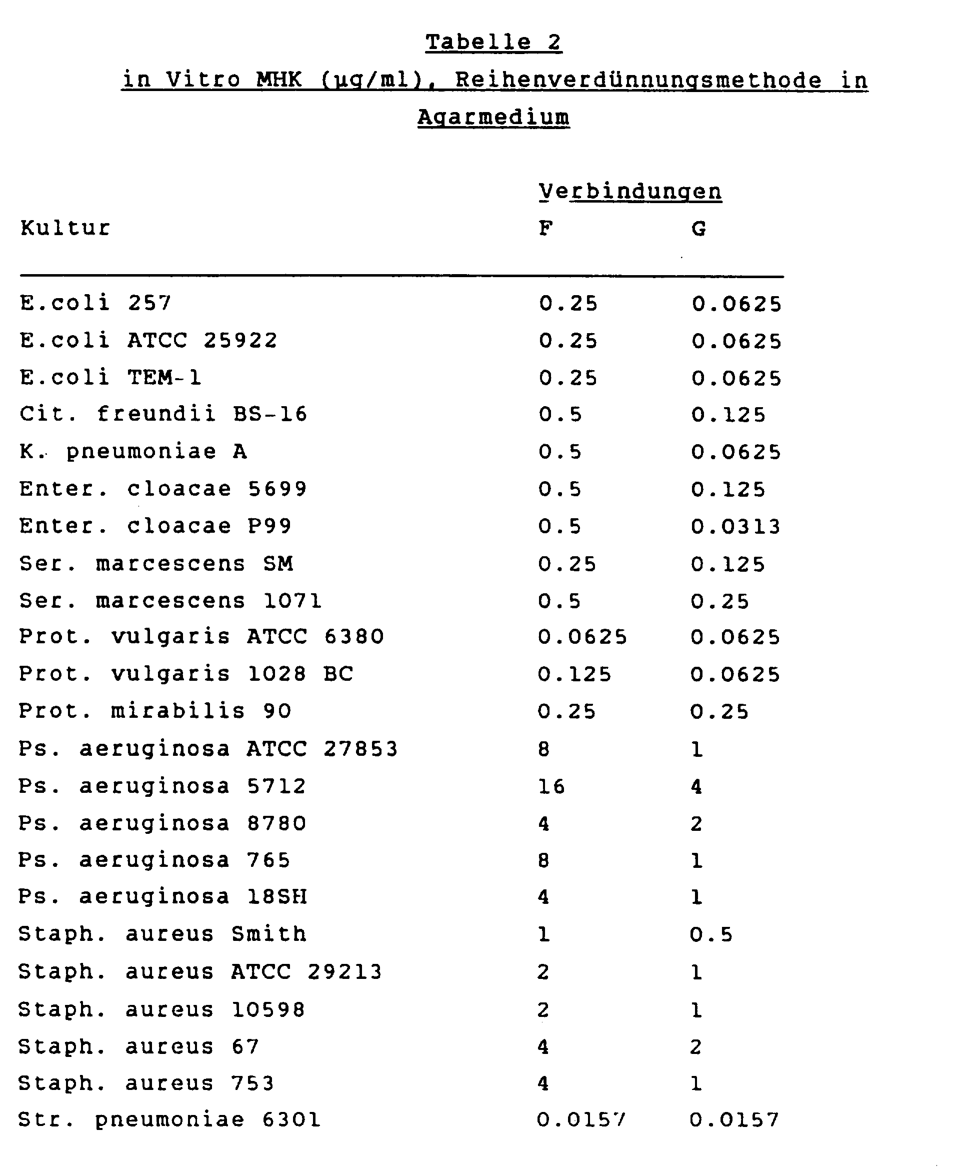

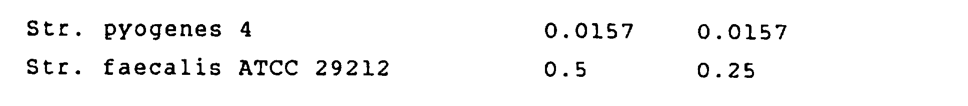

Zur Bekämpfung einer bakteriellen Infektion in einem Säuger kann eine erfindungsgemässe Verbindung mit einer Tagesdosis von etwa 5 bis etwa 500 mg/kg, vorzugsweise etwa 10-100 mg/kg, insbesondere etwa 10-55 mg/kg, behandelt werden. Sämtliche Verabreichungsarten, welche bisher für die Penicillin- und Cephalosporintherapie in Frage kommen, sind ebenfalls für die erfindungsgemässen neuen Cephalosporine verwendbar. Verabreichungsarten sind demnach z.B. intravenöse, intramuskuläre und enterale Administrierungen, z.B. in Form von Suppositorien.

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial wie z.B. Wasser, Gelatine, Gummi arabicum, Milchzucker, Stärke, Magnesiumstearat, Talk, pflanzliche Oele, Polyalkylenglykole, Vaseline usw. enthalten. Die pharmazeutischen Präparate können in fester Form, z.B. als Tabletten, Dragées, Suppositorien, Kapseln oder in flüssiger Form, z.B. als Lösungen, Suspensionen oder Emulsion vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetika oder Puffer. Sie können auch noch andere therapeutisch wertvolle Stoffe enthalten. Die Carbonsäuren der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser und isotonischer Kochsalzlösung, Lösungsmittelvermittler, wie Propylenglykol, zubereitet. Die leicht hydrolysierbaren Ester der Formel I kommen auch für die enterale Verabreichung in Betracht.

### Beispiel 1

### Herstellung von [6R-trans]-4-[3-Carboxy-1-(2-fluoräthyl)-6,8-difluor-1,4-dihydro-4-oxochinolin-7-yl]-1-[[2-carboxy -8-oxo-7-[(phenoxyacetyl)amino]-5-thia-1-azabicyclo[4.2.0]oct -2-en-3-yl]methyl]-1-methylpiperaziniumjiodid

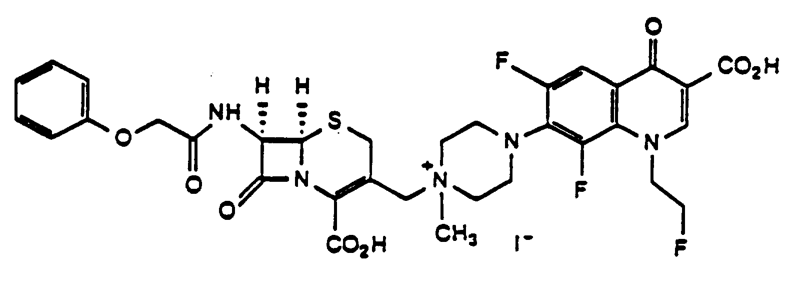

In einer Argonatmosphäre wird ein Gemisch von 406 mg (1 mMol) [6R-trans]-3-(Acetyloxy)methyl]-8-oxo-7-[(phenoxyacetyl)amino]-5-thia-1-azabicyclo[4.2.O]oct-2-en-2-cacbonsäure ,2 ml trockenem Methylenchlorid und 0,60 ml (3 mMol) N-Methyl-N-(trimethylsilyl)trifluoracetamid (MSTFA) eine Stunde gerührt; nach Zugabe von 0,28 ml (2 mMol) Jodtrimethylsilan wird das Gemisch weitere 2 Stunden gerührt. Die Lösung wird anschliessend unter vermindertem Druck zur Trockene eingedampft und das zurückbleibende Oel in 2 ml Acetonitril gelöst. Nach Zugabe von 5 Tropfen wasserfreiem Tetrahydrofuran (THF) wird das Gemisch 5-10 Minuten gerührt. Nach Zugabe einer Lösung aus 111 mg (0,3 mMol) 6,8-Difluor-1-(2-fluoräthyl)-1,4-dihydro-7-(4-methyl -1-piperazinyl)-4-oxo-3-chinolincarbonsäure, 0,11 ml (0,6 mMol) MSTFA und einem Liter Acetonitril wird das Ganze weitere 2 Stunden gerührt. Das Gemisch wird in Eis gekühlt, und etwa 100 mg Methanol werden hinzugefügt. Der erhaltene Niederschlag wird abfiltriert, mit Acetonitril gewaschen und unter vermindertem Druck getrocknet. Man erhält so die Titelverbindung: NMR (Me₂SO-d₆) δ 3,15 (s, 3H, NCH₃), 3,45-3,85 (m, 9H, 4 x NCH₂ und CH von SCH₂), 3,95 (d, 1H, J gem = 16,5 Hz, CH von SCH₂), 4,39 und 4,77 (AB, 2H, J gem = 13Hz, NCH₂), 4,61 und 4,64 (AB, 2H, J gem = 15 Hz, OCH₂CO), 4,83-5,07 (m, 4H, NCH₂CH₂F), 5,24 (d, 1H, J = 5Hz, CH), 5,82 (dd, 1H, J = 5 und 7 Hz, CH), 6,95 (d, 2H, J = 8 Hz, Ar), 6,97 (t, 1H, J = 8 Hz, Ar), 7,29 (t, 2H, J = 8 Hz), 7,96 (d, 1 H, J = 12 Hz, Ar), 8,91 (s, 1H, =CH-), 9,18 (d, 1H, J = 7 Hz, NH); IR (KBr) 3400, 1788, 1728, 1700, 1612, cm⁻¹; Massenspektrum m/z 716 (Kation).

### Beispiel 2

### Herstellung von [6R-[6α,7β(Z)]]-1-[[7-[[[(2-Amino-4-thiazolyl)[1-(1-carboxy-1-methyl)äthoxy]imino]acetyl]amino] -2-carboxy-8-oxo-5-thia-1-azabicyclo[4.2.0]oct-2-en-3-yl] methyl]-4-[3-carboxy-1-(2-fluoräthyl)-6,8-difluor-1,4-dihydro-4-oxochinolin-7-yl]-1-methylpiperaziniumhydroxid-inneres Salz-Mononatriumsalz

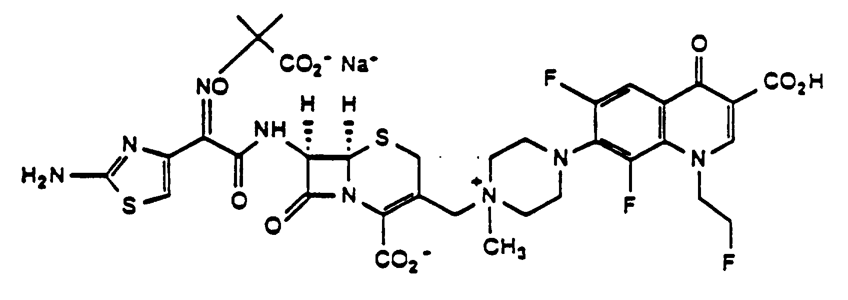

In einer Argonatmosphäre wird ein Gemisch von 5,12 g (8 mMol) [6R-[6α,7β(Z)]]-3-[(Acetyloxy)methyl]-7-[[[(2-amino-4-thiazolyl)[1-(1-carboxy-1-methyl)äthoxy]imino]acetyl]amino]-8-oxo-5-thia-1-azabicyclo[4.2.0]oct-2-en-2-carbonsäure-trifluoressigsäuresalz, 48 ml trockenem Acetonitril und 12 ml (64 mMol) MSTFA 30 Minuten gerührt; nach tropfenweise Zugabe von 2,0 ml (14 mMol) Jodtrimethylsilan wird das Gemisch weitere 30 Minuten gerührt. Das Ganze wird in Eis abgekühlt und mit 1,12 ml (14 mMol) trockenem THF versetzt. Nach 10 Minuten wird eine Lösung aus 2,27 g (6 mMol) 6,8-Difluor-1-(2-fluoräthyl)-1,4-dihydro-7-(4-methyl-1-piperazinyl)-4-oxo-3-chinolincarbonsäure, 24 ml Acetonitril und 1,28 ml (7,2 mMol) MSTFA hinzugefügt und das Gemisch weitere 1,5 Stunden gerührt. Das Gemisch wird unter vermindertem Druck eingedampft und das zurückbleibende Oel in 40 ml Acetonitril gelöst. Nach Kühlung auf Eis werden 4 ml Methanol hinzugefügt, worauf ein dicker Niederschlag ensteht. Nach einigen Minuten hat sich dieser gesetzt und wird abfiltriert und viermal mit je 10 ml Acetonitril gewaschen. Nach dem Trocknen wird der Feststoff mit 60 ml Methanol verrieben, filtriert und viermal mit je 10 ml Methanol gewaschen. Der so erhaltene Feststoff (5,7 g) wird in Wasser aufgeschlämmt und mit wässriger Natriumbicarbonatlösung bis auf pH 7 versetzt. Diese Rohproduktlösung wird in drei Stufen durch C₁₈-reverse-phase-Hochdruckflüssigchromatographie gereinigt. In einer ersten Stufe wird eine Kolonne mit 50 g Waters C₁₈-Kieselgel verwendet mit Wasser, gefolgt von 30% Acetonitril in Wasser, als Eluens. In einer zweiten Stufe wird das Produkt weiter gereinigt durch Hochdruckflüssig- chromatographie an einer C₁₈-Waters-Prep-500A-Kolonne. In einer dritten Stufe wird das Produkt entsalzt an einer Flash-Kolonne mit 60 g C₁₈-Kieselgel und Wasser und 20% Acetonitril in Wasser als Eluens. Nach dem Eindampfen unter vermindertem Druck und dem Gefriertrocknen erhält man 1,0 g Titelverbindung: NMR (Me₂SO-d6-D₂O) δ 1,37 (s, 3H, CH₃), 1,44 (s, 3H, CH₃), 3,10 (s, 3H, NCH₃), 3,39 und 3,88 (AB, 2H, J gem = 16,5 Hz, SCH₂), 3,40-3,70 (m, 8H, 4 x NCH₂), 4,12 und 5,17 (AB, 2H, J gem = 12.5 Hz, NCH₂) 4,62-4,94 (m, 4H, NCH₂CH₂F), 5,15 (d, 1H, J = 5Hz, CH), 5,73 (d, 1H, J = 5Hz, CH) 6,74 (s, 1H, Ar), 7,83 (d, 1H, J = 12 Hz, Ar), 8,47 (brs, 1H, = CH-); IR (KBr) 3400, 1772, 1618, 1595 cm⁻¹; Massenspektrum m/z = 859 (M + H)⁺.

### Beispiel 3

### Herstellung von [6R-[6α,7α(Z)]]-1-[[7-[[(2-Amino-4-thiazolyl)(methoxyimino)acetyl]amino]-2-carboxy-8-oxo-5-thia -1-azabicyclo[4.2.0]oct-2-en-3-yl]methyl]-4-(3-cacboxy-1-äthyl -6-fluor-1,4-dihydro-4-oxo-7-chinolinyl)-1-methylpiperazinium iodid

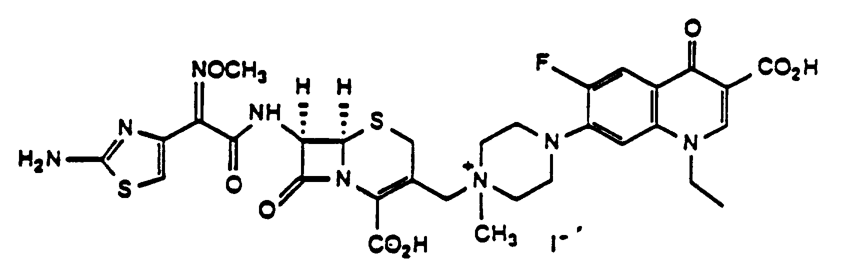

In einer Argonatmosphäre wird ein Gemisch von 273 mg (0,6 mMol) [6R-[6α,7β(Z)]]-3-[(Acetyloxy)methyl]-7-[[(2-amino-4-thiazolyl)(methoxyimino)acetyl]amino]-8-oxo-5-thia-1-azabicyclo[4.2.0]oct-2-en-2-carbonsäure, 2 ml trockenem Methylenchlorid und 0,45 ml (2,4 mMol) MSTFA 2 Stunden gerührt. Nach Zugabe von 0,17 ml (1,2 mMol) Jodtrimethylsilan wird das Ganze weitere 2 Stunden gerührt. Das Gemisch wird unter vermindertem Druck eingedampft und das zurückbleibende Oel in 2 ml trockenem Acetonitril gelöst. Nach Zugabe einiger Tropfen trockenem THF wird das Gemisch weitere 15 Minuten gerührt. Eine Lösung aus 60 mg (0,18 mMol) 1-Aethyl-6-fluor-1,4-dihydro-7-(4-methyl-1-piperazinyl)-4-oxo-3-chinolincarbonsäure, 1 ml trockenem Acetonitril und 0,07 ml (0,36 mMol) MSTFA wird hinzugefügt und das Ganze weitere 2 Stunden gerührt. Nach tropfenweiser Zugabe von 125 mg Methanol fällt das Produkt aus. Der Feststoff wird abfiltriert, viermal mit je 1 ml Acetonitril gewaschen und unter vermindertem Druck eingedampft. Nach dem Verreiben mit Methanol unter vermindertem Druck erhält man die Titelverbindung: NMR (Me₂SO-d6) δ 1,45 (brt, 3H, CH₃ von NEt), 3,15 (s, 3H, NCH₃), 3,50-3,95 (m, 9H, 4 x NCH₂ und CH von SCH₂), 3,85 (s, 3H, OCH₃), 3,99 (d, lH, J gem = 16,5 Hz, CH von SCH₂), 4,43 und 4,70 (AB, 2H, J gem = 14 Hz, NCH₂), 4,61 (brq, 2H, CH₂ von NEt), 5,31 (d, 1H, J = 5 Hz, CH), 5,94 (dd, 1H, J = 5 und 8 Hz, CH), 6,76 (s, 1H, Ar), 7,30 (br, 2H, NH₂), 7,31 (d, 1H, J = 6,5 Hz, Ar), 8,03 (d, 1H, J =12,5 Hz, Ar), 9,03 (s, 1H, = CH-), 9,68 (d, 1H, J = 8 Hz, NH); IR (KBr) 1785, 1720, 1680, 1628, cm⁻¹; Massenspektrum m/z = 729 (Kation).

### Beispiel 4

### Herstellung von [6R-[6α,7β(Z)]]-1-[7-[[[(2-Amino-4-thiazolyl)(methoxyimino)acetyl]lamino]-2-carboxy-8-oxo-5-thia -1-azabicyclo[4.2.0]oct-2-en-3-yl]methyl]-4-[3-carboxy-6,8-di -fluor-1-(2-fluoräthyl)-1,4-dihydro-4-oxo-7-chinolinyl]-1 -methylpiperaziniumjodid

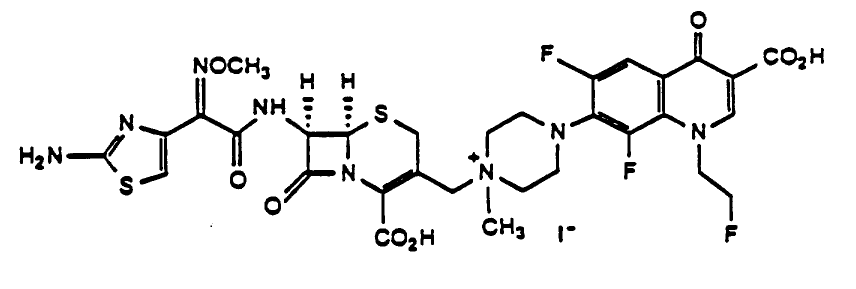

Ein Gemisch aus 84,6 mg (0,186 mMol) [6R-[6α,7β(Z)]]-3-[(Acetyloxy)methyl]-7-[[(2-amino-4-thiazolyl)(methoxyimino)acetyl]amino]-8-oxo-5-thia-1-azabicyclo[4.2.0]oct-2-en-2-carbonsäure, 4 ml trockenem Methylenchlorid und 0,148 ml (0,8 mMol) MSTFA wird eine Stunde gerührt. Die erhaltene Lösung wird mit 0,0625 ml (0,46 mMol) Jodtrimethylsilan versetzt. Das Gemisch wird 2 Stunden gerührt und unter vermindertem Druck zur Trockene eingedampft. Der Rückstand wird in 2 ml trockenem Acetonitril gelöst und mit einigen Tropfen trockenem THF versetzt. Das Gemisch wird 3 Minuten gerührt und anschliessend mit einer Lösung aus 36,9 mg (0,10 mMol) 6,8-Difluor-1-(2-fluoräthyl)-1,4-dihydro-7-(4-methyl-1-piperazinyl)-4-oxo -3-chinolincarbonsäure, 0,040 ml (0,215 mMol) MSTFA und 2,0 ml trockenem Acetonitril versetzt. Das Gemisch wird 3 Stunden gerührt. Nach Zugabe von 0,50 ml Methanol entsteht ein Niederschlag. Der Feststoff wird abfiltriert, wiederholt mit Acetonitril gewaschen und getrocknet. Man erhält 87,5 mg Titelverbindung: NMR (Me₂SO-d6) δ 3,15 (s, 3H, NCH₃), 3,50-3,90 (m, 9H, 4 x NCH₂ und CH von SCH₂), 3,86 (s, 3H, OCH₃), 3,96 (d, 1H, J gem = 17 Hz, CH von SCH₂), 4,42 und 4,73 (AB, 2H, J gem = 14 Hz, NCH₂), 4,85-5,10 (m, 4H, NCH₂CH₂F), 5,29 (d, 1H, J = 5 Hz, CH), 5,93 (dd, 1H, J = 5 und 7 Hz, CH), 6,86 (s, 1H, Ar), 7,24 (s, 2H, NH₂), 7,98 (d, 1H, J = 12 Hz, Ar), 8,94 (s, 1H = CH-), 9,67 (d, 1H, J = 7Hz, NH); IR (KBr) 3420, 1775, 1720, 1675, 1618 cm⁻¹.

### Beispiel 5

### Herstellung von [6R-[6α,7β(Z)-1-[7-[[[(2-Amino-4-thiazolyl) (methoxyimino)acetyl]amino]-2-carboxy-8-oxo-5-thia-1-azabicyclo[4.2.0]oct-2-en-3-yl]methyl]-4-(3-carboxy-6,8 -difluor-1-(2-fluoräthyl)-1,4-dihydro-4-oxo-7-chinolinyl]-1 -methylpiperaziniumhydroxid-inneres Salz-Mononatriumsalz

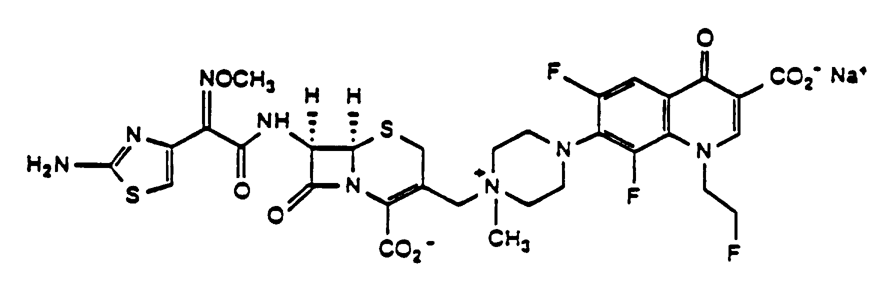

Eine Aufschlämmung von 900 mg gemäss Beispiel 4 hergestellter Verbindung in Wasser wird mit 0,1N wässriger Natriumhydroxidlösung neutralisiert, und die erhaltene Lösung wird gefriergetrocknet. Der Rückstand wird gereinigt durch C18-reverse-phase-Hochdruckflüssigchromatographie an einer Waters-Prep-500A-Kolonne mit einem Wasser-Acetonitril-Gradienten 0-40% als Eluens. Eindampfen und Gefriertrocknen der entsprechenden Fraktionen liefern 344 mg Titelverbindung: NMR (Me₂SO-d6) δ 3,10 (s, 3H, NCH₃), 3,40-3,76 (m, 9H, 4 x NCH₂ und CH von SCH₂), 3,84 (d, 1H, J = 16 Hz, CH von SCH₂), 3,84 (s, 3H, OCH₃), 4,10 und 5,19 (AB, 2H, J gem =14Hz), 4,70-4,94 (m, 4H, NCH₂CH₂F), 5,13 (d, 1H, J = 5Hz, CH), 5,65 (dd, 1H, J = 5 und 7Hz, CH), 6,74 (s, 1H, Ar), 7,23 (s, 2H, NH₂), 7,82 (d, 1H, J = 12Hz, Ar), 8,55 (s, 1H, =CH-), 9,58 (d, 1H, J = 7Hz, NH); IR (KBr) 3410, 1772, 1665, 1618 cm⁻¹; Massenspektrum m/z 787 (M + H)⁺.

### Beispiel 6

### Herstellung von (6R-trans)-4-[3-Carboxy-6,8-difluor-1-(2-fluoräthyl)-1,4-dihydro-7-chinolinyl]-1-[[2-(1,1 -dimethyläthoxy)carbonyl-7-(formylamino)-8-oxo-5-thia-1 -azabicyclo[4.2.0]oct-2-en-3-yl]-methyl]-1-methylpiper aziniumjodid

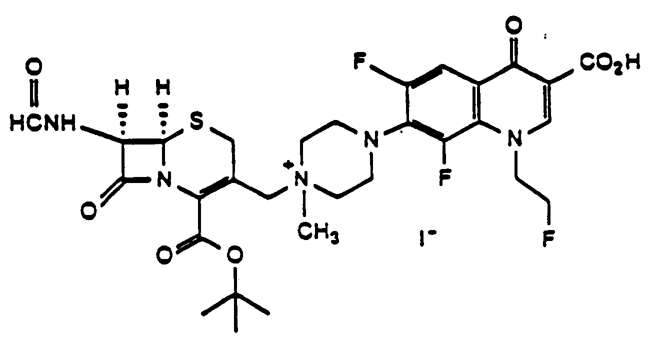

Ein Gemisch aus 0,87 g (2,35 mMol) 6,8-Difluor-1-(2-fluoräthyl)-1,4-dihydro-7-(4-methyl -1-piperazinyl)-4-oxo-3-chinolincarbonsäure, 0,51 ml (2,6 mMol) MSTFA und 5 ml trockenem Acetonitril wird 30 Minuten gerührt; nach Zugabe von 1,00 g [6R(6α,7β]-7-formylamino-3-jodmethyl-8-oxo-5-thia-1-azabicyclo-[4.2.0]oct-2-en-2-carbonsäure-1,1-dimethyläthylester wird das Ganze weitere 24 Stunden gerührt. Der erhaltene Niederschlag wird abfiltriert und verworfen. Die Mutterlauge wird an 5 g einer C₁₈-Kieselgelkolonne absorbiert. Nach dem Eluieren mit Wasser, 10% und 20% wässrigem Methanol werden die entsprechenden Fraktionen vereinigt und unter vermindertem Druck eingedampft. Man erhält einen Niederschlag, der abfiltriert und getrocknet wird. Man erhält so 380 mg Titelverbindung: IR (KBr) 3440, 1785, 1720. 1610 cm⁻¹; Massenspektrum m/z = 666 (Kation).

### Beispiel 7

### Herstellung von (6R-trans)-4-[3-Carboxy-6,8-difluor-1-(2-fluoräthyl)-1-1,4-dihydro-7-chinolinyl]-1-[[2-carboxy-7 -(formylamino)-8-oxo-5-thia-1-azabicyclo[4.2.0]oct-2-en-3-yl] methyl]-1-methylpiperaziniumtrifluoracetat

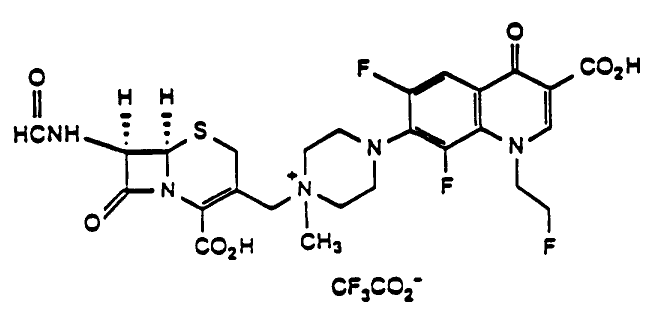

Ein Gemisch aus 200 mg der gemäss Beispiel 6 hergestellten Verbindung, 0,2 ml Anisol und 2,5 ml Trifluoressigsäure wird 3 Stunden bei Zimmertemperatur gerührt. Nach dem Abfiltrieren der unlöslichen Anteile wird die Lösung unter vermindertem Druck eingedampft. Der Rückstand wird in 10 ml Acetonitril gelöst und mit 200 ml Aether zur Ausfällung des Produktes versetzt. Der Niederschlag wird abfiltriert und getrocknet, und man erhält 135 mg Titelverbindung: NMR (Me₂SO-d6) δ 3,14 (s, 3H, NCH₃), 3,50-3,85 (m, 9H, 4 x NCH₂ und CH von SCH₂), 3,95 (d, 1H, J gem = 16,5 Hz, CH von SCH₂), 4,35 (d, 1H, J gem = 13Hz, CH von NCH₂), 4,82-5,05 (m, 5H, NCH₂CH₂F und CH von NCH₂), 5,23 (d, 1H, J = 5Hz, CH), 5,84 (dd, 1H, J = 5 und 7Hz, CH), 7,96 (d, 1H, J = 13Hz, Ar), 8,17 (s, 1H, NCHO), 8,92 (s, 1H, =CH-), 9,11 (d, 1H, J = 7Hz, NH); IR 3400, 1780, 1720, 1685 cm⁻¹; Massenspektrum m/z = 610 (Kation).

### Beispiel 8

### Herstellung von [6R-[6α,7β(Z)]]-1-[[7-[[(2-Amino-4-thiazolyl)[[1,1-dimethyl-2-(1,1-dimethyläthoxy)-2-oxoäthoxy] imino]acetyl]amino]-8-oxo-5-thia-1-azabicyclo[4.2.0]oct-2-en-3-yl]methyl]-4-[3-carboxy-(2-fluoräthyl)-6,8-difluor-1,4 -dihydro-4-oxochinolin-7-yl]-1-methylpiperaziniumjodid

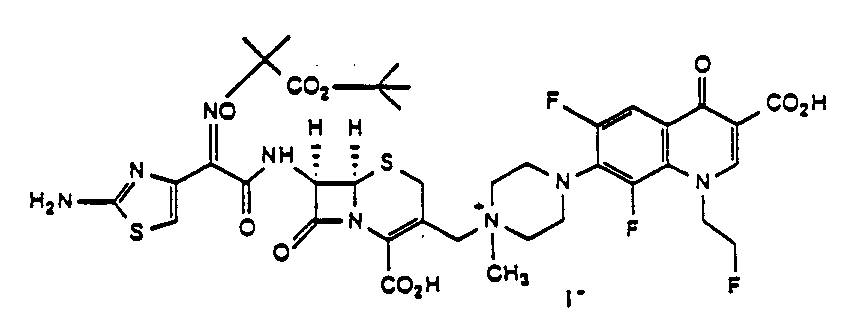

In einer Argonatmosphäre wird ein Gemisch aus 272 mg (1 mMol) 7-Aminocephalosporansäure, 0,67 ml (3,6 mMol) MSTFA und 3 ml trockenem Acetonitril 30 Minuten gerührt; nach Zugabe von 0,25 ml (1,75 mMol) Jodtrimethylsilan wird das Ganze weitere 30 Minuten gerührt. Das Gemisch wird abgekühlt und mit 0,14 ml (1,75 mMol) wasserfreiem THF versetzt. Nach 10 Minuten wird eine Lösung aus 277 mg (0,75 mMol) 6,8-Difluor-1-(2-fluoräthyl)-1,4-dihydro-7-(4-methyl-1-piperazinyl)-4-oxo-3-chinolincarbonsäure, 0,17 ml (0,9 mMol) MSTFA und 3 ml trockenem Acetonitril hinzugefügt. Das Ganze wird weitere 2,5 Stunden bei Zimmertemperatur gerührt, mit 478 mg (1 mMol) 2-[[[1-(2-Amino-4-thiazolyl)-2-[(2-benzothiazolyl)thio]-2-oxoäthyliden]amino]oxy]methylpropansäure- 1,1-Dimethyläthylester und 4 ml trockenem Acetonitril versetzt und das Gemisch 12 Stunden gerührt. Eine geringe Menge unlöslichen Materials wird durch Filtrieren entfernt und das Filtrat unter vermindertem Druck zur Trockene eingedampft. Das zurückbleibende Oel wird in 4 ml Acetonitril gelöst und nach Kühlung mit Eis mit 0,16 ml Methanol versetzt. Das Ganze wird 1 Minute gerührt, 2 Minuten stehen gelassen und der erhaltene Niederschlag abfiltriert. Dieser wird dreimal mit je 3 ml Acetonitril gewaschen und unter vermindertem Druck eingedampft. Man erhält so 530 ml Titelverbindung: NMR (Me₂SO-d6), δ 1,36 (s, 12H, t-Bu und CH₃), 1,40 (s, 3H, CH₃), 3,12 (s, 3H, NCH₃), 3,40-3,86 (m, 9H, 4 x NCH₂ und CH von SCH₂), 3,96 (d, 1H, J gem = 16Hz, CH von SCH₂), 4,40-4,66 (AB, 2H, J gem = 13Hz, NCH₂), 4,62-5,06 (m, 4H, NCH₂CH₂F), 5,26 (d, 1H, J = 5Hz, CH), 5,93 (dd, 1H, J = 5 und 7Hz, CH), 6,68 (s, 1H, Ar), 7,25 (s, 2H, NH₂), 7,92 (d, 1H, J = 12Hz, Ar), 8,88 (s, 1H, =CH-), 9,44 (d, 1H, J = 7Hz, NH).

### Beispiel 9

### Wahlweise Synthese von [6R-[6α,7β(Z)]]-1-[[7-[[[(2-Amino-4-thiazolyl)[1-(1-carboxy-1-methyl)äthoxy]imino]acetyl] amino]-8-oxo-5-thia-1-azabicyclo[4.2.0]oct-2-en-3-yl]methyl] -4-[3-carboxy-1-(2-fluoräthyl)-6,8-difluor-1,4-dihydro-4 -oxochinolin-7-yl]-1-methylpiperaziniumhydroxid-inneres Salz-Mononatriumsalz

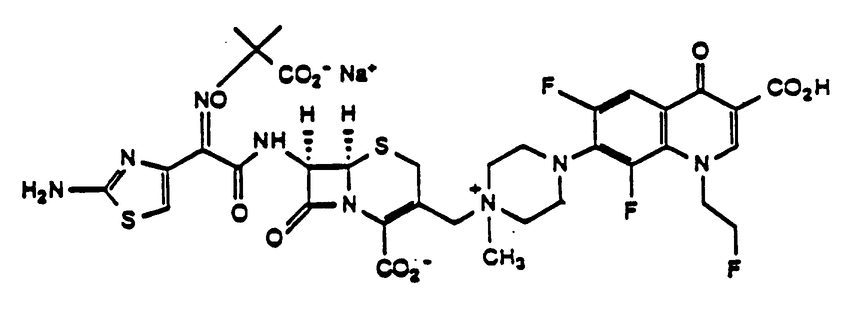

Eine Lösung von 102 mg der gemäss Beispiel 8 hergestellten Verbindung in 0.4 ml Anisol, 1,5 ml Methylenchlorid und 1,5 ml Trifluoressigsäure wird 12 Stunden bei 0°C stehen gelassen. Nach Filtrieren, Eindampfen zur Trockene unter vermindertem Druck, Zugabe von Methylenchlorid und wiederum Eindampfen zur Trockene wird der Rückstand mit Aether verrieben, wobei ein Feststoff erhalten wird. Dieser wird in wässrigen Natriumphosphatpuffer pH 7 gelöst und durch reverse-phase-Hochdruckflüssigchromatographie gereinigt; man erhält die Titelverbindung mit einem NMR-Spektrum wie für die gleiche Verbindung in Beispiel 2 angegeben.

### Beispiel 10

### Herstellung von (6R-trans)-3-Hydroxymethyl-7-[[(1,1-dimethyläthoxy)carbonyl]amino]-8-oxo-5-thia-1-azabicyclo [4,2,0]oct-2-en-2-carbonsäure

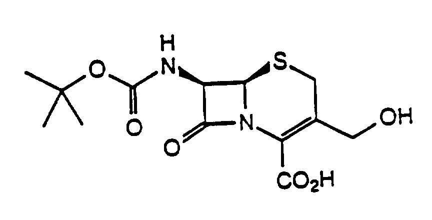

Eine Lösung von 19,2 g (0,48 Mol) Natriumhydroxid in 240 ml Wasser wird unter Rühren auf -5°C in einem Trockeneis/Acetonbad abgekühlt und auf einmal mit 54,4 g (0,20 Mol) 7-Aminocephalosporansäure versetzt. Durch Heben oder Senken des Kühlbads wird die Reaktionstemperatur auf -5 bis 0°C kontrolliert, bis die anfängliche Reaktionshitze verschwunden ist. Das Kühlbad wird anschliessend durch ein Eisbad ersetzt und das Gemisch bei 0-5°C während der restlichen Zeit von insgesamt 30 Minuten gerührt. Der pH-Wert wird durch Zugabe von etwa 2 ml 6N wässriger Chlorwasserstoffsäure auf 9-9,5 eingestellt. Anschliessend werden 700 ml Dioxan, gefolgt von einer Lösung von 87,5 g (0.40 Mol) di-t-Butyldicarbonat in 200 ml Dioxan zugefügt. Nach Zugabe von 33,6 g (0.40 Mol) Natriumbicarbonat wird das Gemisch 70 Stunden gerührt. 750 ml Aethylacetat werden hinzugefügt, die Phasen werden getrennt und die organische Phase zweimal mit je 125 ml Wasser extrahiert. Die vereinigten wässrigen Lösungen werden zweimal mit je 300 ml Aethylacetat gewaschen. Durch Aequilibrieren der wässrigen Lösung mit 750 ml Aethylacetat und 80 ml 6N wässriger Salzsäure, wobei der pH-Wert auf 2,5 absinkt, bildet sich ein Niederschlag, der durch Filtrieren durch Hyflo entfernt wird. Die wässrige Phase wird abgetrennt und mit 300 ml Aethylacetat extrahiert. Die organischen Lösungen werden vereinigt, mit 300 ml Wasser gewaschen, über Natriumsulfat getrocknet und unter vermindertem Druck auf etwa 100 ml konzentriert. Mit abnehmendem Volumen fällt allmählich ein Niederschlag aus. Dieser wird filtriert und mit Aether gewaschen. Das Produkt wird unter vermindertem Druck getrocknet, und man erhält 24,1 g (36%) der Titelverbindung. Dieser wird in Beispiel 11 direkt verwendet.

### Beispiel 11

### Herstellung von (6R-trans)-3-Hydroxymethyl-7-[[(1,1-dimethyläthoxy)carbonyl]amino]-8-oxo-5-thia-1-azabicyclo[4,2,0]oct-2-en-carbonsäure-diphenylmethylester

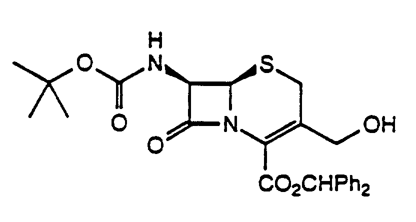

Eine Aufschlämmung von 44,67 g (0.135 Mol) der Hydroxysäure aus Beispiel 10 in 270 ml trockenem Tetrahydrofuran (THF) wird unter Rühren mit 32,51 g (0,167 Mol) Diphenyldiazomethan in 390 ml n-Hexan versetzt und das Gemisch anschliessend 20 Stunden heftig gerührt. Der entstandene Niederschlag wird filtriert. mit n-Hexan gewaschen und unter vermindertem Druck getrocknet. Man erhält 45,74 g (68,2%) Titelverbindung: ¹H NMR (Me₂SO-d₆) δ1,41 (s, 9H), 3,59 (s, 2H), 4,22 (t, J=6Hz, 2H), 5,08 (d, J=5Hz), 5,51 (dd, J=5 and 8 Hz, 1H), 6,88 (s, 1H), 7,26-7,51 (m, 10H), 8,03 (d, J=8Hz, 1H).

### Beispiel 12

### Herstellung von 1-Aethyl-6-fluor-1,4-dihydro-7-[4-[(1,1-dimethyläthoxy)carbonyl]-1-piperazinyl-4-oxo-3-chinolin-carbonsäure

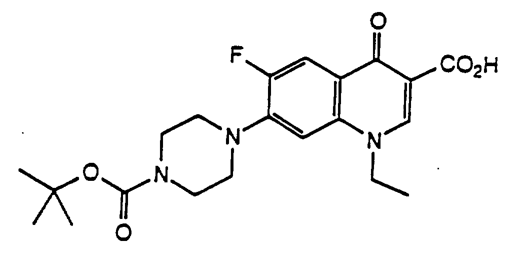

Eine Aufschlämmung von 24,0 g (0,075 Mol) 1-Aethyl-6-fluor-1,4-dihydro-4-oxo-7-(1-piperazinyl)-3-chinolon carbonsäure in 240 ml Dioxan werden unter Rühren mit 130 ml Wasser und 80 ml (0,080 Mol) 1N wässriger Natriumhydroxidlösung versetzt. Das Gemisch wird etwa 30 Minuten auf 100°C erhitzt bis eine klare Lösung entsteht. Die Lösung wird auf 0°C gekühlt und mit einer Lösung von 19,2 g (0,088 Mol) di-t-Butyldicarbonat in 50 ml Dioxan versetzt. Das Gemisch wird bei 0°C 30 Minuten und anschliessend bei Zimmertemperatur weitere 3 Stunden gerührt. Der entstandene Niederschlag wird abfiltriert und in 520 ml 10%ige wässrige Essigsäure aufgeschlämmt. Das Gemisch wird kurz auf 100°C erhitzt und anschliessend abgekühlt und filtriert. Das abfiltrierte Produkt wird unter vermindertem Druck über Phosphorpentoxid getrocknet, wonach man 27,0 g (86%) der Titelverbindung erhält: ¹H NMR (Me₂SO) δ1,39 (t,3H, J=7Hz), 1,40 (S, 9H), 3,25 (m, 4H). 3,48 (m, 4H), 4,54 (q,2H, J=7Hz), 7,08 (d, 1H, J_{HF}=7,5Hz), 7,91 (d, 1H, J_{HF}=14Hz), 8,94 (s,1H).

### Beispiel 13

### Herstellung von 1-Aethyl-6-fluor-1,4-dihydro-7-[4-[(1,1-dimethyläthoxy)carbony]-1-piperazinyl]-4-oxo-3-chinolin carbonsäure (4-nitrophenyl)methylester

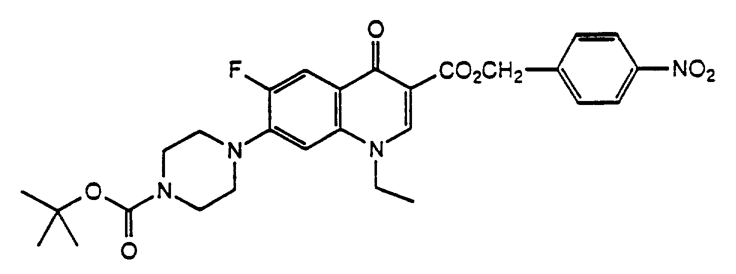

Eine Aufschlämmung von 1,0 g (2,4 mMol) des gemäss Beispiel 12 erhaltenen Produkts in 40 ml Wasser wird mit 2,5 ml (2,5 mMol) 1N wässriger Natriumhydroxidlösung versetzt. Das Gemisch wird bis zur vollständigen Lösung erwärmt. Die Lösung wird filtriert und gefriergetrocknet. Das erhaltene Natriumsalz wird in 10 ml Dimethylformamid (DMF) gelöst, 2 Stunden mit 4Å Molekularsieb gerührt und anschliessend mit 0,55 g (2,5 mMol) 4-Nitrobenzylbromid versetzt. Das Gemisch wird 3 Tage gerührt. Das Molekularsieb wird durch Filtration und das Lösungsmittel unter vermindertem Druck entfernt. Dann wird durch Flash-Chromatographie gereinigt (Kieselgel, Methylenchlorid:Aethylacetat 1:2 als Eluens), wobei 1,0 g (77%) Titelverbindung als weisser Feststoff erhalten wird: ¹H NMR(CDCl₃), δ1,46 (s, 9H), 1,52 (t, 3H, J=7,5 Hz), 1,57 (s, 3H), 3,17 (m, 4H), 3,62 (m, 4H), 4,18 (q, 2H, J=7,5 Hz), 5,46 (s, 2H), 6,74 (d, 1H, J_{HF}=7Hz), 7,71 (d, 2H, J=8,5Hz), 8.12 (d, 1H, J_{HF}=13Hz), 8.23 (d, 2H, J=8.5Hz), 8.45 (s,1H).

### Beispiel 14

### Herstellung von 1-Aethyl-6-fluor-1,4-dihydro-oxo-7-(1-piperazinyl)-3-chinoloncarbonsäure-(4-nitrophenyl)methylester

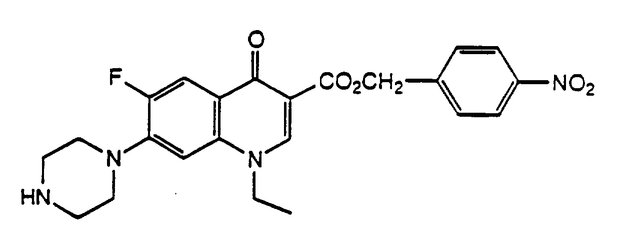

Ein Gemisch von 10 g (0,018 Mol) des gemäss Beispiel 13 hergestellten Esters, 40 ml Anisol und 40 ml Trifluoressigsäure werden bei 0°C 3 Stunden gerührt. Das Gemisch wird unter vermindertem Druck eingedampft und der Rückstand zweimal mit je 100 ml Aether verrieben. Der Aether-Extrakt wird zweimal mit je 100 ml Wasser gewaschen und die wässrigen Flüssigkeiten mit dem Rückstand nach dem obigen Verreiben mit Aether vereinigt. Nach Zugabe von wässriger 1N Natriumhydroxidlösung bis auf pH 11 wird das Produkt siebenmal mit je 100 ml Methylenchlorid extrahiert. Die vereinigten organischen Auszüge werden über Natriumsulfat getrocknet und eingedampft. Man erhält 7,70 g (95%) Titelverbindung: ¹H NMH (CDCl₃) δ1,57 (t, 3H, J=7,5 Hz), 3,11 (m,4H) 3,24 (m,4H), 4,23 (q, 2H, J=7,5 Hz), 5,51 (s, 2H), 6,78 (d, 1H, J_{HF}=7Hz), 7,75 (d, 2H, J=8,5 Hz), 8,15 (d, 1H, H_{HF}=14Hz, 8,26 (d, 2H, J=8,5 Hz),8,49 (s, 1H).

### Beispiel 15

### Herstellung von (6R,trans)-3-[[[[4-[1-Aethyl-6-fluor-1,4-dihydro-3-[[(4-nitrophenyl)methoxy]carbonyl]-4-oxo-7-chinolinyl]-1-piperazinyl]carbonyl]oxy]methyl]-7-[[(1,1-dimethyläthoxy)carbonyl]amino]-8-oxo-5-thia-1-azabicyclo[4.2.0]oct-2-en-2-carbonsäure-diphenylmethylester

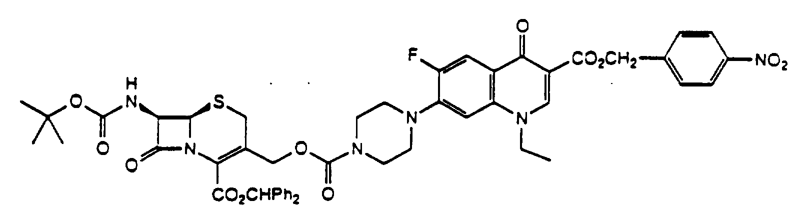

Eine Lösung von 2,00 ml (3,84 mMol) 20%igem Phosgen in Toluol und 68 ml trockenem Methylenchlorid wird in einer Argonatmosphäre auf 3-4°C abgekühlt und mit einer Lösung von 1,71 g (3,44 mMol) (6R-trans)-3-Hydroxymethyl-7-[[(1,1-dimethyläthyoxy)carbonyl]amino]-8-oxo-5-thia-1-azabicyclo[4.2.0]-oct-2-en-2-carbonsäure-diphenylmethylester (Beispiel 11) in 30 ml Methylenchlorid sowie mit 0,66 ml (3,85 mMol) N,N-Diisopropyläthylamin und 5 ml Methylenchlorid versetzt. Das Gemisch wird 15 Minuten kalt und anschliessend 2 Stunden bei Zimmertemperatur gerührt. Die erhaltene Lösung wird zu einer Lösung aus 1,88 g (4,14 mMol) 1-Aethy1-6-fluor-1,4-dihydro-4-oxo-7-(1-piperazinyl)-3-chinolincarbonsäure-(4-nitrophenyl)methylester (Beispiel 14), 0,71 ml (4,15 mMol) N,N-Diisopropyläthylamin und 68 ml Methylenchlorid gegeben. Nach 2 1/2- bis 3-stündigem Rühren in einer Argonatmosphäre wird die Lösung unter vermindertem Druck bis zu etwa 20 ml eingedampft und zu einer 5,0 x 14 cm Kieselgel-Kolonne zwecks druckchromatographischer Reinigung gegeben. Die Kolonne wird zuerst mit 20 ml Methylenchlorid eluiert und anschliessend mit Aethylacetat, wobei Fraktionen zu 20-25 ml aufgefangen werden. Die Fraktionen 26-30 enthalten das reine Produkt und werden vereinigt und unter vermindertem Druck zur Trockene eingedampft, wobei 0.716 g Rückstand erhalten wird. Die Fraktionen 31-55 werden nochmals chromatographiert und das zusätzlich erhaltene, reine Produkt mit dem obigen Rückstand vereinigt, wobei 1.12 g (33,5%) Titelverbindung erhalten werden. IR (KBr) 1789, 1712, 1622 cm⁻¹.

Elementaranalyse: Berechnet für C₅₀H₄₉N₆O₁₂FS: C, 61,47; H,5,06: N,8,60; S, 3,28; F, 1,94. Gefunden: C, 61,23; H, 5,03; N, 8,50; S, 3,35; F, 1,65.

### Beispiel 16

### Herstellung von (6R,trans)-3-[[[4-(3-Carboxy-1-äthyl-6-fluor-1,4-dihydro-4-oxo-7-chinolinyl]-1-piperazinyl]carbonyl] oxy]methyl]-7-[[(1,1-dimethyläthoxy)carbonyl]amino]-8-oxo-5 -thia-1-azabicyclo[4.2.0]oct-2-en-2-carbonsäure-diphenylmethyl ester

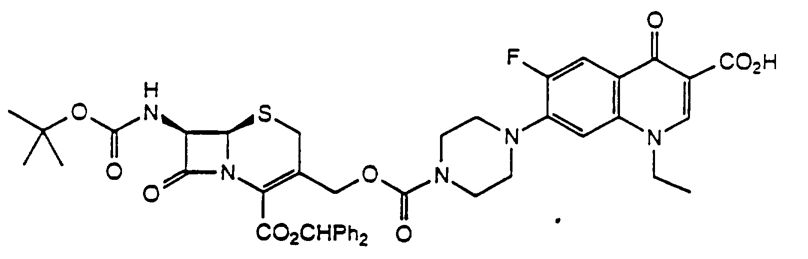

Ein Gemisch von 1,125 g (1,15 mMol) des gemäss Beispiel 15 erhaltenen p-Nitrobenzylesters, 1,54 g 10%igem Pd/C-Katalysator und 115 ml trockenem THF (destilliert aus Natrium-benzophenonketyl) wird in einer Wasserstoffatmosphäre bei atmosphärischem Druck etwa 3 Stunden gerührt. Der Katalysator wird abfiltriert und das Lösungsmittel unter vermindertem Druck eingedampft. Der Rückstand wird chromatographisch an einer 2,2 x 11 cm Kieselgelkolonne gereinigt mit Aethylacetat, gefolgt von Aethylacetat-Aceton-Methanol-Wasser (70:5:2,5:2.5) als Eluens. Die entsprechenden Fraktionen werden vereinigt und unter vermindertem Druck zur Trockene eingedampft, wobei 0,817 g (86,3%) Titelverbindung erhalten werden: IR(KBr) 1785, 1715, 1625, 698 cm⁻¹.

### Beispiel 17

### Herstellung von (6R,trans)-7-Amino-3-[[[[4-(3-carboxy-1-äthyl-6-fluor-1,4-dihydro-4-oxo-7-chinolinyl)-1-piperazinyl] carbonyl]oxy]methyl]-8-oxo-5-thia-1-azabicyclo[4.2.0]oct-2 -en-2-carbonsäure-trifluoracetat

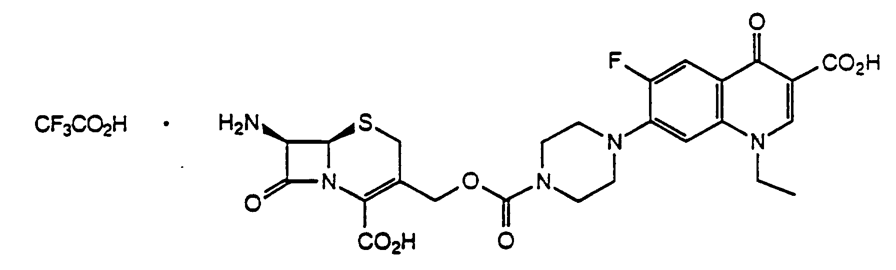

Einer Lösung von 0,811 g (0,963 mMol) des gemäss Beispiel 16 hergestellten Diphenylmethylesters und 1,80 ml Anisol in 15 ml trockenem Methylenchlorid wird in einer Argonatmosphäre auf 0°C abgekühlt; nach Zugabe von 11,8 ml kalter Trifluoressigsäure wird das Gemisch bei 0°C 2 Stunden gerührt. Das Gemisch wird anschliessend unter vermindertem Druck bei 0-5°C eingedampft und sodann mit 5 ml Methylenchlorid, 20 ml Aethylacetat und 60 ml Aether versetzt. Das Gemisch wird 5-10 Minuten gerührt; der erhaltene Niederschlag wird abfiltriert und mit Aether gewaschen. Nach dem Trocknen unter vermindertem Druck erhält man 0,591 g (89,0%) Titelverbindung.

### Beispiel 18

### Herstellung von [6R[6α,7β(z)]]-7-[[(2-Amino-4-thiazolyl) (methoxyimino)acetyl]amino]-3-[[[[4-(3-carboxyl-1-äthyl-6-fluor-1,4-dihydro-4-oxo-7-chinolinyl]-1-piperazinyl]carbonyl] -oxy]methyl]-8-oxo-5-thia-1-azabicyclo[4.2.0]oct-2-en-2 -carbonsäure-Dinatriumsalz

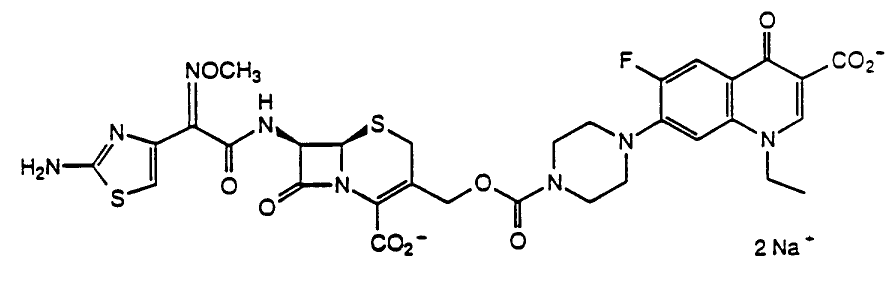

Ein Gemisch von 59,4 mg (0,086 mMol) des nach Beispiel 17 hergestellten Trifluoressigsäuresalzes und 1,5 ml THF wird unter Rühren auf 0-5°C gekühlt. Anschliessend wird eine Lösung von 26,0 mg (0,309 mMol) Natriumbicarbonat in 2 ml Wasser hinzugefügt, gefolgt von einer Lösung von 38,0 mg (0,108 mMol) (Z)-2-Amino-α-(methoxyimino)-4-thiazoläthanthionsäure-S-2-benzothiazolylester in 0,75 ml THF. Nach 25 Minuten wird das Kühlbad entfernt und das Reaktionsgemisch 16,5 Stunden gerührt. Das Gemisch wird nun zur Entfernung von THF unter vermindertem Druck eingedampft. Die zurückbleibende wässrige Lösung wird mit Aethylacetat gewaschen und anschliessend durch Zugabe von 0,025M wässriger Natriumdihydrogenphosphatlösung auf pH 7,55 eingestellt. Die Lösung wird an 1,75 g einer C₁₈-Kieselgelkolonne (Waters) chromatographiert. Das Produkt wird eluiert mit Hilfe eines stufenweise Gradienten von 0,025M Natriumphosphatpuffer- -Acetonitril mit 0-30% Acetonitril. Die entsprechenden Fraktionen werden vereinigt und unter vermindertem Druck zur Entfernung von Acetonitril teilweise eingedampft. Ansäuerung mit IN wässriger Salzsäure bis auf pH 2 liefert einen Niederschlag, der zweimal zentrifugiert und mit Wasser gewaschen wird. Der Niederschlag wird in Wasser durch Zugabe von Natriumbicarbonat bis auf pH 7.5 gelöst. Nach Zugabe von Aceton fällt das Natriumsalz aus. Das Produkt wird isoliert und durch Zentrifugierung mit Aceton gewaschen. Nach dem Trocknen unter vermindertem Druck erhält man 36,2 mg Titelverbindung: IR(KBr) 3420, 1762, 1675, 1622 cm⁻¹; Massenspektrum (FAB) m/z 803 (M+H)⁺

### Beispiel 19

### Herstellung von 1-Cyclopropyl-6-fluor-1,4-dihydro-4-oxo-7-(1-piperazinyl)-3-chinolincarbonsäure-(4-nitrophenyl)methyl ester

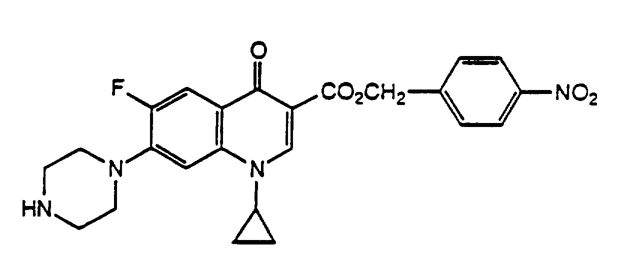

Diese Verbindung wird hergestellt aus 1-Cyclopropyl-6-fluor-1,4-dihydro-4-oxo-7-(1-piperazinyl)-3-chinolincarbonsäure in Analogie zu den Beispielen 12, 13 und 14 für die Herstellung von 1-Aethyl-6-fluor-1,4-dihydro-4-oxo-7-(1-piperazinyl-3-chinolincarbonsäure-(4-nitrophenyl)methylester.

### Beispiel 20

### Herstellung von (6R,trans)-[[7-(1,1-Dimethyläthoxy)carbonyl] amino]-3-[[[[4-[1-cyclopropyl-6-fluor-1,4-dihydro-3-[[(4-nitrophenyl)methoxy]carbonyl]-4-oxo-7-chinolinyl]-1 piperazinyl]carbonyl]oxy]methyl]-8-oxo-5-thia-1-azabicyclo [4.2.0]oct-2-en-2-carbonsäure-diphenylmethylester

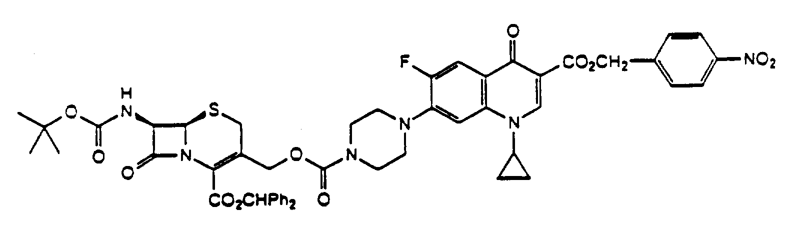

In einer Argonatmosphäre wird eine Lösung von 0,58 ml (1,12 mMol) 20%igem Phosgen in Toluol und 20 ml Methylenchlorid auf 0-5°C gekühlt und mit einer Lösung von 0,497 g (1 mMol) (6R-trans)-3-Hydroxymethyl-7-[[(1,1-dimethyläthoxy)carbonyl]amino]-8-oxo-5-thia-1-azabicyclo[4.2.0]oct-2-en-2-carbonsäure-diphenylmethylester in 8 ml Methylenchlorid und mit 0,195 ml (1,14 mMol) N,N-Diisopropyläthylamin versetzt. Das Gemisch wird 15 Minuten bei 0-5°C gerührt. Anschliessend wird das Eisbad entfernt und das Gemisch 1 Stunde und 40 Minuten bei Zimmertemperatur gerührt. Die erhaltene Lösung wird zu einer Lösung von 0,550 g (1,18 mMol) 1-Cyclopropyl-6-fluor-1,4-dihydro-4-oxo-7-(1-piperazinyl)-3-chinolincarbonsäure-(4-nitrophenyl)methylester und 0,205 ml (1,20 mMol) N,N-Diisopropyläthylamin in 17 ml Methylenchlorid gegeben. Das Gemisch wird bei Zimmertemperatur in einer Argonatmosphäre 3 Stunden und 5 Minuten gerührt, anschliessend durch Eindampfen konzentriert und durch Flash-Chromatographie an 29 g Kieselgel in einer Kolonne von einem Durchmesser von etwa 2,2 cm gereinigt. Das Gemisch wird als Lösung in Methylenchlorid der Kolonne zugeführt und mit Aethylacetat, n-Hexan und anschliessend mit Aethylacetat eluiert. Die entsprechenden Fraktionen werden vereinigt und unter vermindertem Druck zur Trockene eingedampft. Man erhält 0,425 g (42,9%) Titelverbindung: IR (KBr) 1789, 1720, 1622, 1520, 1345, 702 cm⁻¹; Massenspektrum (FAB) m/z 989 (M+H)⁺.

### Beispiel 21

### Herstellung von (6R-trans)-3-[[[[4-(3-Carboxy-1-cyclopropyl-6-fluor-1,4-dihydro-4-oxo-7-chinolinyl)-1-piperazinyl]carbon yl]oxy]methyl]-7-[[(1,1-dimethyläthoxy)carbonyl]amino]-8-oxo -5-thia-1-azabicyclo[4.2.0]oct-2-en-2-carbonsäure-diphenyl methylester

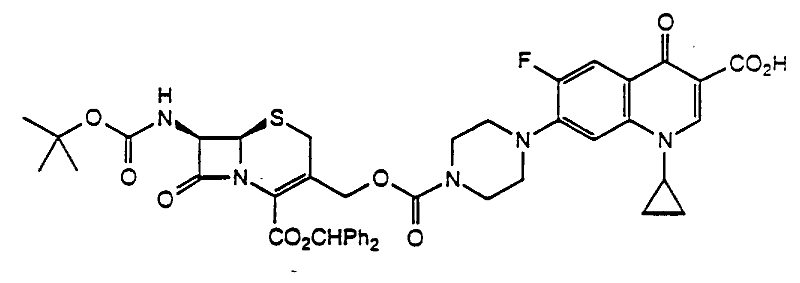

Ein Gemisch von 0,414 g (0,418 mMol) des gemäss Beispiel 20 hergestellten p-Nitrobenzylesters, 0,602 g 10%igem Pd/C-Katalysator und 40 ml THF (destilliert aus Nariumbenzophenonketyl) wird bei atmosphärischem Druck etwa 3,5 Stunden hydriert. Der Katalysator wird abfiltriert und die Lösung unter vermindertem Druck eingedampft. Der Rückstand wird mit Hilfe eines präparativen zentrifugal-beschleunigten radialen Dünnschichtchromatogrammapparats (Chromatron Model 7924) chromatographisch gereinigt, wobei mit Aethylacetat, gefolgt von Aethylacetat-Aceton-Wasser (70:10:5:5) eluiert wird. Man erhält 251,6 mg (70,4%) Titelverbindung: IR (KBr) 1788, 1720, 1628, 702 cm⁻¹: Massenspektrum (FAB) m/z 854 (M + H)⁺.

### Wahlweise Synthese von (6R-trans)-3-[[[[4-(3-Carboxy-1-cyclopropyl-6-fluor-1,4-dihydro-4-oxo-7-chinolinyl)-1 -piperazinyl]carbonyl]oxy]methyl]-7-[[(1,1-dimethyläthoxy) carbonyl]amino]-8-oxo-5-thia-1-azabicyclo[4.2.0]oct-2-en-2 -carbonsäure-diphenylmethylester

In einer Argonatmosphäre werden 124 mg (0,25 mMol) (6R-trans)-3-Hydroxymethyl-7-[[(1,1-dimethyläthoxy)carbonyl]amino]-8-oxo-5-thia-1-azabicyclo[4.2.0]oct-2-en-2-carbonsäurediphenylmethylester in 3 ml Methylenchlorid gelöst und 45 Minuten mit 4A Molekularsieb gerührt. Die Lösung wird anschliessend in Eis gekühlt und zusammen mit 36 mg (0,28 mmol) N,N-Diisopropyläthylamin zu einer kalten Lösung von 0,14 ml 20%igem Phosgen in Toluol in 1,5 ml Methylenchlorid gegebenen. Die Lösung wird 30 Minuten bei 0-5°C und anschliessend 45 Minuten bei Zimmertemperatur gerührt, anschliessend wieder in Eis gekühlt und bei 0-5°C zu einer eiskalten Lösung, vorgängig hergestellt bei Zimmertemperatur durch 30-minütigem Rühren von 83 mg (0,25 mMol) 1-Cyclopropyl-6-fluor-1,4-dihydro-4-oxo-7-(1-piperazinyl)-3-chinolincarbonsäure (getrocknet unter vermindertem Druck bei 100°C) und 125 mg (0,62 mMol) N-Methyl-N-(trimethylsilyl)trifluoracetamid (MSTFA) mit 3 ml Methylenchlorid, gegeben. Das erhaltene Reaktionsgemisch wird 30 Minuten bei 0-5°C und 1 Stunde bei Zimmertemperatur gerührt und anschliessend unter vermindertem Druck eingedampft. Der Rückstand wird chromatographisch gereinigt, und man erhält 63 mg (30%) Titelverbindung mit der gleichen IR- und Massenspektra wie die oben erhaltene Verbindung.

### Beispiel 22

### Herstellung von (6R-trans)-7-Amino-3-[[[[4-(3-carboxy-1-cyclopropyl-6-fluor-1,4-dihydro-4-oxo-7-chinolinyl)-1 -piperazinyl]carbonyl]oxy]methyl]-8-oxo-5-thia-1-azabicyclo [4.2.0]oct-2-en-2-carbonsäuretrifluoracetat

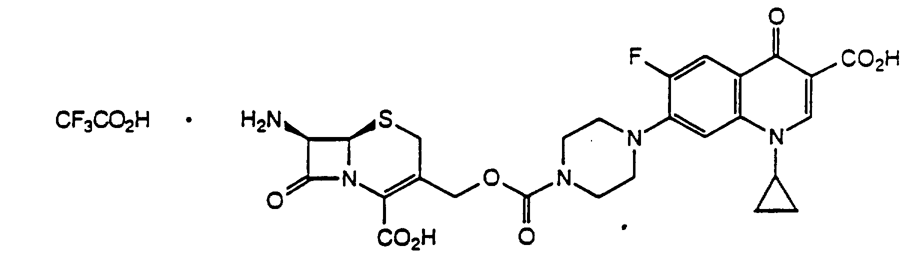

Eine Lösung von 0,251 g (0,294 mMol) des gemäss Beispiel 21 hergestellten Diphenylmethylesters und 0,55 ml Anisol in 4,5 ml trockenem Methylenchlorid wird auf 0°C gekühlt. Nach Zugabe von 3,6 ml kalter Trifluoressigsäure wird das Gemisch bei etwa 0°C in einer Argonatmosphäre 1 Stunde und 45 Minuten gerührt. Das Gemisch wird unter vermindertem Druck zur Trockene eingedampft. Der Rückstand wird mit 1,5 ml Methylenchlorid und anschliessend mit 6 ml Aethylacetat behandelt. Der erhaltene gummiartige Niederschlag erstarrt allmählich. Nach Zugabe von 23 ml Aether wird das Gemisch 10-15 Minuten kalt gerührt und anschliessend filtriert. Das erhaltene feste Produkt wird mit Aether gewaschen und unter vermindertem Druck getrocknet; man erhält 186,2 mg Titelverbindung und zusätzlich 9,0 mg an der Wand des Reaktionsgefässes haftendes Produkt (insgesamt 93,3%).

### Beispiel 23

### Herstellung von [6R-[6α,7β(Z)]]-7-[[(2-Amino-4-thiazolyl)(methoxyimino)acetyl]amino]-3-[[[[4-(3-carboxy-1-cyclopropyl -6-fluor-1,4-dihydro-4-oxo-7-chinolinyl)-1-piperazinyl] carbonyl]oxy]methyl]-8-oxo-5-thia-1-azabicyclo[4.2.0]oct-2 -en-2-carbonsäure-Dinatriumsalz

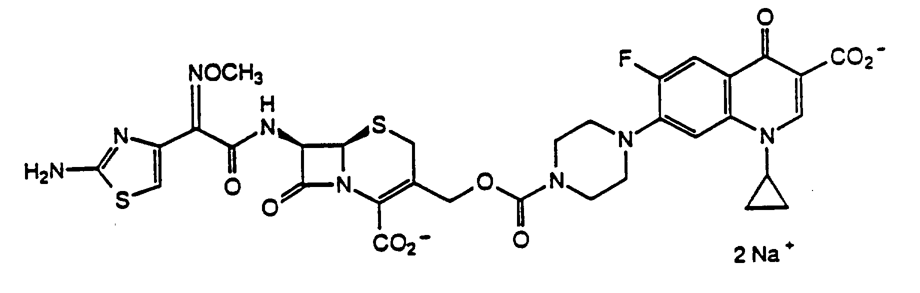

183,7 ml (0,262 mMol) des gemäss Beispiel 22 hergestellten Trifluoracetats werden in 4,5 ml THF aufgeschlämmt und auf 0°C gekühlt. Nach Zugabe einer Lösung von 79,9 mg (0,95 mMol) Natriumbicarbonat in 4,5 ml Wasser wird mit 1,5 ml Wasser gewaschen. Das Gemisch wird 18 Minuten gerührt. Nach Zugabe von 93,9 mg (0,268 mMol) (Z)-2-Amino-α-(methoxyimino)-4-thiazoläthanthionsäure-S-2-benzothiazolylester in 1,5 ml THF sowie 0,7 ml THF wird nach 15 Minuten das Kühlbad entfernt und das Reaktionsgemisch 17 Stunden bei Zimmertemperatur gerührt. Das Gemisch wird unter vermindertem Druck eingedampft und die zurückbleibende wässrige Lösung mit Aethylacetat extrahiert. Der Aethylacetatextrakt wird zweimal mit Wasser zurückgewaschen. Die wässrigen Phasen werden vereinigt (pH 8,70) und mit 0,025M wässriger Natriumdihydrogenphosphatlösung bis auf pH 7,80 versetzt. Die Lösung wird unter vermindertem Druck (zur Entfernung von Spuren von Aethylacetat) eingedampft und anschliessend an einer 9 g C₁₈-Kieselgel (Waters) enthaltenden Kolonne chromatographiert. Die Kolonne wird mit pH 7,8 wässriger Natriumphosphatpuffer gewaschen und anschliessend mit einem stufenweise Gradienten von Wasser-Acetonitril (0-30% Acetonitril) unter Druck eluiert. Die entsprechenden Fraktionen werden vereinigt, eingedampft und gefriergetrocknet; man erhält 12706 mg (59,7%) Titelverbindung. Davon werden 52 mg in 0,5 ml Wasser gelöst und der pH-Wert durch Zugabe von Natriumbicarbonat auf 8,0 eingestellt. Zum Ausfällen des Produkts werden 24 ml kaltes Aceton zugegeben. Nach Zentrifugierung wird die überschüssige Flüssigkeit entfernt und der zurückbleibende Feststoff mit kaltem Aceton verrieben, filtriert und unter vermindertem Druck über Phosphorpentoxid getrocknet. Man erhält 38 mg Titelverbindung: IR (KBr) 3410, 2540, 1762, 1672, 1622; Massenspektrum (FAB) 837 (M + Na)⁺, 815 (M + H)⁺.

### Beispiel 24

### Herstellung von (6R-trans)-3[[[[4-(3-Carboxy-1-cyclopropyl-6-fluor-1,4-dihydro-4-oxo-7-chinolinyl)-1-piperazinyl] carbonyl]oxy]methyl]-8-oxo-7-[(phenoxyacetyl)amino] -5-thia-1-azabicyclo[4.2.0]oct-2-en-2-carbonsäure

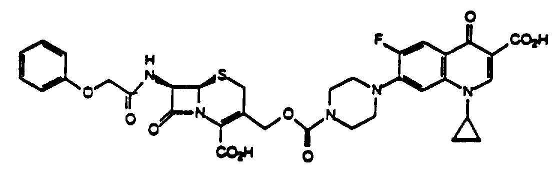

Eine Lösung von 184 mg (0,262 mMol) des gemäss Beispiel 22 hergestellten Trifluoracetats in 5 ml THF wird bei 0-5°C gerührt und mit einer Lösung von 101 mg (1,2 mMol) Natriumbicarbonat in 4,5 ml Wasser und anschliessend mit einer kalten Lösung von 54 mg (0,314 mMol) Phenoxyacetylchlorid in 1,5 ml THF tropfenweise versetzt. Das Ganze wird bei 0-5°C 20 Minuten gerührt und anschliessend 3 Stunden bei Zimmertemperatur. Das Gemisch wird unter vermindertem Druck (zur Entfernung von THF) eingedampft. Der wässrige Rückstand wird mit Wasser verdünnt, mit Aethylacetat gewaschen, in Eis gekühlt und, zur Ausfällung des Produkts, auf pH 2,5 angesäuert. Der erhaltene Niederschlag wird abfiltriert und mit Wasser und Aethylacetat gewaschen; man erhält 130 mg Titelverbindung. Diese wird durch C₁₈-reverse-phase Hochdruckflüssigchromatographie an einer Kieselgelkolonne gereinigt unter Verwendung eines pH 7,5 Natriumphosphatpuffer-Acetonitrilgradienten. Nach dem Abdampfen und Gefriertrocknen der entsprechenden Fraktionen wird der Rückstand in Wasser gelöst. Die Lösung wird filtriert und anschliessend zur Ausfällung des Produkts auf pH 2,5 angesäuert. Massenspektrum (FAB) m/z 722 (M + H)⁺.

### Beispiel 25

### Herstellung von [6R-[6α,7β(Z)]]-7-[[(2-Amino-4-thiazolyl)-[(1-carboxy-1-methyl-äthoxy)imino]acetyl]amino]-3-[[[4-(3 -carboxy-1-cyclopropyl-6-fluor-1,4-dihydro-4-oxo-7 -chinolinyl)-1-piperazinyl]carbonyl]-oxy]methyl]-8-oxo-5-thia -1-azabicyclo[4.2.0]oct-2-en-2-carbonsäure

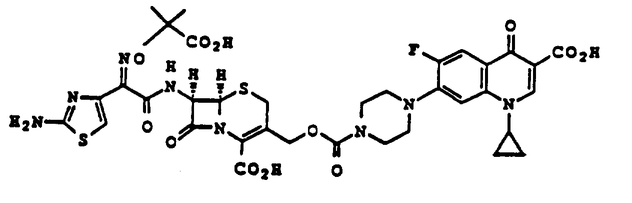

Eine Aufschlämmung von 552 mg (0,786 mMol) des gemäss Beispiel 22 hergestellten Trifluoracetats in 14 ml THF wird in Eis gekühlt und mit einer Lösung von 240 mg (2,85 mMol) Natriumbicarbonat in 15 ml Wasser versetzt. Das Gemisch wird 20 Minuten gerührt und anschliessend mit einer Lösung von 384 mg (0,804 mMol) 2-[[[1-(2-Amino-4-thiazolyl)-2-[(2-benzothiazolyl)thio]-2-oxoäthyliden]amino]oxy]methyl-propansäure-1,1-dimethyläthylester in 6 ml THF versetzt. Das Gemisch wird 15 Minuten unter Eiskühlung und anschliessend etwa 12 Stunden bei Zimmertemperatur gerührt. Zur Entfernung von THF wird das Gemisch unter vermindertem Druck eingedampft. Die zurückbleibende wässrige Lösung wird mit Aethylacetat gewaschen und auf pH 2,7 angesäuert. Der erhaltene Niederschlag wird abfiltriert, mit Wasser gewaschen und unter vermindertem Druck über Calciumsulfat getrocknet. Man erhält 540 mg Zwischenprodukt, das unter Kühlung in 2,4 ml und 6 ml Trifluoressigsäure gelöst und etwa 12 Stunden bei 0°C gehalten wird. Das Gemisch wird anschliessend unter vermindertem Druck eingedampft, mit 10 ml Methylenchlorid versetzt und anschliessend erneut unter vermindertem Druck eingedampft. Nach Zugabe von 4 ml Methylenchlorid und 16 ml Aethylacetat erstarrt der Rückstand. Dieser wird abfiltriert, mit Aethylacetat gewaschen und in der Luft getrocknet; man erhält 520 mg Titelverbindung in Form des Trifluoracetats. Dieses Produkt wird mit vier äquivalenten Natriumbicarbonat in 0,025M pH 7,5 Natriumphosphatpuffer gelöst und an C₁₈-Kieselgel chromatographisch gereinigt mit pH 7,5 Puffer-Acetonitril als Eluens. Die entsprechenden Fraktionen werden vereinigt und auf pH 3 angesäuert, wobei die Titelverbindung ausfällt. Diese wird abfiltriert, mit Wasser gewaschen und unter vermindertem Druck getrocknet: IR 1782, 1703, 1628, cm⁻¹; Massenspektrum (FAB) m/z 843 (M + H)+.

### Beispiel 26 .

### Herstellung von [6R-[6α,7β(Z)]]-7-[[(2-Amino-4-thiazolyl)[(carboxymethoxy)imino]acetyl]amino]-3-[[[[4-(3-carboxy-1 cyclopropyl-6-fluor-1,4-dihydro-4-oxo-7-chinolinyl)-1 -piperazinyl]carbonyl]oxy]methyl]-8-oxo-5-thia-1-azabicyclo [4.2.0]oct-2-en-2-carbonsäure

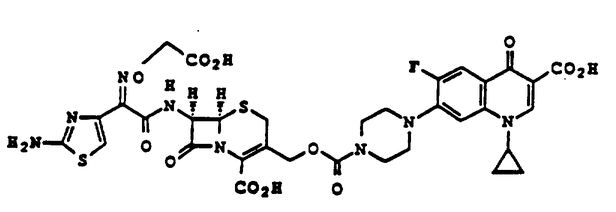

In analoger Weise wie in Beispiel 25 angegeben, jedoch unter Verwendung des entsprechenden Thioesters, erhält man die Titelverbindung: IR 1780, 1698, 1628 cm⁻¹; Massenspektrum (FAB) 815 (M + H)+.

Wie in den vorangehenden Beispielen angegeben können die folgenden Verbindungen hergestellt werden:

[6R-[6α,7β(Z)]]-7-[[(2-Amino-4-thiazolyl)(methoxyimino)acetyl]amino]-3-[[[[[1-(3-carboxy-1-äthyl-6-fluor-1,4-diydro-4-oxo-7-chinolinyl)-3-pyrrolidinyl]amino]carbonyl]oxy]methyl]-8-oxo-5-thia-1-azabicyclo[4.2.0]oct-2-en-2-carbonsäure 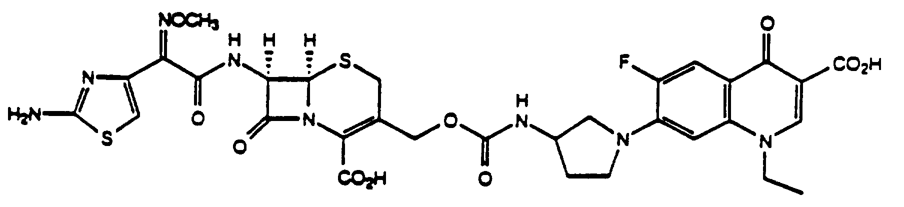 [6R-[6α,7β(Z)]]-7-[[(2-Amino-4-thiazolyl)(methoxyimino)acetyl]amino]-3-[[[[4-[3-carboxy-6-fluor-1-(4-fluorphenyl)-1,4-dihydro-4-oxo-7-chinolinyl]-1-piperazinyl]carbonyl]oxy]methyl]-8-oxo-5-thia-1-azabicyclo[4.2.0]oct-2-en-carbonsäure 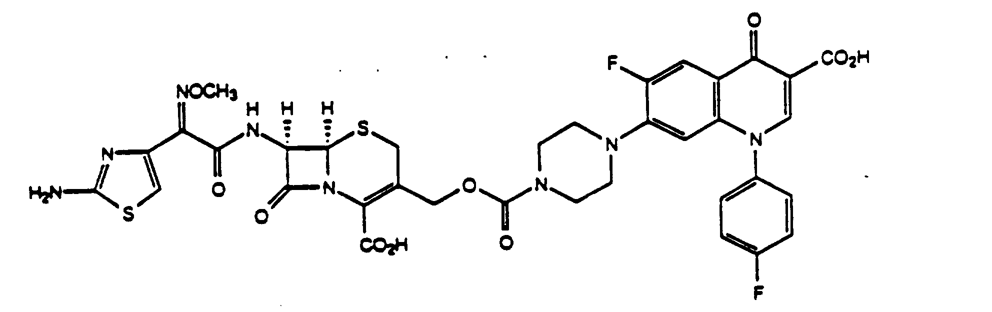 [6R-[6α,7β(Z)]]-7-[[(2-Amino-4-thiazolyl)(methoxyimino)acetyl]amino]-3-[[[[4-(3-carboxy-1-äthyl-6-fluor-1,4-dihydro-4-oxo-1,8-naphthyridin-7-yl)-1-piperazinyl]carbonyloxy]methyl]-8-oxo-5-thia-1-azabicyclo[4.2.0]oct-2-en-2-carbonsäure 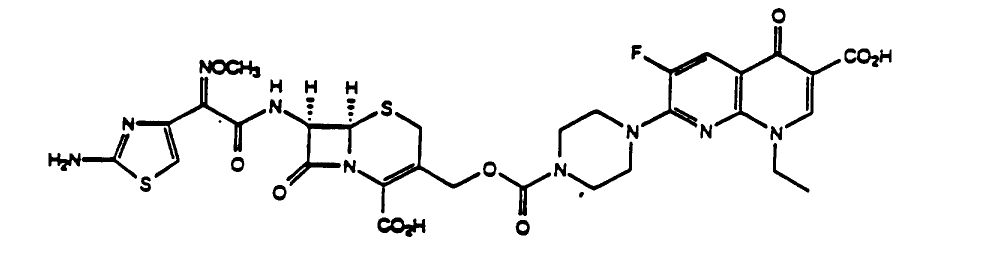 [6R-[6α,7β(Z)]]-7-[[(2-Amino-4-thiazolyl)(methoxyimino)acetyl]amino]-3-[[[[4-(9-cyclopropyl-6-fluor-2,3,4,9-tetrahydro-3,4-dioxoisothiazolo[5,4-b]chinolin-7-yl)-1-piperazinyl] -carbonyl]oxy]methyl]-8-oxo-5-thia-1-azabicyclo[4.2.0]oct-2-en-2-carbonsäure 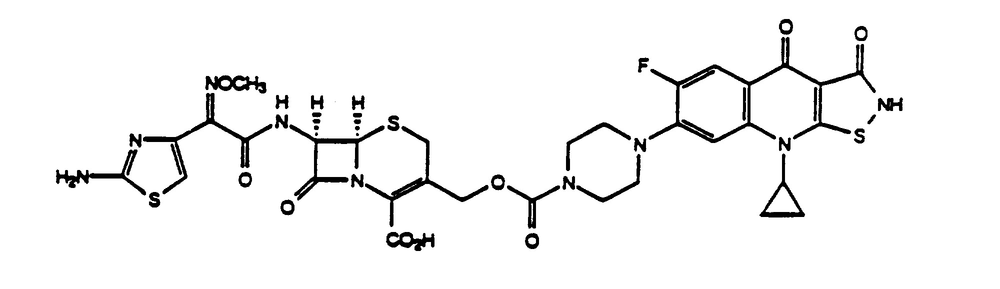 [6R-[6α,7α(Z)]]-7-[[(2-Amino-4-thiazolyl) (methoxyimino)acetyl]amino]-3-[[[[4-(9-cyclopropyl-6-fluor-2,3,4,9-tetrahydro-3,4-dioxoisoxazolo[5,4-b]chinolin-7-yl)-1-piperazinyl]carbonyl]-oxy]methyl]-8-oxo-5-thia-1-azabicyclo[4.2.0]oct-2-en-2-carbonsäure 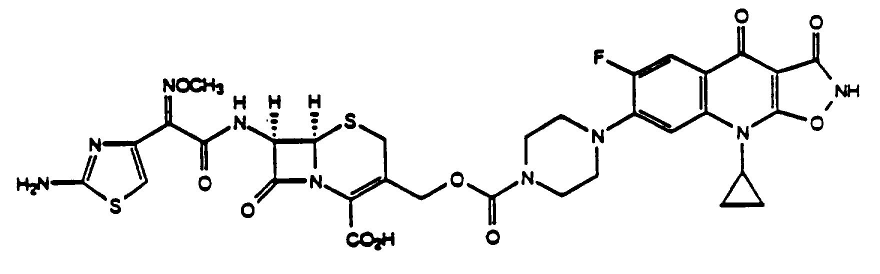 [6R-[6α,7β(Z)]]-7-[[(2-Amino-4-thiazolyl)(methoxyimino)acetyl]amino]-3-[[[[[1-(3-carboxy-8-chloro-1-cyclopropyl-6-fluor-1,4-dihydro-4-oxo-7-chinolinyl)-3-pyrrolidinyl]amino]carbonyl]oxy]-methyl]-8-oxo-5-thia-1-azabicyclo[4.2.0]oct-2-en-2-carbonsäure 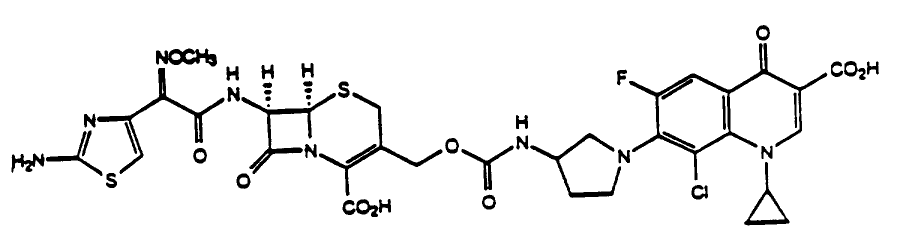 [6R-[6α,7β(Z)]]-7-[[(2-Amino-4-thiazolyl)[(1-carboxy-1-methyläthoxy)imino]acetyl]amino]-3-[[[[4-(3-carboxy-1-äthyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure 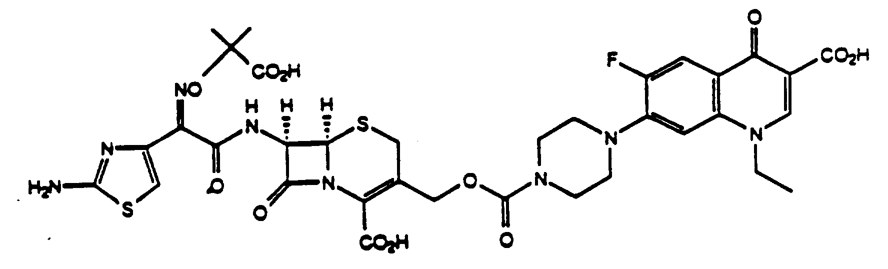 [6R-[6α,7β(Z)]]-7-[[(2-Amino-4-thiazolyl)[(carboxymethoxy)imino]acetyl]amino]-3-[[[[4-(3-carboxy-1-äthyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure 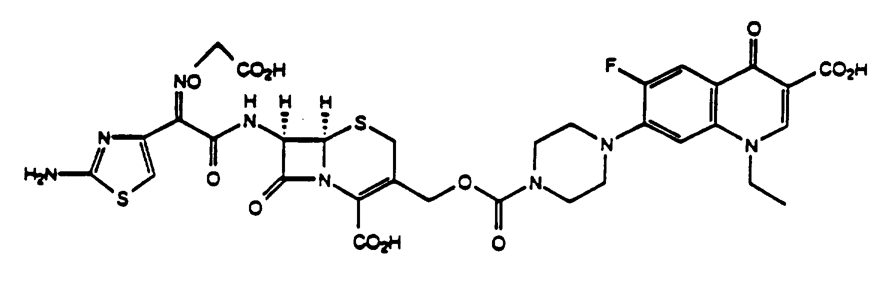 (6R-trans)-3-[[[[4-(3-Carboxy-1-äthyl-6-fluor-1,4-dihydro-4-oxo-7-chinolinyl)-1-piperazinyl]carbonyl]oxy]methyl]-7-[(hydroxyphenylacetyl)amino]-8-oxo-5-thia-1-azabicyclo[4.2.0]oct-2-en-2-carbonsäure 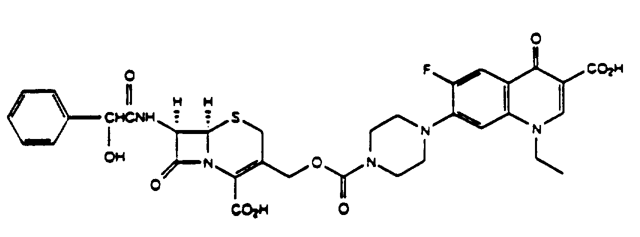 (6R-trans)-3-[[[[4-(3-Carboxy-1-äthyl-6-fluor-1,4-dihydro-4-oxo-7-chinolinyl)-1-piperazinyl]carbonyl]oxy]methyl]-8-oxo-7-[(phenoxyacetyl)amino]-5-thia-1-azabicyclo[4.2.0]oct-2-en-2-carbonsäure 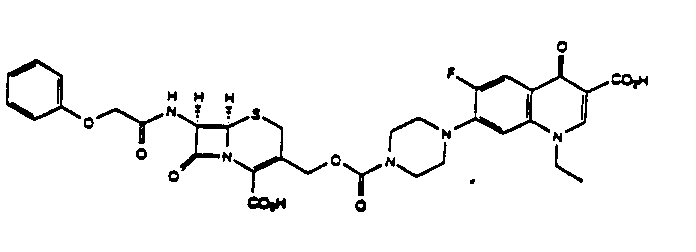 [6R-[6α,7β(R)]]-3-[[[[4-(3-Carboxy-1-äthyl-6-fluor-1,4-dihydro-4-oxo-7-chinolinyl)-1-piperazinyl]carbonyl]oxy]methyl]-7-[[[[(4-äthyl-2,3-dioxo-1-piperazinyl)carbonyl]amino]phenylacetyl]amino]-8-oxo-5-thia-1-azabicyclo[4.2.0]oct-2-en-2-carbonsäure 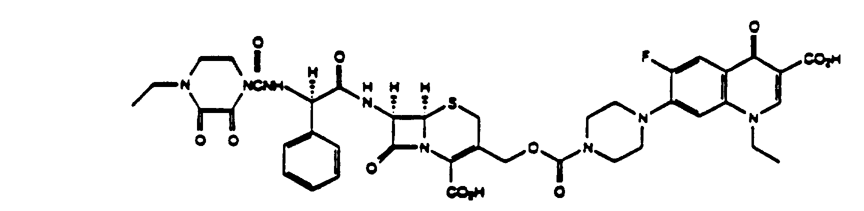 [6R-[6α,7β(Z)]]-7-[[(2-Amino-4-thiazolyl)(methoxyimino)acetyl]amino]-3-[[[[4-(5-amino-3-carboxy-1-cyclopropyl-6,8-difluor-1,4-dihydro-4-oxo-7-chinolinyl)-1-piperazinyl]carbonyl]oxy]methyl]-8-oxo-5-thia-1-azabicyclo[4.2.0]oct-2-on-2-carbonsäure

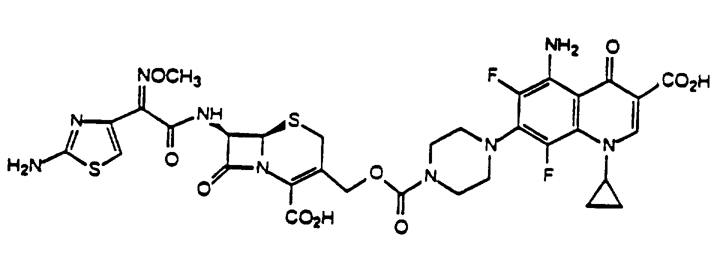

### Beispiel A

Herstellung von Trockenampullen für die intramuskulare Verabreichung:

Es wird in üblicher Weise ein Lyophilisat von 1 g des Wirkstoffes hergestellt und in eine Ampulle abgefüllt. Die sterile Wasserampulle enthält 10% Propylenglycol. Vor der Verabreichung wird das Lyophilisat mit 2,5 ml einer 2%-igen wässrigen Lidocainhydrochloridlösung versetzt.

Als Wirkstoff kann eines der gemäss den Beispielen 1-26 hergestellten Endprodukte eingesetzt werden.

## Patentansprüche (Patentansprüche für folgende(n) Vertragsstaat(en): AT, BE, CH, DE, FR, GB, IT, LI, LU, NL, SE)

1. Acylderivate der allgemeinen Formel 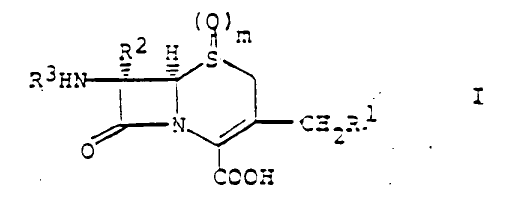 in der R¹ einen substituierten N-Heterocyclus der Formeln 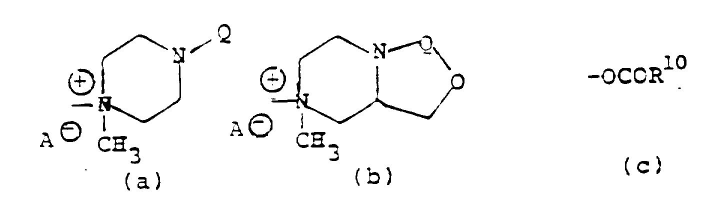 bedeutet, worin R¹⁰ eine der Gruppen 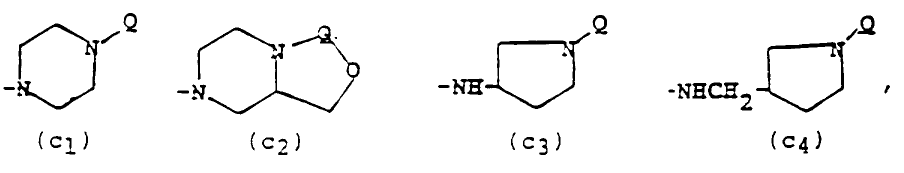 und Q eine substituierte Chinolinyl- oder Naphthyridinylgruppe darstellt, wobei der N-Heterocyclus gegebenenfalls durch eine oder mehrere C₁-C₈-Alkylgruppen substituierts ein kann; und worin ferner R Wasserstoff, C₁-C₈-Alkoxy, C₁-C₈-Alkylthio oder C₁-C₇-Alkanoylamino, R³ eine Acylgruppe, m 0, 1 oder 2 und A ein pharmazeutisch verträgliches Anion darstellt,
sowie leicht hydrolysierbare Ester und pharmazeutisch verträgliche Salze dieser Verbindungen und Hydrate der Verbindungen der Formel I bzw. von deren Estern und Salzen.

2. Verbindungen nach Anspruch 1, dadurch gekennzeichnet, dass R¹ eine der Gruppen (a) und (b) darstellt.

3. Verbindungen nach Anspruch 2, dadurch gekennzeichnet, dass m 0 und R Wasserstoff darstellt.

4. Verbindungen nach Anspruch 2 oder 3, dadurch gekennzeichnet, dass R¹ die nachstehende Formel besitzt: 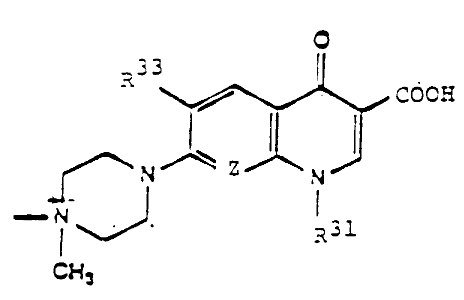 worin Z R³⁰-C oder Stickstoff; R³⁰ Wasserstoff, Halogen oder eine Oxymethylenbrücke (-CH₂O-) zum Piperazinrest unter Bildung eines ankondensierten 6-gliedrigen Ringes; R³¹ Wasserstoff, C₁-C₈-Alkyl, C₂-C₆-Alkenyl, C₃-C₇-Cycloalkyl, Halogen-C₁-C₈-Alkyl oder Mono-, Di- oder Trihalogenphenyl; oder R³⁰ und R³¹ zusammen eine C₃-C₅-Alkylengruppe, eine C₂-C₄-Alkylenmonooxygruppe, eine C₁-C₂-Alkylendioxygruppe oder eine Gruppe der Formel -OCH₂N(CH₃) ; und R³³ Wasserstoff oder Halogen darstellen.

5. Verbindungen nach Anspruch 4, dadurch gekennzeichnet, dass Z die Gruppe R³⁰-C darstellt, worin R³⁰ Wasserstoff, Brom, Chlor oder Fluor, R³¹ C₁-C₈-Alkyl, Halogen-C₁-C₈-Alkyl oder C₃-C₇ Cycloalkyl und R³³ Wasserstoff, Chlor oder Fluor bedeutet.

6. Verbindungen nach Anspruch 5, dadurch gekennzeichnet, dass R³⁰ Wasserstoff oder Fluor, R³¹ Aethyl, Fluoräthyl oder Cyclopropyl und R³³ Wasserstoff oder Fluor darstellt.

7. Verbindungen nach Anspruch 2 oder 3, dadurch gekennzeichnet, dass R¹ die nachstehende Formel besitzt:

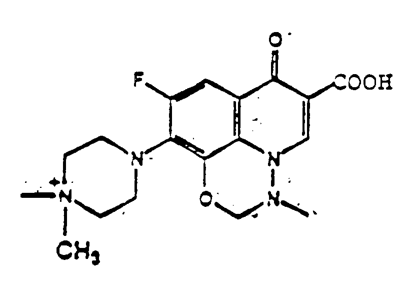

8. Verbindungen nach Anspruch 2 oder 3, dadurch gekennzeichnet, dass R¹ die nachstehende Formel besitzt:

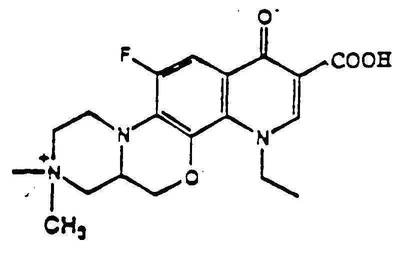

9. Verbindungen nach einem der Ansprüche 2-8, dadurch gekennzeichnet, dass R³ eine aliphatische Acylgruppe der Formel
R⁵-CO-
in der R⁵ Wasserstoff, C₁-C₈-Alkyl, C₃-C₇-Cycloalkyl, C₁-C₈-Alkoxy, C₂-C₆-Alkenyl, C₃-C₇-Cycloalkenyl oder Cyclohexadienyl; oder durch einen oder mehrere der Reste Halogen, Cyan, Nitro, Amino, Mercapto, C₁-C₈-Alkylthio oder Cyanmethylthio substituiertes C₁-C₈-Alkyl oder C₂-C₆- Alkenyl darstellt,
bedeutet.

10. Verbindungen nach einem der Ansprüche 2-8, dadurch gekennzeichnet, dass R³ eine aromatische Acylgruppe der allgemeinen Formeln 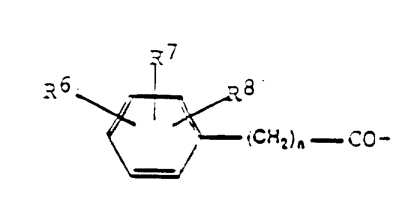 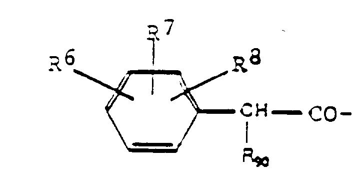 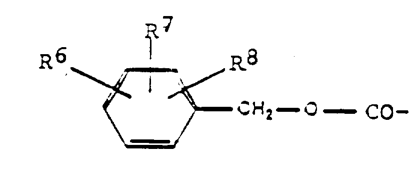 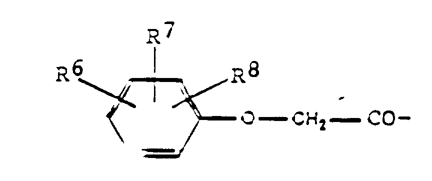 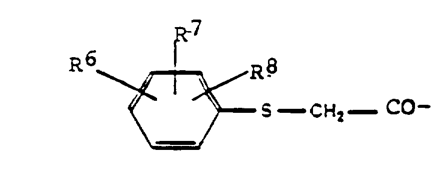 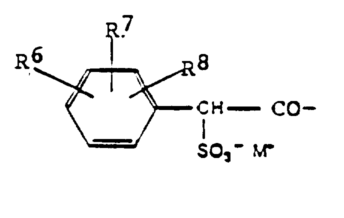 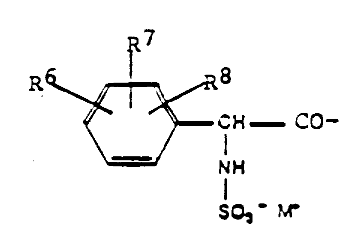 in denen n 0, 1, 2 oder 3; R⁶, R⁷ und R⁸ je Wasserstoff, Halogen, Hydroxy, Nitro, Amino, Cyan, Trifluormethyl, C₁-C₄-Alkyl, C₁-C₄-Alkoxy oder Aminomethyl, R⁹⁰ Amino, Acylamino, Hydroxy, ein Carboxysalz, geschütztes Carboxy, Formyloxy, Azido oder ein Sulfosalz und M ein Kation darstellen, bedeutet.

11. Verbindungen nach einem der Ansprüche 2-8, dadurch gekennzeichnet, dass R³ eine heteroaromatische Acylgruppe der allgemeinen Formeln
R¹⁰¹-(CH₂)ₙ-CO- 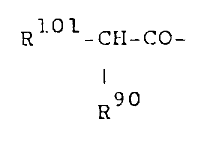
R¹⁰¹-O-CH₂-CO-
R¹⁰¹-S-CH₂-CO-
und
R¹⁰¹-CO-CO-
in denen n 0, 1, 2 oder 3; R⁹⁰ Amino, Acylamino, Hydroxy, ein Carboxysalz, geschütztes Carboxy, Azido oder ein Sulfosalz und R¹⁰¹ einen gegebenenfalls substituierten 5-, 6- oder 7-gliedrigen heterocyclischen Ring mit 1, 2, 3 oder 1 Stickstoff-, Sauerstoff- und/oder Schwefelatomen darstellt,
bedeutet.

12. Verbindungen nach einem der Ansprüche 2-8, dadurch gekennzeichnt, dass R³ eine [[(4-substituierte-2,3-dioxo--1-piperazinyl)carbonyl]amino]acetylgruppe der Formel 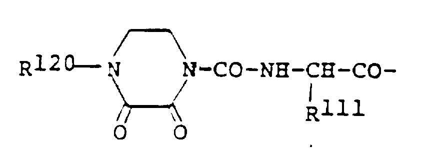 darstellt, in der R¹¹¹ C₁-C₈-Alkyl, Hydroxy-C₁-C₈-Alkyl oder eine aromatische Gruppe der allgemeinen Formel 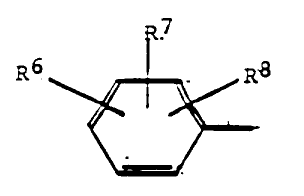 in der R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen und R¹⁰ gegebenenfalls durch einen oder mehrere der Reste halogen, Cyan, Nitro, Amino und/oder Mercapto substituiertes C₁-C₈-Alkyl darstellen.

13. Verbindungen nach einem der Anspcüche 2-8, dadurch gekennzeichnet, dass R³ eine (Acylamino)acetylgruppe der allgemeinen Formel 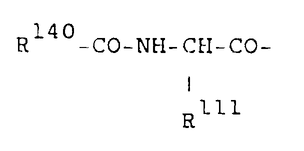 darstellt, worin R¹¹¹ C₁-C₈-Alkyl, Hydroxy-C₁-C₈-Alkyl oder eine aromatische Gruppe der Formel 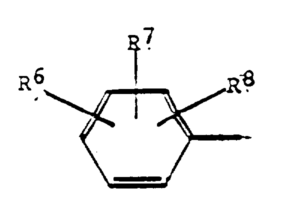 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder schwefelatomen darstellt, und worin ferner R¹⁴⁰ die Gruppe 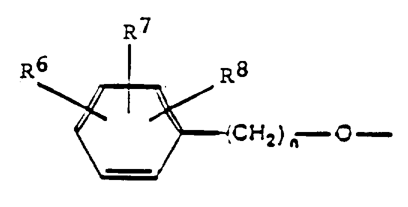 (worin R⁶, R⁷ und R⁸ die obige Bedeutung haben und n 0, 1, 2 oder 3 bedeutet) Wasserstoff, C₁-C₈-Alkyl, substituiertes C₁-C₈-Alkyl, Amino, C₁-C₈-Alkylamino, di-C₁-C₈-Alkyl)-amino, (Cyan-C₁-C₈-Alkyl)-amino, Hydrazino, C₁-C₈-Alkylhydrazino, Arylhydrazino oder Acylhydrazino darstellt.

14. Verbindungen nach einem der Ansprüche 2-8, dadurch gekennzeichnet, dass R³ eine (substituierte Oxyimino)acetylgruppe der allgemeinen Formel 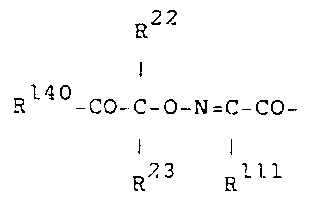 darstellt, worin R¹¹¹ C₁-C₈-Alkyl, Hydroxy-C₁-C₈-Alkyl oder eine aromatische Gruppe der Formel 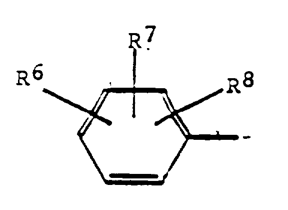 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/odec Schwefelatomen darstellt, und worin ferner R¹⁴⁰ die Gruppe 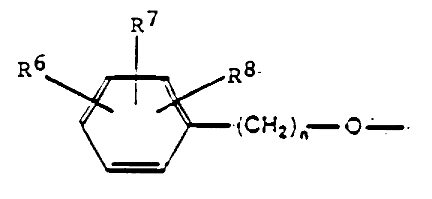 (worin R⁶, R⁷ und R⁸ die obige Bedeutung haben und n 0, 1, 2 oder 3 darstellt), Wasserstoff, C₁-C₈-Alkyl, substituiertes C₁-C₈-Alkyl, Amino, C₁-C₈-Alkylamino, Di-(C₁-C₈-Alkyl)-amino, (Cyan-C₁-C₈-Alkyl)-amino, Hydrazino, C₁-C₈-Alkylhydrazino, Arylhydrazino oder Acylhydrazino und R und R³ Wasserstoff oder C₁-C₈-Alkyl darstellen; oder R und R³ zusammen mit dem benachbarten Kohlenstoffatom einen C₃-C₇ carbocyclischen Ring bilden.

15. Verbindungen nach einem der Ansprüche 2-8, dadurch gekennzeichnet, dass R³ eine [[[3-substituierte-2-oxo-1-imidazolidinyl]carbonyl]amino]acetylgruppe der allgemeinen Formel 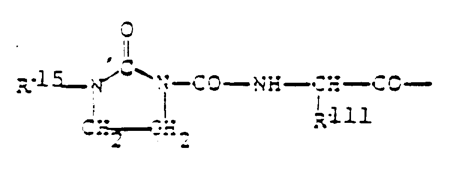 darstellt, worin R¹¹¹ C₁-C₈-Alkyl, Hydroxy-C₁-C₈-Alkyl oder eine aromatische Gruppe der Formel 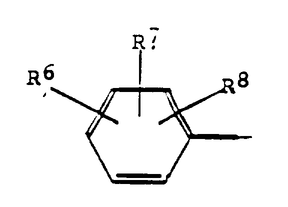 darstellt, worin R⁶, R⁷ und R⁸ Wasserstoff, Halogen, Hydroxy, Nitro, Amino, Cyan, Trifluormethyl, C₁-C₄-Alkyl, C₁-C₄- Alkoxy, Aminomethyl oder einen gegebenenfalls substituierten 5-, 6- oder 7-gliedrigen heterocyclischen Ring mit 1, 2, 3 oder 4. Stickstoff-, Sauerstoff- und/oder Schwefelatomen darstellt; und worin ferner R¹⁵ Wasserstoff, C₁-C₈-Alkylsulfonyl, Arylmethylenamino (d.h. -N=CHR¹¹¹, worin R¹¹¹ die oben gegebene Bedeutung hat), R¹⁶CO- (worin R¹⁶ Wasserstoff oder gegebenenfalls durch Halogen substituiertes C₁-C₈-Alkyl darstellt), eine wie unter R¹¹¹ oben definierte aromatische Gruppe oder gegebenenfalls durch einen oder mehrere der Reste Halogen, Cyan, Nitro, Amino und/oder Mercapto substituiertes C₁-C₈-Alkyl darstellt.

16. Verbindungen nach einem der Ansprüche 2-8, dadurch gekennzeichnet, dass R³ eine (substituierte Oxyimino)arylacetylgruppe der allgemeinen Formel 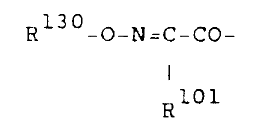 darstellt, worin R¹⁰¹ einen gegebenenfalls substituierten 5, 6- oder 7-gliedrigen heterocyclischen Ring mit 1,2, 3 oder 4, Stickstoff-, Sauerstoff-und/oder Schwefelatomen darstellt, worin der heterocyclische Ring durch Halogen, Hydroxy, Nitro, Amino, Cyan, Trifluormethyl, C₁-C₄-Alkyl oder C₁-C₄-Alkoxy substituiert ist und R¹³⁰ Wasserstoff, C₁-C₈-Alkyl, C₃-C₇-Cycloalkyl, Carboxy-C₃-C₇-cycloalkyl oder substituiertes C₁-C₈-Alkyl darstellt, wobei die C₁-C₈-Alkylgruppe substituiert ist durch einen oder mehrere der Reste Halogen, Cyan, Nitro, Amino, Mercapto, C₁-C₈-Alkylthio, eine durch R¹¹¹ oben definierte aromatische Gruppe, Carboxy (einschliesslich dessen Salze), C₁-C₇-Alkanoylamino, Carbamoyl, C₂-C₉-Alkoxycabonyl, Phenylmethoxycarbonyl, Diphenylmethoxycarbonyl, Hydroxy-C₁-C₈-Alkoxyphosphinyl, Dihydroxyphosphinyl, Hydroxy(phenylmethoxy)phosphinyl oder di-(C₁-C₈-Alkoxy)-phosphinyl.

17. Verbindungen nach Anspruch 16, dadurch gekennzeichnet, dass R¹⁰¹ die Formel 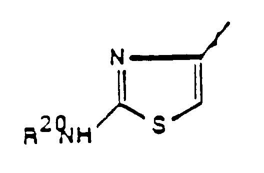 besitzt, worin R⁰ Wasserstoff oder eine Aminoschutzgruppe und R¹³⁰ Wasserstoff, C₁-C₈-Alkyl oder eine Gruppe der allgemeinen Formel 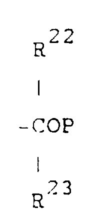 worin R und R³ Wasserstoff oder C₁-C₈-Alkyl oder zusammen mit dem benachbarten Kohlenstoffatom einen C₃-C₇-carbocyclischen Ring darstellen und P Hydroxy oder -NHR¹⁹, worin R¹⁹ Wasserstoff, C₁-C₈-Alkyl, Amino, C₁-C₈-Alkylamino, Arylamino oder Acylamino darstellt, bedeutet.

18. Verbindungen nach Anspruch 17, dadurch gekennzeichnet, dass R⁰ Wasserstoff und R¹³⁰ Methyl oder eine Gruppe der Formel 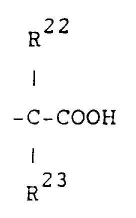 darstellt, worin R und R³ Wasserstoff oder Methyl bedeuten.

19. Verbindungen nach einem der Ansprüche 14 und 16-18 in der syn-Form bzw. als Gemische, worin die syn-Form überwiegt.

20. Verbindungen nach Anspruch 2, dadurch gekennzeichnet, dass R¹ die Gruppe (a) und R³ Phenoxyacetyl darstellt.

21. Verbindungen nach Anspruch 2, gekennzeichnet als
(6R-trans)-4-[3-Carboxy-1-(2-fluoräthyl)-6,8-difluor-1,4-dihydro-4-oxochinolin-7-yl]-[[2-carboxy-8-oxo-7-[(phenoxyacetyl)amino]-5-thia-1-azabicyclo[4.2.0]oct-2-en-3-yl]methyl]-1-methylpiperaziniumjodid,
[6R-[6α,7β(Z)]]-1-[[7-[[[(2-Amino-4-thiazolyl)[1-(1-carboxy-1-methyl)äthoxy]imino]acetyl]amino]-2-carboxy-8-oxo-5-thia-1-azabicyclo[4.2.0]oct-2-en-3-yl]methyl]-4-[3-carboxy-1-(2-fluocoäthyl)-6,8-difluor-1,4-dihydro-4-oxochinolin-7-yl]-1-methylpiperaziniumhydroxid-inneres Salz-Mononatriumsalz und
[6R-[6α,7β(Z)]]-1-[[7-[[(2-Amino-4-thiazolyl)(methoxyimino)acelyl]amino]-2-carboxy-8-oxo-5-thia-1-azabicyclo-[4.2.0]oct-2-en-3-yl]methyl]-4-(3-carboxy-1-äthyl-6-fluor-1,4-dihydro-4-oxo--7-chinolinyl)-1-methylpipecaziniumjodid.

22. [6R-[6α,7β(Z)]]-1-[[[(2-Amino-4-thiazolyl)(methoxyimino)acetyl]amino]-2-carboxy-8-oxo-5-thia-1-azabicyclo-[4.2.0]oct-2-en-3-yl]methyl]-4-[3-carboxy-6,8-difluor-1-(2-fluoräthyl)-1,4-dihydro-4-oxo-7-chinoliny]-1-methyl-piperaziniumjodid.

23. [6R-[6α,7β(Z)]]-1-[[[(2-Amino-4-thiazolyl)(methoxyimino)acetyl]amino]-2-carboxy-8-oxo-5-thia-1-azabicyclo-[4.2.0]oct-2-en-3-yl]methyl]-4-[3-carboxy-6,8-difluor-1-(2-fluoräthyl)-1,4-dihydro-4-oxo-7-chinolinyl]-1-methylpiperaziniumhydroxid-inneres Salz-Mononatriumsalz.

24. (6R-trans)-4-[3-Carboxy-6,8-difluor-1-(2-fluoräthyl)-1,4-dihydro-4-oxo-7-chinolinyl]-1-[[2-carboxy-7-(formylamino)-8-oxo-5-thia-1-azabicyclo[4.2.0]oct-2-en-3-yl]methyl]-1-methylpiperaziniumtrifluoracetat.

25. [6R-[6α,7β(Z)]]-1-[[7-[[(2-Amino-4-thiazolyl)[[1,1-dimethyl-2-(1,1-dimethyläthoxy)-2-oxo-ethoxy]imino]acetyl]amino]-2-carboxy-8-oxo-5-thia-1-azabicyclo[4.2.0]oct-2-en-3-yl]-methyl]-4-[3-carboxy-(2-fluoräthyl)-6,8-difluoro-1,4-dihydro-4-oxochinolin-7-yl]-1-methylpiperaziniumjodid.

26. Verbindungen nach Anspruch 1, dadurch gekennzeichnet, dass R¹ die Gruppe (c) darstellt.

27. Verbindungen nach Anspruch 26, dadurch gekennzeichnet, dass m 0 oder 1 darstellt.

28. Verbindungen nach Anspruch 26 oder 27, dadurch gekennzeichnet, dass Q eine Gruppe der Formel 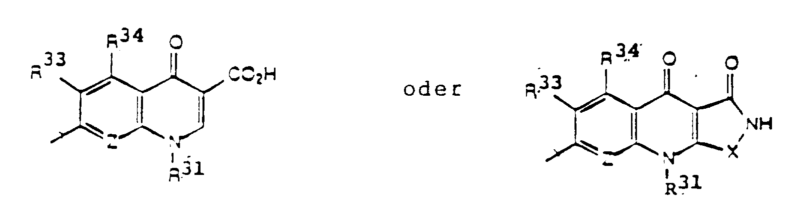 bedeutet, worin Z R³⁰-C oder Stickstoff; X Sauerstoff oder Schwefel; R³⁰ Wasserstoff, Halogen oder eine Oxomethylenbrücke (-CH₂O-) zum Piperazinrest unter Bildung eines ankondensierten 6-gliedrigen Ringes; R³¹ Wasserstoff, C₁-C₈-Alkyl, C₂-C₆-Alkenyl, C₃-C₇-Cycloalkyl, Halogen-C₁-C₈-Alkyl oder Mono-, Di- oder Tri-halogenphenyl; R³⁰ und R³¹ zusammen eine C₃-C₅-Alkylengruppe, eine C₂-C₄-Alkylenmonooxygruppe, eine C₁-C₂-Alkylendioxygruppe oder eine Gruppe der Formel -OCH₂N(CH₃)-; R³³ Wasserstoff oder Halogen; und R³⁴ Wasserstoff oder Amino darstellen.

29. Verbindungen nach Anspruch 28, dadurch gekennzeichnet, dass Z die Gruppe R³⁰-C bedeutet, worin R³⁰ Wasserstoff, Chlor, Brom oder Fluor, R³¹ C₁-C₈-Alkyl, Halogen-C₁-C₈-Alkyl oder C₃-C₇-Cycloalkyl und R³³ Wasserstoff, Chlor oder Fluor bedeutet.

30. Verbindungen nach Anspruch 29, dadurch gekennzeichnet, dass R³⁰ Wasserstoff oder Fluor, R³¹ ethyl, Fluoräthyl oder Cyclopropyl und R³³ Wasserstoff oder Fluor darstellt.

31. Verbindungen nach Anspruch 28, dadurch gekennzeichnet, dass R¹ die nachstehende Formel besitzt:

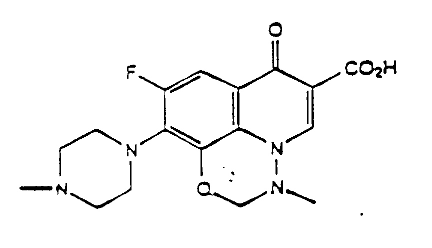

32. Verbindungen nach Anspruch 28, dadurch gekennzeichnet, dass R¹ die nachstehende Formel besitzt:

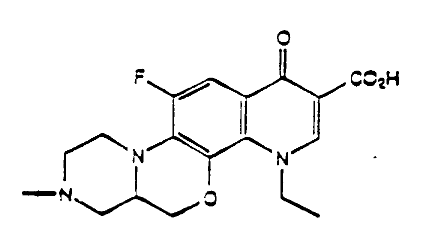

33. Verbindungen nach einem der Ansprüche 26-32, dadurch gekennzeichnet, dass R³ eine aliphatische Acylgruppe der Formel
R⁵-CO-
in der R⁵ Wasserstoff, C₁-C₈-Alkyl, C₃-C₇-Cycloalkyl, C₁-C₈-Alkoxy, C₂-C₆-Alkenyl, C₃-C₇-Cycloalkenyl oder Cyclohexadienyl; oder durch einen oder mehrere der Reste Halogen, Cyan, Nitro, Amino, Mercapto, C₁-C₈-Alkylthio oder Cyanmethylthio substituiertes C₁-C₈-Alkyl oder C₂-C₆-Alkenyl darstellt,
bedeutet.

34. Verbindungen nach einem der Ansprüche 26-32, dadurch gekennzeichnet, dass R³ eine aromatische Acylgruppe der allgemeinen Formeln 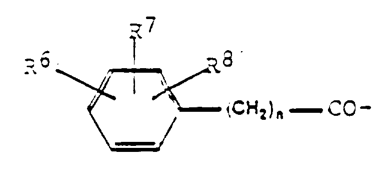 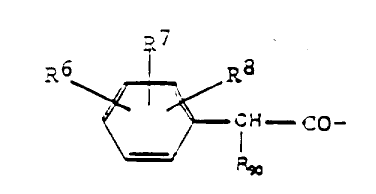 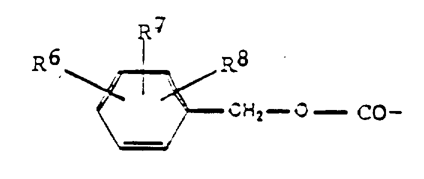 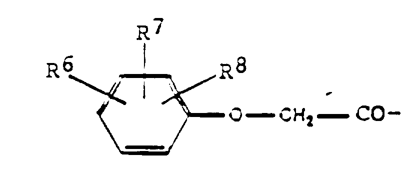 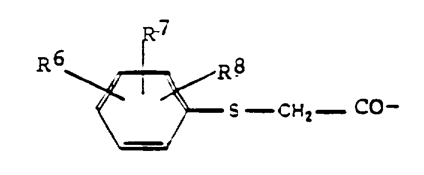 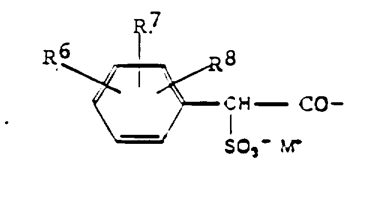 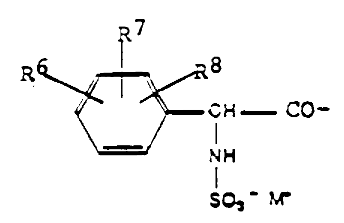 in denen n 0, 1, 2 oder 3; R⁶, R⁷ und R⁸ je Wasserstoff, Halogen, Hydroxy, Nitro, Amino, Cyan, Trifluormethyl, C₁-C₄-Alkyl, C₁-C₄-Alkoxy oder Aminomethyl, R⁹⁰ Amino, Acylamino, Hydcoxy, ein Carboxysalz, geschütztes Carboxy, Formyloxy, Azido oder ein Sulfosalz und M ein Kation darstellen,
bedeutet.

35. Verbindungen nach einem der Ansprüche 26-32, dadurch gekennzeichnet, dass R³ eine heteroaromatische Acylgruppe der allgemeinen Formeln
R¹⁰¹-(CH₂)ₙ-CO- 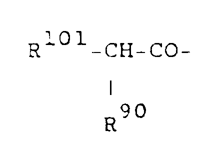
R¹⁰¹-O-CH₂-CO-
R¹⁰¹-S-CH₂-CO-
und
R¹⁰¹-CO-CO-
in denen n 0, 1, 2 oder 3; R⁹⁰ Amino, Acylamino, Hydroxy, ein Carboxysalz, geschütztes Carboxy, Azido oder ein Sulfosalz und R¹⁰¹ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellt,
bedeutet.

36. Verbindungen nach einem der Ansprüche 26-32, dadurch gekennzeichnet, dass R³ eine [[(4-substituierte-2,3-dioxo--1-piperazinyl)carbonyl]amino]acetylgruppe der Formel 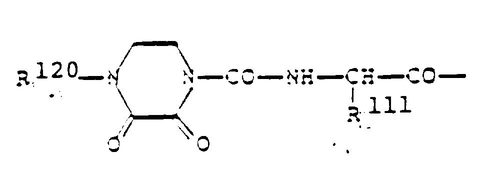 darstellt, in der R¹¹ C₁-C₈-Alkyl, Hydroxy-C₁-C₈-Alkyl oder eine aromatische Gruppe der allgemeinen Formel 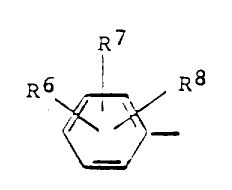 in der R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen und R¹⁰ gegebenenfalls durch einen oder mehrere der Reste Halogen, Cyan, Nitro, Amino und/oder Mercapto substituiertes C₁-C₈-Alkyl darstellen.

37. Verbindungen nach einem der Ansprüche 26-32, dadurch gekennzeichnet, dass R³ eine (Acylamino)acetylgruppe der allgemeinen Formel 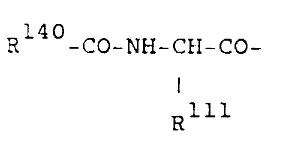 darstellt, worin R¹¹¹ C₁-C₈-Alkyl, Hydroxy-C₁-C₈-Alkyl oder eine aromatische Gruppe der Formel 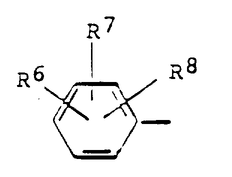 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, stickstoff-, Sauerstoff- und/oder Schwefelatomen darstellt, und worin ferner R¹⁴⁰ die Gruppe 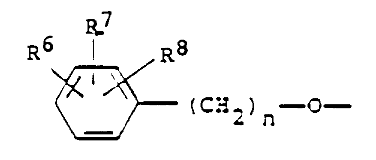 (worin R⁶, R⁷ und R⁸ die obige Bedeutung haben und n 0, 1, 2 oder 3 bedeutet) Wasserstoff, C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkylamino, Hydrazino, C₁-C₈-Alkylhydrazino, Arylhydrazino oder Acylhydrazino darstellt.

38. Verbindungen nach einem der Ansprüche 26-32, dadurch gekennzeichnet, dass R³ eine (substituierte Oxyimino)acetylgruppe der allgemeinen Formel 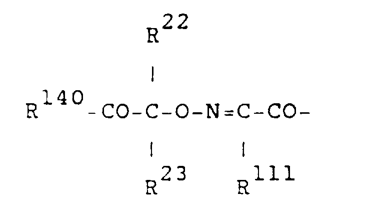 darstellt, worin R¹¹¹ C₁-C₈-Alkyl, Hydroxy-C₁-C₈-Alkyl oder eine aromatische Gruppe der Formel 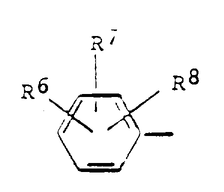 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt, und worin ferner R¹⁴⁰ die Gruppe 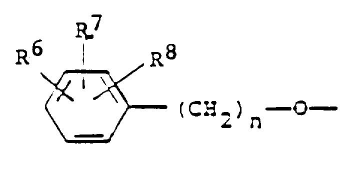 (worin R⁶, R⁷ und R⁸ die obige Bedeutung haben und n 0, 1, 2 oder 3 darstellt), Wasserstoff, C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkyl)-amino, Hydrazino, C₁-C₈-Alkylhydrazino, Arylhydrazino oder Acylhydrazino und R und R³ Wasserstoff oder C₁-C₈-Alkyl darstellen, oder R und R³ zusammen mit dem benachbarten Kohlenstoffatom einen C₃-C₇ carbocyclischen Ring bilden.

39. Verbindungen nach einem der Ansprüche 26-32, dadurch gekennzeichnet, dass R³ eine [[[3-substituierte-2-oxo--1-imidazolidinyl]carbonyl]amino]acetylgruppe der allgemeinen Formel 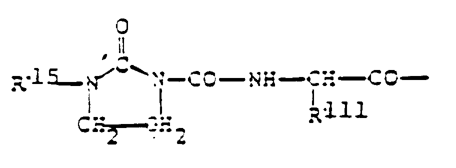 darstellt, worin R¹¹¹ C₁-C₈-Alkyl, Hydroxy-C₁-C₈-Alkyl oder eine aromatische Gruppe der Formel 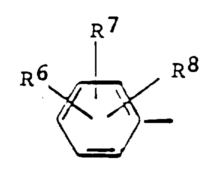 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt; und worin ferner R¹⁵ Wasserstoff, C₁-C₈-Alkylsulfonyl, Arylmethylenamino (d.h. -N=CHR¹¹¹, worin R¹¹¹ die oben gegebene Bedeutung hat), R¹⁶CO- (worin R¹⁶ Wasserstoff oder gegebenenfalls durch Halogen substituiertes C₁-C₈-Alkyl darstellt), eine wie unter R¹¹¹ oben definierte aromatische Gruppe oder gegebenenfalls durch einen oder mehrere der Reste Halogen, Cyan, Nitro, Amino und/oder Mercapto substituiertes C₁-C₈-Alkyl darstellt.

40. Verbindungen nach einem der Ansprüche 26-32, dadurch gekennzeichnet, dass R³ eine (substituierte Oxyimino)arylacetylgruppe der allgemeinen Formel 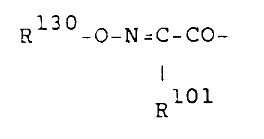 darstellt, worin R¹⁰¹ einen gegebenenfalls substituierten 5, 6- oder 7-gliedrigen heterocyclischen Ring mit 1, 2, 3 oder 4, Stickstoff-, Sauerstoff- und/oder Schwefelatomen darstellt, worin der heterocyclische Ring durch Halogen, Hydroxy, Nitro, Amino, Cyan, Trifluormethyl, C₁-C₄-Alkyl oder C₁-C₄-Alkoxy substituiert ist und R¹³⁰ Wasserstoff, C₁-C₈-Alkyl, C₃-C₇-Cycloalkyl, Carboxy-C₃-C₇-cycloalkyl oder substituiertes C₁-C₈ Alkyl darstellt, wobei die C₁-C₈-Alkylgruppe substituiert ist durch einen oder mehrere der Reste Halogen, Cyan, Nitro, Amino, Mercapto, C₁-C₈-Alkylthio, eine durch R¹¹¹ oben definierte aromatische Gruppe, Carboxy (einschliesslich dessen Salze), C₁-C₇-Alkanoylamino, Carbamoyl, C₂-C₉-Alkoxycabonyl, Phenylmethoxycarbonyl, Diphenylmethoxycarbonyl, Hydroxy-C₁-C₈-Alkoxyphosphinyl, Dihydroxyphosphinyl, Hydroxy(phenylmethoxy)phosphinyl oder di-(C₁-C₈-Alkoxy)-phosphinyl.

41. Verbindungen nach Anspruch 40, dadurch gekennzeichnet, dass R¹⁰¹ die Formel 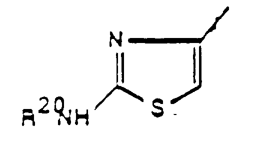 besitzt, worin R⁰ Wasserstoff oder eine Aminoschutzgruppe und R¹³⁰ Wasserstoff, C₁-C₈-Alkyl oder eine Gruppe der allgemeinen Formel 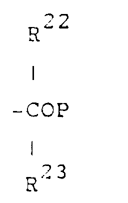 worin R und R³ Wasserstoff oder C₁-C₈-Alkyl oder zusammen mit dem benachbarten Kohlenstoffatom einen C₃-C₇-carbocyclischen Ring darstellen und P Hydroxy oder -NHR¹⁹, worin R¹⁹ Wasserstoff, C₁-C₈-Alkyl, Amino, C₁-C₈-Alkylamino, Arylamino oder Acylamino darstellt, bedeutet.

42. Verbindungen nach Anspruch 41, dadurch gekennzeichnet, dass R⁰ Wasserstoff oder Triphenylmethyl darstellt.

43. Verbindungen nach einem der Ansprüche 38 und 40-42 in der syn-Form bzw. als Gemische, worin die syn-Form überwiegt.

44. Verbindungen nach Anspruch 26, gekennzeichnet als
[6R-[6α,7β(Z)]]-7-[[(2-Amino-4-thiazolyl)(methoxyimino)acetyl]amino]-3-[[[[4-(3-carboxy-1-äthyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure,
[6R-[6α,7β(Z)]]-7-[[(2-Amino-4-thiazolyl)methoxyimino)acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]-oct-2-en-2-carbonsäure,
(6R-trans)-3[[[[4-(3-Carboxy-1-cyclopropyl-6-fluor-1,4-dihydro-4-oxo-7-chinolinyl)-1-piperazinyl]carbonyl]oxy]methyl]-8-oxo-7-[(phenoxyacetyl)amino]-5-thia-1-azabicyclo[4.2.0]oct-2-en-2-carbonsäure,
[6R-[6α,7β(Z)]]-7-[[(2-Amino-4-thiazolyl)-[(1-carboxy-1-methyl-äthoxy)imino]acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure,
[6R-[6α,7β(Z)]]-7-[[(2-Amino-4-thiazolyl)-[(carboxymethoxy)imino]acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure,
[6R-[6α,7β(Z)]]-7-[[(2-Amino-4-thiazolyl)(methoxyimino)acetyl]amino]-3-[[[[4-(9-cyclopropyl-6-fluoro-2,3,4,9-tetrahydro-3,4-dioxoisothiazolo[5,4-b]chinolin-7-yl)-1-piperazinyl]carbonyl]oxy]methyl]-8-oxo-5-thia-1-azabicyclo[4.2.0]-oct-2-en-2-carbonsäure und
[6R-[6α,7β(Z)]]-7-[[(2-Amino-4-thiazolyl)(methoxyimino)acetyl]amino]-3-[[[[4-(9-cyclopropyl-6-fluoro-2,3,4,9-tetrahydro-3,4-dioxoisoxazolo[5,4-b]chinolin-7-yl)-1-piperazinyl]carbonyl]oxy]methyl]-8-oxo-5-thia-1-azabicyclo[4.2.0]oct-2-en-2-carbonsäure
sowie Salze dieser Verbindungen und Hydrate dieser Verbindungen und Salze.

45. Salze der Verbindungen gemäss Anspruch 44, gekennzeichnet als Natriumsalze.

46. Verbindungen der Formel 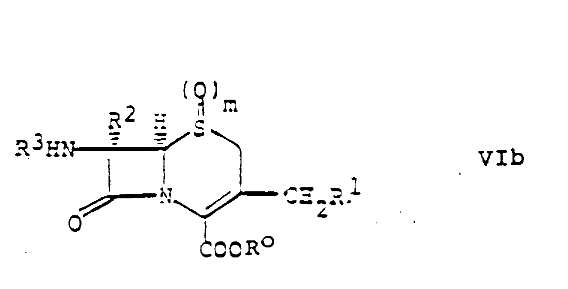 worin R¹, R oder R³ und m die oben gegebene Bedeutung haben und R^{o} t-Butyl, Benzhydryl, p-Nitrobenzyl oder Allyl darstellt.

47. (6R-trans)-4-[3-Carboxy-6,8-difluor-1-(2-fluoräthyl)-1,4-dihydro-4-oxo-7-chinolinyl]-1-[[2-(1,1-dimethyläthoxy)carbonyl-7-(formylamino)-8-oxo-5-thia-1-azabicyclo[4.2.0]oct-2-en-3-yl]methyl]-1-methylpiperaziniumjodid.

48. Verbindungen der Formel 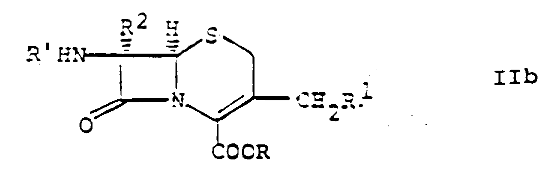 worin R¹ und R die oben gegebene Bedeutung haben, R Wasserstoff oder eine Carbonsäureschutzgruppe und R' Wasserstoff oder eine Leicht abspaltbare Aminoschutzgruppe darstellt.

49. (6R-trans)-3-[[[[4-(3-Carboxy-1-cyclopropyl-6-fluor-1,4-dihydro-4-oxo-7-chinolinyl)-1-piperazinyl]carbonyl]oxy]methyl]-7-[[(1,1-dimethyläthoxy)carbonyl]amino]-8-oxo-5-thia-1-azabicyclo[4.2.0]oct-2-en-2-carbonsäurediphenylmethylester.

50. Verbindungen gemäss einem der Ansprüche 1-45 als pharmazeutische Wirkstoffe.

51. Verbindungen gemäss einem der Ansprüche 1-45 als pharmazeutische Wirkstoffe zur Behandlung und Prophylaxe von Infektionskrankheiten.

52. Verfahren zur Herstellung der Verbindungen gemäss einem der Ansprüche 1-45, dadurch gekennzeichnet, dass man
a) zur Herstellung einer Verbindung der Formel I, in der m 0 darstellt, eine Verbindung der allgemeinen Formel 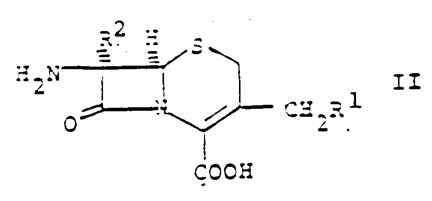 in der R¹ und R die oben gegebene Bedeutung haben, oder einen leicht hydrolysierbaren Ester oder Salz dieser Verbindung mit einer Carbonsäure der allgemeinen Formel R³OH oder mit einem reaktionsfähigen Derivat davon umsetzt, oder dass man
b) zur Herstellung einer Verbindung der Formel I, in der m 0 und R¹ eine der Gruppen (a) und (b) darstellt, eine Verbindung der Formel 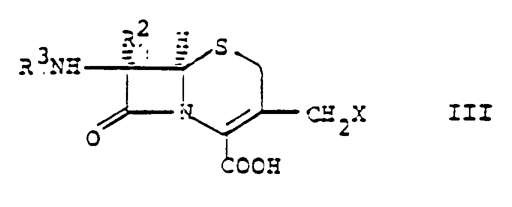 in der R und R³ die oben gegebene Bedeutung haben, X eine Abgangsgruppe darstellt, und Amino-, Hydroxy- und Carboxygruppen geschützt sein können,
oder einen leicht hydrolysierbaren Ester oder Salz davon mit einer Verbindung der Formel 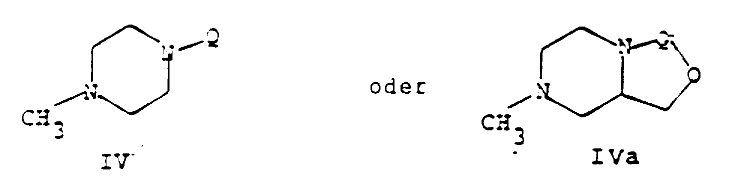 in der Q die oben gegebene Bedeutung hat und die Stickstoffatome geschützt sein können,
oder dass man
c) zur Herstellung eines leicht hydrolysierbaren Esters einer Carbonsäure der Formel I, in der m 0 und R¹ die Gruppe (c) darstellt, eine Verbindung der Formel 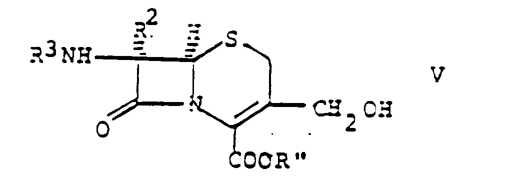 in der R und R³ die oben gegebene Bedeutung haben und R' den Rest eines leicht hydrolysierbaren Esters darstellt,
in Gegenwart einer Base mit Phosgen umsetzt, das erhaltene Reaktionsprodukt mit einer Verbindung der Formel R¹⁰H, worin R¹⁰ die oben gegebene Bedeutung hat, umsetzt, wobei jedoch allfällige Amino-, Hydroxy- und/oder Carboxyfunktionen davon geschützt sein können, wonach man allfällige Schutzgruppen abspaltet, oder dass man
d) zur Herstellung einer Carbonsäure der Formel I einen Ester der Formel 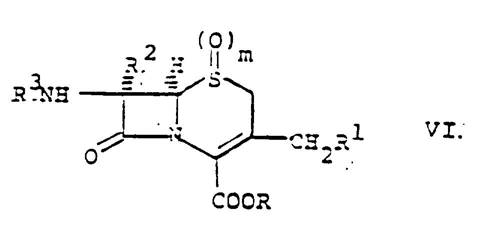 in der R¹, R, R³ und m die oben gegebene Bedeutung haben und R eine Carbonsäureschutzgruppe darstellt, in die Carbonsäure der Formel I umwandelt, oder dass man
e) zur Herstellung einer Verbindung der Formel I, worin m 0 darstellt, oder eines leicht hydrolysierbaren Esters oder Salzes davon eine Verbindung der Formel 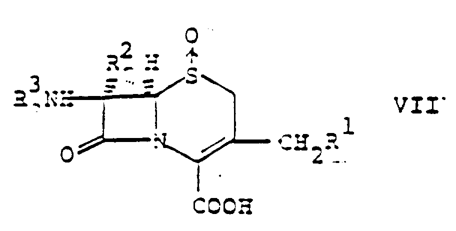 in der R¹, R und R³ die oben gegebene Bedeutung haben,
oder einen leicht hydrolyzierbaren Ester oder Salz davon reduziert, oder dass man
f) zur Herstellung einer Verbindung der Formel I, worin m 1 oder 2 darstellt oder eines leicht hydrolysierbaren Esters oder Salzes davon eine Verbindung der Formel 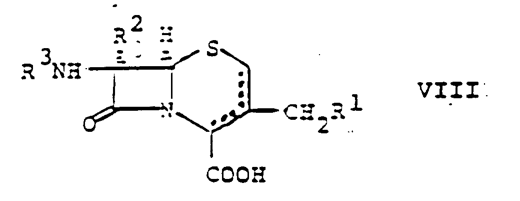 in der R¹, R und R³ die oben gegebene Bedeutung haben und die gestrichelten Linien die Anwesenheit einer Δ2- oder Δ3-Doppelbindung anzeigen,
oder eines ihrer Ester oder Salze oxidiert, oder dass man
g) zur Herstellung einer Verbindung der Formel I, worin R³ einen Aminosubstituenten enthält oder eines ihrer Ester oder Salze die Aminoschutzgruppe in Substituenten R³⁰ einer Verbindung der allgemeinen Formel 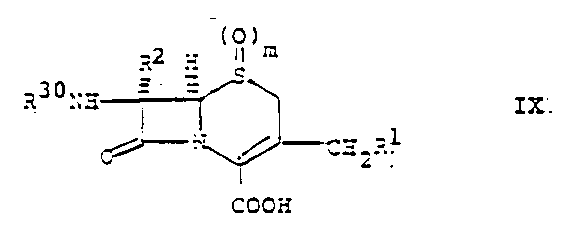 in der R¹, R und m die oben gegebene Bedeutung haben und R³⁰ einen eine geschützte Aminogruppe enthaltenden Acylrest darstellt,
oder eines ihrer Ester oder Salze abspaltet, oder dass man
h) zur Herstellung eines leicht hydrolysierbaren Esters einer Verbindung der Formel I eine Carbonsäure der Formel I einer entsprechenden Veresterung unterwirft, oder dass man
i) zur überstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

53. Verfahren nach Anspruch 52, dadurch gekennzeichnet, dass man die Verfahrensalternative (b) ausführt.

54. Verfahren nach Anspruch 52, dadurch gekennzeichnet, dass man eine der Verfahrensalternativen (a), (c), (d), (f), (g) und (i) ausführt.

55. Pharmazeutische Präparate, gekennzeichnet durch einen Gehalt an einer Verbindung gemäss einem der Ansprüche 1-45.

56. Pharmazeutische Präparate zur Behandlung und Prophylaxe von Infektionskrankheiten, gekennzeichnt durch einen Gehalt an einer Verbindung gemäss einem der Ansprüche 1-45.

57. Verwendung von Verbindungen gemäss einem der Ansprüche 1-45 bei der Herstellung von Arzneimitteln für die Behandlung bzw. Prophylaxe von Infektionskrankheiten.

## Patentansprüche (Patentansprüche für folgende(n) Vertragsstaat(en): ES, GR)

1. Verfahren zur Herstellung von Acylderivaten der allgemeinen Formel 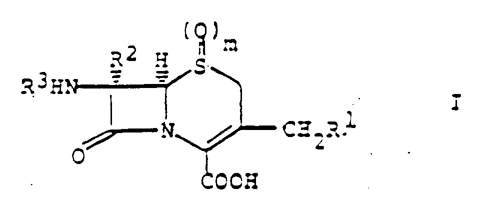 in der R¹ einen substituierten N-Herocyclus der Formeln 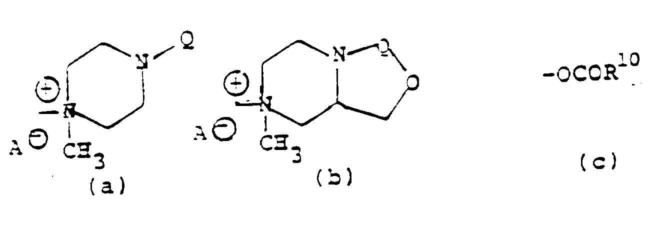 bedeutet, worin R¹⁰ eine der Gruppen 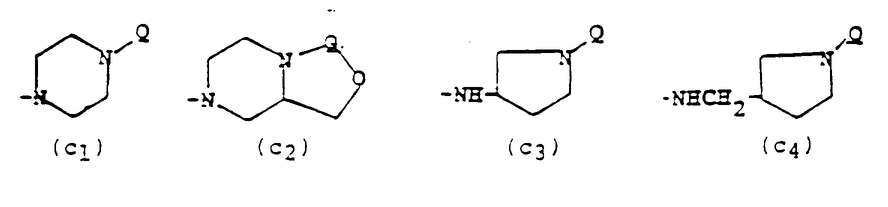 und Q eine substituierte Chinolinyl- oder Naphlhyridinylgruppe darstellt, wobei der N-Heterocyclus gegebenenfalls durch eine oder mehrere C₁-C₈-Alkylgruppen substituiert sein kann; und worin ferner R Wasserstoff, C₁-C₈-Alkoxy, C₁-C₈-Alkylthio oder C₁-C₇-Alkanoylamino, R³ eine Acylgruppe, m 0, 1 oder 2 und A ein pharmazeutisch verträgliches Anion darstellt,
sowie von leicht hydrolysierbaren Estern und pharmazeutisch verträglichen Salzen dieser Verbindungen und von Hydraten der Verbindungen der Formel I bzw. von deren Estern und Salzen, dadurch gekennzeichnet. dass man
a) zur Herstellung einer Verbindung der Formel I, in der m 0 darstellt. eine Verbindung der allgemeinen Formel 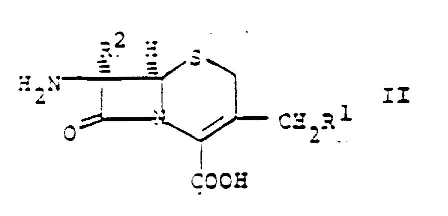 in der R¹ und R die oben gegebene Bedeutung haben, oder einen leicht hydrolysierbaren Ester oder Salz dieser Verbindung mit einer Carbonsäure der allgemeinen Formel R³OH oder mit einem reaktionsfähigen Derivat davon umsetzt, oder dass man
b) zur Herstellung einer Verbindung der Formel I, in der m 0 und R¹ eine der Gruppen (a) und (b) darstellt, eine Verbindung der Formel 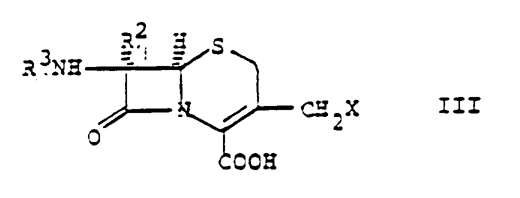 in der R und R³ die oben gegebene Bedeutung haben, X eine Abgangsgruppe darstellt, und Amino-, Hydroxy- und Carboxygruppen geschützt sein können,
oder einen leicht hydrolysierbaren Ester oder Salz davon mit einer Verbindung der Formel 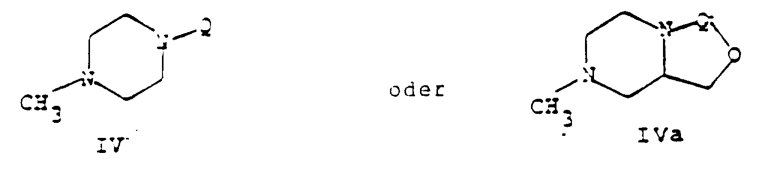 in der Q die oben gegebene Bedeutung hat und die Stickstoffatome geschützt sein können,
oder dass man
c) zur Herstellung eines leicht hydrolysierbaren Esters einer Carbonsäure der Formel I, in der m 0 und R¹ die Gruppe (c) darstellt, eine Verbindung der Formel 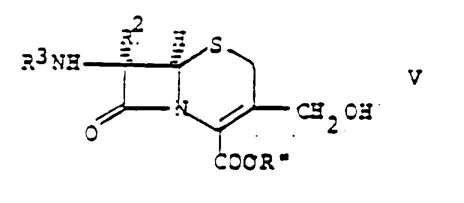 in der R und R³ die oben gegebene Bedeutung haben und R" den Rest eines leicht hydrolysierbaren Esters darstellt,
in Gegenwart einer Base mit Phosgen umsetzt, das erhaltene Reaktionsprodukt mit einer Verbindung der Formel R¹⁰H, worin R¹⁰ die oben gegebene Bedeutung hat, umsetzt, wobei jedoch allfällige Amino-, Hydroxy- und/oder Carboxyfunktionen davon geschützt sein können, wonach man allfällige Schutzgruppen abspaltet, oder dass man
d) zur Herstellung einer Carbonsäure der Formel I einen Ester der Formel 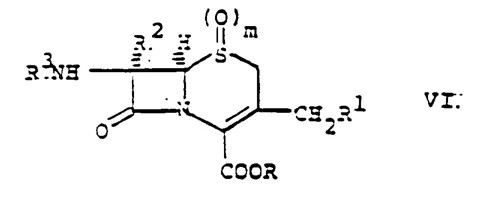 in der R¹, R, R³ und m die oben gegebene Bedeutung haben und R eine Carbonsäureschutzgruppe darstellt, in die Carbonsäure der Formel I umwandelt, oder dass man
e) zur Herstellung einer Verbindung der Formel I, worin m 0 darstellt, oder eines leicht hydrolysierbaren Esters oder Salzes davon eine Verbindung der Formel 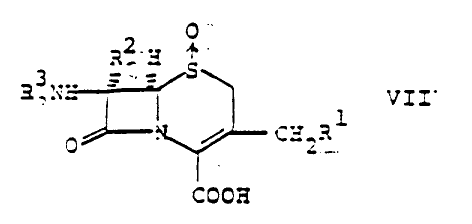 in der R¹, R und R³ die oben gegebene Bedeutung haben,
oder einen leicht hydrolysierbaren Ester oder Salz davon reduziert, oder dass man
f) zur Herstellung einer Verbindung der Formel I, worin m 1 oder 2 darstellt oder eines leicht hydrolysierbaren Esters oder Salzes davon eine Verbindung der Formel 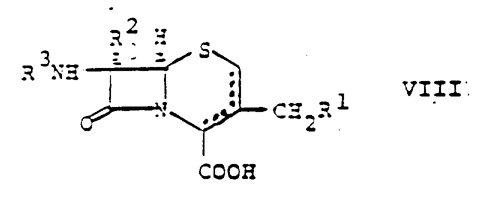 in der R¹, R und R³ die oben gegebene Bedeutung haben und die gestrichelten Linien die Anwesenheit einer Δ2- oder Δ3-Doppelbindung anzeigen.
oder eines ihrer Ester oder Salze oxidiert, oder dass man
g) zur Herstellung einer Verbindung der Formel I, worin R³ einen Aminosubstituenten enthält oder eines ihrer Ester oder Salze die Aminoschutzgruppe in Substituenten R³⁰ einer Verbindung der allgemeinen Formel 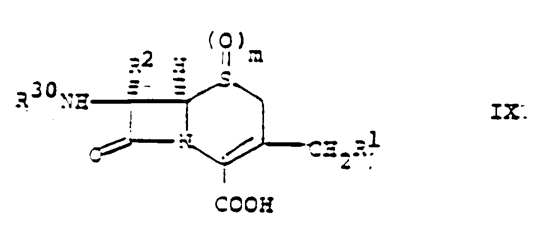 in der R¹, R und m die oben gegebene Bedeutung haben und R³⁰ einen eine geschützte Aminogruppe enthaltenden Acylrest darstellt,
oder eines ihrer Ester oder Salze abspaltet, oder dass man
h) zur Herstellung eines leicht hydrolysierbaren Esters einer Verbindung der Formel I eine Carbonsäure der Formel I einer entsprechenden Veresterung unterwirft, oder dass man
i) zur Herstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

2. Verfahren zur Herstellung von Verbindungen nach Anspruch 1, worin R¹ eine der Gruppen (a) und (b) darstellt, dadurch gekennzeichnet, dass man entsprechend substituierte Ausgangsverbindungen einsetzt.

3. Verfahren zur Herstellung von Verbindungen nach Anspruch 2, worin m 0 und R Wasserstoff darstellt, dadurch gekennzeichnet, dass man entsprechend substituierte Ausgangsverbindungen einsetzt.

4. Verfahren zur Herstellung von Verbindungen nach Anspruch 2 oder 3, worin R¹ die nachstehende Formel besitzt: 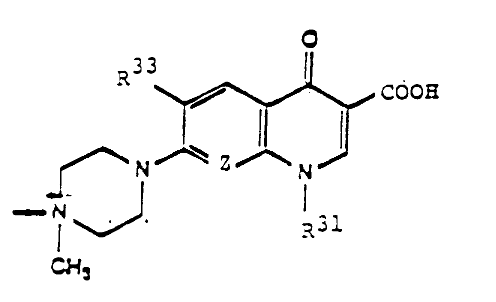 worin Z R³⁰-C oder Stickstoff; R³⁰ Wasserstoff, Halogen oder eine Oxymethylenbrücke (-CH₂O-) zum Piperazinrest unter Bildung eines ankondensierten 6-gliedrigen Ringes; R³¹ Wasserstoff, C₁-C₈-Alkyl, C₂-C₆-Alkenyl, C₃-C₇-Cycloalkyl, Halogen-C₁-C₈-Alkyl oder Mono-, Di- oder Trihalogenphenyl; oder R³⁰ und R³¹ zusammen eine C₃-C₅-Alkylengruppe, eine C₂-C₄-Alkylenmonooxygruppe, eine C₁-C₂-Alkylendioxygruppe oder eine Gruppe der Formel -OCH₂N(CH₃)-; und R³³ Wasserstoff oder Halogen darstellen,
dadurch gekennzeichnet, dass man entsprechend substituierte Ausgangsverbindungen einsetzt.

5. Verfahren zur Herstellung von Verbindungen nach Anspruch 4, worin Z die Gruppe R³⁰-C darstellt, worin R³⁰ Wasserstoff, Brom, Chlor oder Fluor, R³¹ C₁-C₈-Alkyl, Halogen-C₁-C₈-Alkyl oder C₃-C₇ -Cycloalkyl und R³³ Wasserstoff, Chlor oder Fluor bedeutet, dadurch gekennzeichnet, dass man entsprechend substituierte Ausgangsverbindungen einsetzt.

6. Verfahren zur Herstellung von Verbindungen nach Anspruch 5, worin R³⁰ Wasserstoff oder Fluor, R³¹ Aethyl, Fluoräthyl oder Cyclopropyl und R³³ Wasserstoff oder Fluor darstellt, dadurch gekennzeichnet, dass man entsprechend substituierte Ausgangsverbindungen einsetzt.

7. Verfahren zur Herstellung von Verbindungen nach Anspruch 2 oder 3, worin R¹ die nachstehende Formel besitzt: 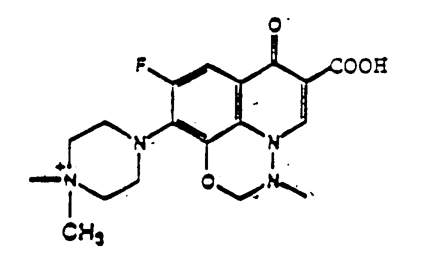 dadurch gekennzeichnet, dass man entsprechend substituierte Ausgangsverbindungen einsetzt.

8. Verfahren zur Herstellung von Verbindungen nach Anspruch 2 oder 3, worin R¹ die nachstehende Formel besitzt: 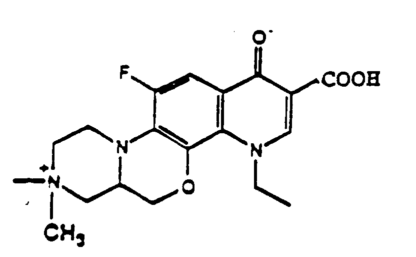 dadurch gekennzeichnet, dass man entsprechend substituierte Ausgangsverbindungen einsetzt.

9. Verfahren zur Herstellung von Verbindungen nach einem der Ansprüche 2-8, worin R³ eine aliphatische Acylgruppe der Formel
R⁵-CO-
in der R⁵ Wasserstoff, C₁-C₈-Alkyl, C₃-C₇-Cycloalkyl, C₁-C₈-Alkoxy, C₂-C₆-Alkenyl, C₃-C₇-Cycloalkenyl oder Cyclohexadienyl; oder durch einen oder mehrere der Reste Halogen, Cyan, Nitro, Amino, Mercapto, C₁-C₈-Alkylthio oder Cyanmethylthio substituiertes C₁-C₈-Alkyl oder C₂-C₆-Alkenyl darstellt,
bedeutet, dadurch gekennzeichnet, dass man entsprechend substituierte Ausgangsverbindungen einsetzt.

10. Verfahren zur Herstellung von Verbindungen nach einem der Ansprüche 2-8, worin R³ eine aromatische Acylgruppe der allgemeinen Formeln 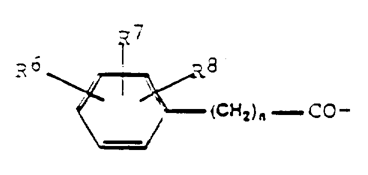 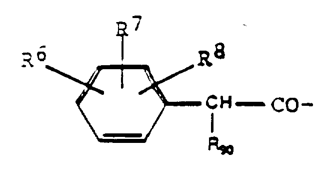 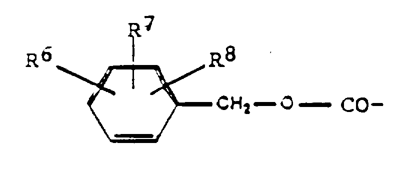 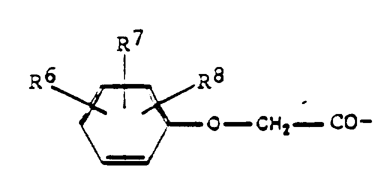 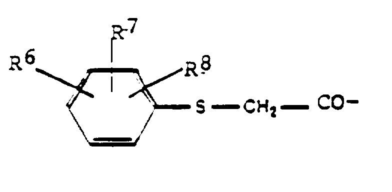 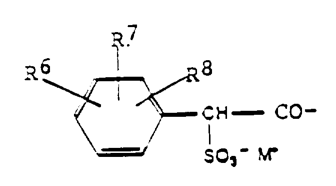 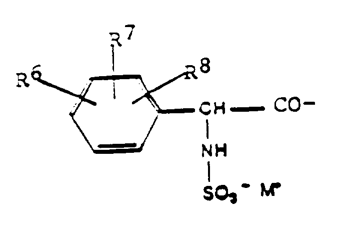 in denen n 0, 1, 2 oder 3; R⁶, R⁷ und R⁸ je Wasserstoff, Halogen, Hydroxy, Nitro, Amino, Cyan, Trifluormethyl, C₁-C₄-Alkyl, C₁-C₄-Alkoxy oder Aminomethyl, R⁹⁰ Amino, Acylamino, Hydroxy, ein Carboxysalz, geschütztes Carboxy, Formyloxy, Azido oder ein Sulfosalz und M ein Kation darstellen,
bedeutet, dadurch gekennzeichnet, dass man entsprechend substituierte Ausgangsverbindungen einsetzt.

11. Verfahren zur Herstellung von Verbindungen nach einem der Ansprüche 2-8, worin R³ eine heteroaromatische Acylgruppe der allgemeinen Formeln
R¹⁰¹-(CH₂)ₙ-CO- 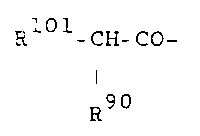
R¹⁰¹-O-CH₂-CO-
R¹⁰¹-S-CH₂-CO-
und
R¹⁰¹-CO-CO-
in denen n 0, 1, 2 oder 3; R⁹⁰ Amino, Acylamino, Hydroxy, ein carboxysalz, geschütztes carboxy, Azido oder ein Sulfosalz und R¹⁰¹ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellt,
bedeutet, dadurch gekennzeichnet, dass man entsprechend substituierte Ausgangsverbindungen einsetzt.

12. Verfahren zur Herstellung von Verbindungen nach einem der Ansprüche 2-8, worin R³ eine [[(4-substituierte-2,3-dioxo-1-piperazinyl)carbonyl]amino]acetylgruppe der Formel 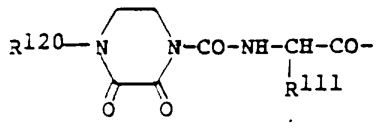 darstellt, in der R¹¹¹ C₁-C₈-Alkyl, Hydroxy-C₁-C₈-Alkyl oder eine aromatische Gruppe der allgemeinen Formel 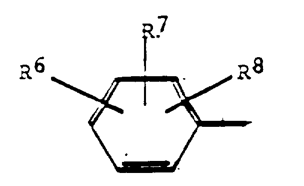 in der R⁶, R⁷ ʼund R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen und R¹⁰ gegebenenfalls durch einen oder mehrere der Reste Halogen, Cyan, Nitro, Amino und/oder Mercapto substituiertes C₁-C₈-Alkyl darstellen;
dadurch gekennzeichnet, dass man entsprechend substituierte Ausgangsverbindungen einsetzt.

13. Verfahren zur Herstellung von Verbindungen nach einem der Ansprüche 2-8, worin R³ eine (Acylamino)acetylgruppe der allgemeinen Formel 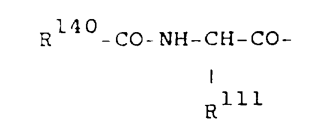 darstellt, worin R¹¹¹ C₁-C₈-Alkyl, Hydroxy-C₁-C₈-Alkyl oder eine aromatische Gruppe der Formel 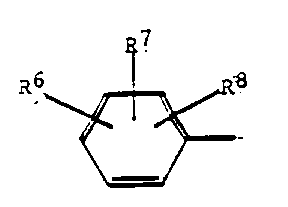 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt, und worin ferner R¹⁴⁰ die Gruppe 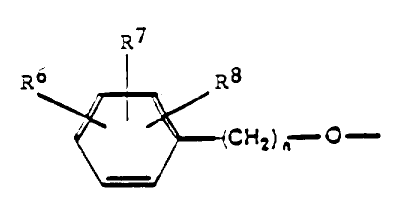 (worin R⁶, R⁷ und R⁸ die obige Bedeutung haben und n 0, 1, 2 oder 3 bedeutet) Wasserstoff, C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkyl)-amino, Hydrazino, C₁-C₈-Alkylhydrazino, Arylhydrazino oder Acylhydrazino darstellt, dadurch gekennzeichnet, dass man entsprechend substituierte Ausgangsverbindungen einsetzt.

14. Verfahren zur Herstellung von Verbindungen nach einem der Ansprüche 2-8, worin R³ eine (substituierte Oxyimino)acetylgruppe der allgemeinen Formel 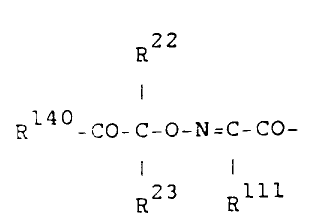 darstellt, worin R¹¹¹ C₁-C₈-Alkyl, Hydroxy-C₁-C₈-Alkyl oder eine aromatische Gruppe der Formel 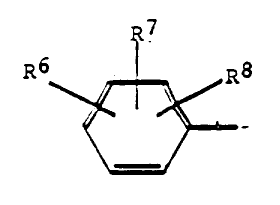 darstellt, worin R⁶, R⁷ und R⁸ Wasserstoff, Halogen, Hydroxy, Nitro Amino, cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt, und worin ferner R¹⁴⁰ die Gruppe 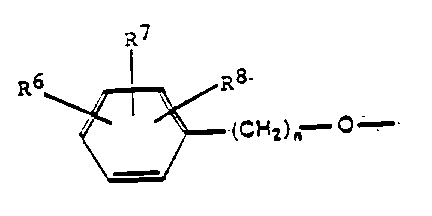 (worin R⁶, R⁷ und R⁸ die obige Bedeutung haben und n 0, 1, 2 oder 3 darstellt), Wasserstoff, C₁-C₈-Alkyl, substituiertes C₁-C₈-Alkyl, Amino, C₁-C₈-Alkylamino, Di-(C₁-C₈-Alkyl)-amino, (Cyan-C₁-C₈-Alkyl)-amino, Hydrazino, C₁-C₈-Alkylhydrazino, Arylhydrazino oder Acylhydrazino und R und R³ Wasserstoff oder C₁-C₈ Alkyl darstellen; oder R und R³ zusammen mit dem benachbarten Kohlenstoffatom einen C₃-C₇ carbocyclischen Ring bilden,
dadurch gekennzeichnet, dass man entsprechend substituierte Ausgangsverbindungen einsetzt.

15. Verfahren zur Herstellung von Verbindungen nach einem der Anspcüche 2-8, worin R³ eine [[[3-substituierte-2-oxo-1-imidazolidinyl]carbonyl]amino]acetylgruppe der allgemeinen Formel 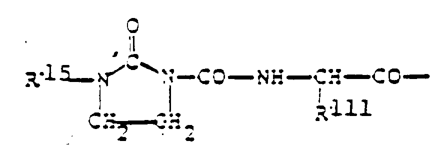 darstellt, worin R¹¹¹ C₁-C₈-Alkyl, Hydroxy-C₁-C₈-Alkyl oder eine aromatische Gruppe der Formel 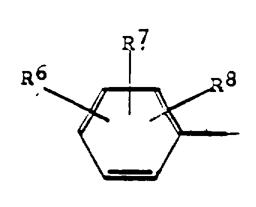 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt; und worin ferner R¹⁵ Wasserstoff, C₁-C₈-Alkylsulfonyl, Arylmethylenamino (d.h. -N=CHR¹¹¹, worin R¹¹¹ die oben gegebene Bedeutung hat), R¹⁶CO- (worin R¹⁶ Wasserstoff oder gegebenenfalls durch Halogen substituiertes C₁-C₈-Alkyl darstellt), eine wie unter R¹¹¹ oben definierte aromatische Gruppe oder gegebenenfalls durch einen oder mehrere der Reste Halogen, Cyan, Nitro, Amino und/oder Mercapto substituiertes C₁-C₈-Alkyl darstellt,
dadurch gekennzeichnet, dass man entsprechend substituierte Ausgangsverbindungen einsetzt.

16. Verfahren zur Herstellung von Verbindungen nach einem der Ansprüche 2-8, worin R³ eine (substituierte Oxyimino)arylacetylgruppe der allgemeinen Formel 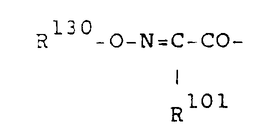 darstellt, worin R¹⁰¹ einen gegebenenfalls substituierten 5, 6- oder 7-gliedrigen heterocyclischen Ring mit 1, 2, 3 oder 4, Stickstoff-, Sauerstoff- und/oder Schwefelatomen darstellt, worin der heterocyclische Ring durch Halogen, Hydroxy, Nitro, Amino, Cyan, Trifluormethyl, C₁-C₄-Alkyl oder C₁-C₄-Alkoxy substituiert ist und R¹³⁰ Wasserstoff, C₁-C₈-Alkyl, C₃-C₇-Cycloalkyl, Carboxy-C₃-C₇-cycloalkyl oder substituiertes C₁-C₈-Alkyl darstellt, wobei die C₁-C₈-Alkylgruppe substituiert ist durch einen oder mehrere der Reste Halogen, Cyan, Nitro, Amino, Mercapto, C₁-C₈-Alkylthio, eine durch R¹¹¹ oben definierte aromatische Gruppe, Carboxy (einschliesslich dessen Salze), C₁-C₇-Alkanoylamino, Carbamoyl, C₂-C₉-Alkoxycabonyl, Phenylmethoxycarbonyl, Diphenylmethoxycarbonyl, Hydroxy-C₁-C₈-Alkoxyphosphinyl, Dihydroxyphophinyl, Hydroxy(phenylmethoxy)phosphinyl oder di-(C₁-C₈-Alkoxy)-phosphinyl, dadurch gekennzeichnet, dass man entsprechend substituierte Ausgangsverbindungen einsetzt.

17. Verfahren zur Herstellung von Verbindungen nach Anspruch 16, worin R¹⁰¹ die Formel 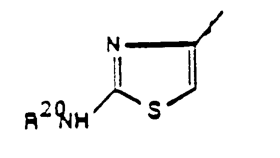 besitzt, worin R⁰ Wasserstoff oder eine Aminoschutzgruppe und R¹³⁰ Wasserstoff, C₁-C₈-Alkyl oder eine Gruppe der allgemeinen Formel 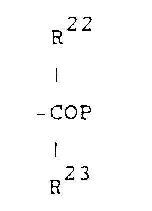 worin R und R³ Wasserstoff oder C₁-C₈-Alkyl oder zusammen mit dem benachbarten Kohlenstoffatom einen C₃-C₇-carbocyclischen Ring darstellen und P Hydroxy oder -NHR¹⁹, worin R¹⁹ Wasserstoff, C₁-C₈-Alkyl, Amino, C₁-C₈-Alkylamino, Arylamino oder Acylamino darstellt, bedeutet,
dadurch gekennzeichnet, dass man entsprechend substituierte Ausgangsverbindungen einsetzt.

18. Verfahren zur Herstellung von Verbindungen nach Anspruch 17, worin R⁰ Wasserstoff und R¹³⁰ Methyl oder eine Gruppe der Formel 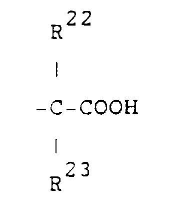 darstellt, worin R und R³ Wasserstoff oder Methyl bedeuten,
dadurch gekennzeichnet, dass man entsprechend substituierte Ausgangsverbindungen einsetzt.

19. Verfahren zur Herstellung von Verbindungen nach einem der Ansprüche 14 und 16-18 in der syn-Form bzw. als Gemische, worin die syn-Form überwiegt, dadurch gekennzeichnet, dass man entsprechend substituierte Ausgangsverbindungen einsetzt.

20. Verfahren zur Herstellung von Verbindungen nach Anspruch 2, worin R¹ die Gruppe (a) und R³ Phenoxyacetyl darstellt, dadurch gekennzeichnet, dass man entsprechend substituierte Ausgangsverbindungen einsetzt.

21. Verfahren zur Herstellung von Verbindungen nach Anspruch 2,
(6R-trans)-4-[3-carboxy-1-(2-fluoräthyl)-6,8-difluor-1,4-dihydro-4-oxochinolin-7-yl]-[[2-carboxy-8-oxo-7-[(phenoxyacetyl)amino]-5-thia-1-azabicyclo[4,2.0]oct-2-en-3-yl]methyl]-1-methylpiperaziniumjodid,
[6R-[6α,7β(Z)]]-1-[[7-[[[(2-Amino-4-thiazolyl) [1-(1-carboxy-1-methyl)äthoxy]imino]acetyl]amino]-2-carboxy-8-oxo-5-thia-1-azabicyclo[4.2.0]oct-2-en-3-yl]methyl]-4-[3-carboxy-1-(2-fluoroäthyl)-6,8-difluor-1,4-dihydro-4-oxochinolin-7-yl]-1-methylpiperaziniumhydroxid-inneres Salz-Mononatriumsalz und
[6R-[6α,7β(Z)]]-1-[[7-[[(2-Amino-4-thiazolyl)(methoxyimino)acetyl]amino]-2-carboxy-8-oxo-5-thia-1-azabicyclo[4.2.0]oct-2-en-3-yl]methyl]-4-(3-carboxy-1-äthyl-6-fluor-1,4-dihydro-4-oxo-7-chinolinyl)-1-methylpiperaziniumjodid, dadurch gekennzeichnet, dass man entsprechend substituierte Ausgangsverbindungen einsetzt.

22. Verfahren zur Herstellung einer Verbindung nach Anspruch 2, [6R-[6α,7β(Z)]]-1-[[[(2-Amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluor-1-(2-fluoräthyl)-1,4-dihydro-4-oxo-7-chinolinyl]-1-methylpiperaziniumjodid, dadurch gekennzeichnet, dass man entsprechend substituierte Ausgangsverbindungen einsetzt.

23. Verfahren zur Herstellung einer Verbindung nach Anspruch 2, [6R-[6α,7β(Z)]]-1-[[[(2-Amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluor-1-(2-fluoräthyl)-1,4-dihydro-4-oxo-7-chinolinyl]-1-methylpiperaziniumhydroxid-inneres Salz-Mononatriumsalz, dadurch gekennzeichnet, dass man entsprechend substituierte Ausgangsverbindungen einsetzt.

24. Verfahren zur Herstellung einer Verbindung nach Ansprucn 2, (6R-trans)-4-[3-Carboxy-6,8-difluor-1-(2-fluoräthyl)-1,4-dihydro-4-oxo-7-chinolinyl]-1-[[2-carboxy-7-(formylamino)-8-oxo-5-thia-1-azabicyclo[4.2.0]oct-2-en-3-yl]methyl]-1-methylpiperaziniumtrifluoracetat, dadurch gekennzeichnet, dass man entsprechend substituierte Ausgangsverbindungen einsetzt.

25. Verfahren zur Herstellung einer Verbindung nach Anspruch 2, [6R-[6α,7β(Z)]]-1-[[7-[[(2-Amino-4-thiazolyl)[[1,1-dimethyl-2-(1,1-dimethyläthoxy)-2-oxo-ethoxy]imino]acetyl]amino]-2-carboxy-8-oxo-5-thia-1-azabicyclo[4.2.0]oct-2-en-3-yl]-methyl]-4-[3-carboxy-(2-fluoräthyl)-6,8-difluoro-1,4-dihydro-4-oxochinolin-7-yl]-1-methylpiperaziniumjodid, dadurch gekennzeichnet, dass man entsprechend substituierte Ausgangsverbindungen einsetzt.

26. Verfahren zur Herstellung von Verbindungen nach Anspruch 1, worin R¹ die Gruppe (c) darstellt, dadurch gekennzeichnet, dass man entsprechend substituierte Ausgangsverbindungen einsetzt.

27. Verfahren zur Herstellung von Verbindungen nach Anspruch 26, worin m 0 oder 1 darstellt, dadurch gekennzeichnet, dass man entsprechend substituierte Ausgangsverbindungen einsetzt.

28. Verfahren zur Herstellung von Verbindungen nach Anspruch 26 oder 27, worin Q eine Gruppe der Formel 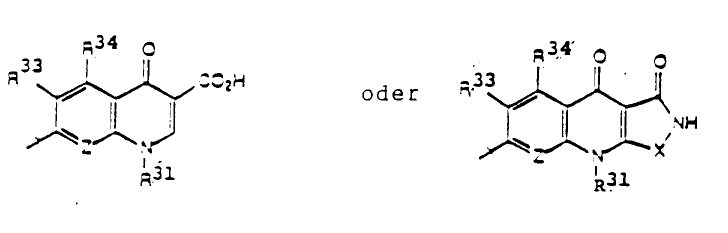 bedeutet, worin Z R³⁰-C oder Stickstoff; X Sauerstoff oder Schwefel; R³⁰ Wasserstoff, Halogen oder eine Oxomethylenbrücke (-CH₂O-) zum Piperazinrest unter Bildung eines ankondensierten 6-gliedrigen Ringes; R³¹ Wasserstoff, C₁-C₈-Alkyl, C₂-C₆-Alkenyl, C₃-C₇-Cycloalkyl, Halogen-C₁-C₈-Alkyl oder Mono-, Di- oder Tri-halogenphenyl; R³⁰ und R³¹ zusammen eine C₃-C₅-Alkylengruppe, eine C₂-C₄-Alkylenmonooxygruppe, eine C₁-C₂-Alkylendioxygruppe oder eine Gruppe der Formel -OCH₂N(CH₃)- ; R³³ Wasserstoff oder Halogen; und R³⁴ Wasserstoff oder Amino darstellen,
dadurch gekennzeichnet, dass man entsprechend substituierte Ausgangsverbindungen einsetzt.

29. Verfahren zur Herstellung von Verbindungen nach Anspruch 28, worin Z die Gruppe R³⁰-C bedeutet, worin R³⁰ Wasserstoff, Chlor, Brom oder Fluor, R³¹ C₁-C₈-Alkyl, Halogen-C₁-C₈-Alkyl oder C₃-C₇-Cycloalkyl und R³³ Wasserstoff, Chlor oder Fluor bedeutet, dadurch gekennzeichnet, dass man entsprechend substituierte Ausgangsverbindungen einsetzt.

30. Verfahren zur Herstellung von Verbindungen nach Anspruch 29, worin R³⁰ Wasserstoff oder Fluor, R³¹ Aethyl, Fluoräthyl oder Cyclopropyl und R³³ Wasserstoff oder Fluor darstellt, dadurch gekennzeichnet, dass man entsprechend substituierte Ausgangsverbindungen einsetzt.

31. Verfahren zur Herstellung von Verbindungen nach Anspruch 28, worin R¹ die nachstehende Formel besitzt: 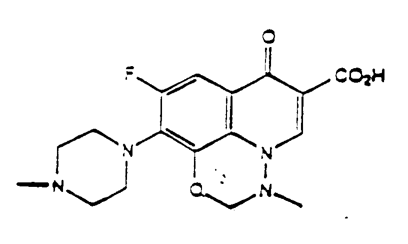 , dadurch gekennzeichnet, dass man entsprechend substituierte Ausgangsverbindungen einsetzt.

32. Verfahren zur Herstellung von Verbindungen nach Anspruch 28, worin R¹ die nachstehende Formel besitzt: 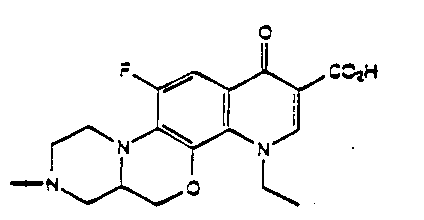 , dadurch gekennzeichnet, dass man entsprechend substituierte Ausgangsverbindungen einsetzt.

33. Verfahren zur Herstellung von Verbindungen nach einem der Ansprüche 26-32, worin R³ eine aliphatische Acylgruppe der Formel
R⁵-CO-
in der R⁵ Wasserstoff, C₁-C₈-Alkyl, C₃-C₇-Cycloalkyl, C₁-C₈-Alkoxy, C₂-C₆-Alkenyl, C₃-C₇-Cycloalkenyl oder Cyclohexadienyl; oder durch einen oder mehrere der Reste Halogen, Cyan, Nitro, Amino, Mercapto, C₁-C₈-Alkylthio oder Cyanmethylthio substituiertes C₁-C₈-Alkyl oder C₂-C₆-Alkenyl darstellt,
bedeutet, dadurch gekennzeichnet, dass man entsprechend substituierte Ausgangsverbindungen einsetzt.

34. Verfahren zur Herstellung von Verbindungen nach einem der Ansprüche 26-32, worin R³ eine aromatische Acylgruppe der allgemeinen Formeln 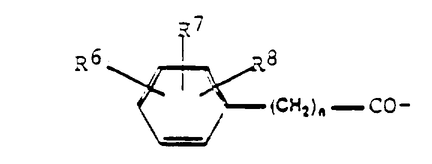 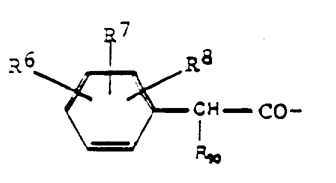 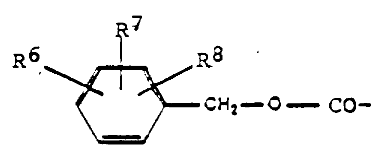 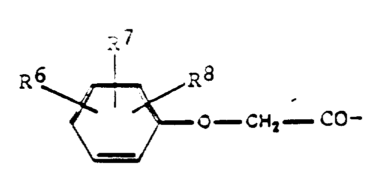 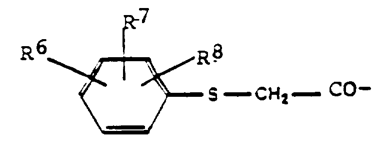 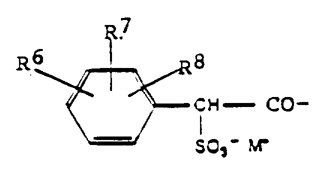 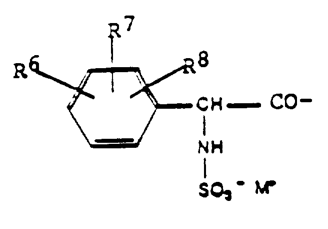 in denen n 0, 1, 2 oder 3; R⁶, R⁷ und R⁸ je Wasserstoff, Halogen, Hydroxy. Nitro, Amino, Cyan, Trifluormethyl, C₁-C₄-Alkyl, C₁-C₄-Alkoxy oder Aminomethyl, R⁹⁰ Amino, Acylamino, Hydroxy, ein Carboxysalz, geschütztes Carboxy, Formyloxy, Azido oder ein Sulfosalz und M ein Kation darstellen,
bedeutet, dadurch gekennzeichnet, dass man entsprechend substituierte Ausgangsverbindungen einsetzt.

35. Verfahren zur Herstellung von Verbindungen nach einem der Ansprüche 26-32, worin R³ eine heteroaromatische Acylgruppe der allgemeinen Formeln
R¹⁰¹- (CH₂)ₙ-CO- 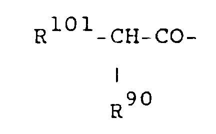
R¹⁰¹-O-CH₂-CO-
R¹⁰¹-S-CH₂-CO-
und
R¹⁰¹-CO-CO-
in denen n 0, 1, 2 oder 3; R⁹⁰ Amino, Acylamino, Hydroxy, ein Carboxysalz, geschütztes Carboxy, Azido oder ein Sulfosalz und R¹⁰¹ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellt,
bedeutet, dadurch gekennzeichnet, dass man entsprechend substituierte Ausgangsverbindungen einsetzt.

36. Verfahren zur Herstellung von Verbindungen nach einem der Ansprüche 26-32, worin R³ eine [[(4-substituierte-2,3-dioxo-1-piperazinyl)carbonyl]amino]acetylgruppe der Formel 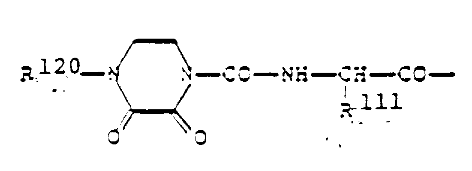 darstellt, in der R¹¹ C₁-C₈-Alkyl, Hydroxy-C₁-C₈-Alkyl oder eine aromatische Gruppe der allgemeinen Formel 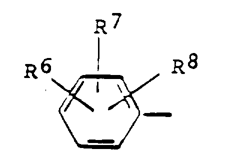 in der R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen und R¹⁰ gegebenenfalls durch einen oder mehrere der Reste Halogen, Cyan, Nitro, Amino und/oder Mercapto substituiertes C₁-C₈-Alkyl darstellen,
dadurch gekennzeichnet, dass man entsprechend substituierte Ausgangsverbindungen einsetzt.

37. Verfahren zur Herstellung von Verbindungen nach einem der Ansprüche 26-32, worin R³ eine (Acylamino)acetylgruppe der allgemeinen Formel 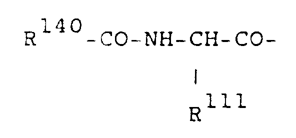 darstellt, worin R¹¹¹ C₁-C₈-Alkyl, Hydroxy-C₁-C₈-Alkyl oder eine aromatische Gruppe der Formel 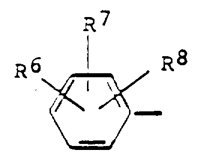 darstellt, worin R⁶, R⁷ und R⁸ Wasserstoff, Halogen, Hydroxy, Nitro, Amino, cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt, und worin ferner R¹⁴⁰ die Gruppe 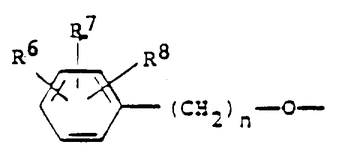 (worin R⁶, R⁷ und R⁸ die obige Bedeutung haben und n 0, 1, 2 oder 3 bedeutet) Wasserstoff C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkyl)-amino, Hydrazino, C₁-C₈-Alkylhydrazino, Arylhydrazino oder Acylhydrazino darstellt,
dadurch gekennzeichnet, dass man entsprechend substituierte Ausgangsverbindungen einsetzt.

38. Verfahren zur Herstellung von Verbindungen nach einem der Ansprüche 26-32, worin R³ eine (substituierte Oxyimino)acetylgruppe der allgemeinen Formel 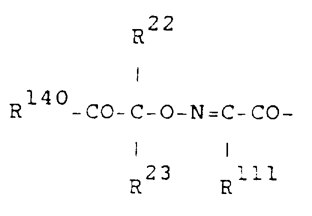 darstellt, worin R¹¹¹ C₁-C₈-Alkyl, Hydroxy-C₁-C₈-Alkyl oder eine aromatische Gruppe der Formel 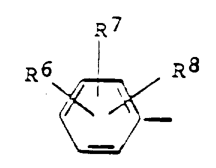 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy oder Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt, und worin ferner R¹⁴⁰ die Gruppe 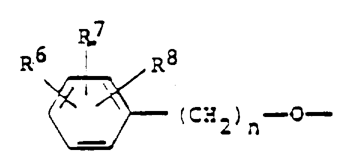 (worin R⁶, R⁷ und R⁸ die obige Bedeutung haben und n 0, 1, 2 oder 3 darstellt), Wasserstoff, C₁-C₈-Alkyl, substituiertes C₁-C₈-Alkyl, Amino, C₁-C₈-Alkylamino, di-(C₁-C₈-Alkyl)-amino, (Cyan-C₁-C₈-Alkyl)-amino, Hydrazino, C₁-C₈-Alkylhydrazino, Arylhydrazino oder Acylhydrazino und R und R³ Wasserstoff oder C₁-C₈-Alkyl darstellen, oder R und R³ zusammen mit dem benachbarten Kohlenstoffatom einen C₃-C₇ carbocyclischen Ring bilden,
dadurch gekennzeichnet, dass man entsprechend substituierte Ausgangsverbindungen einsetzt.

39. Verfahren zur Herstellung von Verbindungen nach einem der Ansprüche 26-32, worin R³ eine [[[3-substituierte-2-oxo-1-imidazolidinyl]cacbonyl]amino]acetylgruppe der allgemeinen Formel 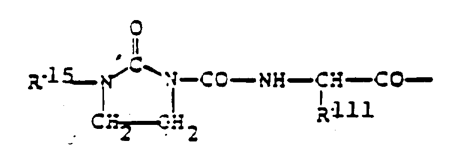 darstellt, worin R¹¹¹ C₁-C₈-Alkyl, Hydroxy-C₁-C₈-Alkyl oder eine aromatische Gruppe der Formel 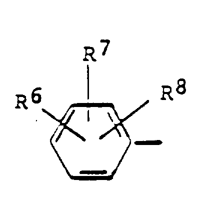 darstellt, worin R⁶, R⁷ und R⁸ Wasserstoff, Halogen, Hydroxy, Nitro, Amino, Cyan, Trifluormethyl, C₁-C₄-Alkyl, C₁-C₄-Alkoxy, Aminomethyl oder einen gegebenenfalls substituierten 5-, 6- oder 7-gliedrigen heterocyclischen Ring mit 1, 2, 3 oder 4, Stickstoff-, Sauerstoff- und/oder Schwefelatomen darstellt; und worin ferner R¹⁵ Wasserstoff, C₁-C₈-Alkylsulfonyl, Arylmethylenamino (d.h. -N=CHR¹¹¹, worin R¹¹¹ die oben gegebene Bedeutung hat), R¹⁶CO- (worin R¹⁶ Wasserstoff oder gegebenenfalls durch Halogen substituiertes C₁-C₈-Alkyl darstellt), eine wie unter R¹¹¹ oben definierte aromatische Gruppe oder gegebenenfalls durch einen oder mehrere der Reste Halogen, Cyan, Nitro, Amino und/oder Mercapto substituiertes C₁-C₈-Alkyl darstellt,
dadurch gekennzeichnet, dass man entsprechend substituierte Ausgangsverbindungen einsetzt.

40. Verfahren zur Herstellung von Verbindungen nach einem der Ansprüche 26-32, worin R³ eine (substituierte Oxyimino)arylacetylgruppe der allgemeinen Formel 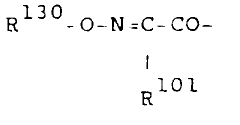 darstellt, worin R¹⁰¹ einen gegebenenfalls substituierten 5, 6- oder 7-gliedrigen heterocyclischen Ring mit 1, 2, 3 oder 4, Stickstoff-, Sauerstoff- und/oder Schwefelatomen darstellt, worin der heterocyclische Ring durch Halogen, Hydroxy, Nitro, Amino, Cyan, Trifluormethyl, C₁-C₄-Alkyl oder C₁-C₄-Alkoxy substituiert ist und R¹³⁰ Wasserstoff, C₁-C₈-Alkyl, C₃-C₇-Cycloalkyl, Carboxy-C₃-C₇-cycloalkyl oder substituiertes C₁-C₈-Alkyl darstellt, wobei die C₁-C₈-Alkylgruppe substituiert ist durch einen oder mehrere der Reste Halogen, Cyan, Nitro, Amino Mercapto, C₁-C₈-Alkylthio, eine durch R¹¹¹ oben definierte aromatische Gruppe, Carboxy (einschliesslich dessen Salze), C₁-C₇-Alkanoylamino, Carbamoyl, C₂-C₉-Alkoxycabonyl, Phenylmethoxycarbonyl, Diphenylmethoxycarbonyl, Hydroxy-C₁-C₈-Alkoxyphosphinyl, Dihydroxyphosphinyl, Hydroxy(phenylmethoxy)phosphinyl oder di-(C₁-C₈-Alkoxy)-phosphinyl,
dadurch gekennzeichnet, dass man entsprechend substituierte Ausgangsverbindungen einsetzt.

41. Verfahren zur Herstellung von Verbindungen nach Anspruch 40, worin R¹⁰¹ die Formel 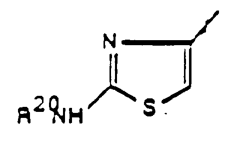 besitzt, worin R⁰ Wasserstoff oder eine Aminoschutzgruppe und R¹³⁰ Wasserstoff, C₁-C₈-Alkyl oder eine Gruppe der allgemeinen Formel 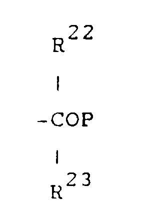 worin R und R³ Wasserstoff oder C₁-C₈-Alkyl oder zusammen mit dem benachbarten Kohlenstoffatom einen C₃-C₇-carbocyclischen Ring darstellen und P Hydroxy oder -NHR¹⁹, worin R¹⁹ Wasserstoff, C₁-C₈-Alkyl, Amino, C₁-C₈-Alkylamino, Arylamino oder Acylamino darstellt, bedeutet,
dadurch gekennzeichnet, dass man entsprechend substituierte Ausgangsverbindungen einsetzt.

42. Verfahren zur Herstellung von Verbindungen nach Anspruch 41, worin R⁰ Wasserstoff oder Triphenylmethyl darstellt, dadurch gekennzeichnet, dass man entsprechend substituierte Ausgangsverbindungen einsetzt.

43. Verfahren zur Herstellung von Verbindungen nach einem der Ansprüche 38 und 40-42 in der syn-Form bzw. als Gemische, worin die syn-Form überwiegt, dadurch gekennzeichnet, dass man entsprechend substituierte Ausgangsverbindungen einsetzt.

44. Verfahren zur Herstellung von Verbindungen nach Anspruch 26,
[6R-[6α,7β(Z)]]-7-[[(2-Amino-4-thiazolyl)(methoxyimino)acetyl]amino]-3-[[[[4-(3-carboxy-1-äthyl-6-fluor-1,4-dihydro-4-oxo-7-chinolinyl)-1-piperazinyl]carbonyl]oxy]methyl]-8-oxo-5-thia-1-azabicycLo[4,2.0]oct-2-en-2-carbonsäure,
[6R-[6α,7β(Z)]]-7-[[(2-Amino-4-thiazolyl)methoxyimino)acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]-oct-2-en-2-carbonsäure,
(6R-trans)-3[[[[4-(3-Carboxy-1-cyclopropyl-6-fluor-1,4-dihydro-4-oxo-7-chinolinyl)-1-piperazinyl]carbonyl]oxy]methyl]-8-oxo-7-[(phenoxyacetyl)amino]-5-thia-1-azabicyclo[4.2.0]oct-2-en-2-carbonsäure,
[6R-[6α,7β(Z)]]-7-[[(2-Amino-4-thiazolyl)-[(1-carboxy-1-methyl-äthoxy)imino]acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4,2.0]oct-2-en-2-carbonsäure,
[6R-[6α,7β(Z)]]-7-[[(2-Amino-4-thiazolyl)-[(carboxymethoxy)imino]acetyl]amino]-3-[[[[4-(3-carboxy-1-cyclopropyl-6-fluor-1,4-dihydro-4-oxo-7-chinolinyl)-1-piperazinyl]carbonyl]oxy]methyl]-8-oxo-5-thia-1-azabicyclo[4.2.0]oct-2-en-2-carbonsäure,
[6R-[6α,7β(Z)]]-7-[[(2-Amino-4-thiazolyl)(methoxyimino)acetyl]amino]-3-[[[[4-(9-cyclopropyl-6-fluoro-2,3,4,9-tetrahydro-3,4-dioxoisothiazolo[5,4-b]chinolin-7-yl)-1-piperazinyl]carbonyl]oxy]methyl]-8-oxo-5-thia-1-azabicyclo[4.2.0]oct-2-en-2-carbonsäure und
[6R-[6α,7β(Z)]]-7-[[(2-Amino-4-thiazolyl)(methoxyimino)acetyl]amino]-3-[[[[4-(9-cyclopropyl-6-fluoro-2,3,4,9-tetrahydro-3,4-dioxoisoxazolo[5,4-b]chinolin-7-yl)-1-piperazinyl]carbonyl]oxy]methyl]-8-oxo-5-thia-1-azabicyclo[4.2.0] oct-2-en-2-carbonsäure
sowie von Salzen dieser Verbindungen und von Hydraten dieser Verbindungen und Salze, dadurch gekennzeichnet, dass man entsprechend substituierte AUsgangsverbindungen einsetzt.

45. Verfahren zur Herstellung von Natriumsalzen der Verbindungen gemäss Anspruch 44, dadurch gekennzeichnet, dass man entsprechend substituierte Ausgangsverbindungen einsetzt.

46. Verfahren nach einem der Ansprüche 1-45, dadurch gekennzeichnet, dass man die Verfahrensalternative (b) ausführt.

47. Verfahren nach einem der Ansprüche 1-45, dadurch gekennzeichnet, dass man eine der Verfahrensalternativen (a), (c), (d), (f), (g) und (i) ausführt.

48. Verfahren zur Herstellung von pharmazeutischen Präparaten, dadurch gekennzeichnet, das man ein in Anspruch 1 definiertes Acylderivat der Formel I oder einen leicht hydrolysierbaren Ester oder ein Salz dieser Verbindung oder ein Hydrat einer Verbindung der Formel I bzw. eines Esters oder Salzes davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

49. Verwendung von Verbindungen gemäss einem der Ansprüche 1-47 bei der Herstellung von Arzneimitteln für die Behandlung bzw. Prophylaxe von Infektionskrankheiten.

50. Verbindungen der Formel 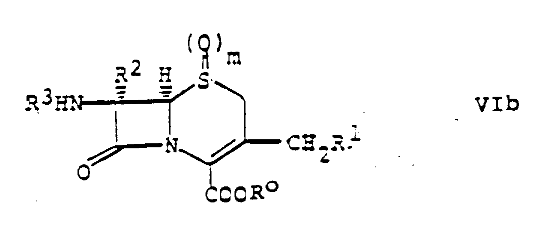 worin R¹, R oder R³ und m die oben gegebene Bedeutung haben und R^{o} t-Butyl, Benzhydryl, p-Nitrobenzyl oder Allyl darstellt.

51. (6R-trans)-4-[3-Carboxy-6,8-difluor-1-(2-fluoräthyl)-1,4-dihydro-4-oxo-7-chinolinyl]-1-[[2-(1,1-dimethyläthoxy)carbonyl-7-(formylamino)-8-oxo-5-thia-1-azabicyclo[4.2.0]oct-2-en-3-yl]methyl]-1-methylpiperaziniumjodid.

52. Verbindungen der Formel 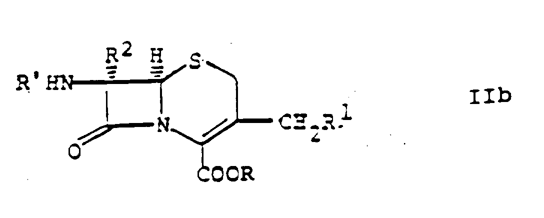 worin R¹ und R die oben gegebene Bedeutung haben, R Wasserstoff oder eine Carbonsäureschutzgruppe und R' Wasserstoff oder eine leicht abspaltbare Aminoschutzgruppe darstellt.

53. (6R-trans)-3-[[[[4-(3-Carboxy-1-cyclopropyl-6-fluor-1,4-dihydro-4-oxo-7-chinolinyl)-1-piperazinyl]carbonyl]oxy]methyl]-7-[[(1,1-dimethyläthoxy)carbonyl]amino]-8-oxo-5-thia-1-azabicyclo[4.2.0]oct-2-en-2-carbonsäurediphenylmethylester.

## Claims (Claims for the following Contracting State(s): AT, BE, CH, DE, FR, GB, IT, LI, LU, NL, SE)

1. Acyl derivatives of the general formula 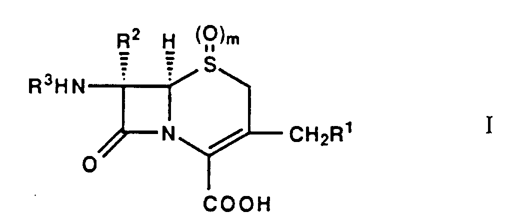 in which R¹ represents a substituted N-heterocycle of the formulae 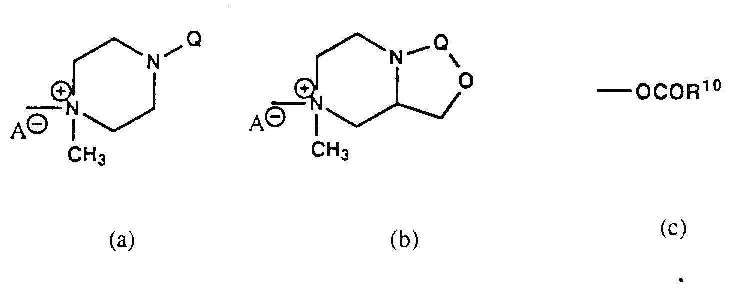 wherein R¹⁰ represents one of the groups 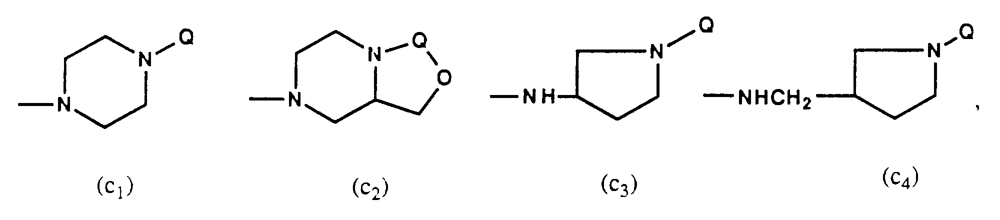
and Q represents a substituted quinolinyl or naphthyridinyl group, whereby the N-heterocycle can be optionally substituted by one or more C₁-C₈-alkyl groups; and wherein R represents hydrogen, C₁-C₈-alkoxy, C₁-C₈-alkylthio or C₁-C₇-alkanoylamino, R³ represents an acyl group, m represents 0, 1 or 2 and A represents a pharmaceutically acceptable anion,
as well as readily hydrolyzable esters and pharmaceutically acceptable salts of these compounds and hydrates of the compounds of formula I or of their esters and salts.

2. Compounds according to claim 1, characterized in that R¹ represents one of groups (a) and (b).

3. Compounds according to claim 2, characterized in that m represents 0 and R represents hydrogen.

4. Compounds according to claim 2 or 3, characterized in that R¹ has the following formula: 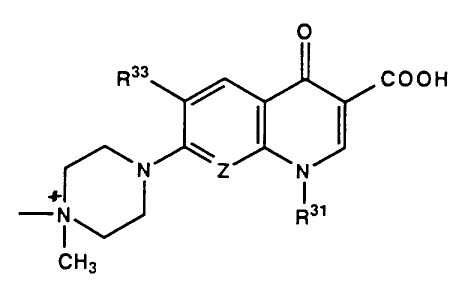 wherein Z represents R³⁰-C or nitrogen; R³⁰ represents hydrogen, halogen or an oxymethylene (-CH₂O-) bridge to the piperazine residue with the formation of a fused 6-membered ring; R³¹ represents hydrogen, C₁-C₈-alkyl, C₂-C₆-alkenyl, C₃-C₇-cycloalkyl, halo-C₁-C₈-alkyl- or mono-, di- or tri-halophenyl; or R³⁰ and R³¹ together represent a C₃-C₅-alkylene group, a C₂-C₄-alkylenemonooxy group, a C₁-C₂-alkylenedioxy group or a group of the formula -OCH₂N(CH₃)-; and R³³ represents hydrogen or halogen.

5. Compounds according to claim 4, characterized in that Z represents the group R³⁰-C in which R³⁰ signifies hydrogen, bromine, chlorine or fluorine, R³¹ signifies C₁-C₈-alkyl, halo-C₁-C₈-alkyl or C₃-C₇-cycloalkyl and R³³ signifies hydrogen, chlorine or fluorine.

6. Compounds according to claim 5, characterized in that R³⁰ represents hydrogen or fluorine, R³¹ represents ethyl, fluoroethyl or cyclopropyl and R³³ represents hydrogen or fluorine.

7. Compounds according to claim 2 or 3, characterized in that R¹ has the following formula:

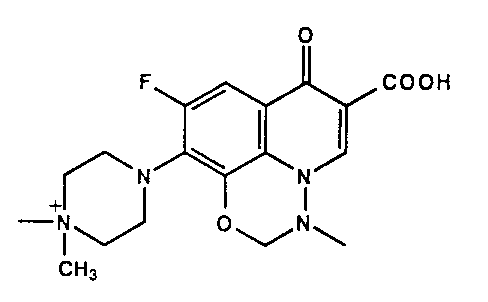

8. Compounds according to claim 2 or 3, characterized in that R¹ has the following formula:

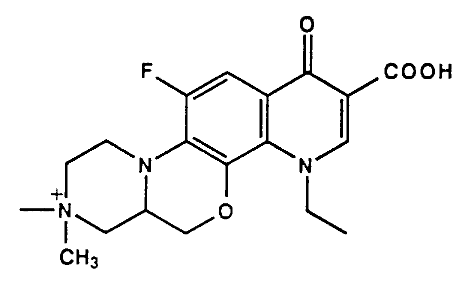

9. Compounds according to any one of claims 2-8, characterized in that R³ signifies an aliphatic acyl group of the formula
R⁵-CO-
in which R⁵ represents hydrogen, C₁-C₈-alkyl, C₃-C₇-cycloalkyl, C₁-C₈-alkoxy, C₂-C₆-alkenyl, C₃-C₇-cycloalkenyl or cyclohexadienyl; or C₁-C₈-alkyl or C₂-C₆-alkenyl substituted by one or more halogen, cyano, nitro, amino, mercapto, C₁-C₈-alkylthio or cyanomethylthio residues.

10. Compounds according to any one of claims 2-8, characterized in that R³ signifies an aromatic acyl group of the general formulae 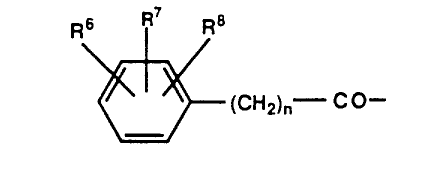 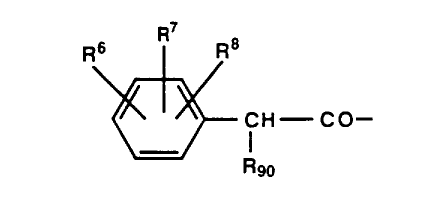 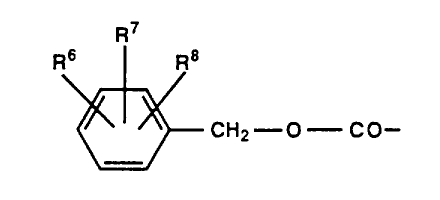 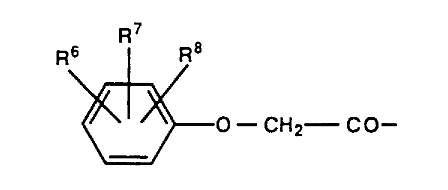 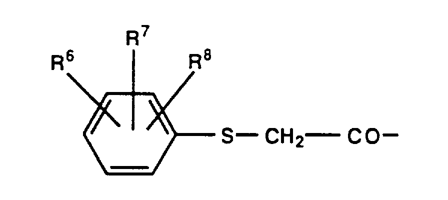 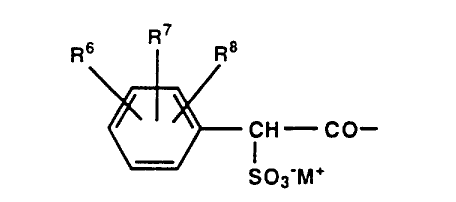 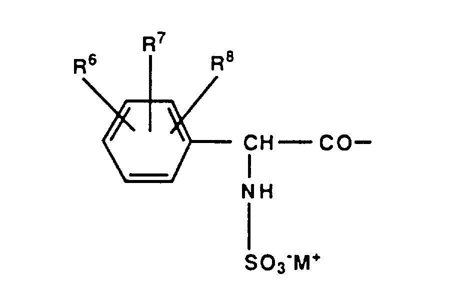 in which n represents 0, 1, 2 or 3; R⁶, R⁷, and R⁸ each represent hydrogen, halogen, hydroxy, nitro, amino, cyano, trifluoromethyl, C₁-C₄-alkyl, C₁-C₄-alkoxy or aminomethyl, R⁹⁰ represents amino, acylamino, hydroxy, a carboxy salt, protected carboxy, formyloxy, azido or a sulpho salt and M represents a cation.

11. Compounds according to any one of claims 2-8, characterized in that R³ signifies a heteroaromatic acyl group of the general formulae
R¹⁰¹-(CH₂)ₙ-CO- 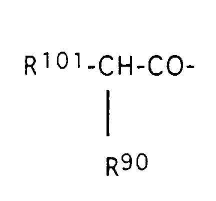
R¹⁰¹-O-CH₂-CO-
R¹⁰¹-S-CH₂-CO-
and
R¹⁰¹-CO-CO-
in which n represents 0, 1, 2 or 3; R⁹⁰ represents amino, acylamino, hydroxy, a carboxy salt, protected carboxy, azido or a sulpho salt and R¹⁰¹ represents an optionally substituted 5-, 6- or 7-membered heterocyclic ring with 1, 2, 3 or 4 nitrogen, oxygen and/or sulphur atoms.

12. Compounds according to any one of claims 2-8, characterized in that R³ represents a [[(4-substituted-2,3-dioxo-1-piperazinyl)carbonyl]amino]acetyl group of the formula 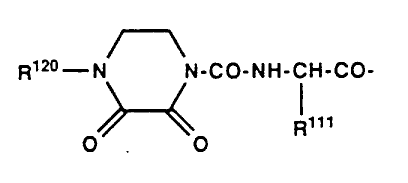 in which R¹¹¹ represents C₁-C₈-alkyl, hydroxy-C₁-C₈-alkyl or an aromatic group of the general formula 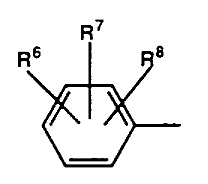 in which R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms and R¹⁰ represents C₁-C₈-alkyl optionally substituted by one or more halogen, cyano, nitro, amino and/or mercapto residues.

13. Compounds according to any one of claims 2-8, characterized in that R³ represents an (acylamino)acetyl group of the general formula 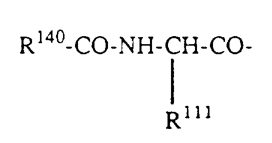 wherein R¹¹¹ represents C₁-C₈-alkyl, hydroxy-C₁-C₈-alkyl or an aromatic group of the formula 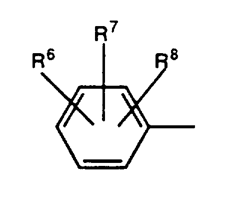 in which R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 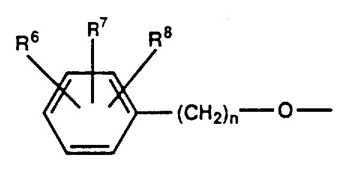 (wherein R⁶, R⁷ and R⁸ have the above significance and n signifies 0, 1, 2 or 3) hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino.

14. Compounds according to any one of claims 2-8, characterized in that R³ represents a (substituted oxyimino)acetyl group of the general formula 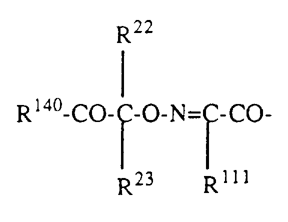 wherein R¹¹¹ represents C₁-C₈-alkyl, hydroxy-C₁-C₈-alkyl or an aromatic group of the formula 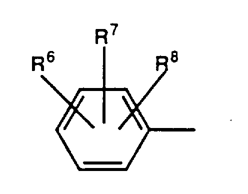 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 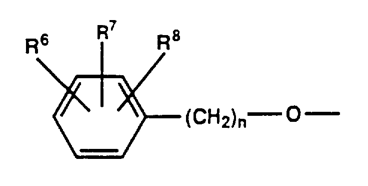 (wherein R⁶, R⁷ and R⁸ have the above significance and n represents 0, 1, 2 or 3), hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino and R and R³ represent hydrogen or C₁-C₈-alkyl; or R and R³ together with the adjacent carbon atom form a C₃-C₇ carbocyclic ring.

15. Compounds according to any one of claims 2-8, characterized in that R³ represents a [[[3-substituted-2-oxo-1-imidazolidinyl]carbonyl]amino]acetyl group of the general formula 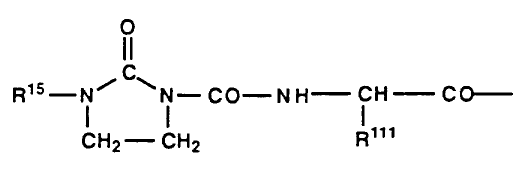 wherein R¹¹¹ represents C₁-C₈-alkyl, hydroxy-C₁-C₈-alkyl or an aromatic group of the formula 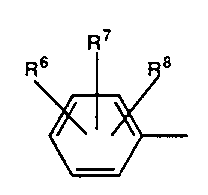 wherein R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms; and wherein R¹⁵ represents hydrogen, C₁-C₈-alkylsulphonyl, arylmethyleneamino (i.e. -N=CHR¹¹¹ wherein R¹¹¹ has the significance given above), R¹⁶CO - (wherein R¹⁶ represent hydrogen or C₁-C₈-alkyl optionally substituted by halogen), an aromatic group as defined above under R¹¹¹ or C₁-C₈-alkyl optionally substituted by one or more halogen, cyano, nitro, amino and/or mercapto residues.

16. Compounds according to any one of claims 2-8, characterized in that R³ represents a (substituted oxyimino)arylacetyl group of the general formula 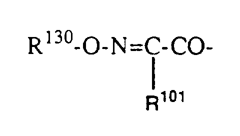 wherein R¹⁰¹ represents an optionally substituted 5, 6- or 7-membered heterocyclic ring with 1, 2, 3 or 4 nitrogen, oxygen and/or sulphur atoms, wherein the heterocyclic ring is substituted by halogen, hydroxy, nitro, amino, cyano, trifluoromethyl, C₁-C₄-alkyl or C₁-C₄-alkoxy, and R¹³⁰ represents hydrogen, C₁-C₈-alkyl, C₃-C₇-cycloalkyl, carboxy-C₃-C₇-cycloalkyl or substituted C₁-C₈-alkyl whereby the C₁-C₈-alkyl group is substituted by one or more halogen, cyano, nitro, amino, mercapto, C₁-C₈-alkylthiol an aromatic group defined above by R¹¹¹, carboxy (including salts thereof), C₁-C₇-alkanoylamino, carbamoyl, C₂-C₉-alkoxycarbonyl, phenylmethoxycarbonyl, diphenylmethoxycarbonyl, hydroxyC₁-C₈-alkoxyphosphinyl, dihydroxyphosphinyl, hydroxy(phenylmethoxy)phosphinyl or di-(C₁-C₈-alkoxy)-phosphinyl residues.

17. Compounds according to claim 16, characterized in that R¹⁰¹ has the formula 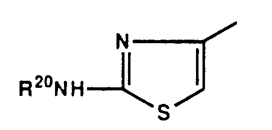 wherein R⁰ signifies hydrogen or an amino protecting group and R¹³⁰ signifies hydrogen, C₁-C₈-alkyl or a group of the formula 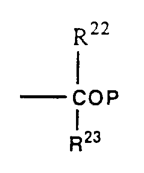 wherein R and R³ signify hydrogen or C₁-C₈-alkyl or together with the adjacent carbon atom signify a C₃-C₇-carbocyclic ring and P signifies hydroxy or -NHR¹⁹, wherein R¹⁹ represents hydrogen, C₁-C₈-alkyl amino, C₁-C₈-alkylamino, arylamino or acylamino.

18. Compounds according to claim 17, characterized in that R⁰ signifies hydrogen and R¹³⁰ signifies methyl or a group of the formula 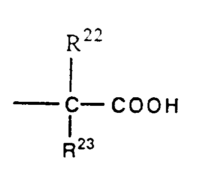 wherein R and R³ represent hydrogen or methyl.

19. Compounds according to any one of claims 14 and 16-18 in the syn form or as a mixture in which the syn form predominates.

20. Compounds according to claim 2, characterized in that R¹ represents group (a) and R³ represents phenoxyacetyl.

21. Compounds according to claim 2, characterized as
(6R-trans)-4-[3-carboxy-1-(2-fluoroethyl)-6,8-difluoro-1,4-dihydro-4-oxoquinolin-7-yl]-[[2-carboxy-8-oxo-7-[(phenoxyacetyl)amino]-5-thia-1-azabicyclo[4.2.0]oct-2-en-3-yl]methyl]-1-methylpiperazinium iodide,
[6R-[6α,7β(Z)]]-1 -[[7-[[[(2-amino-4-thiazolyl)[1-(1-carboxy-1-methyl)ethoxy]imino]acetyl]amino]-2-carboxy-8-oxo-5-thia-1-azabicyclo[4.2.0]oct-2-en-3-yl]methyl]-4-[3-carboxy-1-(2-fluoroethyl)-6,8-difluoro-1,4-dihydro-4-oxoquinolin-7-yl]-1-methylpiperazinium hydroxide inner salt monosodium salt and
[6R-[6α,7β(Z)]]-1-[[7-[[(2-amino-4-thiazolyl)(methoxyimino)acetyl]amino]-2-carboxy-8-oxo-5-thia-1-azabicyclo[4.2.0]oct-2-en-3-yl]methyl]-4-(3-carboxy-1-ethyl-6-fluoro-1,4-dihydro-4-oxo-7-quinolinyl)-1-methylpiperazinium iodide.

22. [6R-[6α,7β(Z)]]-1-[[[(2-Amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluoro-1-(2-fluoroethyl)-1,4-dihydro-4-oxo-7-quinolinyl]-1-methylpiperazinium iodide.

23. [6R-[6α,7β(Z)]]-1-[[[(2-Amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluoro-1-(2-fluoroethyl)-1,4-dihydro-4-oxo-7-quinolinyl]-1-methylpiperazinium hydroxide inner salt monosodium salt.

24. [6R-trans)-4-[3-Carboxy-6,8-difluoro-1-(2-fluoroethyl)-1,4-dihydro-4-oxo-7-quinolinyl]-1-[[2-carboxy-7-(formylamino)-8-oxo-5-thia-1-azabicyclo[4.2.0]oct-2-en-3-yl]-methyl-1-methylpiperazinium trifluoroacetate.

25. [6R-[6α,7β(Z)]]-1-[[7-[[(2-Amino-4-thiazolyl)[[1,1-dimethyl-2-(1,1-dimethylethoxy)-2-oxo-ethoxy]imino]acetyl]amino]-2-carboxy-8-oxo-5-thia-1-azabicyclo[4.2.0]oct-2-en-3-yl]-methyl]-4-[3-carboxy-(2-fluoroethyl)-6,8-difluoro-1,4-dihydro-4-oxoquinolin-7-yl]-1-methylpiperazinium iodide.

26. Compounds according to claim 1, characterized in that R¹ represents group (c).

27. Compounds according to claim 26, characterized in that m represents 0 or 1.

28. Compounds according to claim 26 or 27, characterized in that Q signifies a group of the formula 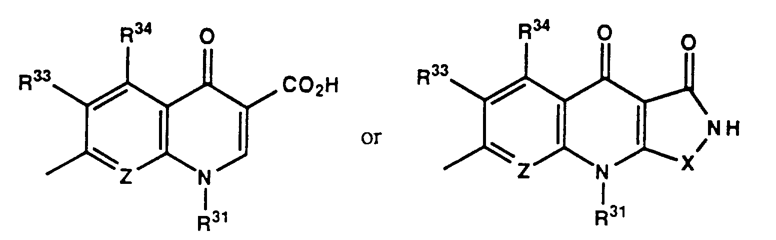 wherein Z represents R³⁰-C or nitrogen; X represents oxygen or sulphur; R³⁰ represents hydrogen, halogen or an oxymethylene (-CH₂O-) bridge to the piperazine residue with the formation of a fused 6-membered ring; R³¹ represents hydrogen, C₁-C₈-alkyl, C₂-C₆-alkenyl, C₃-C₇-cycloalkyl, halo-C₁-C₈-alkyl or mono-, di- or tri-halophenyl; or R³⁰ and R³¹ together represent a C₃-C₅-alkylene group, a C₂-C₄-alkylenemonooxy group, a C₁-C₂-alkylenedioxy group or a group of the formula -OCH₂N(CH₃)-; R³³ represents hydrogen or halogen; and R³⁴ represents hydrogen or amino.

29. Compounds according to claim 28, characterized in that Z signifies the group R³⁰-C, wherein R³⁰ signifies hydrogen, chlorine, bromine or fluorine, R³¹ signifies C₁-C₈-alkyl, halo-C₁-C₈-alkyl or C₃-C₇-cycloalkyl and R³³ signifies hydrogen, chlorine or fluorine.

30. Compounds according to claim 29, characterized in that R³⁰ represents hydrogen or fluorine, R³¹ represents ethyl, fluoroethyl or cyclopropyl and R³³ represents hydrogen or fluorine.

31. Compounds according to claim 28, characterized in that R¹ has the following formula:

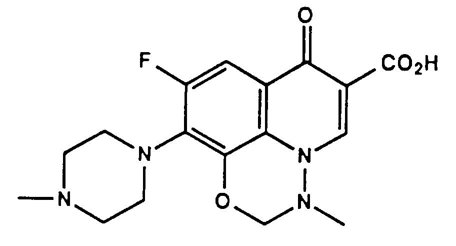

32. Compounds according to claim 28, characterized in that R¹ has the following formula:

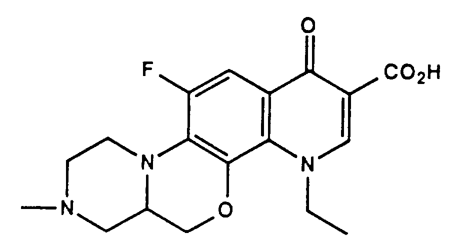

33. Compounds according to any one of claims 26-32, characterized in that R³ signifies an aliphatic acyl group of the formula the
R⁵-CO-
in which R⁵ represents hydrogen, C₁-C₈-alkyl, C₃-C₇-cycloalkyl, C₁-C₈-alkoxy, C₂-C₆-alkenyl, C₃-C₇-cycloalkenyl or cyclohexadienyl; or C₁-C₈-alkyl or C₂-C₆-alkenyl substituted by one or more halogen, cyano, nitro, amino, mercapto, C₁-C₈-alkylthio or cyanomethylthio residues.

34. Compounds according to any one of claims 26-32, characterized in that R³ signifies an aromatic acyl group of the general formulae 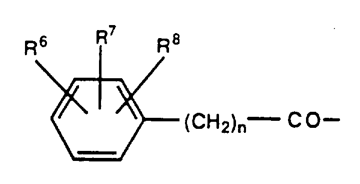 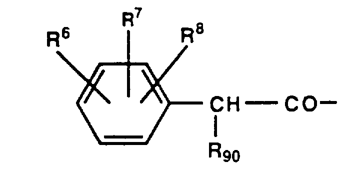 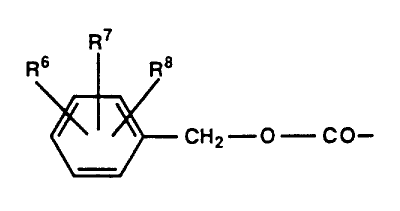 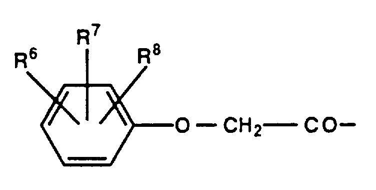 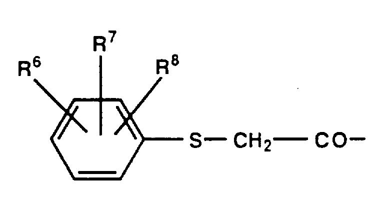 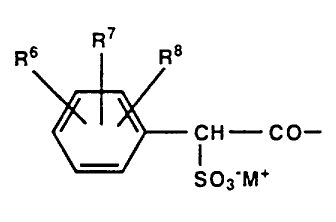 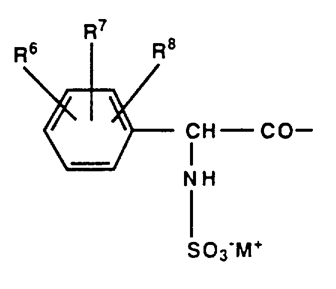 in which n represents 0, 1, 2 or 3; R⁶, R⁷, and R⁸ each represent hydrogen, halogen, hydroxy, nitro, amino, cyano, trifluoromethyl, C₁-C₄-alkyl, C₁-C₄-alkoxy or aminomethyl, R⁹⁰ represents amino, acylamino, hydroxy, a carboxy salt, protected carboxy, formyloxy, azido or a sulpho salt and M represents a cation.

35. Compounds according to any one of claims 26-32, characterized in that R³ signifies a heteroaromatic acyl group of the general formulae
R¹⁰¹-(CH₂)ₙ-CO- 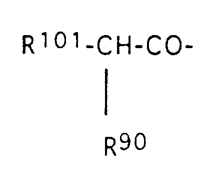
R¹⁰¹-O-CH₂-CO-
R¹⁰¹-S-CH₂-CO-
and
R¹⁰¹-CO-CO-
in which n represents 0, 1, 2 or 3; R⁹⁰ represents amino, acylamino, hydroxy, a carboxy salt, protected carboxy, azido or a sulpho salt and R¹⁰¹ represents an optionally substituted 5-, 6- or 7-membered heterocyclic ring with 1, 2, 3 or 4 nitrogen, oxygen and/or sulphur atoms.

36. Compounds according to any one of claims 26-32, characterized in that R³ represents a [[(4-substituted-2,3-dioxo-1-piperazinyl)carbonyl]amino]acetyl group of the formula 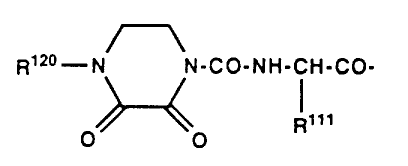 in which R¹¹¹ represents C₁-C₈-alkyl, hydroxy-C₁-C₈-alkyl or an aromatic group of the general formula 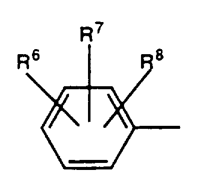 in which R⁶, R⁷ and R⁸ represent hydrogen, halogen, hydroxy, nitro, amino, cyano, trifluoromethyl, C₁-C₄-alkyl C₁-C₄-alkoxy, aminomethyl or an optionally substituted 5-, 6- or 7-membered heterocyclic ring with 1, 2, 3 or 4 nitrogen, oxygen and/or sulphur atoms and R¹⁰ represents C₁-C₈-alkyl optionally substituted by one or more halogen, cyano, nitro, amino and/or mercapto residues.

37. Compounds according to any one of claims 26-32, characterized in that R³ represents an (acylamino)acetyl group of the formula 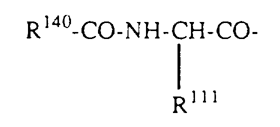 wherein R¹¹¹ represents C₁-C₈-alkyl, hydroxy-C₁-C₈-alkyl or an aromatic group of the formula 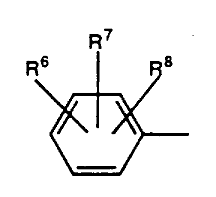 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 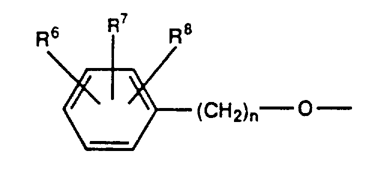 (wherein R⁶, R⁷ and R⁸ have the above significance and n signifies 0, 1, 2 or 3) hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino.

38. Compounds according to any one of claims 26-32, characterized in that R³ represents a (substituted oxyimino)acetyl group of the general formula 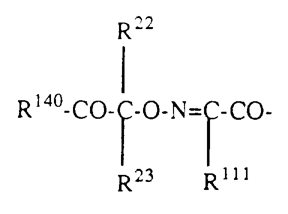 wherein R¹¹¹ represents C₁-C₈-alkyl, hydroxy-C₁-C₈-alkyl or an aromatic group of the formula 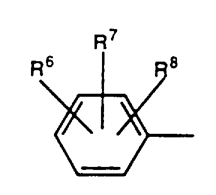 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 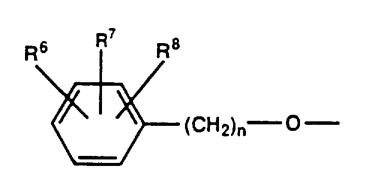 (wherein R⁶, R⁷ and R⁸ have the above significance and n represents 0, 1, 2 or 3), hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkyl-hydrazino, arylhydrazino or acylhydrazino and R and R³ represent hydrogen or lower alkyl; or R and R³ together with the adjacent carbon atom form a C₃-C₇ carbocyclic ring.

39. Compounds according to any one of claims 26-32, characterized in that R³ represents a [[[3-substituted-2-oxo-1-imidazolidinyl]carbonyl]amino]acetyl group of the general formula 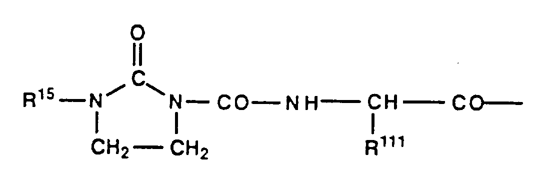 wherein R¹¹¹ represents C₁-C₈-alkyl, hydroxy-C₁-C₈-alkyl or an aromatic group of the formula 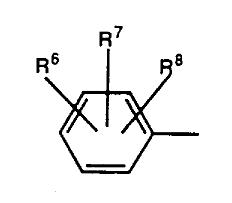 wherein R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms; and wherein R¹⁵ represents hydrogen, C₁-C₈-alkylsulphonyl, arylmethyleneamino (i.e. -N-CHR¹¹¹ wherein R¹¹¹ has the significance given above), R¹⁶CO- (wherein R¹⁶ represents hydrogen or C₁-C₈-alkyl optionally substituted by halogen), an aromatic group as defined above under R¹¹¹ or C₁-C₈-alkyl optionally substituted by one or more halogen, cyano, nitro, amino and/or mercapto residues.

40. Compounds according to any one of claims 26-32, characterized in that R³ represents a (substituted oxyimino)aryl-acetyl group of the general formula 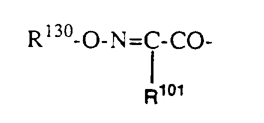 wherein R¹⁰¹ represents an optionally substituted 5, 6- or 7-membered heterocyclic ring with 1, 2, 3 or 4 nitrogen, oxygen and/or sulphur atoms, wherein the heterocyclic ring is substituted by halogen, hydroxy, nitro, amino, cyano, trifluoromethyl, C₁-C₄-alkyl or C₁-C₄-alkoxy, and R¹³⁰ represents hydrogen, C₁-C₈-alkyl, C₃-C₇-cycloalkyl, carboxy-C₃-C₇-cycloalkyl or substituted C₁-C₈-alkyl, whereby the C₁-C₈-alkyl group is substituted by one or more halogen, cyano, nitro, amino, mercapto, C₁-C₈-alkylthio, an aromatic group defined above by R¹¹¹, carboxy (including salts thereof), C₁-C₇-alkanoylamino, carbamoyl, C₂-C₉-alkoxycarbonyl, phenylmethoxycarbonyl, diphenylmethoxycarbonyl, hydroxy-C₁-C₈-alkoxyphosphinyl, dihydroxyphosphinyl, hydroxy-(phenylmethoxy)phosphinyl or di-(C₁-C₈-alkoxy)-phosphinyl residues.

41. Compounds according to claim 40, characterized in that R¹⁰¹ has the formula 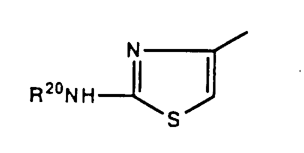 wherein R⁰ signifies hydrogen or an amino protecting group and R¹³⁰ signifies hydrogen, C₁-C₈-alkyl or a group of the formula 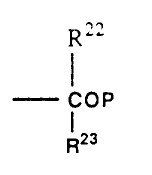 wherein R and R³ signify hydrogen or C₁-C₈-alkyl or together with the adjacent carbon atom signify a C₃-C₇ carbocyclic ring and P signifies hydroxy or -NHR¹⁹, wherein R¹⁹ represents hydrogen, C₁-C₈-alkyl, amino, C₁-C₈-alkylamino, arylamino or acylamino.

42. Compounds according to claim 41, characterized in that R⁰ signifies hydrogen or triphenylmethyl.

43. Compounds according to any one of claims 38 and 40-42 in the syn form or as a mixture in which the syn form predominates.

44. Compounds according to claim 26, characterized as
[6R-[6α,7β(Z)]]-7-[[(2-amino-4-thiazolyl)(methoxyimino)acetyl]amino]-3-[[[[4-(3-carboxy-1-ethyl-6-fluoro-1,4-dihydro-4-oxo-7-quinolinyl)-1-piperazinyl]-carbonyl]oxy]methyl]-8-oxo-5-thia-1-azabicyclo[4.2.0]oct-2-ene-2-carboxylic acid,
[6R-[6α,7β(Z)]]-7-[[(2-amino-4-thiazolyl)methoxyimino)acetyl]amino]-3-[[[[4-(3-carboxy-1-cyclopropyl-6-fluoro-1,4-dihydro-4-oxo-7-quinolinyl)-1-piperazinyl]carbonyl]oxy]methyl]-8-oxo-5-thia-1-azabicyclo[4.2.0]-oct-2-ene-2-carboxylic acid,
(6R-trans)-3[[[[4-(3-carboxy-1-cyclopropyl-6-fluoro-1,4-dihydro-4-oxo-7-quinolinyl)-1-piperazinyl]carbonyl]oxy]methyl]-8-oxo-7-[(phenoxyacetyl)amino]-5-thia-1-azabicyclo-[4.2.0]oct-2-ene-2-carboxylic acid,
[6R-[6α,7β(Z)]]-7-[[(2-amino-4-thiazolyl)-[(1-carboxy-1-methyl-ethoxy)imino]acetyl]amino]-3-[[[[4-(3-carboxy-1-cyclopropyl-6-fluoro-1,4-dihydro-4-oxo-7-quinolinyl)-1-piperazinyl]carbonyl]oxy]methyl]-8-oxo-5-thia-1-azabicyclo[4.2.0]-oct-2-ene-2-carboxylic acid,
[6R-[6α,7β(Z)]]-7-[[(2-amino-4-thiazolyl)-[(carboxymethoxy)imino]acetyl]amino]-3-[[[[4-(3-carboxy-1-cyclo-propyl-6-fluoro-1,4-dihydro-4-oxo-7-quinolinyl)-1-piperazinyl]carbonyl]oxy]methyl]-8-oxo-5-thia-1-azabicyclo[4.2.0]oct-2-ene-2-carboxylic acid,
[6R-[6α,7β(Z)]]-7-[[(2-amino-4-thiazolyl)(methoxyimino)acetyl]amino]-3-[[[[4-(9-cyclopropyl-6-fluoro-2,3,4,9-tetrahydro-3,4-dioxoisothiazolo[5,4-b]quinolin-7-yl)-1-piperazinyl]carbonyl]oxy]methyl]-8-oxo-5-thia-1-azabicyclo[4.2.0]-oct-2-ene-2-carboxylic acid and
(6R-[6α,7β(Z)]]-7-[[(2-amino-4-thiazolyl)(methoxyimino)acetyl]amino]-3-[[[[4-(9-cyclopropyl-6-fluoro-2,3,4,9-tetrahydro-3,4-dioxoisooxazolo[5,4-b]quinolin-7-yl)-1-piperazinyl]carbonyl]oxy]methyl]-8-oxo-5-thia-1-azabicyclo[4.2.0]-oct-2-ene-2-carboxylic acid
as well as salts of these compounds and hydrates of these compounds and salts.

45. Salts of the compounds according to claim 44, characterized as sodium salts.

46. Compounds of the formula 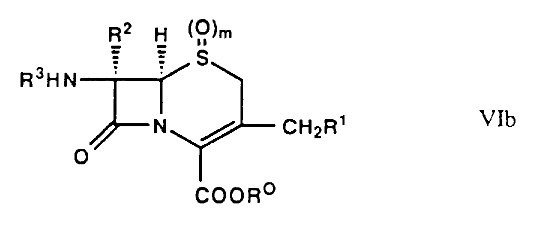 wherein R¹, R or R³ and m have the significance given above and R^{o} represents t-butyl, benzhydryl, p-nitrobenzyl or allyl.

47. (6R-trans)-4-[3-Carboxy-6,8-difluoro-1-(2-fluoroethyl)-1,4-dihydro-4-oxo-7-quinolinyl]-1-[[2-(1,1-dimethylethoxy)carbonyl-7-(formylamino)-8-oxo-5-thia-1-azabicyclo[4.2.O]oct-2-en-3-yl]methyl]-1-methylpiperazinium iodide.

48. Compounds of the formula 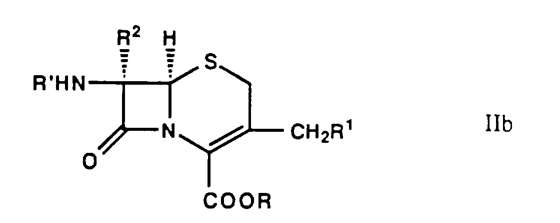 wherein R¹ and R have the significance given above, R represents hydrogen or a carboxylic acid protecting group and R' represents hydrogen or a readily cleavable amino protecting group.

49. (6R-trans)-3-[[[[4-(3-Carboxy-1-cyclopropyl-6-fluoro-1,4-dihydro-4-oxo-7-quinolinyl)-1-piperazinyl]carbonyl]oxy]methyl]-7-[[(1,1-dimethylethoxy)carbonyl]amino]-8-oxo-5-thia-1-azabicyclo[4.2.0]oct-2-ene-2-carboxylic acid diphenylmethyl ester.

50. Compounds according to any one of claims 1-45 as pharmaceutically active substances.

51. Compounds as set forth in any one of claims 1-45 as pharmaceutically active substances for the treatment and prophylaxis of infectious diseases.

52. A process for the manufacture of the compounds according to any one of claims 1-45, characterized by
a) for the manufacture of a compound of formula I in which m represents 0, reacting a compound of the general formula 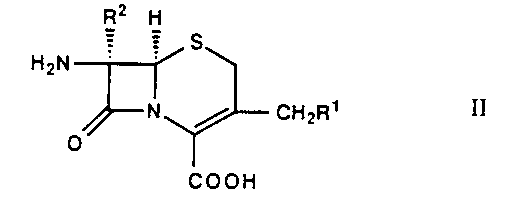 in which R¹ and R have the significance given above, or a readily hydrolyzable ester or salt of this compound with a carboxylic acid of the general formula R³OH or with a reactive derivative thereof, or
b) for the manufacture of a compound of formula I in which m represents 0 and R¹ represents one of groups (a) and (b), [reacting] a compound of the formula 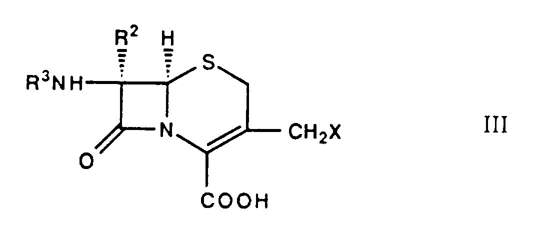 in which R and R³ have the significance given above, X represents a leaving group and amino, hydroxy and carboxy groups present may be protected,
or a readily hydrolyzable ester or salt thereof with a compound of the formula 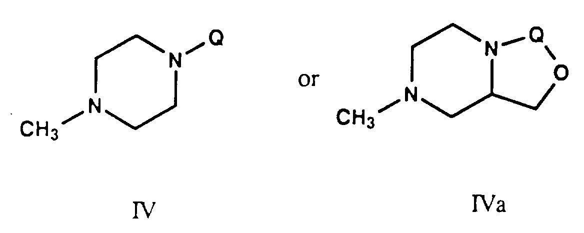 in which Q has the significance given above and the nitrogen atoms may be protected,
or
c) for the manufacture of a readily hydrolyzable ester of a carboxylic acid of formula I in which m represents 0 and R¹ represents group (c), reacting a compound of the formula 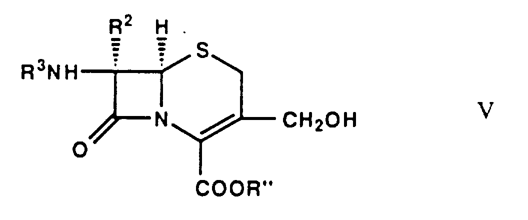 in which R and R³ have the significance given above and R" represents the residue of a readily hydrolyzable ester,
in the presence of a base with phosgene [and] reacting the reaction product obtained with a compound of formula R¹⁰H, wherein R¹⁰ has the significance given above, whereby any amino, hydroxy and/or carboxy functions present may be protected and any protecting groups present are subsequently cleaved off, or
d) for the manufacture of a carboxylic acid of formula I, converting an ester of the formula 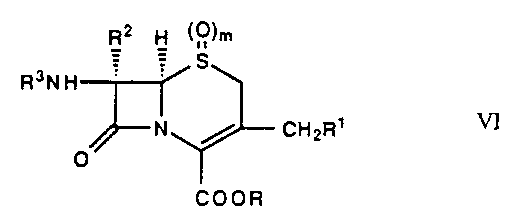 in which R¹, R, R³ and m have the significance given above and R represents a carboxylic acid protecting group,
into the carboxylic acid of formula I, or
e) for the manufacture of the compound of formula I in which m represents 0 or a readily hydrolyzable ester or salt thereof, reducing a compound of the formula 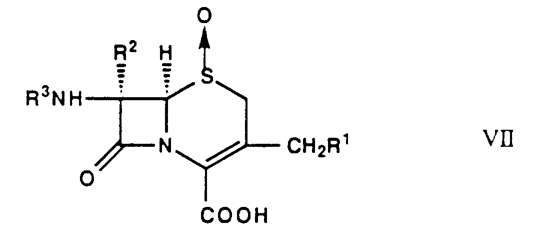 in which R¹, R and R³ have the significance given above, or a readily hydrolyzable ester or salt thereof, or
f) for the manufacture of a compound of formula I in which m represents 1 or 2 or a readily hydrolyzable ester or salt thereof, oxidizing a compound of the formula 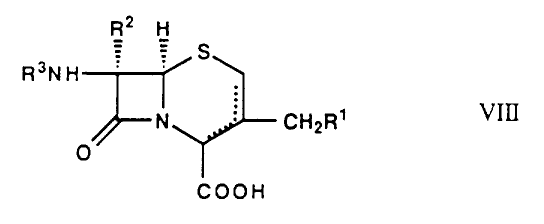 in which R¹, R and R³ have the significance given above and the dotted lines indicate the presence of a Δ2 or Δ3 double bond,
or one of its esters of salts, or
g) for the manufacture of a compound of formula I in which R³ contains an amino substituent or one of its esters or salts, cleaving off the amino protecting group in the substituent R³⁰ of a compound of the general formula 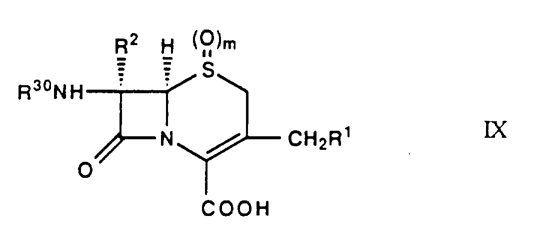 in which R¹, R and m have the significance given above and R³⁰ represents an acyl group containing a protected amino group,
or of one of its esters or salts, or
h) for the manufacture of a readily hydrolyzable ester of a compound of formula I, subjecting a carboxylic acid of formula I to a corresponding esterification, or
i) for the manufacture of salts or hydrates of a compound of formula I or of hydrates of these salts, converting a compound of formula I into a salt or hydrate or into a hydrate of this salt.

53. A process according to claim 52, characterized by carrying out process alternative (b).

54. A process according to claim 52, characterized by carrying out one of process alternatives (a), (c), (d), (f), (g) and (i).

55. A pharmaceutical preparation, characterized in that it contains a compound according to any one of claims 1-45.

56. A pharmaceutical preparation for the treatment and prophylaxis of infectious diseases, characterized in that it contains a compound according to any one of claims 1-45.

57. The use of compounds according to any one of claims 1-45 for the manufacture of medicaments for the treatment or prophylaxis of infectious diseases.

## Claims (Claims for the following Contracting State(s): ES, GR)

1. A process for the manufacture of acyl derivatives of the general formula 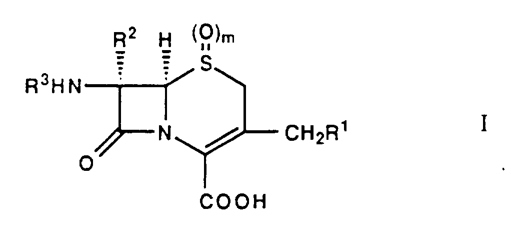 in which R¹ represents a substituted N-heterocycle of the formulae 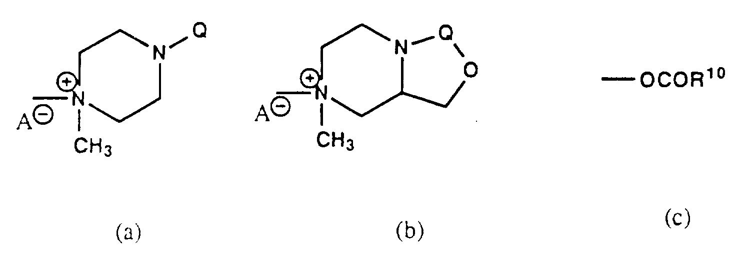 wherein R¹⁰ represents one of the groups 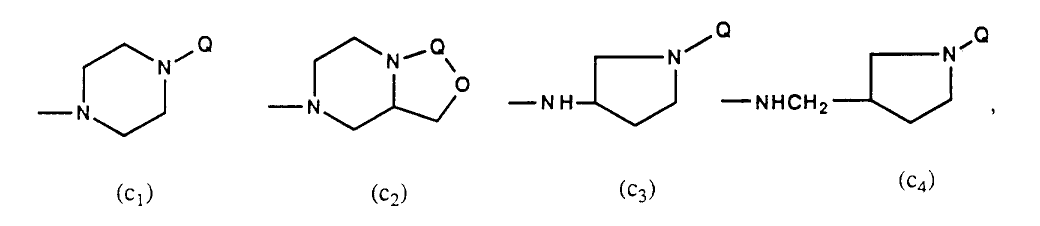
and Q represents a substituted quinolinyl or naphthyridinyl group, whereby the N-heterocycle can be optionally substituted by one or more C₁-C₈-alkyl groups; and wherein R represents hydrogen, C₁-C₈-alkoxy C₁-C₈-alkylthio or C₁-C₇-alkanoylamino, R³ represents an acyl group, m represents 0, 1 or 2 and A represents a pharmaceutically acceptable anion,
as well as of readily hydrolyzable esters and pharmaceutically acceptable salts of these compounds and of hydrates of the compounds of formula I or of their esters and salts, characterized by
a) for the manufacture of a compound of formula I in which m represents 0, reacting a compound of the general formula 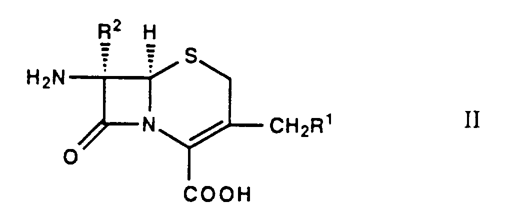 in which R¹ and R have the significance given above, or a readily hydrolyzable ester or salt of this compound with a carboxylic acid of the general formula R³OH or with a reactive derivative thereof, or
b) for the manufacture of a compound of formula I in which m represents 0 and R¹ represents one of groups (a) and (b), [reacting] a compound of the formula 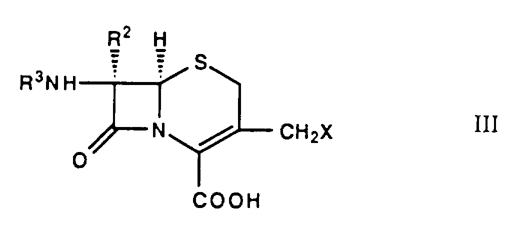 in which R and R³ have the significance given above, X represents a leaving group and amino, hydroxy and carboxy groups present may be protected,
or a readily hydrolyzable ester or salt thereof with a compound of the formula 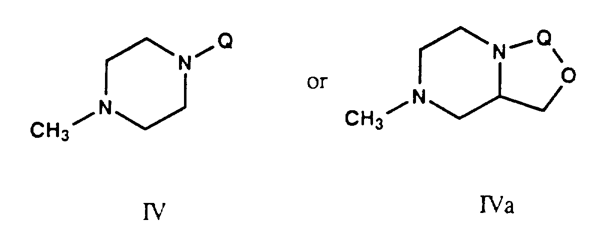 in which Q has the significance given above and the nitrogen atoms may be protected,
or
c) for the manufacture of a readily hydrolyzable ester of a carboxylic acid of formula I in which m represents 0 and R¹ represents group (c), reacting a compound of the formula 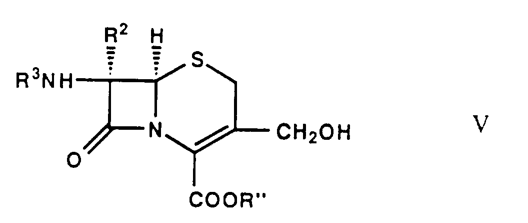 in which R and R³ have the significance given above and R" represents the residue of a readily hydrolyzable ester, in the presence of a base with phosgene [and] reacting the reaction product obtained with a compound of formula R¹⁰H, wherein R¹⁰ has the significance given above, whereby any amino, hydroxy and/or carboxy functions present may be protected and any protecting groups present are subsequently cleaved off, or
d) for the manufacture of a carboxylic acid of formula I, converting an ester of the formula 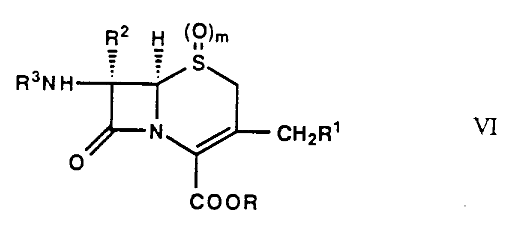 in which R¹, R, R³ and m have the significance given above and R represents a carboxylic acid protecting group,
into the carboxylic acid of formula I, or
e) for the manufacture of the compound of formula I in which m represents 0 or a readily hydrolyzable ester or salt thereof, reducing a compound of the formula 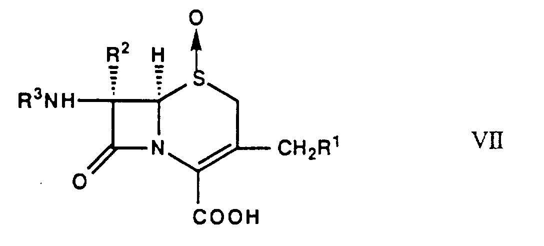 in which R¹, R and R³ have the significance given above, or a readily hydrolyzable ester or salt thereof, or
f) for the manufacture of a compound of formula I in which m represents 1 or 2 or a readily hydrolyzable ester or salt thereof, oxidizing a compound of the formula 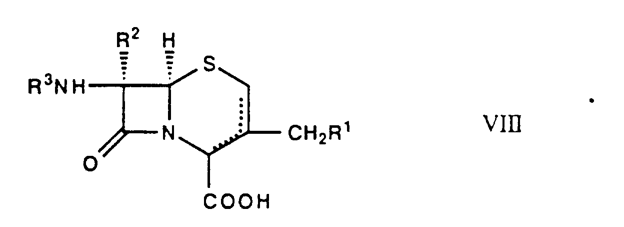 in which R¹, R and R³ have the significance given above and the dotted lines indicate the presence of a Δ2 or Δ3 double bond,
or one of its esters of salts, or
g) for the manufacture of a compound of formula I in which R³ contains an amino substituent or one of its esters or salts, cleaving off the amino protecting group in the substituent R³⁰ of a compound of the general formula 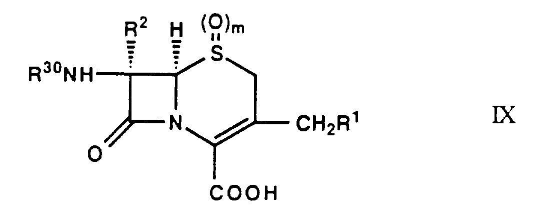 in which R¹, R and m have the significance given above and R³⁰ represents an acyl group containing a protected amino group,
or of one of its esters or salts, or
h) for the manufacture of a readily hydrolyzable ester of a compound of formula I, subjecting a carboxylic acid of formula I to a corresponding esterification, or
i) for the manufacture of salts or hydrates of a compound of formula I or of hydrates of these salts, converting a compound of formula I into a salt or hydrate or into a hydrate of this salt.

2. A process for the manufacture of compounds according to claim 1 in which R¹ represents one of groups (a) and (b), characterized in that correspondingly substituted starting compounds are used.

3. A process for the manufacture of compounds according to claim 2 in which m represents 0 and R represents hydrogen, characterized in that correspondingly substituted starting compounds are used.

4. A process for the manufacture of compounds according to claim 2 or 3 in which R¹ has the following formula: 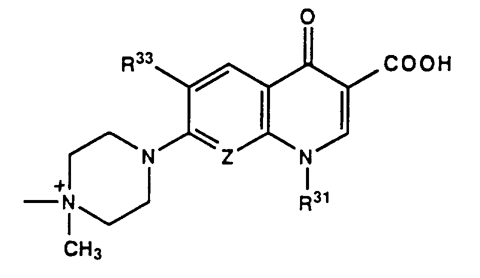 wherein Z represents R³⁰⁻C or nitrogen; R³⁰ represents hydrogen, halogen or an oxymethylene (-CH₂O-) bridge to the piperazine residue with the formation of a fused 6-membered ring; R³¹ represents hydrogen, C₁-C₈-alkyl, C₂-C₆-alkenyl, C₃-C₇-cycloalkyl, halo-C₁-C₈-alkyl- or mono-, di- or tri-halophenyl; or R³⁰ and R³¹ together represent a C₃-C₅-alkylene group, a C₂-C₄-alkylenemonooxy group, a C₁-C₂-alkylenedioxy group or a group of the formula -OCH₂N(CH₃)-; and R³³ represents hydrogen or halogen,
characterized in that correspondingly substituted starting compounds are used.

5. A process for the manufacture of compounds according to claim 4 in which Z represents the group R³⁰-C, wherein R³⁰ signifies hydrogen, bromine, chlorine or fluorine, R³¹ signifies C₁-C₈-alkyl, halo-C₁-C₈-alkyl or C₃-C₇-cycloalkyl and R³³ signifies hydrogen, chlorine or fluorine, characterized in that correspondingly substituted starting compounds are used.

6. A process for the manufacture of compounds according to claim 5 in which R³⁰ represents hydrogen or fluorine, R³¹ represents ethyl, fluoroethyl or cyclopropyl and R³³ represents hydrogen or fluorine, characterized in that correspondingly substituted starting compounds are used.

7. A process for the manufacture of compounds according to claim 2 or 3 in which R¹ has the following formula: 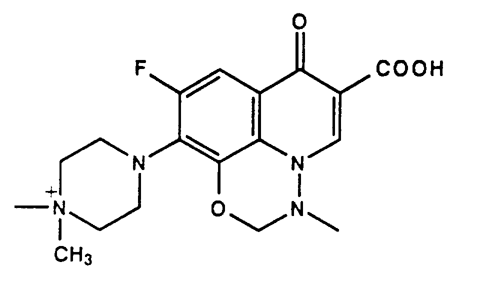 characterized in that correspondingly substituted starting compounds are used.

8. A process for the manufacture of compounds according to claim 2 or 3 in which R¹ has the following formula: 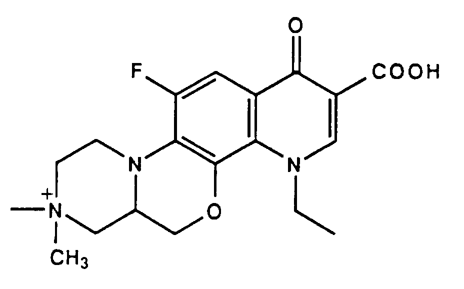 characterized in that correspondingly substituted starting compounds are used.

9. A process for the manufacture of compounds according to any one of claims 2-8 in which R³ signifies an aliphatic acyl group of the formula
R⁵-CO-
in which R⁵ represents hydrogen, C₁-C₈-alkyl, C₃-C₇-cycloalkyl, C₁-C₈-alkoxy, C₂-C₆-alkenyl, C₃-C₇-cycloalkenyl or cyclohexadienyl; or C₁-C₈-alkyl or C₂-C₆-alkenyl substituted by one or more halogen, cyano, nitro, amino, mercapto, C₁-C₈-alkylthio or cyanomethylthio residues,
characterized in that correspondingly substituted starting compounds are used.

10. A process for the manufacture of compounds according to any one of claims 2-8 in which R³ signifies an aromatic acyl group of the general formulae 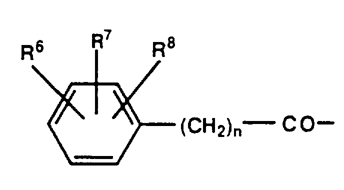 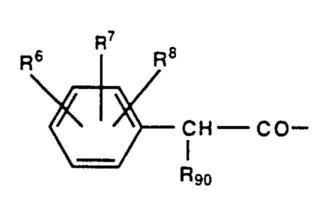 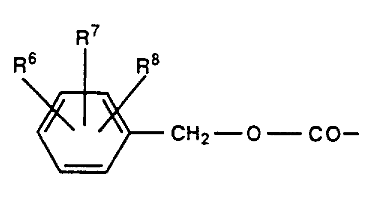 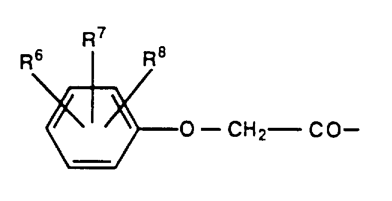 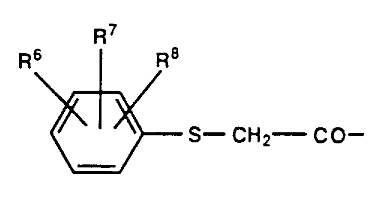 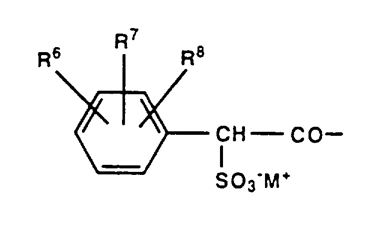 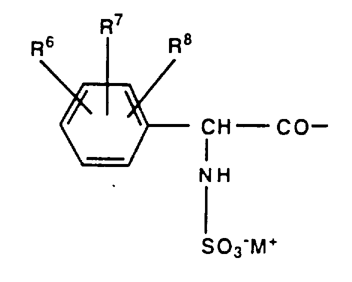 in which n represents 0, 1, 2 or 3; R⁶, R⁷, and R⁸ each represent hydrogen, halogen, hydroxy, nitro, amino, cyano, trifluoromethyl, C₁-C₄-alkyl, C₁-C₄-alkoxy or aminomethyl, R⁹⁰ represents amino, acylamino, hydroxy, a carboxy salt, protected carboxy, formyloxy, azido or a sulpho salt and M represents a cation,
characterized in that correspondingly substituted starting compounds are used.

11. A process for the manufacture of compounds according to any one of claims 2-8 in which R³ signifies a heteroaromatic acyl group of the general formulae
R¹⁰¹-(CH₂)ₙ-CO- 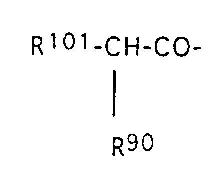
R¹⁰¹-O-CH₂-CO-
R¹⁰¹-S-CH₂-CO-
and
R¹⁰¹-CO-CO-
in which n represents 0, 1, 2 or 3; R⁹⁰ represents amino, acylamino, hydroxy, a carboxy salt, protected carboxy, azido or a sulpho salt and R¹⁰¹ represents an optionally substituted 5-, 6- or 7-membered heterocyclic ring with 1, 2, 3 or 4 nitrogen, oxygen and/or sulphur atoms,
characterized in that correspondingly substituted starting compounds are used.

12. A process for the manufacture of compounds according to any one of claims 2-8 in which R³ represents a [[(4-substituted-2,3-dioxo-1-piperazinyl)carbonyl]amino]acetyl group of the formula 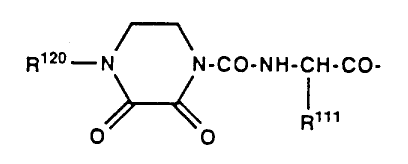 in which R¹¹¹ represents C₁-C₈-alkyl, hydroxy-C₁-C₈-alkyl or an aromatic group of the general formula 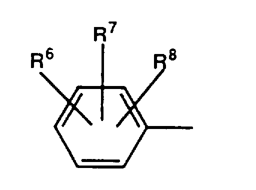 in which R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms and R¹⁰ represents C₁-C₈-alkyl optionally substituted by one or more halogen, cyano, nitro, amino and/or mercapto residues,
characterized in that correspondingly substituted starting compounds are used.

13. A process for the manufacture of compounds according to any one of claims 2-8 in which R³ represents an (acylamino)acetyl group of the general formula 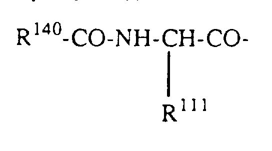 wherein R¹¹¹ represents C₁-C₈-alkyl, hydroxy-C₁-C₈-alkyl or an aromatic group of the formula 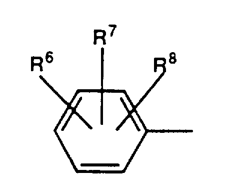 in which R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 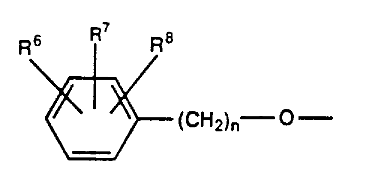
(wherein R⁶, R⁷ and R⁸ have the above significance and n signifies 0, 1, 2 or 3) hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino,
characterized in that correspondingly substituted starting compounds are used.

14. A process for the manufacture of compounds according to any one of claims 2-8 in which R³ represents a (substituted oxyimino)acetyl group of the general formula 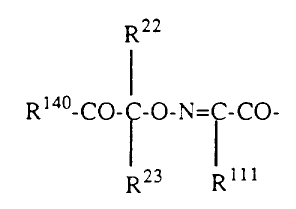 wherein R¹¹¹ represents C₁-C₈-alkyl, hydroxy-C₁-C₈-alkyl or an aromatic group of the formula 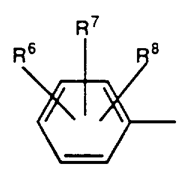 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 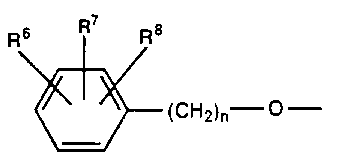 (wherein R⁶, R⁷ and R⁸ have the above significance and n represents 0, 1, 2 or 3), hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino and R and R³ represent hydrogen or C₁-C₈-alkyl; or R and R³ together with the adjacent carbon atom form a C₃-C₇ carbocyclic ring,
characterized in that correspondingly substituted starting compounds are used.

15. A process for the manufacture of compounds according to any one of claims 2-8 in which R³ represents a [[[3-substituted-2-oxo-1-imidazolidinyl]carbonyl]amino]acetyl group of the general formula 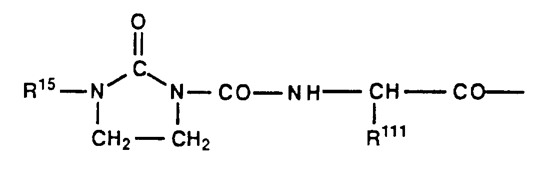 wherein R¹¹¹ represents C₁-C₈-alkyl, hydroxy-C₁-C₈-alkyl or an aromatic group of the formula 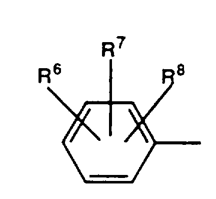 wherein R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms; and wherein R¹⁵ represents hydrogen, C₁-C₈-alkylsulphonyl, arylmethyleneamino (i.e. -N=CHR¹¹¹ wherein R¹¹¹ has the significance given above), R¹⁶CO- (wherein R¹⁶ represent hydrogen or C₁-C₈-alkyl optionally substituted by halogen), an aromatic group as defined above under R¹¹¹ or C₁-C₈-alkyl optionally substituted by one or more halogen, cyano, nitro, amino and/or mercapto residues,
characterized in that correspondingly substituted starting compounds are used.

16. A process for the manufacture of compounds according to any one of claims 2-8 in which R³ represents a (substituted oxyimino)arylacetyl group of the general formula 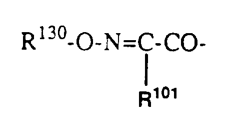 wherein R¹⁰¹ represents an optionally substituted 5, 6- or 7-membered heterocyclic ring with 1, 2, 3 or 4 nitrogen, oxygen and/or sulphur atoms, wherein the heterocyclic ring is substituted by halogen, hydroxy, nitro, amino, cyano, trifluoromethyl, C₁-C₄-alkyl or C₁-C₄-alkoxy, and R¹³⁰ represents hydrogen, C₁-C₈-alkyl, C₃-C₇-cycloalkyl, carboxy-C₃-C₇-cycloalkyl or substituted C₁-C₈-alkyl, whereby the C₁-C₈-alkyl group is substituted by one or more halogen, cyano, nitro, amino, mercapto, C₁-C₈-alkylthio, an aromatic group defined above by R¹¹¹, carboxy (including salts thereof), C₁-C₇-alkanoylamino, carbamoyl, C₂-C₉-alkoxycarbonyl, phenylmethoxycarbonyl, diphenylmethoxycarbonyl, hydroxy- C₁-C₈-alkoxyphosphinyl, dihydroxyphosphinyl, hydroxy-(phenylmethoxy)phosphinyl or di-(C₁-C₈-alkoxy)-phosphinyl residues,
characterized in that correspondingly substituted starting compounds are used.

17. A process for the manufacture of compounds according to claim 16 in which R¹⁰¹ has the formula 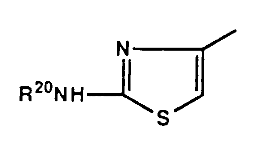 wherein R⁰ signifies hydrogen or an amino protecting group and R¹³⁰ signifies hydrogen, C₁-C₈-alkyl or a group of the formula 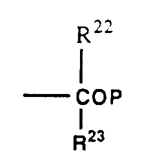 wherein R and R³ signify hydrogen or C₁-C₈-alkyl or together with the adjacent carbon atom signify a C₃-C₇-carbocyclic ring and P signifies hydroxy or -NHR¹⁹, wherein R¹⁹ represents hydrogen, C₁-C₈-alkyl, amino, C₁-C₈-alkylamino, arylamino or acylamino, characterized in that correspondingly substituted starting compounds are used.

18. A process for the manufacture of compounds according to claim 17 in which R⁰ signifies hydrogen and R¹³⁰ signifies methyl or a group of the formula 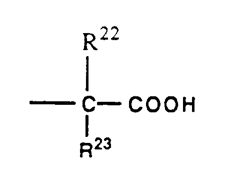 wherein R and R³ represent hydrogen or methyl, characterized in that correspondingly substituted starting compounds are used.

19. A process for the manufacture of compounds according to any one of claims 14 and 16-18 in the syn form or as a mixture in which the syn form predominates, characterized in that correspondingly substituted starting compounds are used.

20. A process for the manufacture of compounds according to claim 2 in which R¹ represents group (a) and R³ represents phenoxyacetyl, characterized in that correspondingly substituted starting compounds are used.

21. A process for the manufacture of compounds according to claim 2,
(6R-trans)-4-[3-carboxy-1-(2-fluoroethyl)-6,8-difluoro-1,4-dihydro-4-oxoquinolin-7-yl]-[[2-carboxy-8-oxo-7-[(phenoxyacetyl)amino]-5-thia-1-azabicyclo[4.2.0]oct-2-en-3-yl]methyl]-1-methylpiperazinium iodide,
[6R-[6α,7β(Z)]]-1-[[7-[[[(2-amino-4-thiazolyl)[1-(1-carboxy-1-methyl)ethoxy]imino]acetyl]amino]-2-carboxy-8-oxo-5-thia-1-azabicyclo[4.2.0]oct-2-en-3-yl]methyl]-4-[3-carboxyl-1-(2-fluoroethyl)-6,8-difluoro-1,4-dihydro-4-oxoquinolin-7-yl]-1-methylpiperazinium hydroxide inner salt monosodium salt and
[6R-[6α,7β[3(Z)]]-1-[[7-[[(2-amino-4-thiazolyl)(methoxyimino)acetyl]amino]-2-carboxy-8-oxo-5-thia-1-azabicyclo[4.2.0]oct-2-en-3-yl]methyl]-4-(3-carboxy-1-ethyl-6-fluoro-1,4-dihydro-4-oxo-7-quinolinyl)-1-methylpiperazinium iodide, characterized in that correspondingly substituted starting compounds are used.

22. A process for the manufacture of a compound according to claim 2, [6R-[6α,7β(Z)]]-1-[[[(2-amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluoro-1-(2-fluoroethyl)-1,4-dihydro-4-oxo-7-quinolinyl]-1-methylpiperazinium iodide, characterized in that correspondingly substituted starting compounds are used.

23. A process for the manufacture of a compound according to claim 2, [6R-[6α,7β(Z)]]-1-[[[(2-amino-4-thiazolyl)(methoxyimino)acetyl]amino]-2-carboxy-8-oxo-5-thia-1-azabicyclo[4.2.0]oct-2-en-3-yl]methyl]-4-[3-carboxy-6,8-difluoro-1-(2-fluoroethyl)-1,4-dihydro-4-oxo-7-quinolinyl]-1-methyl-piperazinium hydroxide inner salt monosodium salt, characterized in that correspondingly substituted starting compounds are used.

24. A process for the manufacture of a compound according to claim 2, [6R-trans)-4-[3-carboxy-6,8-difluoro-1-(2-fluoroethyl)-1,4-dihydro-4-oxo-7-quinolinyl]-1-[[2-carboxy-7-(formylamino)-8-oxo-5-thia-1-azabicyclo[4.2.0]oct-2-en-3-yl]-methyl-1-methylpiperazinium trifluoroacetate, characterized in that correspondingly substituted starting compounds are used.

25. A process for the manufacture of a compound according to claim 2, [6R-[6α,7β(Z)]]-1-[[7-[[(2-amino-4-thiazolyl)[[1,1-dimethyl-2-(1,1-dimethylethoxy)-2-oxo-ethoxy]imino]acetyl]amino]-2-carboxy-8-oxo-5-thia-1-azabicyclo[4.2.0]oct-2-en-3-yl]-methyl]-4-[3-carboxy-(2-fluoroethyl)-6,8-difluoro-1,4-dihydro-4-oxoquinolin-7-yl]-1-methylpiperazinium iodide, characterized in that correspondingly substituted starting compounds are used.

26. A process for the manufacture of compounds according to claim 1 in which R¹ represents group (c), characterized in that correspondingly substituted starting compounds are used.

27. A process for the manufacture of compounds according to claim 26 in which m represents 0 or 1, characterized in that correspondingly substituted starting compounds are used.

28. A process for the manufacture of compounds according to claim 26 or 27 in which Q signifies a group of the formula 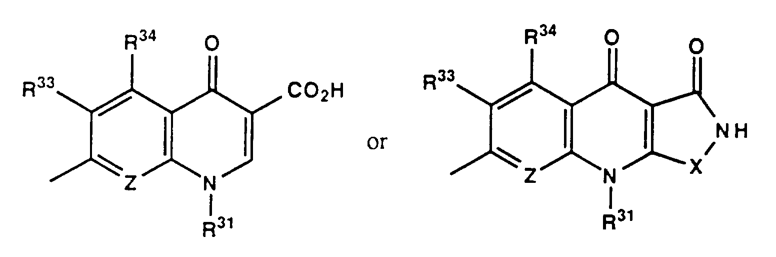 wherein Z represents R³⁰-C or nitrogen; X represents oxygen or sulphur; R³⁰ represents hydrogen, halogen or an oxymethylene (-CH₂O-) bridge to the piperazine residue with the formation of a fused 6-membered ring; R³¹ represents hydrogen, C₁-C₈-alkyl, C₂-C₆-alkenyl, C₃-C₇-cycloalkyl, halo-C₁-C₈-alkyl or mono-, di- or tri-halophenyl; or R³⁰ and R³¹ together represent a C₃-C₅-alkylene group, a C₂-C₄-alkylenemonooxy group, a C₁-C₂-alkylenedioxy group or a group of the formula -OCH₂N(CH₃)-; R³³ represents hydrogen or halogen; and R³⁴ represents hydrogen or amino,
characterized in that correspondingly substituted starting compounds are used.

29. A process for the manufacture of compounds according to claim 28 in which Z signifies the group R³⁰⁻C, wherein R³⁰ signifies hydrogen, chlorine, bromine or fluorine, R³¹ signifies C₁-C₈-alkyl, halo-C₁-C₈-alkyl or C₃-C₇-cycloalkyl and R³³ signifies hydrogen, chlorine or fluorine, characterized in that correspondingly substituted starting compounds are used.

30. A process for the manufacture of compounds according to claim 29 in which R³⁰ represents hydrogen or fluorine, R³¹ represents ethyl, fluoroethyl or cyclopropyl and R³³ represents hydrogen or fluorine, characterized in that correspondingly substituted starting compounds are used.

31. A process for the manufacture of compounds according to claim 28 in which R¹ has the following formula: 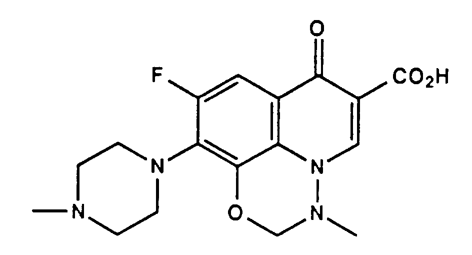 characterized in that correspondingly substituted starting compounds are used.

32. A process for the manufacture of compounds according to claim 28 in which R¹ has the following formula: 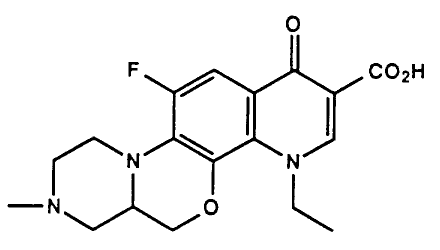 characterized in that correspondingly substituted starting compounds are used.

33. A process for the manufacture of compounds according to any one of claims 26-32 in which R³ signifies an aliphatic acyl group of the formula
R⁵-CO-
in which R⁵ represents hydrogen, C₁-C₈-alkyl, C₃-C₇-cycloalkyl, C₁-C₈-alkoxy, C₂-C₆-alkenyl, C₃-C₇-cycloalkenyl or cyclohexadienyl; or C₁-C₈-alkyl or C₂-C₆-alkenyl substituted by one or more halogen, cyano, nitro, amino, mercapto, C₁-C₈-alkylthio or cyanomethylthio residues,
characterized in that correspondingly substituted starting compounds are used.

34. A process for the manufacture of compounds according to any one of claims 26-32 in which R³ signifies an aromatic acyl group of the general formulae 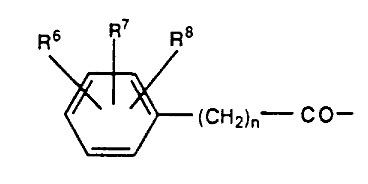 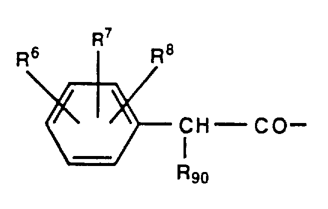 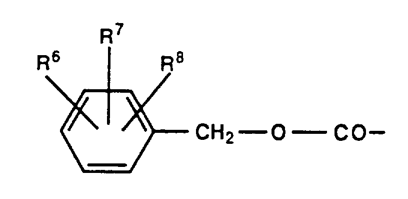 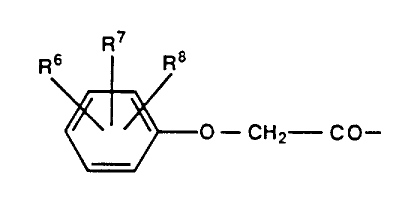 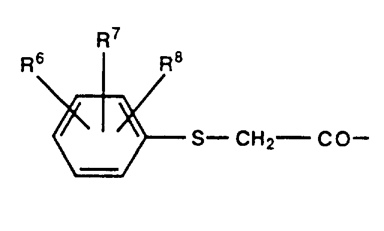 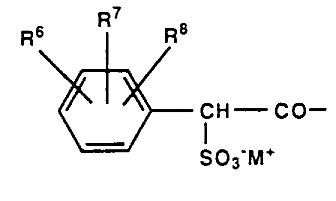 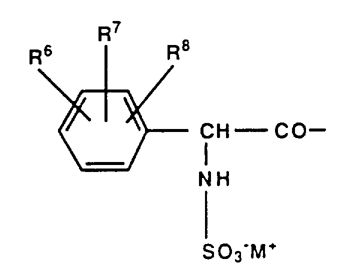 in which n represents 0, 1, 2 or 3; R⁶, R⁷, and R⁸ each represent hydrogen, halogen, hydroxy, nitro, amino, cyano, trifluoromethyl, C₁-C₄-alkyl C₁-C₄-alkoxy or aminomethyl, R⁹⁰ represents amino, acylamino, hydroxy, a carboxy salt, protected carboxy, formyloxy, azido or a sulpho salt and M represents a cation,
characterized in that correspondingly substituted starting compounds are used.

35. A process for the manufacture of compounds according to any one of claims 26-32 in which R³ signifies a heteroaromatic acyl group of the general formulae
R¹⁰¹-(CH₂)ₙ-CO- 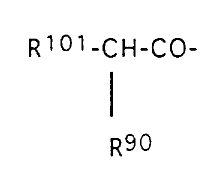
R¹⁰¹-O-CH₂-CO-
R¹⁰¹-S-CH₂-CO-
and
R¹⁰¹-CO-CO-
in which n represents 0, 1, 2 or 3; R⁹⁰ represents amino, acylamino, hydroxy, a carboxy salt, protected carboxy, azido or a sulpho salt and R¹⁰¹ represents an optionally substituted 5-, 6- or 7-membered heterocyclic ring with 1, 2, 3 or 4 nitrogen, oxygen and/or sulphur atoms,
characterized in that correspondingly substituted starting compounds are used.

36. A process for the manufacture of compounds according to any one of claims 26-32 in which R³ represents a [[(4-substituted-2,3-dioxo-1-piperazinyl)carbonyl]amino]acetyl group of the formula 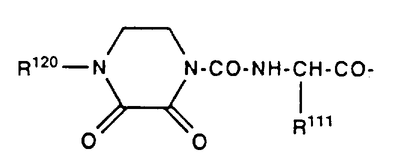 in which R¹¹¹ represents C₁-C₈-alkyl, hydroxy-C₁-C₈-alkyl or an aromatic group of the general formula 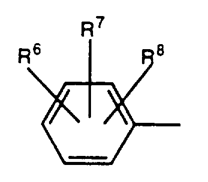 in which R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms and R¹⁰ represents C₁-C₈-alkyl optionally substituted by one or more halogen, cyano, nitro, amino and/or mercapto residues, characterized in that correspondingly substituted starting compounds are used.

37. A process for the manufacture of compounds according to any one of claims 26-32 in which R³ represents an (acylamino)acetyl group of the formula 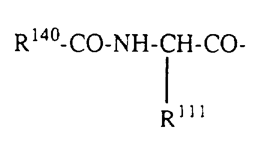 wherein R¹¹¹ represents C₁-C₈-alkyl, hydroxy-C₁-C₈-alkyl or an aromatic group of the formula 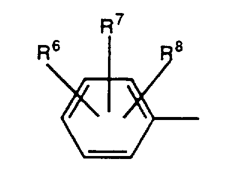 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 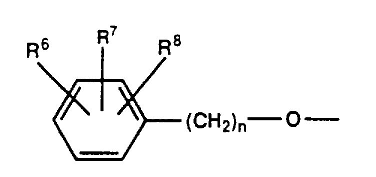 (wherein R⁶, R⁷ and R⁸ have the above significance and n signifies 0, 1, 2 or 3) hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino,
characterized in that correspondingly substituted starting compounds are used.

38. A process for the manufacture of compounds according to any one of claims 26-32 in which R³ represents a (substituted oxyimino)acetyl group of the general formula 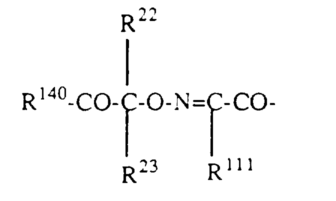 wherein R¹¹¹ represents C₁-C₈-alkyl, hydroxy-C₁-C₈-alkyl or an aromatic group of the formula 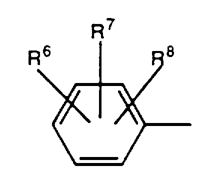 wherein R⁶, R⁷ and R⁸ represent hydrogen, halogen, hydroxy, nitro, amino, cyano, trifluoromethyl, C₁-C₄-alkyl, C₁-C₄-alkoxy or aminomethyl or an optionally substituted 5-, 6- or 7-membered heterocyclic ring with 1, 2, 3 or 4 nitrogen, oxygen and/or sulphur atoms and wherein R¹⁴⁰ represents the group 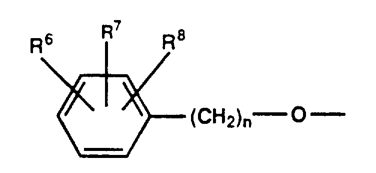 (wherein R⁶, R⁷ and R⁸ have the above significance and n represents 0, 1, 2 or 3), hydrogen, C₁-C₈-alkyl, substituted C₁-C₈-alkyl, amino, C₁-C₈-alkylamino, di-(C₁-C₈-alkyl)-amino, (cyano-C₁-C₈-alkyl)-amino, hydrazino, C₁-C₈-alkylhydrazino, arylhydrazino or acylhydrazino and R and R³ represent hydrogen or lower alkyl; or R and R³ together
with the adjacent carbon atom form a C₃-C₇ carbocyclic ring,
characterized in that correspondingly substituted starting compounds are used.

39. A process for the manufacture of compounds according to any one of claims 26-32 in which R³ represents a [[[3-substituted-2-oxo-1-imidazolidinyl]carbonyl]amino]acetyl group of the general formula 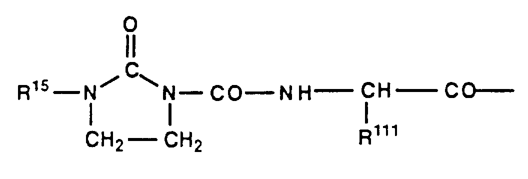 wherein R¹¹¹ represents C₁-C₈-alkyl, hydroxy-C₁-C₈-alkyl or an aromatic group of the formula 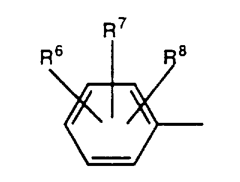 wherein R⁶, R⁷ and R⁸ represent hydrogen, halogen, hydroxy, nitro, amino, cyano, trifluoromethyl, C₁-C₄-alkyl, C₁-C₄-alkoxy, aminomethyl or an optionally substituted 5-, 6- or 7-membered heterocyclic ring with 1, 2, 3 or 4 nitrogen, oxygen and/or sulphur atoms; and wherein R¹⁵ represents hydrogen, C₁-C₈-alkylsulphonyl, arylmethyleneamino (i.e. -N-CHR¹¹¹ wherein R¹¹¹ has the significance given above), R¹⁶CO- (wherein R¹⁶ represents hydrogen or C₁-C₈-alkyl optionally substituted by halogen), an aromatic group as defined above under R¹¹¹ or C₁-C₈-alkyl optionally substituted by one or more halogen, cyano, nitro, amino and/or mercapto residues,
characterized in that correspondingly substituted starting compounds are used.

40. A process for the manufacture of compounds according to any one of claims 26-32 in which R³ represents a (substituted oxyimino)arylacetyl group of the general formula 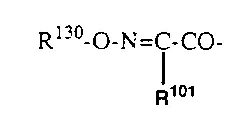 wherein R¹⁰¹ represents an optionally substituted 5, 6- or 7-membered heterocyclic ring with 1, 2, 3 or 4 nitrogen, oxygen and/or sulphur atoms, wherein the heterocyclic ring is substituted by halogen, hydroxy, nitro, amino, cyano, trifluoromethyl, C₁-C₄-alkyl or C₁-C₄-alkoxy, and R¹³⁰ represents hydrogen, C₁-C₈-alkyl, C₃-C₇-cycloalkyl, carboxy-C₃-C₇-cycloalkyl or substituted C₁-C₈-alkyl whereby the C₁-C₈-alkyl group is substituted by one or more halogen, cyano, nitro, amino, mercapto, C₁-C₈-alkylthio, an aromatic group defined above by R¹¹¹, carboxy (including salts thereof), C₁-C₇-alkanoylamino, carbamoyl, C₂-C₉-alkoxycarbonyl, phenylmethoxycarbonyl, diphenylmethoxycarbonyl, hydroxy-C₁-C₈-alkoxyphosphinyl, dihydroxyphosphinyl, hydroxy(phenylmethoxy)phosphinyl or di-(C₁-C₈-alkoxy)-phosphinyl residues,
characterized in that correspondingly substituted starting compounds are used.

41. A process for the manufacture of compounds according to claim 40 in which R¹⁰¹ has the formula 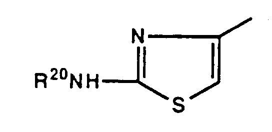 wherein R⁰ signifies hydrogen or an amino protecting group and R¹³⁰ signifies hydrogen, C₁-C₈-alkyl or a group of the formula 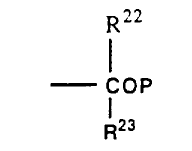 wherein R and R³ signify hydrogen or C₁-C₈-alkyl or together with the adjacent carbon atom signify a C₃-C₇ carbocyclic ring and P signifies hydroxy or -NHR¹⁹, wherein R¹⁹ represents hydrogen, C₁-C₈-alkyl, amino, C₁-C₈-alkylamino, arylamino or acylamino,
characterized in that correspondingly substituted starting compounds are used.

42. A process for the manufacture of compounds according to claim 41 in which R⁰ signifies hydrogen or triphenylmethyl, characterized in that correspondingly substituted starting compounds are used.

43. A process for the manufacture of compounds according to any one of claims 38 and 40-42 in the syn form or as a mixture in which the syn form predominates, characterized in that correspondingly substituted starting compounds are used.

44. A process for the manufacture of compounds according to claim 26,
[6R-[6α,7β(Z)]]-7-[[(2-amino-4-thiazolyl)(methoxyimino)acetyl]amino]-3-[[[[4-(3-carboxy-1-ethyl-6-fluoro-1,4-dihydro-4-oxo-7-quinolinyl)-1-piperazinyl]-carbonyl]oxy]methyl]-8-oxo-5-thia-1-azabicyclo[4.2.0]oct-2-ene-2-carboxylic acid,
[6R-[6α,7β(Z)]]-7-[[(2-amino-4-thiazolyl)methoxyimino)acetyl]amino]-3-[[[[4-(3-carboxy-1-cyclopropyl-6-fluoro-1,4-dihydro-4-oxo-7-quinolinyl)-1-piperazinyl]carbonyl]oxy]methyl]-8-oxo-5-thia-1-azabicyclo[4.2.0]-oct-2-ene-2-carboxylic acid,
(6R-trans)-3[[[[4-(3-carboxy-1-cyclopropyl-6-fluoro-1,4-dihydro-4-oxo-7-quinolinyl)-1-piperazinyl]carbonyl]oxy]methyl]-8-oxo-7-[(phenoxyacetyl)amino]-5-thia-1-azabicyclo-[4.2.0]oct-2-ene-2-carboxylic acid,
[6R-[6α,7β(Z)]]-7-[[(2-amino-4-thiazolyl)-[(1-carboxy-1-methyl-ethoxy)imino]acetyl]amino]-3-[[[[4-(3-carboxy-1-cyclopropyl-6-fluoro-1,4-dihydro-4-oxo-7-quinolinyl)-1-piperazinyl]carbonyl]oxy]methyl]-8-oxo-5-thia-1-azabicyclo[4.2.0]-oct-2-ene-2-carboxylic acid,
[6R-[6α,7β(Z)]]-7-[[(2-amino-4-thiazolyl)-[(carboxymethoxy)imino]acetyl]amino]-3-[[[[4-(3-carboxy-1-cyclo-propyl-6-fluoro-1,4-dihydro-4-oxo-7-quinolinyl)-1-piperazinyl]carbonyl]oxy]methyl]-8-oxo-5-thia-1-azabicyclo[4.2.0]oct-2-ene-2-carboxylic acid,
[6R-[6α,7β(Z)]]-7-[[(2-amino-4-thiazolyl)(methoxyimino)acetyl]amino]-3-[[[[4-(9-cyclopropyl-6-fluoro-2,3,4,9-tetrahydro-3,4-dioxoisothiazolo[5,4-b]quinolin-7-yl)-1-piperazinyl]carbonyl]oxy]methyl]-8-oxo-5-thia-1-azabicyclo[4.2.0]-oct-2-ene-2-carboxylic acid and
[6R-[6α,7β(Z)]]-7-[[(2-amino-4-thiazolyl)(methoxyimino)acetyl]amino]-3-[[[[4-(9-cyclopropyl-6-fluoro-2,3,4,9-tetrahydro-3,4-dioxoisooxazolo[5,4-b]quinolin-7-yl)-1-piperazinyl]carbonyl]oxy]methyl]-8-oxo-5-thia-1-azabicyclo[4.2.0]-oct-2-ene-2-carboxylic acid
as well as salts of these compounds and hydrates of these compounds and salts, characterized in that correspondingly substituted starting compounds are used.

45. A process for the manufacture of sodium salts of the compounds according to claim 44, characterized in that correspondingly substituted starting compounds are used.

46. A process according to any one of claims 1-45, characterized in that process alternative (e) is carried out.

47. A process according to any one of claims 1-45, characterized in that one of process alternatives (a), (c), (d), (f), (g) and (i) is carried out.

48. A process for the manufacture of pharmaceutical preparations, characterized by mixing an acyl derivative of formula I defined in claim 1 or a readily hydrolyzable ester or a salt of this compound or a hydrate of a compound of formula I or of an ester or salt thereof as the active ingredient with non-toxic, inert, solid and liquid carriers and/or excipients which are usual in such preparations and which are suitable for therapeutic administration.

49. The use of compounds according to any one of claims 1-47 for the manufacture of medicaments for the treatment or prophylaxis of infectious diseases.

50. Compounds of the formula 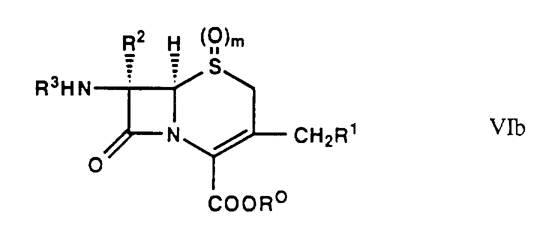 wherein R¹, R or R³ and m have the significance given above and R^{o} represents t-butyl, benzhydryl, p-nitrobenzyl or allyl.

51. (6R-trans)-4-[3-Carboxy-6,8-difluoro-1-(2-fluoroethyl)-1,4-dihydro-4-oxo-7-quinolinyl]-1-[[2-(1,1 -dimethylethoxy)carbonyl-7-(formylamino)-8-oxo-5-thia-1-azabicyclo-[4.2.0]oct-2-en-3-yl]methyl]-1-methylpiperazinium iodide.

52. Compounds of the formula 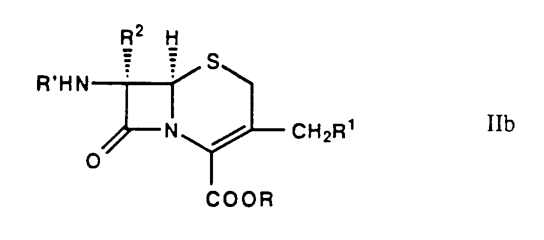 wherein R¹ and R have the significance given above, R represents hydrogen or a carboxylic acid protecting group and R' represents hydrogen or a readily cleavable amino protecting group.

53. (6R-trans)-3-[[[[4-(3-Carboxy-1-cyclopropyl-6-fluoro-1,4-dihydro-4-oxo-7-quinolinyl)-1-piperazinyl]carbonyl]oxy]methyl]-7-[[(1,1-dimethylethoxy)carbonyl]amino]-8-oxo-5-thia-1-azabicyclo[4.2.0]oct-2-ene-2-carboxylic acid diphenylmethyl ester.

## Revendications (Revendications pour l'(les) Etat(s) contractant(s) suivant(s): AT, BE, CH, DE, FR, GB, IT, LI, LU, NL, SE)

1. Dérivés acylés de formule générale 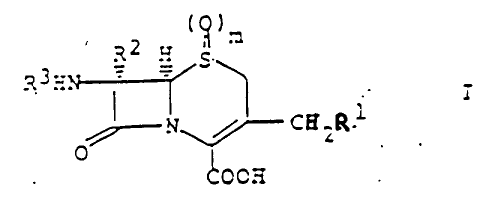 où R¹ représente un N-hétérocycle substitué de formules 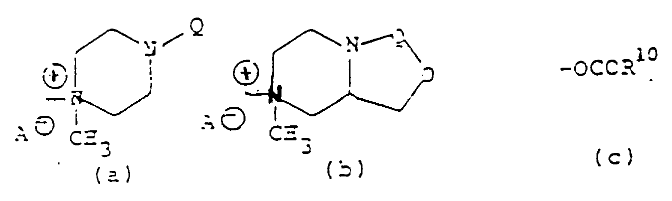 où R¹⁰ représente l'un des groupes 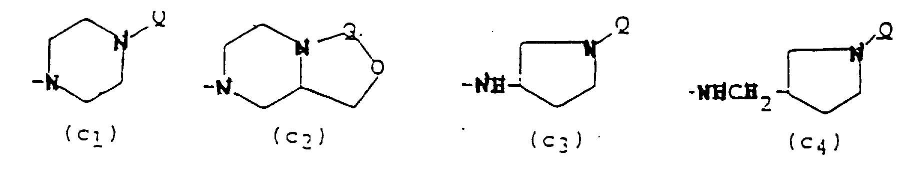 et Q représente un groupe quinolyle substitué ou un groupe naphtyridinyle, où le N-hétérocycle peut être substitué le cas échéant par un ou plusieurs groupes alkyle C₁₋₈ ; et où en outre R représente un hydrogène, alcoxy C₁₋₈, un alkylthio C₁₋₈ ou un alcanoylamino C₁₋₇, R³ représente un groupe acyle, m 0, 1 ou 2 et A un anion compatible pharmaceutiquement,
ainsi que des esters facilement hydrolysables et dès sels pharmaceutiquement compatibles de ces composés et des hydrates des composés de formule I ou de leurs esters et leurs sels.

2. Composés selon la revendication 1, caractérisés en ce que R¹ représente l'un des groupes (a) et (b).

3. Composés selon la revendication 2, caractérisés en ce que m représente 0 et R l'hydrogène.

4. Composés selon la revendication 2 ou 3, caractérisés en ce que R¹ possède la formule suivante : 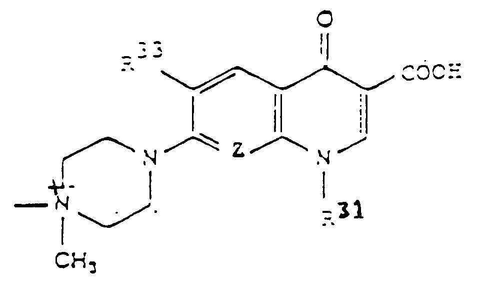 où Z représente R³⁰-C ou l'azote ; R³⁰ est l'hydrogène, un halogène ou un pont oxyméthylène (-CH₂O-) avec le reste pipérazine en formant un cycle condensé à 6 maillons ; R³¹ est l'hydrogène, un alkyle C₁₋₈, alcényle C₂₋₆, cycloalkyle C₃₋₇, halogénoalkyle C₁₋₈ ou mono-, di- ou trihalogénophényle ; ou R³⁰ et R³¹ forment ensemble un groupe alkylène C₃₋₅, un groupe alkylène monooxy C₂₋₄, un groupe alkylène dioxy C₁₋₂ ou un groupe de formule -OCH₂N(CH₃) ; et R³³ est un hydrogène ou un halogène.

5. Composés selon la revendication 4, caractérisés en ce que le Z représente le groupe R³⁰-C où R³⁰ est l'hydrogénie, le chlore, le brome ou le fluor, R³¹ est un alkyle C₁₋₈, un halogénoalkyle C₁₋₈ ou un cycloalkyle C₃₋₇ et R³³ est un hydrogène, chlore ou fluor.

6. Composés selon la revendication 5, caractérisés en ce que R³⁰ représente un hydrogène ou un fluor, R³¹ est un éthyle, fluoroéthyle ou cyclopropyle et R³³ est un hydrogène ou un fluor.

7. Composés selon la revendication 2 ou 3, caractérisés en ce que R¹ a la formule suivante :

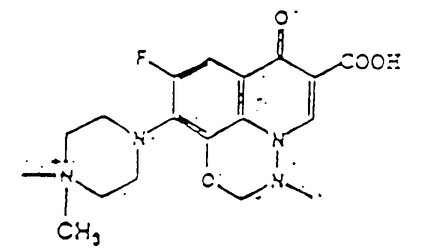

8. Composés selon la revendication 2 ou 3, caractérisés en ce que R¹ a la formule suivante :

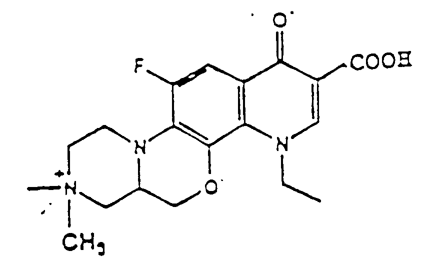

9. Composés selon l'une des revendications 2-8, caractérisés en ce que R³ est un groupe acyle aliphatique de formule :
R⁵-CO-
où R⁵ est un hydrogène, alkyle C₁₋₈, cycloalkyle C₃₋₇, alcoxy C₁₋₈, alcényle C₂₋₆, cycloalcényle C₃₋₇ ou cyclohexadiényle ; ou un alkyle C₁₋₈ substitué par un ou plusieurs des restes halogène, cyano, nitro, amino, mercapto, alkylthio C₁₋₈ ou cyanométhylthio ou un alcényle C₂₋₆.

10. Composés selon l'une des revendications 2-8, caractérisés en ce que R³ est un groupe acyle aromatique de formules générales 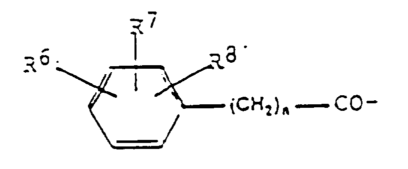 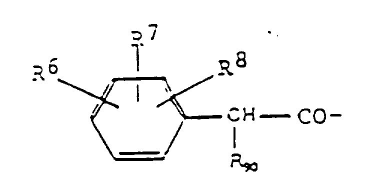 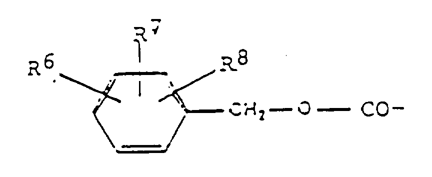 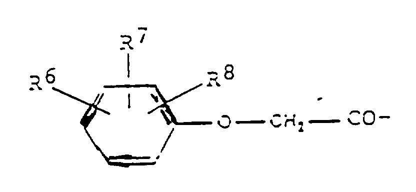 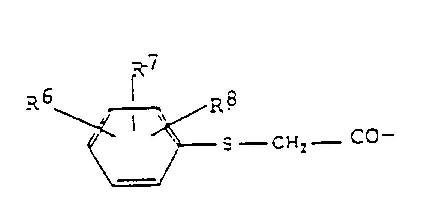 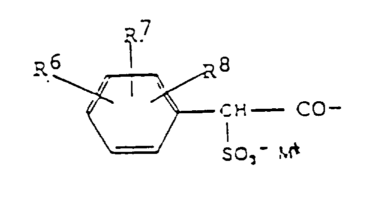 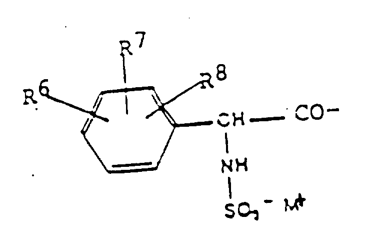 où n représente 0, 1, 2 ou 3 ; R⁶, R⁷ et R⁸ représentent chacun un hydrogène, halogène, hydroxy, nitro, amino, cyano, trifluorométhyle, alkyle C₁₋₄, alcoxy C₁₋₄ ou aminométhyle, R⁹⁰ représente un amino, acylamino, hydroxy, un sel carboxylique, un carboxy protégé, formyloxy, azido ou un sel sulfo et M est un cation.

11. Composés selon l'une des revendications 2-8, caractérisés en ce que R³ représente un groupe acyle hétéroaromatique de formules générales
R¹⁰¹-(CH₂)ₙ-CO- 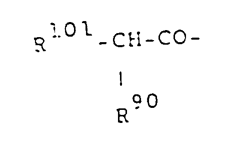
R¹⁰¹-O-CH₂-CO-
R¹⁰¹-S-CH₂-CO-
et
R¹⁰¹-CO-CO-
où n représente 0, 1, 2 ou 3 ; R⁹⁰ représente un amino, acylamino, hydroxy, un sel carboxylique, un carboxy protégé, azido ou un sel sulfo et R¹⁰¹ représente un cycle hétérocyclique à 5, 6 ou 7 maillons substitués le cas échéant avec 1, 2, 3, ou 4 atomes d'azote, d'oxygène et/ou de soufre.

12. Composés selon l'une des revendications 2-8, caractérisés en ce que R³ représente un groupe [[(4-substitué-2,3-dioxo-1-pipérazinyl)carbonyl]amino]acétyle de formule 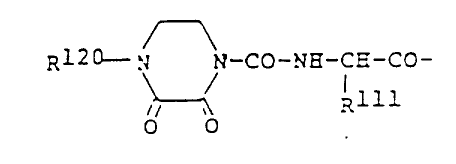 où R¹¹¹ représente un alkyle C₁₋₈, un hydroxy-alkyle C₁₋₈ ou un groupe aromatique de formule générale 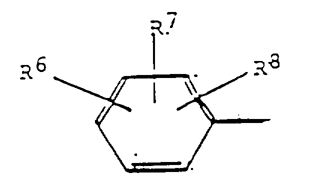 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitués le cas échéant avec 1, 2, 3 ou 4 atomes d'azote, d'oxygène et/ou de soufre et R¹⁰ représente un groupe alkyle C₁₋₈ substitué par un ou plusieurs des restes halogène, cyano, nitro, amine et/ou mercapto.

13. Composés selon l'une des revendications 2-8, caractérisés en ce que R³ représente un groupe (acylamino)acétyle de formule générale 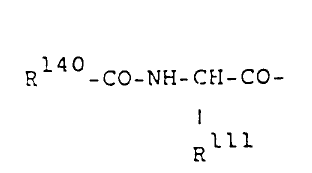 où R¹¹¹ représente un groupe alkyle C₁₋₈, un hydroxyalkyle C₁₋₈ ou un groupe aromatique de formule 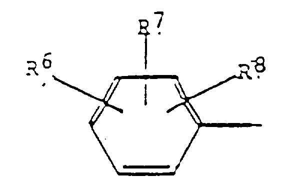 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitués le cas échéant avec 1, 2, 3, ou 4 atomes d'azote, d'oxygène et/ou de soufre, et où R¹⁴⁰ représente le groupe 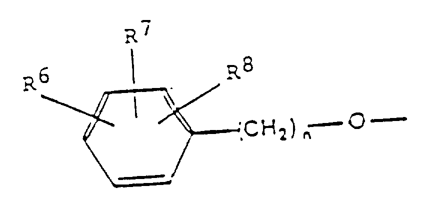 (où R⁶, R⁷ et R⁸ ont la signification ci-dessus et n représente 0, 1, 2 ou 3), un hydrogène, un alkyle C₁₋₈, alkyle C₁₋₈ substitué, amino, alkylamino C₁₋₈, di(alkyle C₁₋₈) amino, (cyano-alkyle C₁₋₈)amino, hydrazino, alkylhydrazino C₁₋₈, arylhydrazino ou acylhydrazino.

14. Composés selon l'une des revendications 2-8, caractérisés en ce que R³ représente un groupe (oxyimino substitué)acétyle de formule générale 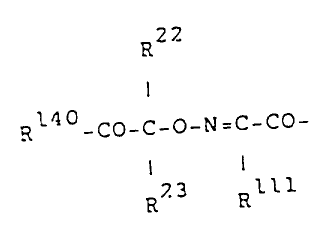 où R¹¹¹ représente un alkyle C₁₋₈, hydroxy-alkyle C₁₋₈ ou un groupe aromatique de formule 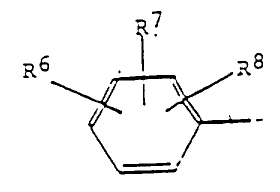 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitués le cas échéant avec 1, 2, 3 ou 4 atomes d'azote, d'oxygène et/ou de soufre , et où en outre R¹⁴⁰ représente le groupe 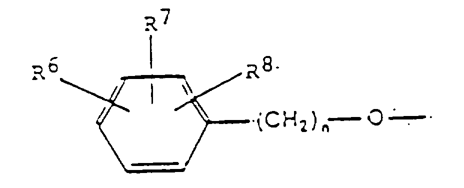 (où R⁶, R⁷ et R⁸ ont la signification ci-dessus et n représente 0, 1, 2 ou 3), un hydrogène, un alkyle C₁₋₈, un alkyle substitué C₁₋₈, amino, alkylamino C₁₋₈, di(alkyle C₁₋₈)amino, (cyanoalkyle C₁₋₈)amino, hydrazino, alkylhydrazino C₁₋₈, arylhydrazino ou acylhydrazino et R et R³ représentent un hydrogène ou un alkyle C₁₋₈ ; ou R et R³ forment ensemble avec l'atome de carbone voisin un cycle carbocyclique C₃₋₇.

15. Composés selon l'une des revendications 2-8, caractérisés en ce que R³ représente un groupe [[[3-substitué-2-oxo-1-imidazolidinyl]carbonyl]amino]acétyle de formule générale 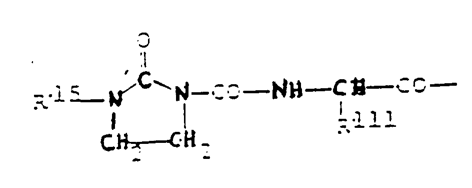 où R¹¹¹ représente un alkyle en C₁₋₈, hydroxy-alkyle en C₁₋₈ ou un groupe aromatique de formule 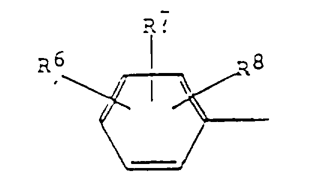 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons le cas échéant substitué avec 1, 2, 3 ou 4 atomes d'azote, d'oxygène et/ou de soufre ; et où en outre R¹⁵ représente un hydrogène, un alkylesulfonyle C₁₋₈, arylméthylèneamino (c'est-à-dire -N=CHR¹¹¹, où R¹¹¹ a la signification donnée ci-dessus), R¹⁶-CO- (où R¹⁶ représente un hydrogène ou un alkyle C₁₋₈ substitué par un halogène), un groupe aromatique défini ci-dessus pour R¹¹¹ ou un alkyle C₁₋₈ substitué par un ou plusisieurs des restes halogène, cyano, nitro, amino et/ou mercapto.

16. Composés selon l'une des revendications 2-8, caractérisés en ce que R³ représente un groupe (oxyimino substitué)arylacétyle de formule générale 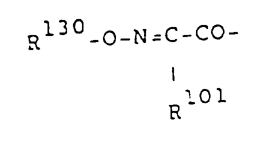 où R¹⁰¹ représente un cycle hétérocyclique à 5, 6 ou 7 maillons substitué le cas échéant avec 1, 2, 3 ou 4 atomes d'azote, d'oxygène et/ou de soufre, où le cycle hétérocyclique est substitué par un halogène, hydroxy, nitro, amino, cyano, trifluorométhyle, alkyle C₁₋₄ ou alcoxy C₁₋₄ et R¹³⁰ est un hydrogène, alkyle C₁₋₈, cycloalkyle C₃₋₇, carboxy cycloalkyle C₃₋₇ ou un alkyle C₁₋₈ substitué, où le groupe alkyle C₁₋₈ est substitué par un ou plusieurs des restes halogène, cyano, nitro, amino, mercapto, alkylthio C₁₋₈, un groupe aromatique défini ci-dessus par R¹¹¹, carboxy (y compris ses sels), alcanoylamino C₁₋₇, carbamoyle, alcoxycarbonyle C₂₋₉, phénylméthoxycarbonyle, diphénylméthoxycarbonyle, hydroxy, alcoxyphosphinyle C₁₋₈, dihydroxyphosphinyle, hydroxy(phénylméthoxy)phosphinyle ou di(alcoxy C₁₋₈)phosphinyle.

17. Composés selon la revendication 16, caractérisés en ce que R¹⁰¹ a la formule 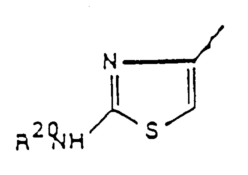 où R⁰ représente un hydrogène ou un groupe protecteur d'amino et R¹³⁰ représente un hydrogène, alkyle C₁₋₈ ou un groupe de formule générale 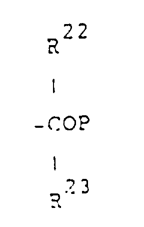 où R et R³ représentent un hydrogène ou un alkyle C₁₋₈ ou forment ensemble avec l'atome de carbone voisin un cycle carbocyclique C₃₋₇ et P est un hydroxy ou -NHR¹⁹ où R¹⁹ est un hydrogène, alkyle C₁₋₈, amino, alkylamino C₁₋₈, arylamino ou acylamino.

18. Composés selon la revendication 17, caractérisés en ce que R⁰ est un hydrogène, et R¹³⁰ est un méthyle ou un groupe de formule 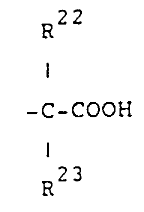 où R et R³ représentent un hydrogène ou un méthyle.

19. Composés selon l'une des revendications 14 et 16-18 sous la forme syn, ou sous forme de mélanges, où la forme syn est prépondérante.

20. Composés selon la revendication 2, caractérisés en ce que R¹ représente le groupe (a) et R³ représente le phénoxyacétyle.

21. Composés selon la revendication 2, caractérisés en ce qu'ils sont :
Iodure de (6R-trans)-4-[3-carboxy-1-(2-fluoroéthyl)-6,8-difluoro-1,4-dihydro-4-oxoquinol-7-yl]-[[2-carboxy-8-oxo-7-[(phénoxyacétyl)amino]-5-thia-1-azabicyclo[4.2.0]oct-2-ène-3-yl]méthyl]-1-méthylpipérazinium. [6R-[6α,7β(Z)]]-1-[[7-[[[(2-amino-4-thiazolyl)[1-(1-carboxy-1-méthyl)éthoxy]imino]acétyl]amino]-2-carboxy-8-oxo-5-thia-1-azabicyclo[4.2.0]oct-2-én-3-yl]méthyl]-4-[3-carboxy-1-(2-fluoroéthyl)-6,8-difluoro-1,4-dihydro-4-oxoquinol-7-yl]-1-méthylpipérazinium hydroxyde sel monosodique interne et
iodure de [6R-[6α,7β(Z)]]-1-[[7-[[(2-amino-4-thiazolyl)(méthoxyimino)acétyl]amino]-2-carboxy-8-oxo-5-thia-1-azabicyclo-[4.2.0]oct-2-én-3-yl]méthyl]-4-(3-carboxy-1-éthyl-6-fluoro-1,4-dihydro-4-oxo-7-quinolyl)-1-méthylpipérazinium.

22. Iodure de [6R-[6α,7β(Z)]-1-[[[(2-amino-4-thiazolyl)méthoxyimino)acétyl]amino]-2-carboxy-8-oxo-5-thia-1-azabicyclo[4.2.0]oct-2-én-3-yl]méthyl]-4-[3-carboxy-6,8-difluoro-1-(2-fluoroéthyl)-1,4-dihydro-4-oxo-7-quinolyl]-1-méthylpipérazinium.

23. [6R-[6α,7β(Z)]]-1-[[[(2-amino-4-thiazolyl)(méthoxyimino)acétyl]amino]-2-carboxy-8-oxo-5-thia-1-azabicyclo[4.2.0]oct-2-én-3-yl]méthyl]-4-[3-carboxy-6,8-difluoro-1-(2-fluoroéthy1)-1,4-dihydro-4-oxo-7-quinolyl]-1-méthyl-pipérazinium hydroxyde sel interne monosodique.

24. Trifluoroacétate de (6R-trans)-4-[3-carboxy-6,8-difluoro-1-(2-fluoroéthyl)-1,4-dihydro-4-oxo-7-quinolyl]-1-[[2-carboxy-7-(formylamino)-8-oxo-5-thia-1-azabicyclo(4.2.0]oct-2-én-3-yl]méthyl]-1-méthylpipérazinium.

25. Iodure de [6R-[6α,7β(Z)]]-1-[[7-[[(2-amino-4-thiazolyl) [[1,1-diméthyl-2-(1,1-diméthyléthoxy)-2-oxoéthoxy]imino]acétyl]amino]-2-carboxy-8-oxo-5-thia-1-azabicyclo-[4.2.0]oct-2-én-3-yl]méthyl]-4-[3-carboxy-(2-fluoroéthyl)-6,8-difluoro-1,4-dihydro-4-oxoquinol-7-yl]-1-méthylpipérazinium.

26. Composés selon la revendication 1, caractérisés en ce que R¹ représente le groupe (c).

27. Composés selon la revendication 26, caractérisés en ce que m représente 0 ou 1.

28. Composés selon la revendication 26 ou 27, caractérisés en ce que Q représente un groupe de formule 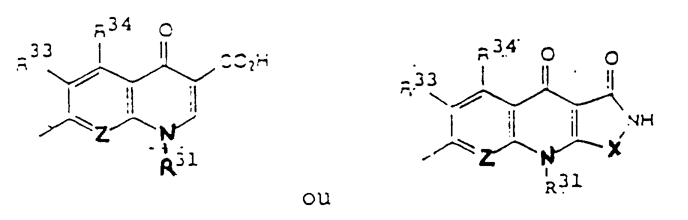 où Z représente R³⁰-C ou un azote ; X représente un oxygène ou un soufre ; R³⁰ est un hydrogène, halogène ou un pont oxométhylène (-CH₂O-) fixé au reste pipérazine en formant un cycle condensé à six maillons ; R³¹ est un hydrogène, alkyle C₁₋₈, alcényle C₂₋₆, cycloalkyle C₃₋₇, halogénoalkyle C₁₋₈ ou mono-, di- ou trihalogénophényle ; R³⁰ et R³¹ forment ensemble un groupe alkylène C₃₋₅, un groupe monoxy alkylène C₂₋₄, un groupe alkylène dioxy C₁₋₂ ou un groupe de formule -OCH₂N(CH₃)- ; R³³ est un hydrogène ou un halogène ; et R³⁴ est un hydrogène ou un amino.

29. Composés selon la revendication 28, caractérisés en ce que Z représente le groupe R³⁰-C, où R³⁰ est un hydrogène, chlore, brome ou fluor, R³¹ est un alkyle C₁₋₈, halogénoalkyle C₁₋₈ ou cycloalkyle C₃₋₇ et R³³ est un hydrogène, chlore ou fluor.

30. Composés selon la revendication 29, caractérisés en ce que R³⁰ représente un hydrogène ou fluor, R³¹ est un éthyle, fluoroéthyle ou cyclopropyle et R³³ est un hydrogène ou fluor.

31. Composés selon la revendication 28, caractérisés en ce que R¹ a la formule suivante

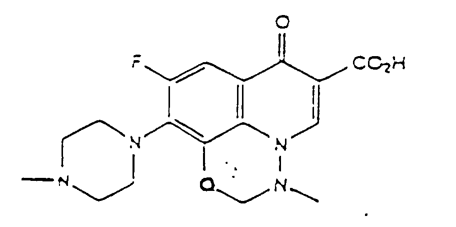

32. Composés selon la revendication 28, caractérisés en ce que R¹ a la formule suivante :

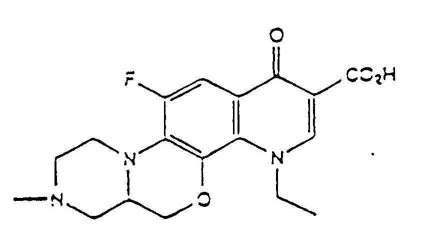

33. Composés selon l'une des revendications 26-32, caractérisés en ce que R³ est un groupe acyle aliphatique de formule
R⁵-CO-
où R⁵ est un hydrogène, alkyle C₁₋₈, cycloalkyle C₃₋₇, alcoxy C₁₋₈, alcényle C₂₋₆, cycloalcényle C₃₋₇ ou cyclohexadiényle ; ou un groupe alkyle C₁₋₈ substitué par un ou plusieurs des restes halogène, cyano, nitro, amino, mercapto, alkylthio C₁₋₈ ou cyanométhylthio ou alcényle C₂₋₆.

34. Composés selon l'une des revendications 26-32 caractérisés en ce que R³ est un groupe acyle aromatique de formules générales 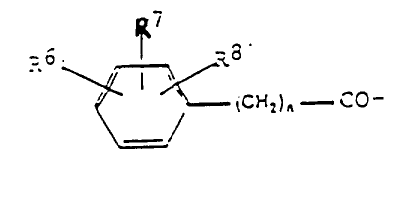 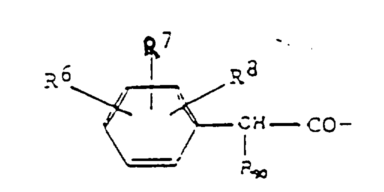 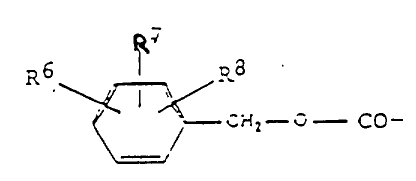 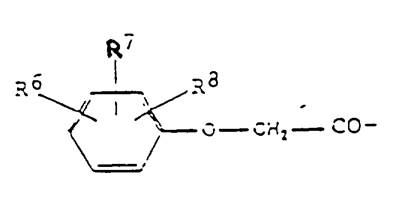 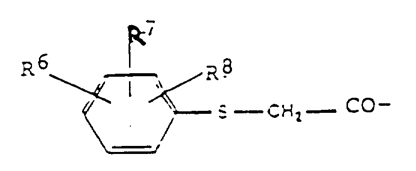 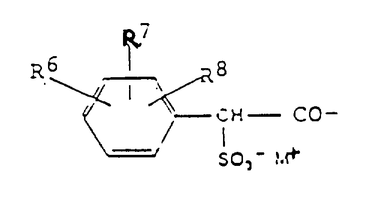 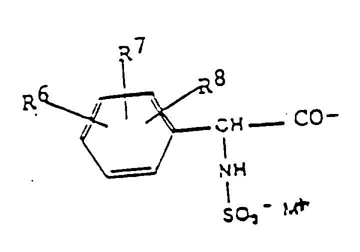 où n représente 0, 1, 2 ou 3 ; R⁶, R⁷ et R⁸ représentent chacun un hydrogène, halogène, hydroxy, nitro, amino, cyano, trifluorométhyle, alkyle C₁₋₄, alcoxy C₁₋₄ ou aminométhyle, R⁹⁰ est un amino, acylamino, hydroxy, un sel carboxylique, un carboxy protégé, formyloxy, azido ou un sel sulfo et M est un cation.

35. Composés selon l'une des revendications 26-32, caractérisés en ce que R³ est un groupe acyle hétéroaromatique de formules générales
R¹⁰¹-(CH₂)ₙ-CO- 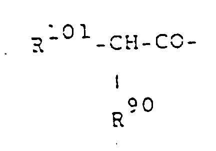
R¹⁰¹-O-CH₂-CO-
R¹⁰¹-S-CH₂-CO-
et
R¹⁰¹-CO-CO-
où n représente 0, 1, 2 ou 3 ; R⁹⁰ représente un amino, acylamino, hydroxy, un sel carboxylique, carboxy protégé, azido, ou un sel sulfo et R¹⁰¹ représente un cycle hétérocyclique à 5, 6 ou 7 maillons le cas échéant substitué avec 1, 2, 3 ou 4 atomes d'azote, d'oxygène et/ou de soufre.

36. Composés selon l'une des revendications 2632, caractérisés en ce que R³ représente un groupe [[(4-substitué-2,3-dioxo-1-pipérazinyl)carbonyl]amino]-acétyle de formule 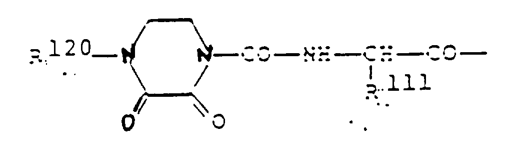 où le groupe R¹¹ représente un groupe alkyle C₁₋₈, hydroalkyle C₁₋₈ ou un groupe aromatique de formule générale 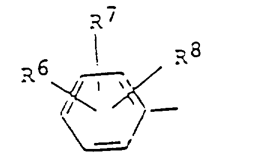 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitué le cas échéant avec 1, 2, 3 ou 4 atomes d'azote, d'oxygène et/ou de soufre et R¹⁰ représente un groupe alkyle C₁₋₈ substitué le cas échéant par un ou plusieurs des restes halogène, cyano, nitro, amino et/ou mercapto.

37. Composés selon l'une des revendications 26-32, caractérisés en ce que R³ représente un groupe (acétylamino)acétyle de formule générale 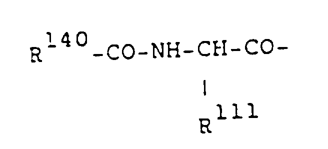 où R¹¹¹ représente un alkyle C₁₋₈, hydroxyalkyle C₁₋₈ ou un groupe aromatique de formule 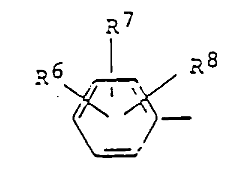 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitué le cas échéant avec 1, 2, 3 ou 4 atomes d'azote, d'oxygène et/ou de soufre, et où en outre R¹⁴⁰ représente le groupe 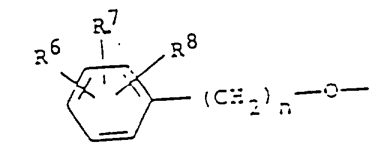 (où R⁶, R⁷ et R⁸ ont la signification ci-dessus, et n représente 0, 1, 2 ou 3), est un hydrogène, alkyle C₁₋₈, alkyle C₁₋₈ substitué, amino, alkylamino C₁₋₈, di-(alkyle C₁₋₈)-amino, (cyanoalkyle C₁₋₈)amino, hydrazino, alkylhydrazino C₁₋₈, arylhydrazino ou acylhydrazino.

38. Composés selon l'une des revendications 26-32, caractérisés en ce que R³ représente un groupe (oxyimino substitué)acétyle de formule générale 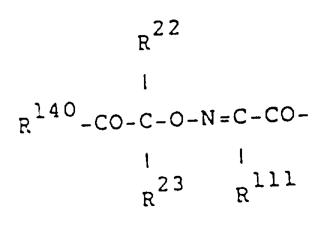 où R¹¹¹ représente un alkyle C₁₋₈, hydroxyalkyle C₁₋₈ ou un groupe aromatique de formule 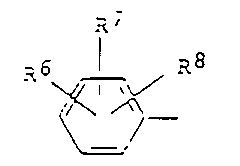 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitué le cas échéant avec 1, 2, 3 ou 4 atomes d'azote, d'oxygène et/ou de soufre, où en outre R¹⁴⁰ représente le groupe 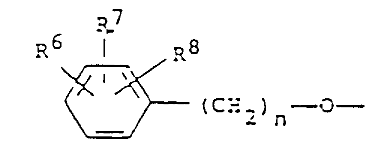 (où R⁶, R⁷ et R⁸ ont la signification ci-dessus, et n représente 0, 1, 2 ou 3), est un hydrogène, alkyle C₁₋₈, alkyle C₁₋₈ substitué, amino, alkylamino C₁₋₈, di(alkyle C₁₋₈)amino, (cyanoalkyle C₁₋₈)-amino, hydrazino, alkylhydrazino C₁₋₈, arylhydrazino ou acylhydrazino et R et R³ représentent un hydrogène ou un alkyle C₁-₈ ou R et R³ forment ensemble avec l'atome de carbone voisin un cycle carbocyclique C_{3-7.}

39. Composés selon l'une des revendications 26-32, caractérisés en ce que R³ représente un groupe [[[3-substitué-2-oxo-1-imidazolidinyl]carbonyl]amino]acétyle de formule générale 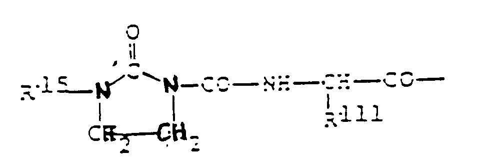 où R¹¹¹ représente un groupe alkyle C₁₋₈, hydroxyalkyle C₁₋₈ ou un groupe aromatique de formule 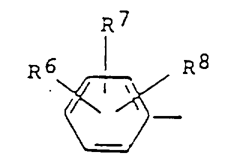 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitué le cas échéant avec 1, 2, 3 ou 4 atomes d'azote, d'oxygène et/ou de soufre ; et où en outre R¹⁵ représente un hydrogène, un alkylsulfonyle C₁₋₈, un arylméthylèneamino (c'est-à-dire -N=CHR¹¹¹, où R¹¹¹ a la signification donnée ci-dessus), R¹⁶CO- (où R¹⁶ représente un hydrogène ou le cas échéant un alkyle C₁₋₈ substitué par un halogène), un groupe aromatique comme c'est défini pour R¹¹¹ ou un groupe alkyle C₁₋₈ substitué le cas échéant par un ou plusieurs des testes halogène, cyano, nitro, amino et/ou mercapto.

40. Composés selon l'une des revendications 26-32, caractérisés en ce que R³ représente un groupe (oxyimino substitué)arylacétyle de formule générale 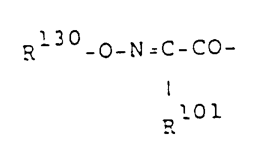 où R¹⁰¹ représente un cycle hétérocyclique à 5, 6 ou 7 maillons substitué le cas échéant avec 1, 2, 3 ou 4 atomes d'azote, d'oxygène et/ou de soufre, où le cycle hétérocyclique est substitué par un halogène, hydroxy, nitro, amino, cyano, trifluorométhyle, alkyle C₁₋₄ ou alcoxy C₁₋₄ et R¹³⁰ représente un hydrogène, alkyle C₁₋₈, cycloalkyle C₃₋₇, carboxycycloalkyle C₃₋₇ ou un alkyle C₁₋₈ substitué où le groupe alkyle C₁₋₈ est substitué par un ou plusieurs des restes halogène, cyano, nitro, amino, mercapto, alkylthio C₁₋₈, un groupe aromatique défini par R¹¹¹ ci-dessus, carboxy (y compris ses sels), alcanoylamino C₁₋₇, carbamoyle, alcoxycarbonyle C₂₋₉, phénylméthoxycarbonyle, diphénylméthoxycarbonyle, hydroxy-alcoxyphosphinyle C₁₋₈, dihydroxyphosphinyle, hydroxy(phénylméthoxy)phosphinyle ou un di(alcoxy C₁₋₈)phosphinyle.

41. Composés selon la revendication 40, caractérisés en ce que R¹⁰¹ a la formule 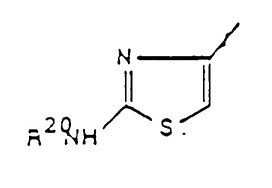 où R⁰ représente un hydrogène ou un groupe protecteur d'amino, R¹³⁰ est un hydrogène, alkyle C₁₋₈ ou un groupe de formule générale 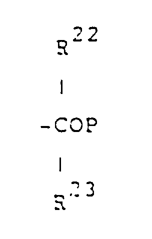 où R et R³ représentent un hydrogène, ou un alkyle C₁₋₈ ou forment ensemble avec l'atome de carbone voisin un cycle carbocyclique C₃₋₇ et P représente un hydroxy ou -NHR¹⁹ où R¹⁹ est un hydrogène, alkyle C₁₋₈, amino, alkylamino C₁₋₈, arylamino ou acylamino.

42. Composés selon la revendication 41, caractérisés en ce que R⁰ représente un hydrogène ou triphénylméthyle.

43. Composés selon l'une des revendications 38 et 40-42 qui sont sous la forme syn ou sous forme de mélanges, où la forme syn est prépondérante.

44. Composés selon la revendication 26, caractérisés par les composés :
Acide [6R-[6α,7β(Z)]]-7-[[(2-amino-4-thiazolyl)(méthoxyimino)acétyl]amino]-3-[[[(4-(3-carboxy-1-éthyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique
Acide [6R-[6α,7β(Z)]]-7-[[(2-amino-4-thiazolyl)méthoxyimino)acétyl]amino]-3-[[[[4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide (6R-trans)-3-[[[[4-(3-carboxy-1-cyclopropyl-6-fluoro-1,4-dihydro-4-oxo-7-quinolyl)-1-pipérazinyl]carbonyl]oxy]méthyl]-8-oxo-7-[(phénoxyacétyl)-amino]-5-thia-1-azabicyclo[4.2.0]oct-2-èn-2-carboxylique.
Acide [6R, [6α, 7β-Z)]-7-[[(2-amino-4-thiazolyl)-[(1-carboxy-1-méthyl-éthoxy)imino]acétyl]amino]-3-[[[[(4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide [6R-[6α,7β(Z)]]-7-[[(2-amino-4-thiazolyl)[(carboxyméthoxy)imino]acétyl]amino]-3-[[[[(4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide [6R-[6α, 7β(Z)]-7-[[(2-amino-4-thiazolyl) (méthoxyimino)acétyl]amino]-3-[[[[(4-(9-cyclopropyl-6-fluoro-2,3,4-tétrahydro-3,4-dioxoisothiazolo[5,4-b]quinol-7-yl)-1-pipérazinyl]-carbonyl]oxy]méthyl]-8-oxo-5-thia-1-azabicyclo-[4.2.0]-oct-2-èn-2-carboxylique
Acide [6R-[6α,7β(Z)]]-7-[[(2-amino-4-thiazolyl)(méthoxyimino)acétyl]amino]-3-[[[[4-(9-cyclopropyl-6-fluoro-2,3,4,9-tétrahydro-3,4-dioxoisoxazolo-[5,4-b]quinol-7-yl]-1-pipérazinyl]carbonyl]oxy]méthyl]-8-oxo-5-thia-1-azabicyclo[4.2.0]oct-2-èn-2-carboxylique ainsi que les sels de ces composés et les hydrates et les sels de ces composés.

45. Sels des composés selon la revendication 44, caractérisés en ce que ce sont les sels de sodium.

46. Composés de formule 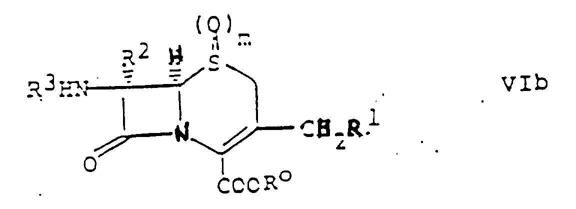 où R¹, R ou R³ et m ont les significations données ci-dessus et R⁰ représente un t-butyle, benzhydryle, p-nitrobenzyle ou allyle.

47. Iodure de (6R-trans)-4-[3-carboxy-6,8-difluoro-1-(2-fluoroéthyl)-1,4-dihydro-4-oxo-7-quinolyl]-1-[[2-(1,1-diméthyléthoxy)carbonyl-7-(formylamino-8-oxo-5-thia-1-azabicyclo[4.2.0]oct-2-èn-3-yl]méthyl]-1-méthylpipérazinium.

48. Composés de formule 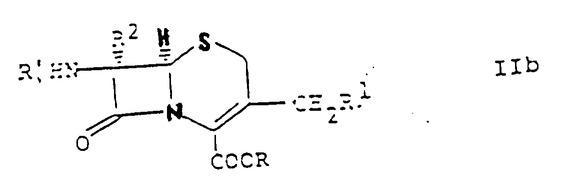 où R¹ et R ont la signification donnée ci-dessus, R représente un hydrogène ou un groupe protecteur d'acide carboxylique et R' est un hydrogène ou un groupe protecteur d'amino facile à éliminer.

49. (6R-trans)-3-[[[[(4-(3-carboxy-1-cyclopropyl-6-fluoro-1,4-dihydro-4-oxo-7-quinolyl)-1-pipérazinyl]carbonyl]oxy]méthyl]-7-[[(1,1-diméthyléthoxy)carbonyl]amino-8-oxo-5-thia-1-azabicyclo[4.2.0]oct-2-èn-2-carboxylate de diphénylméthyle.

50. Composés selon l'une des revendications 1-45, en tant que principes actifs pharmaceutiques.

51. Composés selon l'une des revendications 1-45, comme principes actifs pharmaceutiques pour le traitement et la prophylaxie de maladies infectieuses.

52. Procédé de préparation de composés selon l'une des revendications 1-45, caractérisé en ce que
a) pour préparer un composé de formule I, où m représente 0, on fait réagir un composé de formule générale 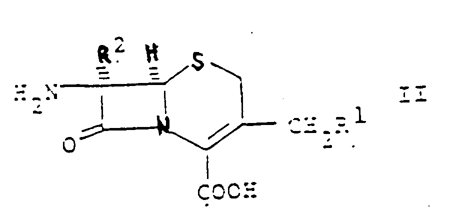 où R¹ et R ont la signification donnée ci-dessus, ou un de ses esters ou sels facilement hydrolysables de ces composés avec un acide carboxylique de formule générale R³OH ou avec un de ses dérivés réactionnels,
ou
b) pour préparer un composé de formule I, où m représente 0 et R¹ l'un des groupes (a) et (b), on fait réagir un composé de formule III 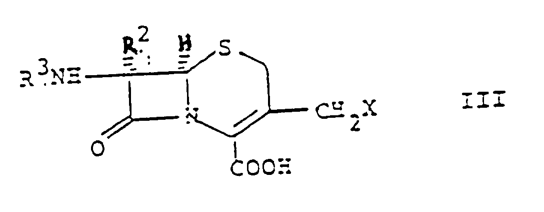 où les groupes R et R³ ont la signification donnée, X représente un groupe éliminable, et les groupes amino, hydroxy et carboxy peuvent être protégés,
ou un de ses sels ou esters facilement hydrolysables, avec un composé de formule 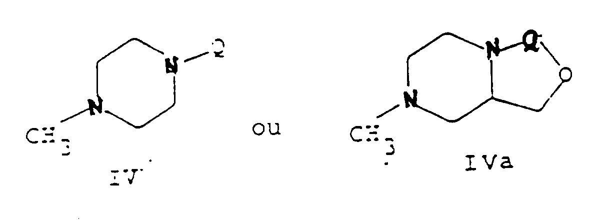 où Q a la signification donnée ci-dessus et les atomes d'azote peuvent être protégés, ou
c) pour préparer un sel facilement hydrolysable d'un acide carboxylique de formule I, où m représente 0 et R¹ représente le groupe (c), on fait réagir un composé de formule 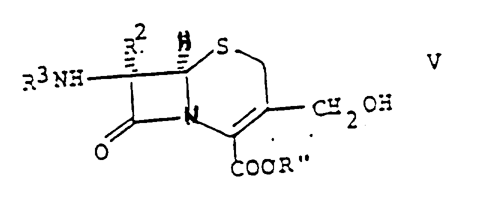 où R et R³ ont les significations données ci-dessus et R' représente le reste d'un ester facilement hydrolysable,
en présence d'une base avec du phosgène, on fait réagir le produit de réaction obtenu avec un composé de formule R¹⁰H, où R¹⁰ a la signification ci-dessus, ou cependant les fonctions éventuelles amino, hydroxy et/ou carboxy peuvent être protégées, après quoi on élimine les groupes protecteurs éventuels, ou
d) pour préparer un acide carboxylique de formule I, on transforme un ester de formule 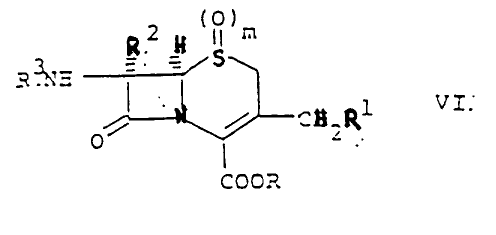 où R¹, R, R³ et m ont la signification indiquée ci-dessus et R représente un groupe protecteur d'acide carboxylique,
en l'acide carboxylique de formule I, ou
e) pour préparer un composé de formule I, où m représente 0, ou un de ses sels ou esters facilement hydrolysables, on réduit un composé de formule 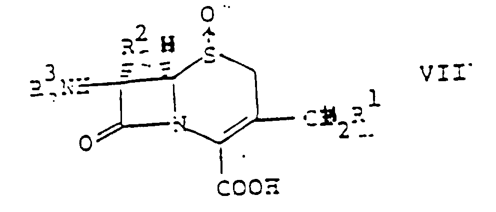 où les groupes R¹, R et R³ ont la signification donnée ci-dessus, ou un de ses sels ou esters facilement hydrolysables, ou
f) pour préparer un composé de formule I où m représente 1 ou 2 ou un de ses sels ou esters facilement hydrolysables, on oxyde un composé de formule 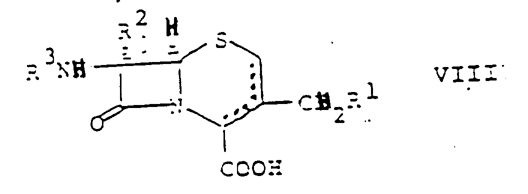 où R¹, R et R³ ont la signification donnée ci-dessus et les lignes en pointillé indiquent la présence d'une double liaison Δ2 ou Δ3,
ou un de ses esters ou de ses sels, ou
g) pour préparer un composé de formule I où R³ contient un substituant d'amino ou l'un de ses esters ou de ses sels on élimine les groupes protecteurs d'amino dans les substituants R³⁰ d'un composé de formule générale 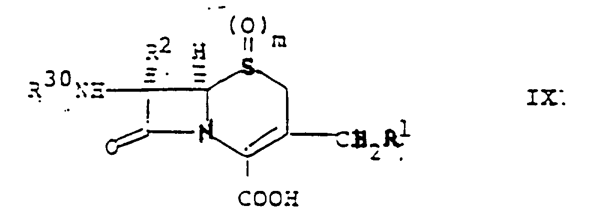 où R¹, R et m ont la signification donnée ci-dessus et R³⁰ représente un reste acyle contenant un groupe amino protégé,
ou d'un de ses sels ou de ses esters, ou
h) pour préparer un ester facilement hydrolysable d'un composé de formule I, on estérifie un acide carboxylique de formule I de façon appropriée, ou
i) pour préparer des sels ou des hydrates d'un composé de formule I ou des hydrates de ses sels, on transforme un composé de formule I en un sel ou en un hydrate, ou en un hydrate de ce sel.

53. Procédé selon la revendication 52, caractérisé en ce qu'on réalise la variante de procédé b).

54. Procédé selon la revendication 52, caractérisé en ce qu'on réalise l'une des variantes de procédé a), c), d), f), g) et i).

55. Préparations pharmaceutiques caractérisées par une teneur en un composé selon l'une des revendications 1-45.

56. Préparations pharmaceutiques pour le traitement et la prophylaxie de maladies infectieuses, caractérisées en ce qu'elles contiennent un composé selon l'une des revendications 1-45.

57. Utilisation des composés selon l'une des revendications 1-45 pour préparer des médicaments pour le traitement et la prophylaxie de maladies infectieuses.

## Revendications (Revendications pour l'(les) Etat(s) contractant(s) suivant(s): ES, GR)

1. Procédé de préparation de dérivés acylés de formule générale 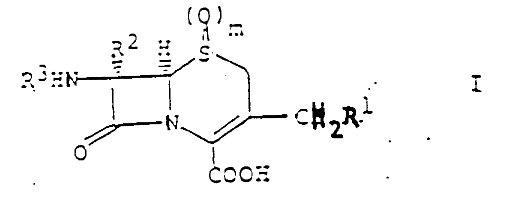 où R¹ représente un N-hétérocycle substitué de formules 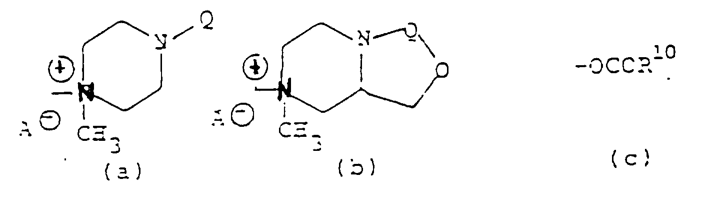 où R¹⁰ représente l'un des groupes 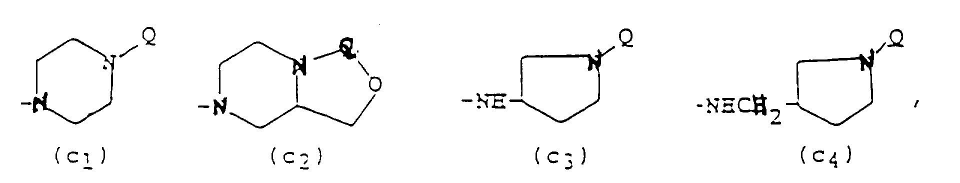 et Q représente un groupe quinolyle substitué ou un groupe naphtyridinyle, où le N-hétérocycle peut être substitué le cas échéant par un ou plusieurs groupes alkyle C₁₋₈ ; et où en outre R représente un hydrogène, alcoxy C₁₋₈, un alkylthio C₁₋₈ ou un alcanoylamino C₁₋₇, R³ représente un groupe acyle, m 0, 1 ou 2 et A un anion compatible pharmaceutiquement,
ainsi que des esters facilement hydrolysables et des sels pharmaceutiquement compatibles de ces composés et des hydrates des composés de formule I ou de leurs esters et leurs sels, caractérisé en ce que
a) pour préparer un composé de formule I, où m représente 0, on fait réagir un composé de formule générale 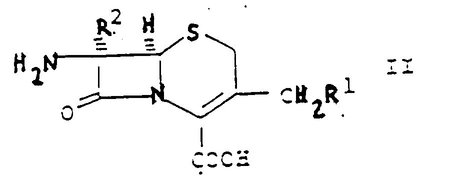 où R¹ et R ont la signification donnée ci-dessus, ou un de ses esters ou sels facilement hydrolysables de ces composés avec un acide carboxylique de formule générale R³OH ou avec un de ses dérivés réactionnels, ou
b) pour préparer un composé de formule I, où m représente 0 et R¹ l'un des groupes (a) et (b), on fait réagir un composé de formule III 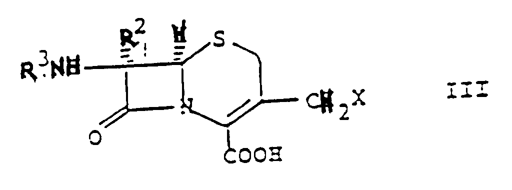 où les groupes R et R³ ont la signification donnée, X représente un groupe éliminable, et les groupes amino, hydroxy et carboxy peuvent être protégés, ou un de ses sels ou esters facilement hydrolysables, avec un composé de formule 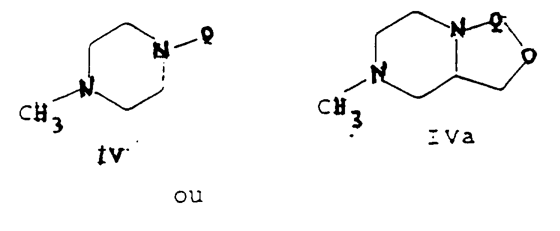 où Q a la signification donnée ci-dessus et les atomes d'azote peuvent être protégés,
ou
c) pour préparer un sel facilement hydrolysable d'un acide carboxylique de formule I, où m représente 0 et R¹ représente le groupe (c), on fait réagir un composé de formule 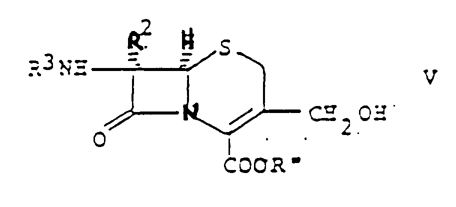
où R et R³ ont les significations données ci-dessus et R¹¹ représente le reste d'un ester facilement hydrolysable,
en présence d'une base avec du phosgène, on fait réagir le produit de réaction obtenu avec un composé de formule R¹⁰H, où R¹⁰ a la signification ci-dessus, ou cependant les fonctions éventuelles amino, hydroxy et/ou carboxy peuvent être protégées, après quoi on élimine les groupes protecteurs éventuels, ou
d) pour préparer un acide carboxylique de formule I, on transforme un ester de formule 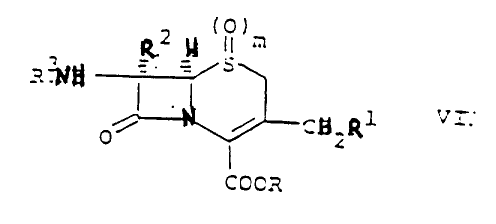 où R¹, R, R³ et m ont la signification indiquée ci-dessus et R représente un groupe protecteur d'acide carboxylique,
en l'acide carboxylique de formule I, ou
e) pour préparer un composé de formule I, où m représente 0, ou un de ses sels ou esters facilement hydrolysables, on réduit un composé de formule 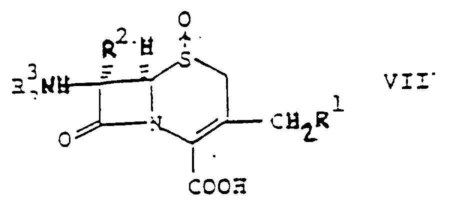 où les groupes R¹, R et R³ ont la signification donnée ci-dessus, ou un de ses sels ou esters facilement hydrolysables, ou
f) pour préparer un composé de formule I où m représente 1 ou 2 ou un de ses sels ou esters facilement hydrolysables, on oxyde un composé de formule 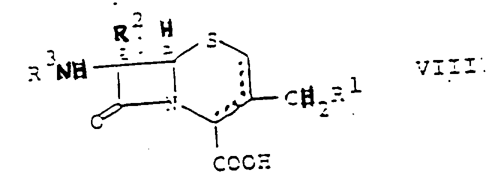 où R¹, R et R³ ont la signification donnée ci-dessus et les lignes en pointillé indiquent la présence d'une double liaison Δ2 ou Δ3,
ou un de ses esters ou de ses sels, ou
g) pour préparer un composé de formule I où R³ contient un substituant d'amino ou l'un de ses esters ou de ses sels on élimine les groupes protecteurs d'amino dans les substituants R³⁰ d'un composé de formule générale 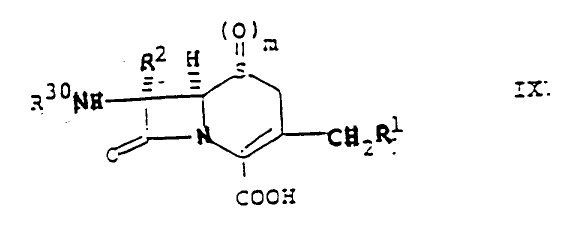 où R¹, R et m ont la signification donnée ci-dessus et R³⁰ représente un reste acyle contenant un groupe amino protégé,
ou d'un de ses sels ou de ses esters, ou
h) pour préparer un ester facilement hydrolysable d'un composé de formule I, on estérifie un acide carboxylique de formule I de façon appropriée, ou
i) pour préparer des sels ou des hydrates d'un composé de formule I ou des hydrates de ses sels, on transforme un composé de formule I en un sel ou en un hydrate, ou en un hydrate de ce sel.

2. Procédé de préparation de composés selon la revendication 1, où R¹ représente l'un des groupes (a) et (b), caractérisé en ce qu'on utilise des composés de départ substitués de façon appropriée.

3. Procédé de préparation de composés selon la revendication 2, où m représente 0 et R représente un hydrogène, caractérisé en ce qu'on utilise les composés de départ substitués de façon appropriée.

4. Procédé de préparation de composés selon la revendication 2 ou 3, où R¹ possède la formule suivante : 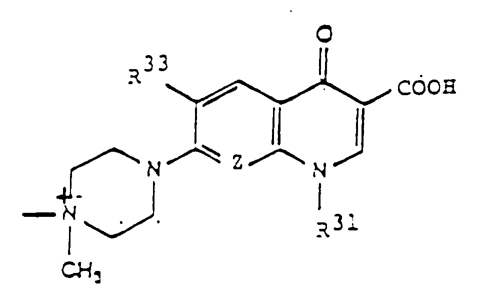 où Z représente R³⁰-C ou l'azote ; R³⁰ est l'hydrogène, un halogène ou un pont oxyméthylène (-CH₂O-) avec le reste pipérazine en formant un cycle condensé à 6 maillons ; R³¹ est l'hydrogène, un alkyle C₁₋₈, alcényle C₂₋₆, cycloalkyle C₃₋₇, halogénoalkyle C₁₋₈ ou mono-, di- ou trihalogénophényle ; ou R³⁰ et R³¹ forment ensemble un groupe alkylène C₃₋₅, un groupe alkylène monooxy C₂₋₄, un groupe alkylène dioxy C₁₋₂ ou un groupe de formule -OCH₂N(CH₃) ; et R³³ est un hydrogène ou un halogène, caractérisé en ce qu'on utilise des composés de départ substitués de manière appropriée.

5. Procédé de préparation de composés selon la revendication 4, où Z représente le groupe R³⁰-C où R³⁰ est l'hydrogène, le chlore, le brome ou le fluor, R³¹ est un alkyle C₁₋₈, un halogénoalkyle C₁₋₈ ou un cycloalkyle C₃₋₇ et R³³ est un hydrogène, chlore ou fluor, caractérisé en ce qu'on utilise les composés de départ substitués de manière appropriée.

6. Procédé de préparation de composés selon la revendication 5, où R³⁰ représente un hydrogène ou un fluor, R³¹ est un éthyle, fluoroéthyle, ou cyclopropyle et R³³ est un hydrogène ou un fluor, caractérisé en ce qu'on utilise les composés de départ substitués de manière appropriée.

7. Procédé de préparation de composés selon la revendication 2 ou 3, où R¹ possède la formule suivante : 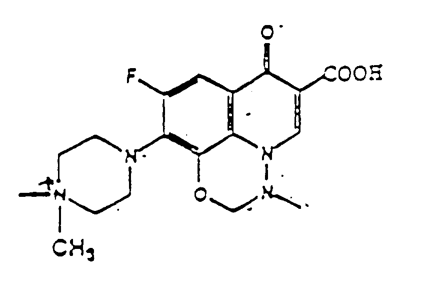 caractérisé en ce qu'on utilise des composés de départ substitués de manière appropriée.

8. Procédé de préparation de composés selon la revendication 2 ou 3, où R¹ a la formule suivante : 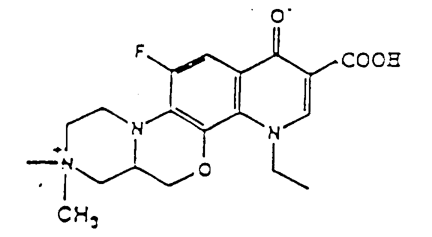 caractérisé en ce qu'on utilise des composés de départ substitués de manière appropriée.

9. Procédé de préparation de composés selon l'une des revendications 2-8, où R³ représente un groupe aliphatique acyle de formule
R⁵-CO-
où R⁵ est un hydrogène, alkyle C₁₋₈, cycloalkyle C₃₋₇, alcoxy C₁₋₈, alcényle C₂₋₆, cycloalcényle C₃₋₇ ou cyclohexadiényle ; ou un alkyle C₁₋₈ substitué par un ou plusieurs des restes halogène, cyano, nitro, amino, mercapto, alkylthio C₁₋₈ ou cyanométhylthio ou un alcényle C₂₋₆,
caractérisé en ce qu'on utilise des composés de départ substitués de manière appropriée.

10. Procédé de préparation de composés selon l'une des revendications 2-8, où R³ représente un groupe acyle aromatique de formules générales 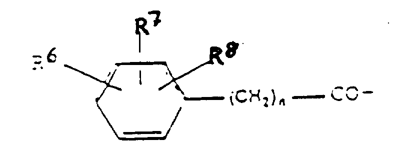 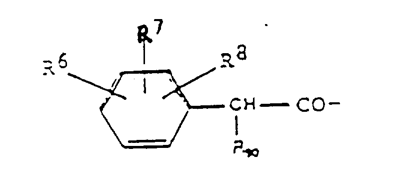 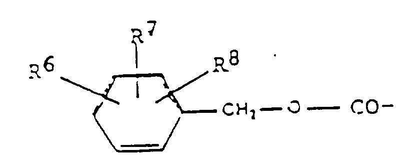 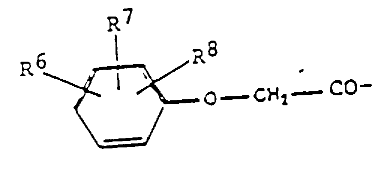 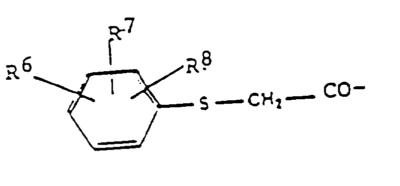 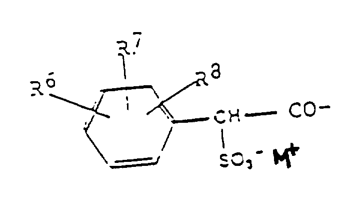 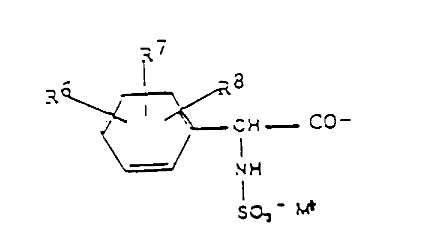 où n représente 0, 1, 2 ou 3 ; R⁶, R⁷ et R⁸ représentent chacun un hydrogène, halogène, hydroxy, nitro, amino, cyano, trifluorométhyle, alkyle C₁₋₄' alcoxy C₁₋₄ ou aminométhyle, R⁹⁰ représente un amino, acylamino, hydroxy, un sel carboxylique, un carboxy protégé, formyloxy, azido ou un sel sulfo et M est un cation, caractérisé en ce qu'on utilise des composés de départ substitués de manière appropriée.

11. Procédé de préparation de composés selon l'une des revendications 2-8, où R³ représente un groupe acyle hétéroaromatique de formules générales
R¹⁰¹-(CH₂)ₙ-CO- 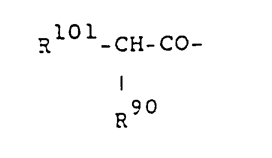
R¹⁰¹-O-CH₂-CO-
R¹⁰¹-S-CH₂-CO-
et
R¹⁰¹-CO-CO-
où n représente 0, 1, 2, ou 3 ; R⁹⁰ représente un amino, acylamino, hydroxy, un sel carboxylique, un carboxy protégé, azido ou un sel sulfo et R¹⁰¹ représente un cycle hétérocyclique à 5, 6 ou 7 maillons substitué le cas échéant avec 1, 2, 3 ou 4 atomes d'azote, d'oxygène et/ou de soufre,
caractérisé en ce qu'on utilise des composés de départ substitués de manière appropriée.

12. Procédé de préparation de composés selon l'une des revendications 2-8, où R³ représente un groupe [[(4-substitué-2,3-dioxo-1-pipérazinyl)carbonyl]amino]acétyle de formule 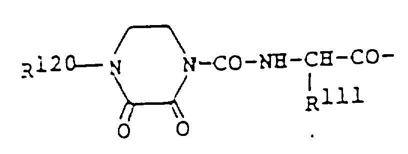 où R¹¹¹ représente un alkyle C₁₋₈, un hydroxyalkyle C₁₋₈ ou un groupe aromatique de formule générale 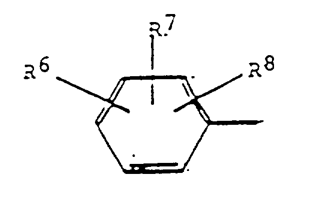 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitué le cas échéant avec 1, 2, 3 ou 4 atomes d'azote, d'oxygène et/ou de soufre et R¹⁰ représente un groupe alkyle C₁₋₈ substitué par un ou plusieurs des restes halogène, cyano, nitro, amine et/ou mercapto,
caractérisé en ce qu'on utilise des composés de départ substitués de manière appropriée.

13. Procédé de préparation de composés selon l'une des revendications 2-8, où R³ représente un groupe (acylamino)acétyl de formule générale 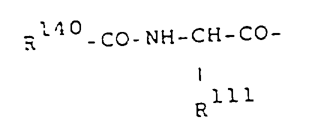 où R¹¹¹ représente un groupe alkyle C₁₋₈, un hydroxyalkyle C₁₋₈ ou un groupe aromatique de formule : 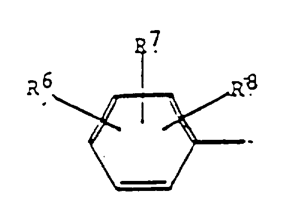 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6, ou 7 maillons substitué le cas échéant avec 1, 2, 3 ou 4 atomes d'azote, d'oxygène et/ou de soufre, et où R¹⁴⁰ représente le groupe 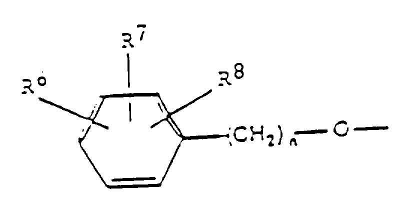 (où R⁶, R⁷ et R⁸ ont la signification ci-dessus et n représente 0, 1, 2, ou 3), un hydrogène, un alkyle C₁₋₈, alkyle C₁₋₈ substitué, amino, alkylamino C₁₋₆, di(alkyle C₁₋₈)amino, (cyano-alkyle C₁₋₈)amino, hydrazino, alkylhydrazino C₁₋₈, arylhydrazino ou acylhydrazino, caractérisé en ce qu'on utilise des composés de départ substitués de manière appropriée.

14. Procédé de préparation de composés selon l'une des revendications 2-8, où R³ représente un groupe (oxyimino substitué)acétyle de formule générale 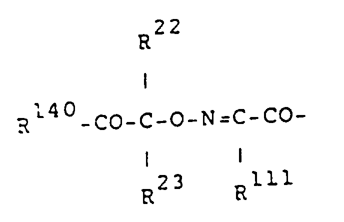 où R¹¹¹ représente un alkyle C₁₋₈, hydroxyalkyle C₁₋₈ ou un groupe aromatique de formule 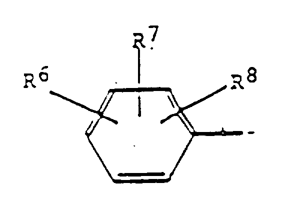 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitué le cas échéant avec 1, 2, 3 ou 4 atomes d'azote, d'oxygène et/ou de soufre, et où en outre R¹⁴⁰ représente le groupe 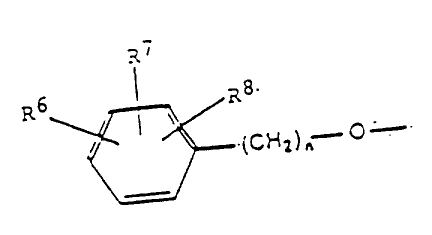 (où R⁶, R⁷ et R⁸ ont la signification ci-dessus et n représente 0, 1, 2 ou 3), un hydrogène, un alkyle C₁₋₈, un alkyle substitué C₁₋₈, amino, alkylamino C₁₋₈, di(alkyle C₁₋₈)amino, (cyanoalkyle C₁₋₈)amino, hydrazino, alkylhydrazino C₁₋₈, arylhydrazino ou acylhydrazino et R et R³ représentent un hydrogène ou un alkyle C₁₋₈ ; ou R et R³ forment ensemble avec l'atome de carbone voisin un cycle carbocyclique C₃₋₇,
caractérisé en ce qu'on utilise des composés de départ substitués de manière appropriée.

15. Procédé de préparation de composés selon l'une des revendications 2-8, où R³ représente un groupe [[[3-(substitué-2-oxo-1-imidazolidinyl]carbonyl]almino]acétyle de formule générale 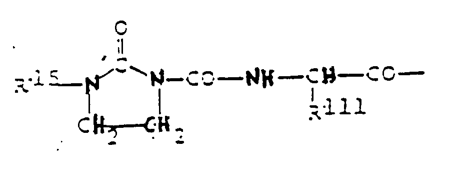 où R¹¹¹ représente un alkyle en C₁₋₈, hydroxy-alkyle en C₁₋₈ ou un groupe aromatique de formule 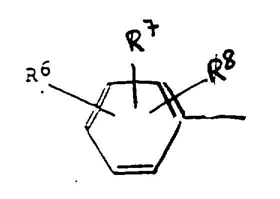 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons le cas échéant substitué avec 1, 2, 3 ou 4 atomes d'azote, d'oxygène et/ou de soufre ; et où en outre R¹⁵ représente un hydrogène, un alkylesulfonyle C₁₋₈, arylméthylèneamino (c'est-à-dire -N=CHR¹¹¹, où R¹¹¹ a la signification donnée ci-dessus), R¹⁶ CO- (où R¹⁶ représente un hydrogène ou un alkyle C₁₋₈ substitué par un halogène), un groupe aromatique défini ci-dessus pour R¹¹¹ ou un alkyle C₁₋₈ substitué par un ou plusieurs des restes halogène, cyano, nitro, amino et/ou mercapto.
caractérisé en ce qu'on utilise des composés de départ substitués de manière appropriée.

16. Procédé de préparation de composés selon l'une des revendications 2-8, où R³ représente un groupe (oxyimino substitué)arylacétyle de formule générale : 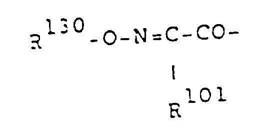 où R¹⁰¹ représente un cycle hétérocyclique à 5, 6 ou 7 maillons substitué le cas échéant avec 1, 2, 3 ou 4 atomes d'azote, d'oxygène et/ou de soufre, où le cycle hétérocyclique est substitué par un halogène, hydroxy, nitro, amino, cyano, trifluorométhyle, alkyle C₁₋₄ ou alcoxy C₁₋₄ et R¹³⁰ est un hydrogène, alkyle C₁₋₈, cycloalkyle C₃₋₇, carboxy cycloalkyle C₃₋₇ ou un alkyle C₁₋₈ substitué, où le groupe alkyle C₁₋₈ est substitué par un ou plusieurs des restes halogène, cyano, nitro, amino, mercapto, alkylthio C₁₋₈, un groupe aromatique défini ci-dessus par R¹¹¹, carboxy (y compris ses sels), alcanoylamino C₁₋₇, carbamoyle, alcoxycarbonyle C₂₋₉, phénylméthoxycarbonyle, diphénylméthoxycarbonyle, hydroxy, alcoxyphosphinyle C₁₋₈, dihydroxyphosphinyle, hydroxy (phénylméthoxy)phosphinyle ou di (alcoxy C₁₋₈)phosphinyle,
caractérisé en ce qu'on utilise des composés de départ substitués de manière appropriée.

17. Procédé de préparation de composés selon la revendication 16, où R¹⁰¹ a la formule 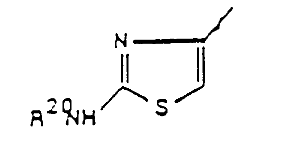 où R⁰ représente un hydrogène ou un groupe protecteur d'amino et R¹³⁰ représente un hydrogène, alkyle C₁₋₈ ou un groupe de formule générale 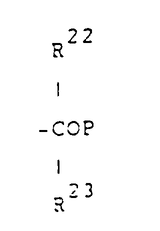 où R et R³ représentent un hydrogène ou un alkyle C₁₋₈ ou forment ensemble avec l'atome de carbone voisin un cycle carbocyclique C₃₋₇ et P est un hydroxy ou -NHR¹⁹ où R¹⁹ est un hydrogène, alkyle C₁₋₈, amino, alkylamino C₁₋₈, arylamino ou acylamino,
caractérisé en ce qu'on utilise des composés de départ substitués de manière appropriée.

18. Procédé de préparation de composés selon la revendication 17, où R⁰ est un hydrogène et R¹³⁰ est un méthyl, ou un groupe de formule 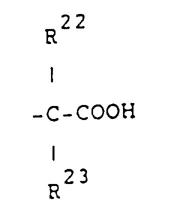 où R et R³ représentent un hydrogène ou un méthyle, caractérisé en ce qu'on utilise des composés de départ substitués de manière appropriée.

19. Procédé de préparation de composés selon l'une des revendications 14 et 16-18 sous la forme syn, ou sous forme de mélange, où la forme syn est prépondérante,
caractérisé en ce qu'on utilise des composés de départ substitués de manière appropriée.

20. Procédé de préparation de composés selon la revendication 2, où R¹ représente le groupe (a) et R³ représente le phénoxyacétyle, caractérisé en ce qu'on utilise des composés de départ substitués de manière appropriée.

21. Procédé de préparation de composés selon la revendication 2,
Iodure de (6R-trans)·4·[3-carboxy-1-(2-fluoroéthyl)-6,8-difluoro-1,4-dihydro-4-oxoquinol-7-yl]-[[2-carboxy-8-oxo-7-[(phénoxyacétyl)amino]-5-thia-1-azabicyclo[4.2.0]oct-2-ène-3-yl]méthyl]-1-méthylpipérazinium,
[6R-[6α,7β(Z)]]-1-[[7-[[[(2-amino-4-thiazolyl)[(1-(1-carboxy-1-méthyl)éthoxy]imino]acétyl]amino]-2-carboxy-8-oxo-5-thia-1-azabicyclo[4.2.0]oct-2-én-3-yl]méthyl]-4-[3-carboxy-1-(2-fluoroéthyl)-6,8-difluoro-1,4-dihydro-4-oxoquinol-7-yl]-1-méthylpipérazinium hydroxyde sel monosodique interne et
iodure de [6R-[6α,7β(Z)]]-1-[[7-[[(2-amino-4-thiazolyl)(méthoxyimino)acétyl]amino]-2-carboxy-8-oxo-5-thia-1-azabicyclo-[4.2.0]oct-2-én-3-yl]méthyl]-4-(3-carboxyl-1-éthyl-6-fluoro-1,4-dihydro-4-oxo-7-quinolyl)-1-méthylpipérazinium.
caractérisé en ce qu'on utilise des composés de départ substitués de manière appropriée.

22. Procédé de préparation de composé selon la revendication 2,
Iodure de [6R-[6α,7β(Z)]-1-[[[(2-amino-4-thiazolyl)méthoxyimino)acétyl]amino]-2-carboxy-8-oxo-5-thia-1-azabicyclo[4.2.0]oct-2-én-3-yl]méthyl]-4-[3-carboxy-6,8-difluoro-1-(2-fluoroéthyl)-1,4-dihydro-4-oxo-7-quinolyl]-1-méthylpipérazinium,
caractérisé en ce qu'on utilise des composés de départ substitués de manière appropriée.

23. Procédé de préparation de composés selon la revendication 2,
[6R-[6α,7β(Z)]]-1-[[[(2-amino-4-thiazolyl)-(méthoxyimino)acétyl]amino]-2-carboxy-8-oxo-5-thia-1-azabicyclo[4.2.0]oct-2-én-3-yl]méthyl]-4-[3-carboxy-6,8-difluoro-1-(2-fluoroéthyl)-1,4-dihydro-4-oxo-7-quinolyl]-1-méthyl-pipérazinium hydroxyde sel interne monosodique,
caractérisé en ce qu'on utilise des composés de départ substitués de manière appropriée.

24. Procédé de préparation de composés selon la revendication 2,
Trifluoroacétate de (6R-trans)-4-[3-carboxy-6,8-difluoro-1-(2-fluoroéthyl)-1,4-dihydro-4-oxo-7-quinolyl]-1-[[2-carboxy-7-(formylamino)-8-oxo-5-thia-1-azabicyclo{4.2.0]oct-2-én-3-yl]méthyl]-1-méthylpipérazinium,
caractérisé en ce qu'on utilise des composés de départ substitués de manière appropriée.

25. Procédé de préparation de composés selon la revendication 2,
Iodure de [6R-[6α,7β(Z)]]-1-[[7-[[(2-amino-4-thiazolyl)[[1,1-diméthyl-2-(1,1-diméthyléthoxy)-2-oxoéthoxy]imino]acétyl]amino]-2-carboxy-8-oxo-5-thia-1-azabicyclo[4.2.0]oct-2-én-3-yl]méthyl]-4-[3-carboxy-(2-fluoroéthyl)-6,8-difluoro-1,4-dihydro-4-oxoquinol-7-yl]-1-méthylpipérazinium,
caractérisé en ce qu'on utilise des composés de départ substitués de manière appropriée.

26. Procédé de préparation de composés selon la revendication 1, où R¹ représente le groupe (c), caractérisé en ce qu'on utilise des composés de départ substitués de manière appropriée.

27. Procédé de préparation de composés selon la revendication 26, où m représente 0 ou 1, caractérisé en ce qu'on utilise des composés de départ substitués de manière appropriée.

28. Procédé de préparation de composés selon la revendication 26 ou 27, où Q représente un groupe de formule 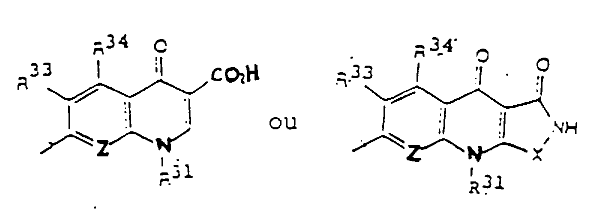 où Z représente R³⁰-C ou un azote ; X représente un oxygène ou un soufre ; R³⁰ est un hydrogène, halogène ou un pont oxométhylène (-CH₂O-) fixé au reste pipérazine en formant un cycle condensé à six maillons ; R³¹ est un hydrogène, alkyle C₁₋₈, alcényle C₂₋₆, cycloalkyle C₃₋₇, halogénoalkyle C₁₋₈ ou mono-, di- ou trihalogénophényle ; R³⁰ et R³¹ forment ensemble un groupe alkylène C₃₋₅, un groupe monoxy alkylène C₂₋₄, un groupe alkylène dioxy C₁₋₂ ou un groupe de formule -OCH₂N(CH₃)- ; R³³ est un hydrogène ou un halogène ; et R³⁴ est un hydrogène ou un amino,
caractérisé en ce qu'on utilise des composés de départ substitués de manière appropriée.

29. Procédé de préparation de composés selon la revendication 28, où Z représente le groupe R³⁰-C où R³⁰ est un hydrogène, chlore, brome ou fluor, R³¹ est un alkyle C₁₋₈, halogénoalkyle C₁₋₈ ou cycloalkyle C₃₋₇ et R³³ est un hydrogène, chlore ou fluor,
caractérisé en ce qu'on utilise des composés de départ substitués de manière appropriée.

30. Procédé de préparation de composés selon la revendicatio 29, où R³⁰ représente un hydrogène ou un fluor, R³¹ est un éthyle, fluoroéthyle ou cyclopropyle et R³³ est un hydrogène ou un fluor,
caractérisé en ce qu'on utilise des composés de départ substitués de manière appropriée.

31. Procédé de préparation de composés selon la revendication 28, où R¹ a la formule suivante : 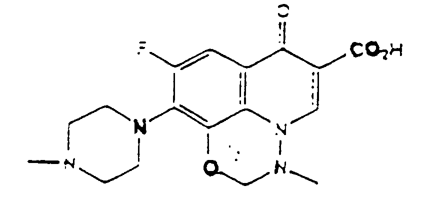 caractérisé en ce qu'on utilise des composés de départ substitués de manière appropriée.

32. Procédé de préparation de composés selon la revendication 28, où R¹ possède la formule suivante : 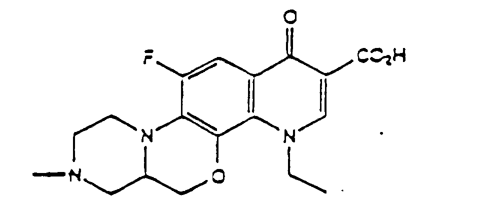 caractérisé en qu'on utilise des composés de départ substitués de manière appropriée.

33. Procédé de préparation de composés selon l'une des revendications 26-32, où R³ est un groupe acyle aliphatique de formule
R⁵-CO-
où R⁵ est un hydrogène, alkyle C₁₋₈, cycloalkyle C₃₋₇, alcoxy C₁₋₈, alcényle C₂₋₆, cycloalcényle C₃₋₇, ou cyclohexadiényle ; ou un groupe alkyle C₁₋₈ substitué par un ou plusieurs des restes halogène, cyano, nitro, amino, mercapto, alkylthio C₁₋₈, ou cyanométhylthio ou alcényle C₂₋₆,
caractérisé en ce qu'on utilise des composés de départ substitués de manière appropriée.

34. Procédé de préparation de composés selon l'une des revendications 26-32, où R³ représente un groupe acyle aromatique de formules générales 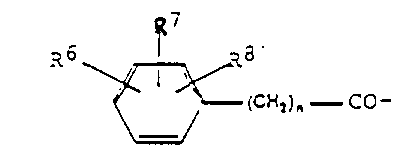 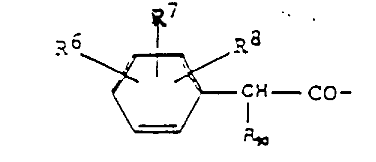 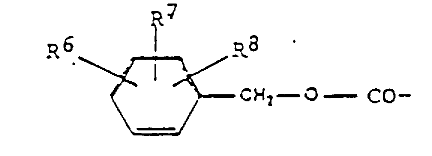 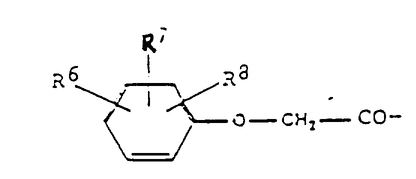 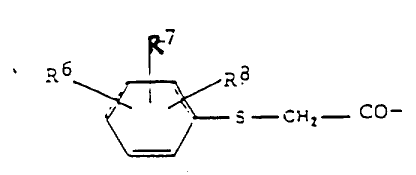 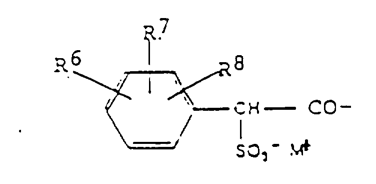 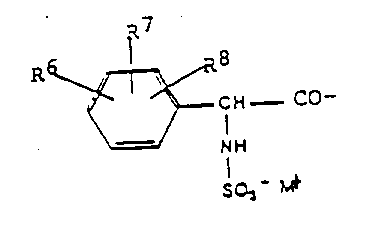 où n représente 0, 1, 2 ou 3 ; R⁶, R⁷ et R⁸ représentent chacun un hydrogène, halogène, hydroxy, nitro, amino, cyano, trifluorométhyle, alkyle C₁₋₄, alcoxy C₁₋₄, ou aminométhyle,R⁹⁰ est un amino, acylamino, hydroxy, un sel carboxylique, un carboxy protégé, formyloxy, azido ou un sel sulfo et M est un cation,
caractérisé en ce qu'on utilise des composés de départ substitués de manière appropriée.

35. Procédé de préparation de composés selon l'une des revendications 26-32, où R³ est un groupe acyle hétéroaromatique de formules générales
R¹⁰¹-(CH₂)ₙ-CO- 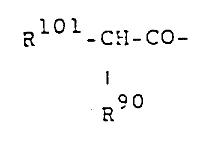
R¹⁰¹-O-CH₂-CO-
R¹⁰¹-S-CH₂-CO-
et
R¹⁰¹-CO-CO-
où n représente 0, 1, 2 ou 3 ; R⁹⁰ représente un amino, acylamino, hydroxy, un sel carboxylique, carboxy protégé, azido, ou un sel sulfo et R¹⁰¹ représente un cycle hétérocyclique à 5, 6 ou 7 maillons le cas échéant substitué avec 1, 2, 3 ou 4 atomes d'azote, d'oxygène et/ou de soufre,
caractérisé en ce qu'on utilise des composés de départ substitués de manière appropriée.

36. Procédé de préparation de composés selon l'une des revendications 26-32, où R³ est un groupe [[(4-substitué-2,3-dioxo-1-pipérazinyl)carbonyl]amino]acétyle de formule 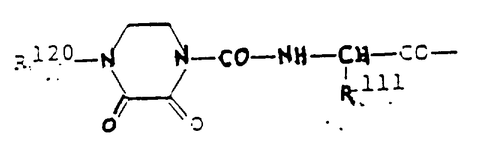 où le groupe R¹¹ représente un groupe alkyle C₁₋₈, hydroalkyle C₁₋₈, ou un groupe aromatique de formule générale 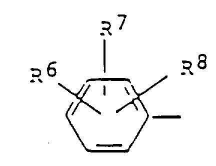 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitué le cas échéant avec 1, 2, 3 ou 4 atomes d'azote, d'oxygène et/ou de soufre et R¹⁰ représente un groupe alkyle C₁₋₈ substitué le cas échéant par un ou plusieurs des restes halogène, cyano, nitro, amino et/ou mercapto, caractérisé en ce qu'on utilise des composés de départ substitués de manière appropriée.

37. Procédé de préparation de composés selon l'une des revendications 26-32, où R³ représente un groupe de formule 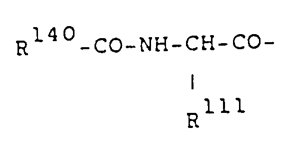 où R¹¹¹ représente un alkyle C₁₋₈, hydroxyalkyle C₁₋₈ ou un groupe aromatique de formule 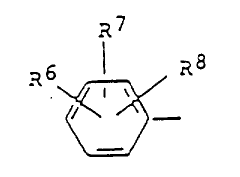 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitué le cas échéant avec 1, 2, 3 ou 4 atomes d'azote, d'oxygène et/ou de soufre, et où en outre R¹⁴⁰ représente le groupe 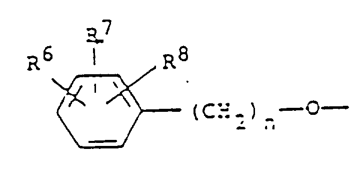 (où R⁶, R⁷ et R⁸ ont la signification ci-dessus, et n représente 0, 1, 2 ou 3), est un hydrogène, alkyle C₁₋₈, alkyle C₁₋₈ substitué, amino, alkylamino C₁₋₈, di(alkyle C₁₋₈) -amino, (cyanoalkyle C₁₋₈)amino, hydrazino, alkylhydrazino C₁₋₈, arylhydrazino ou acylhydrazino, caractérisé en ce qu'on utilise des composés de départ substitués de manière appropriée.

38. Procédé de préparation de composés selon l'une des revendications 26-32, où R³ représente un groupe (oxyimino substitué)acétyle de formule générale 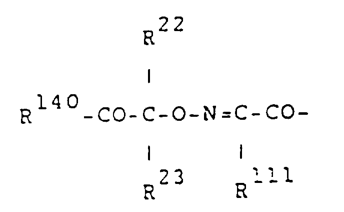 où R¹¹¹ représente un alkyle C₁₋₈, hydroxyalkyle C₁₋₈ ou un groupe aromatique de formule 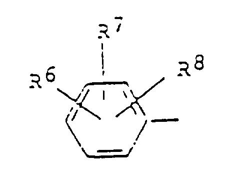 où R⁶, R⁷ et R⁸ représentent un hydrogène, halogène, hydroxy, nitro, amino, cyano, trifluorométhyle, alkyle C₁₋₄, alcoxy C₁₋₄ ou aminométhyle ou un cycle hétérocyclique à 5, 6 ou 7 maillons substitué le cas échéant avec 1, 2, 3 ou 4 atomes d'azote, d'oxygène et/ou de soufre, où en outre R¹⁴⁰ représente le groupe 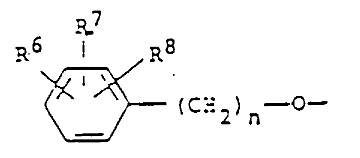 (où R⁶, R⁷ et R⁸ ont la signification ci-dessus, et n représente 0, 1, 2 ou 3), est un hydrogène, alkyle C₁₋₈, alkyle C₁₋₈ substitué, amino, alkylamino C₁₋₈, di(alkyle C₁₋₈)amino, (cyanoalkyle C₁₋₈)-amino, hydrazino, alkylhydrazino C₁₋₈, arylhydrazino ou acylhydrazino et R et R³ représentent un hydrogène ou un alkyle C₁₋₈ ou R et R³ forment ensemble avec l'atome de carbone voisin un cycle carbocyclique C₃₋₇,
caractérisé en ce qu'on utilise des composés de départ substitués de manière appropriée.

39. Procédé de préparation de composés selon l'une des revendications 26-32, où R³ représente un groupe [[[3-substitué-2-oxo-1-imidazolidinyl]carbonyl]amino]-acétyle de formule générale 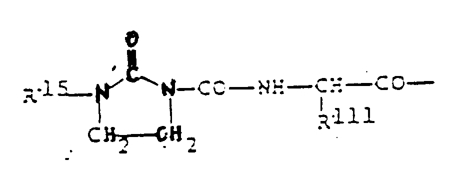 où R¹¹¹ représente un groupe alkyle C₁₋₈, hydroxyalkyle C₁₋₈ ou un groupe aromatique de formule 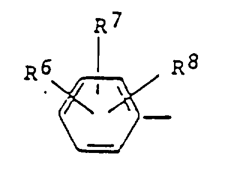 où R⁶, R⁷ et R⁸ représentent un hydrogène, halogène, hydroxy, nitro, amino, cyano, trifluorométhyle, alkyle C₁₋₄, alcoxy C₁₋₄, aminométhyle ou un cycle hétérocyclique à 5, 6 ou 7 maillons substitué le cas échéant avec 1, 2, 3 ou 4 atomes d'azote, d'oxygène et/ou de soufre ; et où en outre R¹⁵ représente un hydrogène, un alkylsulfonyle C₁₋₈, un arylméthylèneamino (c'est-à-dire -N=CHR¹¹¹, où R¹¹¹ a la signification donnée ci-dessus), R¹⁶CO- (où R¹⁶ représente un hydrogène ou le cas échéant un alkyle C₁₋₈ substitué par un halogène), un groupe aromatique comme c'est défini pour R¹¹¹ ou un groupe alkyle C₁₋₈ substitué le cas échéant par un ou plusieurs des restes halogène, cyano, nitro, amino et/ou mercapto,
caractérisé en ce qu'on utilise des composés de départ substitués de manière appropriée.

40. Procédé de préparation de composés selon l'une des revendications 26-32, où R³ représente un groupe (oxyimino substitué)arylacétyle de formule générale 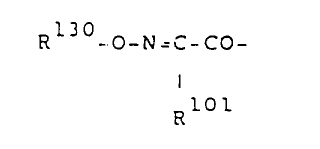 où R¹⁰¹ représente un cycle hétérocyclique à 5, 6 ou 7 maillons substitué le cas échéant avec 1, 2, 3 ou 4 atomes d'azote, d'oxygène et/ou de soufre, où le cycle hétérocyclique est substitué par un halogène, hydroxy, nitro, amino, cyano, trifluorométhyle, alkyle C₁₋₄ ou alcoxy C₁₋₄ et R¹³⁰ représente un hydrogène, alkyle C₁₋₈, cycloalkyle C₃₋₇, carboxycycloalkyle C₃₋₇ ou un alkyle C₁₋₈ substitué où le groupe alkyle C₁₋₈ est substitué par un ou plusieurs des restes halogène, cyano, nitro, amino, mercapto, alkylthio C₁₋₈, un groupe aromatique défini par R¹¹¹ ci-dessus, carboxy (y compris ses sels), alcanoylamino C₁₋₇, carbamoyle, alcoxycarbonyle C₂₋₉, phénylméthoxycarbonyle, diphénylméthoxycarbonyle, hydroxy-alcoxyphosphinyle C₁₋₈, dihydroxyphosphinyle, hydroxy(phénylméthoxy)phosphinyle ou un di(alcoxy C₁₋₈)phosphinyle,
caractérisé en ce qu'on utilise des composés de départ substitués de manière appropriée.

41. Procédé de préparation de composés selon la revendication 40 où R¹⁰¹ a la formule 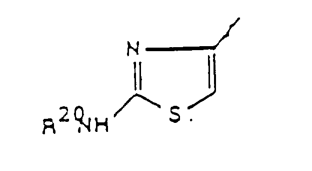 où R⁰ représente un hydrogène ou un groupe protecteur d'amino, R¹³⁰ est un hydrogène, alkyle C₁₋₈ ou un groupe de formule générale 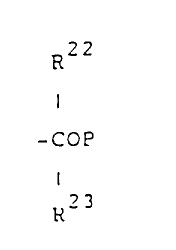 où R et R³ représentent un hydrogène, ou un alkyle C₁₋₈ ou forment ensemble avec l'atome de carbone voisin un cycle carbocyclique C₃₋₇ et P représente un hydroxy ou -NHR¹⁹ où R¹⁹ est un hydrogène, alkyle C₁₋₈, amino, alkylamino C₁₋₈, arylamino ou acylamino,
caractérisé en ce qu'on utilise des composés de départ substitués de manière appropriée.

42. Procédé de préparation de composés selon la revendication 41, où R⁰ représente un hydrogène ou un triphénylméthyle,
caractérisé en ce qu'on utilise des composés de départ substitués de manière appropriée.

43. Procédé de préparation de composés selon l'une des revendications 38 et 40-42 sous la forme syn ou sous forme de mélanges, où la forme syn est prépondérante,
caractérisé en ce qu'on utilise des composés de départ substitués de manière appropriée.

44. Procédé de préparation de composés selon la revendication 26,
Acide [6R-[6α,7β(Z)]]-7-[[(2-amino-4-thiazolyl)(méthoxyimino)acétyl]amino]-3-[[[(4-(3-carboxy-1-éthyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique
Acide [6R-[6α,7β(Z)]]-7-[[(2-amino-4-thiazolyl)méthoxyimino)acétyl]amino]-3-[[[[4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide (6R-trans)-3-[[[[4-(3-carboxy-1-cyclopropyl-6-fluoro-1,4-dihydro-4-oxo-7-quinolyl)-1-pipérazinyl]carbonyl]oxy]méthyl]-8-oxo-7-[(phénoxyacétyl)-amino]-5-thia-1-azabicyclo[4.2.0]oct-2-èn-2-carboxylique.
Acide [6R, [6α,7β-Z)]-7-[[(2-amino-4-thiazolyl)-[(1-carboxy-1-méthyl-éthoxy)imino]acétyl]amino]-3-[[[[(4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide [6R-[6α,7β(Z)]]-7-[[(2-amino-4-thiazolyl)[(carboxyméthoxy)imino]acétyl]amine]-3-[[[[(4-(3-carboxy-1-cyclopropyl-6-fluoro-1,4-dihydro-4-oxo-7-quinolyl)-1-pipérazinyl]carbonyl]oxy]méthyl]-8-oxo-5-thia-1-azabicyclo[4.2.0]oct-2-èn-2-carboxylique.
Acide [6R-[6α,7β(Z)]-7-[[(2-amino-4-thiazolyl)(méthoxyimino)acétyl]amino]-3-[[[[(4-(9-cyclopropyl-6-fluoro-2,3,4-tétrahydro-3,4-dioxoisothiazolo[5,4-b]quinol-7-yl)-1-pipérazinyl]-carbonyl]oxy]méthyl]-8-oxo-5-thia-1-azabicyclo-[4.2.0]-oct-2-èn-2-carboxylique
Acide [6R-[6α,7β(Z)]]-7-[[(2-amino-4-thiazolyl)(méthoxyimino)acétyl]amino]-3-[[[[4-(9-cyclopropyl-6-fluoro-2,3,4,9-tétrahydro-3,4-dioxoisoxazolo-[5,4-b]-quinol-7-yl]-1-pipérazinyl]carbonyl]oxy]méthyl]-8-oxo-5-thia-1-azabicyclo[4.2.0]oct-2-èn-2-carboxylique ainsi que les sels de ces composés et les hydrates et les sels de ces composés,
caractérisé en ce qu'on utilise des composés de départ substitués de manière appropriée.

45. Procédé de préparation des sels de sodium des composés selon la revendication 44, caractérisé en ce qu'on utilise des composés de départ substitués de manière appropriée.

46. Procédé selon l'une des revendications 1-45, caractérisé en ce qu'on réalise la variante de procédé (b).

47. Procédé selon l'une des revendications 1-45, caractérisé en ce qu'on réalise l'une des variantes de procédé (a), (c), (d), (f), (g) et (i).

48. Procédé de préparation de préparations pharmaceutiques, caractérisé en ce qu'on mélange un dérivé acyle défini dans la revendication 1 de formule I ou un de ses sels facilement hydrolysables ou un ester de ce composé ou un hydrate d'un composé de formule I ou d'un ester ou d'un sel de ce composé comme composant actif avec des véhicules et/ou des excipients solides et liquides usuels dans de telles préparations inertes, non toxiques appropriés à l'administration thérapeutique.

49. Utilisation de composés selon l'une des revendications 1-47 lors de la production de médicaments pour le traitement ou la prophylaxie de maladies infectieuses.

50. Composés de formule 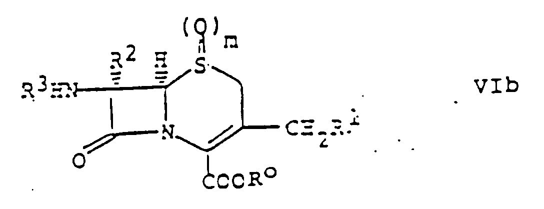 où R¹, R ou R³ et m ont les significations données ci-dessus et R⁰ représente un t-butyle, benzhydryle, p-nitrobenzyle ou allyle.

51. Iodure de (6R-trans)-4-[3-carboxy-6,8-difluoro-1-(2-fluoroéthyl)-1,4-dihydro-4-oxo-7-quinolyl]-1-[[2-(1,1-diméthyléthoxy)carbonyl-7-(formylamino-8-oxo-5-thia-1-azabicyclo[4.2.0]oct-2-èn-3-yl]méthyl]-1-méthylpipérazinium.

52. Composés de formule 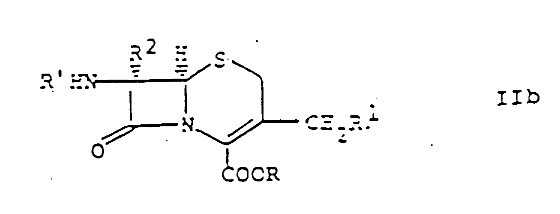 où R¹ et R ont la signification donnée ci-dessus, R représente un hydrogène ou un groupe protecteur d'acide carboxylique et R' est un hydrogène ou un groupe protecteur d'amino facile à éliminer.

53. (6R-trans)-3-[[[[(4-(3-carboxy-1-cyclopropyl-6-fluoro-1,4-dihydro-4-oxo-7-quinolyl)-1-pipérazinyl]carbonyl]oxy]méthyl]-7-[[(1,1-diméthyléthoxy)carbonyl]amino-8-oxo-5-thia-1-azabicyclo[4.2.0]oct-2-èn-2-carboxylate de diphénylméthyle.
